# EUROPEAN PATENT APPLICATION

(11) **EP 3 915 977 A1**
(43) Date of publication of application: **01.12.2021**
(21) Application number: 19910981.0
(22) Date of filing: 12.09.2019
(51) Int. Cl.: C07D 241/44, A61K 31/498, A61P 35/00

(54) **1,2,3,4-TETRAHYDROQUINOXALINE DERIVATIVE, PREPARATION METHOD THEREFOR AND APPLICATION THEREOF**

(30) Priority: 23.01.2019 CN 201910064417
(71) Applicant: Abbisko Therapeutics Co., Ltd., Shanghai 201203 (CN)
(72) Inventor: ZHAO, Baowei, Shanghai 201203 (CN); XUN, Guoliang, Shanghai 201203 (CN); ZHAO, Yuan, Shanghai 201203 (CN); FENG, Tao, Shanghai 201203 (CN); YU, Hongping, Shanghai 201203 (CN); CHEN, Zhui, Shanghai 201203 (CN); XU, Yaochang, Shanghai 201203 (CN)
(74) Representative: dompatent von Kreisler Selting Werner - Partnerschaft von Patent- und Rechtsanwälten mbB
(86) International application number: PCT/CN2019/105557
(87) International publication number: WO 2020/151232

(57) **Abstract**

A 1,2,3,4-tetrahydroquinoxaline derivative having a structure as represented by formula (I), preparation method therefor and application thereof, and the definition on substituents is as stated in the description and the claims. Compounds in the present invention can be widely applied to preparation of drugs for treating one or more tumors, cancers, metabolic diseases, autoimmune diseases or disorders, and a new generation of RORyt agonist drugs is expected to be developed.

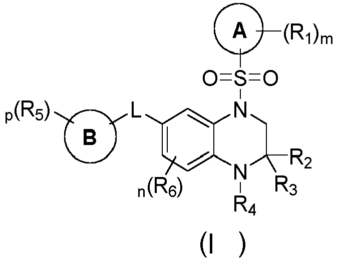

## Description

### TECHNICAL FIELD

The present invention belongs to the field of pharmaceutical synthesis, and particularly relates to a 1,2,3,4-tetrahydroquinoxaline derivative, preparation method therefor and application thereof.

### BACKGROUND

The retinoic-acid-related (RAR) orphan receptor (ROR) family comprises three members of RORα, RORβ and PORγ RORα is indispensable for cerebellum development, while RORβ is mainly expressed in brain and retina. They both play important roles in normal development of the retina. Depending on different splicing sites during transcription, RORγ has two subtypes, RORγ1 and RORγ2 (RORγt), the former of which is mainly expressed in liver, skeletal muscle, and kidney. RORγt is mainly expressed in immune organs. Mice with RORγt-deficiency lack lymph nodes, Peyer's patches, and other lymphoid organs.Their T cell development and maturation processes are also influenced, and the number of various T cells is reduced compared with those of normal mice.

T helper cells play an essential and important role in human immune system. Under the induction of different cytokines during development, the CD4 positive T helper cells can differentiate into a series of regulatory helper cells, such as Th1, Th2, Th17, and Treg. Th1 and Th2 play important roles in the processes of antigen recognition, antigen presentation, and T effector cell activation. Tregs are a class of regulatory cells that promote immunosuppression. Th17 is a type of relatively new T helper cell discovered in recent years, characterized by the secretion of interleukin 17 (IL-17) cytokine. Th17 cells were originally thought to exert immune functions mainly in fighting against bacterial and fungal infections by recruiting neutrophils. Subsequent studies found that these cells were closely linked to the development of autoimmune diseases and malignant tumors. Therefore, the treatment of autoimmune diseases by inhibiting the differentiation of Th17 cells and the treatment of malignant tumors by activating the differentiation of Th17 cells have become hot spots in basic and translational research on immune-related diseases and oncology.

RORγt is a key transcription factor in the differentiation of CD4+ Th17 cells, and the modulation of RORγt activity through small molecule compound can directly influence the abundance and activity of Th17 cells. After RORγt is activated, the level of cytokines secreted by Th17 cells (such as IL-17A) is significantly increased, and the survival and immune activation capability of Th17 cells are greatly enhanced. Meanwhile, the enhanced activation of Th17 cells can reduce the number of immunosuppressive Treg cells and the expression of immunosuppressive receptors (such as PD-1) in tumor infiltrating lymphocytes. Based on the mechanism of action, an orally available, small molecule RORyt agonist can enhance the capability of immune system to recognize and kill tumor cells via activating Th17 cells, which could become a novel anti-tumor small molecule drug following the success of anti-PD-1 and PD-L1 antibodies.

### SUMMARY

The objective of the present invention is to provide a RORyt small molecule agonist.

The first aspect of the present invention provides a compound of formula (I), a stereoisomer, prodrug or pharmaceutically acceptable salt thereof: 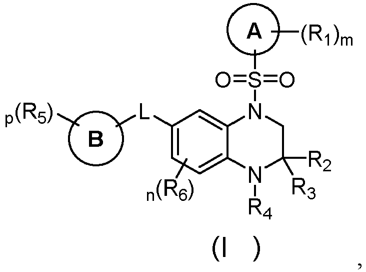 wherein,
L is selected from the group consisting of a bond, -C(R₇)=C(R₈)-, -(CR₉R₁₀)ₘ₁-, -(CR₁₁R₁₂)ₘ₂-O-, -O-(CR₁₃R₁₄)ₘ₃-, -N(R₁₅)-C(O)-, -C(O)-N(R₁₆)-, -(CR₁₇R₁₈)ₘ₄-N(R₁₉)-, -N(R₂₀)-(CR₂₁R₂₂)ₘ₅-, - (CR₂₃R₂₄)ₘ₆-S(O)ᵣ- and -S(O)ᵣ-(CR₂₅R₂₆)ₘ₇-;
ring A is 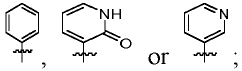
ring B is 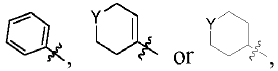 wherein Y is -O- or -N(R₂₇)-;
R₁ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, - C₀₋₈-C(O)NR₃₁R₃₂ and -C₀₋₈-N(R₃₁)-C(O)R₃₀;
R₂ and R₃ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, or R₂ and R₃, together with the carbon atom directly attached thereto, form C(O), 3-10 membered cycloalkyl or 3-10 membered heterocyclyl, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
R₄ is selected from the group consisting of hydrogen, deuterium, hydroxy, C₁₋₄ alkyl, vinyl, propenyl, allyl, ethynyl, C₃₋₆ cycloalkyl, 3-6 membered heterocyclyl, phenyl, benzyl, diazole, triazole, methylsulfonyl, isopropylsulfonyl, aminosulfonyl, carboxyl, methoxycarbonyl, ethoxycarbonyl and acetyl, said C₁₋₄ alkyl, C₃₋₆ cycloalkyl, 3-6 membered heterocyclyl, phenyl, benzyl, diazole and triazole are unsubstituted or substituted by one or more substituents selected from the group consisting of deuterium, halogen, cyano, methyl, ethyl, isopropyl, trifluoromethyl, difluoromethyl, trideuteriomethyl, cyclopropyl, oxacyclobutyl, =O, methoxy, carboxyl, methoxycarbonyl, acetyl, amino, dimethylamino and acetylamino,
or R₄ and R₃, together with the carbon atom directly attached thereto, form 5-10 membered heterocyclyl, the 5-10 membered heterocyclyl is unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-C(S)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-C(S)R₃₀, -C₀₋₈-O-C(O)R₃₀, -C₀₋₈-NR₃₁R₃₂, -C₀₋₈-C(O)NR₃₁R₃₂ and - C₀₋₈-N(R₃₁)-C(O)R₃₀;
each R₅ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -SF₅, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
each R₆ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, - C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
R₇ and R₈ are each independently selected from the group consisting of hydrogen, deuterium, fluorine, C₁₋₄ alkyl, C₁₋₄ deuterioalkyl and C₁₋₄ fluoroalkyl;
R₉, R₁₀, R₁₁, R₁₂, R₁₃, R₁₄, R₁₇, R₁₈, R₂₁, R₂₂, R₂₃, R₂₄, R₂₅ and R₂₆ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, or R₉ and R₁₀, R₁₁ and R₁₂, R₁₃ and R₁₄, R₁₇ and R₁₈, R₂₁ and R₂₂, R₂₃ and R₂₄, R₂₅ and R₂₆, together with the carbon atom directly attached thereto, each independently form C(O), 3-6 membered cycloalkyl, 3-6 membered heterocyclyl, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
R₁₅, R₁₆, R₁₉, R₂₀ and R₂₇ are each independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-C(O)OR₂₉ and -C₀₋₈-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
each R₂₈ is independently selected from the group consisting of hydrogen, deuterium, hydroxy, C₁₋₁₀ alkyl, C₁₋₁₀ alkoxy, C₂₋₁₀ alkenyl, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂, above groups are unsubstituted or substituted by one or more substituents selected from the group consisting of deuterium, halogen, hydroxy, =O, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂;
each R₂₉ is independently selected from the group consisting of hydrogen, deuterium, C₁₋₁₀ alkyl, C₂₋₁₀ alkenyl, C₃₋₁₀ cycloalkyl, 3-10 membered heterocyclyl, C₅₋₁₀ aryl and 5-10 membered heteroaryl, above groups are unsubstituted or substituted by one or more substituents selected from the group consisting of deuterium, halogen, hydroxy, =O, cyano, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂;
each R₃₀ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂, above groups are unsubstituted or substituted by one or more substituents selected from the group consisting of deuterium, halogen, hydroxy, cyano, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂;
each R₃₁ and each R₃₂ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, sulfonyl, methylsulfonyl, isopropylsulfonyl, cyclopropylsulfonyl, p-toluenesulfonyl, amino, monoalkylamino, dialkylamino and C₁₋₁₀ alkanoyl, above groups are unsubstituted or substituted by one or more substituents selected from the group consisting of deuterium, halogen, hydroxy, carboxyl, C₁₋₈ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, amino, monoalkylamino, dialkylamino and C₁₋₁₀ alkanoyl;
or R₃₁ and R₃₂, together with nitrogen atom directly attached thereto, form 4-10 membered heterocyclyl, above groups are unsubstituted or substituted by one or more substituents selected from the group consisting of deuterium, halogen, hydroxy, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, amino, monoalkylamino, dialkylamino and C₁₋₁₀ alkanoyl;
m is an integer of 0 to 5; n is an integer of 0 to 3; p is an integer of 0 to 5;
m1, m3, m5 and m7 are each independently 1 or 2;
m2, m4 and m6 are each independently 0, 1 or 2;
each r is independently 0, 1 or 2.

As a preferred embodiment, in the compound of formula (I), the stereoisomer, prodrug or pharmaceutically acceptable salt thereof, each R₆ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -C₀₋₄-S(O)ᵣR₂₈, - C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀; R₂₈, R₂₉, R₃₀, R₃₁ and R₃₂ are defined as those in the compound of formula (I).

As a further preferred embodiment, in the compound of formula (I), the stereoisomer, prodrug or pharmaceutically acceptable salt thereof, each R₆ is selected from the group consisting of hydrogen, deuterium, fluorine, chlorine, cyano, methyl, ethyl, isopropyl, vinyl, allyl, ethynyl, cyclopropyl, 3-oxacyclobutyl, 3-azacyclobutyl, phenyl, pyridyl, diazole, triazole, methylsulfonyl, aminosulfonyl, methoxy, methoxyacyl, carboxyl, acetyl, acetoxy, amino, dimethylamino, aminoacyl and acetylamino, above groups are unsubstituted or substituted by one or more substituents selected from the group consisting of deuterium, fluorine, chlorine, cyano, methyl, trifluoromethyl, cyclopropyl, phenyl, pyridyl, methylsulfonyl, hydroxy, methoxy, carboxyl and amino.

As a more further preferred embodiment, in the compound of formula (I), the stereoisomer, prodrug or pharmaceutically acceptable salt thereof, the compound of formula (I) is a compound having formula (II a): 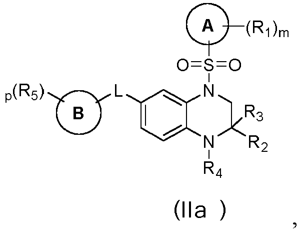 wherein R₂ and R₃ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀, or R₂ and R₃, together with the carbon atom directly attached thereto, form C(O), 3-10 membered cycloalkyl or 3-10 membered heterocyclyl;
R₄ is selected from the group consisting of hydrogen, deuterium, hydroxy, C₁₋₄ alkyl, C₃₋₆ cycloalkyl, 3-6 membered heterocyclyl, phenyl, methylsulfonyl, isopropylsulfonyl, aminosulfonyl, carboxy, methoxycarbonyl, ethoxycarbonyl and acetyl, and said C₁₋₄ alkyl, C₃₋₆ cycloalkyl, 3-6 membered heterocyclyl and phenyl are unsubstituted or substituted by one or more substituents selected from the group consisting of deuterium, fluoro, chloro, cyano, methyl, ethyl, isopropyl, trifluoromethyl, difluoromethyl, trideuteromethyl, cyclopropyl, oxacyclobutyl, methoxy, carboxy, methoxycarbonyl, acetyl, amino, dimethylamino and acetylamino;
ring A, ring B, L, R₁, R₅, R₂₈, R₂₉, R₃₀, R₃₁, R₃₂, r, m and p are defined as those in the compound of formula (I).

As a still further preferred embodiment, in the compound of formula (I), the stereoisomer, prodrug or pharmaceutically acceptable salt thereof, the compound of formula (I) is a compound having formula (IIIa1), formula (IIIa2), formula (IIIa3) or formula (IIIa4): 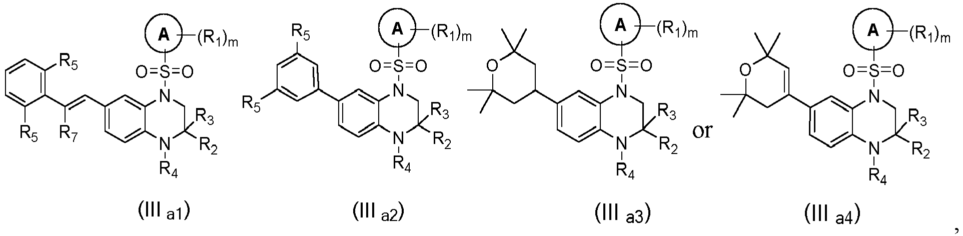 wherein R₂ and R₃ are each independently selected from the group consisting of hydrogen, deuterium, C₁₋₄ alkyl, C₃₋₆ cycloalkyl, 3-6 membered heterocyclyl, -C₀₋₄-O-R₂₉, -C₀₋₄-C(O)OR₂₉, - C₀₋₄-C(O)R₃₀ and -C₀₋₄-O-C(O)R₃₀, or R₂ and R₃, together with the carbon atom directly attached thereto, form C(O), 3-6 membered cycloalkyl or 3-6 membered heterocyclyl;
R₄ is selected from the group consisting of hydrogen, deuterium, C₁₋₄ alkyl, C₃₋₆ cycloalkyl and 3-6 membered heterocyclyl, said C₁₋₄ alkyl, C₃₋₆ cycloalkyl, 3-6 membered heterocyclyl are unsubstituted or substituted by one or more substituents selected from the group consisting of deuterium, fluoro, chloro, cyano, methyl, ethyl, isopropyl, trifluoromethyl, difluoromethyl, trideuteromethyl, cyclopropyl, oxacyclobutyl, methoxy, carboxy, methoxycarbonyl, acetyl, amino, dimethylamino and acetylamino;
each R₅ is independently selected from the group consisting of hydrogen, deuterium, halogen, cyano, C₁₋₄ alkyl, C₃₋₆ cycloalkyl, 3-6 membered heterocyclyl and -O-R₂₉, above groups are unsubstituted or substituted by one or more substituents selected from the group consisting of deuterium, fluoro, chloro, cyano, methyl, ethyl, isopropyl, trifluoromethyl, difluoromethyl, trideuteromethyl, dideuteromethyl, cyclopropyl, oxacyclobutyl, =O, methoxy and carboxy;
R₇ is selected from the group consisting of hydrogen, deuterium, fluoro, methyl, ethyl, trifluoromethyl, difluoromethyl, trideuteromethyl and dideuteromethyl;
ring A, R₁, R₂₉, R₃₀ and m are defined as those in the compound of formula (I).

As a further preferred embodiment, in the compound of formula (I), the stereoisomer, prodrug or pharmaceutically acceptable salt thereof, the compound of formula (I) is a compound having formula (IIb): 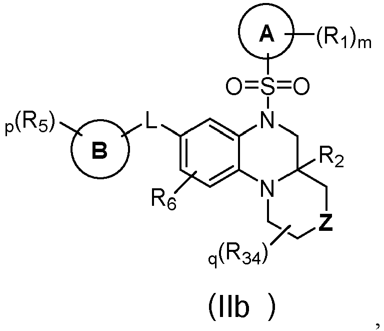 wherein Z is selected from the group consisting of a bond, -O-, -S-, -S(O)-, -S(O)₂-, -N(R₃₃)- and -(CR₃₅R₃₆)-;
R₃₃ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-C(S)R₃₀, -C₀₋₄-O-C(O)R₃₀ and -C₀₋₄-C(O)NR₃₁R₃₂, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, - C₀₋₄-C(O)R₃₀, -C₀₋₄-C(S)R₃₀, -C₀₋₄-O-C(O)R₃₀ and -C₀₋₄-C(O)NR₃₁R₃₂;
each R₃₄ is independently selected from the group consisting of hydrogen, deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and - C₀₋₄-N(R₃₁)-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀;
R₃₅ is selected from the group consisting of hydrogen, deuterium, halogen, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, - C₀₋₄-C(S)R₃₀ and -C₀₋₄-O-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀;
R₃₆ is selected from the group consisting of hydrogen, deuterium, halogen, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, - C₀₋₄-C(S)R₃₀ and -C₀₋₄-O-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀;
q is an integer of 0 to 4; ring A, ring B, L, R₁, R₂, R₅, R₆, R₂₈, R₂₉, R₃₀, R₃₁, R₃₂, m, r and p are defined as those in the compound of formula (I).

As a still further preferred embodiment, in the compound of formula (I), the stereoisomer, prodrug or pharmaceutically acceptable salt thereof, the compound of formula (I) is a compound having formula (IIIb1), formula (IIIb2), formula (IIIb3), formula (IIIb4) or formula (IIIb5): 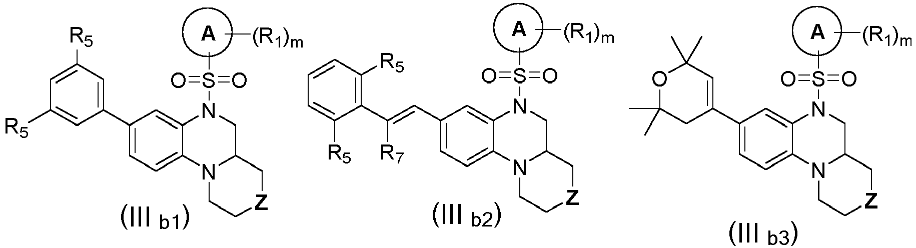 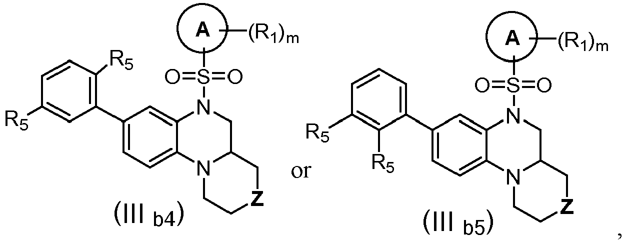 wherein Z is selected from the group consisting of a bond, -O-, -S-, -S(O)-, -S(O)₂-, -N(R₃₃)- and -(CR₃₅R₃₆)-;
each R₅ is independently selected from the group consisting of hydrogen, deuterium, halogen, cyano, C₁₋₄ alkyl, C₃₋₆ cycloalkyl, 3-6 membered heterocyclyl and -O-R₂₉, above groups are unsubstituted or substituted by one or more substituents selected from the group consisting of deuterium, fluoro, chloro, cyano, methyl, ethyl, isopropyl, trifluoromethyl, difluoromethyl, trideuteromethyl, dideuteromethyl, cyclopropyl, oxacyclobutyl, =O, methoxy and carboxy;
R₇ is selected from the group consisting of hydrogen, deuterium, fluoro, methyl, ethyl, trifluoromethyl, difluoromethyl, trideuteromethyl and dideuteromethyl;
R₃₃ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-C(S)R₃₀, -C₀₋₄-O-C(O)R₃₀ and -C₀₋₄-C(O)NR₃₁R₃₂, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀ and -C₀₋₄-C(O)NR₃₁R₃₂;
R₃₅ is selected from the group consisting of hydrogen, deuterium, halogen, C₁₋₄ alkyl, C₁₋₄ haloalkyl, C₁₋₄ deuterioalkyl, C₃₋₆ cycloalkyl, 3-6 membered heterocyclyl, -O-R₂₉, -C(O)OR₂₉, -O-C(O)R₃₀ and -C(O)NR₃₁R₃₂, above groups are unsubstituted or substituted by one or more substituents selected from the group consisting of deuterium, halogen, cyano, nitro, azido, C₁₋₄ alkyl, C₁₋₄ haloalkyl, C₁₋₄ deuterioalkyl, C₃₋₆ cycloalkyl, 3-6 membered heterocyclyl, =O, -S(O)ᵣR₂₈, -C₀₋₄-O-R₂₉, -C(O)OR₂₉, -C(O)R₃₀ and -C(O)NR₃₁R₃₂;
R₃₆ is selected from the group consisting of hydrogen, deuterium, halogen, C₁₋₄ alkyl, C₂₋₄ alkenyl, C₂₋₄ alkynyl, C₁₋₄ haloalkyl, C₁₋₄ deuterioalkyl and C₃₋₆ cycloalkyl, above groups are unsubstituted or substituted by one or more substituents selected from the group consisting of deuterium, fluoro, chloro, cyano, nitro, azido, methyl, ethyl, hydroxy, methoxy and carboxy;
ring A, R₁, R₂₈, R₂₉, R₃₀, R₃₁, R₃₂ and m are defined as those in the compound of formula (I).

As a still further preferred embodiment, in the compound of formula (I), the stereoisomer, prodrug or pharmaceutically acceptable salt thereof, ring A, together with -(R₁)ₘ, forms the following structures: 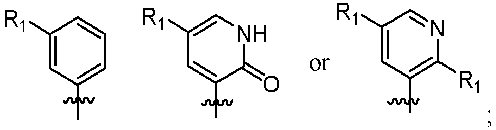
wherein each R₁ is independently selected from the group consisting of hydrogen, deuterium, halogen, cyano, C₁₋₄ alkyl, C₃₋₆ cycloalkyl, 3-6 membered heterocyclyl and -O-R₂₉, above groups are unsubstituted or substituted by one or more substituents selected from the group consisting of deuterium, fluoro, chloro, cyano, methyl, ethyl, isopropyl, trifluoromethyl, difluoromethyl, trideuteromethyl, dideuteromethyl, cyclopropyl, oxacyclobutyl, =O, methoxy, carboxy, methoxycarbonyl, acetyl, amino, dimethylamino and acetylamino;
each R₂₈ is independently selected from the group consisting of hydrogen, deuterium, C₁₋₄ alkyl, C₂₋₄ alkenyl, C₃₋₈ cycloalkyl, 3-8 membered heterocyclyl, C₅₋₈ aryl, 5-8 membered heteroaryl and - NR₃₁R₃₂, above groups are unsubstituted or substituted by one or more substituents selected from the group consisting of deuterium, halogen, hydroxy, =O, cyano, C₁₋₄ alkyl, C₁₋₄ alkoxy, C₃₋₈ cycloalkyl, C₃₋₈ cycloalkoxy and 3-8 membered heterocyclyl;
each R₂₉ is independently selected from the group consisting of hydrogen, deuterium, C₁₋₄ alkyl, C₂₋₄ alkenyl, C₃₋₈ cycloalkyl, 3-8 membered heterocyclyl, C₅₋₈ aryl and 5-8 membered heteroaryl, above groups are unsubstituted or substituted by one or more substituents selected from the group consisting of deuterium, halogen, hydroxy, =O, cyano, C₁₋₄ alkyl, C₁₋₄ alkoxy, C₃₋₈ cycloalkyl, C₃₋₈ cycloalkoxy and 3-8 membered heterocyclyl;
each R₃₀ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₈ cycloalkyl, C₃₋₈ cycloalkyloxy, 3-8 membered heterocyclyl, 3-8 membered heterocyclyloxy, C₅₋₈ aryl, C₅₋₈ aryloxy, 5-8 membered heteroaryl, 5-8 membered heteroaryloxy and -NR₃₁R₃₂, above groups are unsubstituted or substituted by one or more substituents selected from the group consisting of deuterium, halogen, hydroxy, cyano, C₁₋₄ alkyl, C₁₋₄ alkoxy, C₃₋₈ cycloalkyl, C₃₋₈ cycloalkyloxy, 3-8 membered heterocyclyl, 3-8 membered heterocyclyloxy, C₅₋₈ aryl, C₅₋₈ aryloxy, 5-8 membered heteroaryl, 5-8 membered heteroaryloxy and -NR₃₁R₃₂;
each R₃₁ and each R₃₂ are independently selected from the group consisting of hydrogen, deuterium, hydroxy, C₁₋₄ alkyl, C₂₋₄ alkenyl, C₂₋₄ alkynyl, C₃₋₈ cycloalkyl, 3-8 membered heterocyclyl, amino, monoalkylamino and dialkylamino.

As the most preferred embodiment, the compound of formula (I), the stereoisomer, prodrug or pharmaceutically acceptable salt thereof includes, but is not limited to, the following compounds:

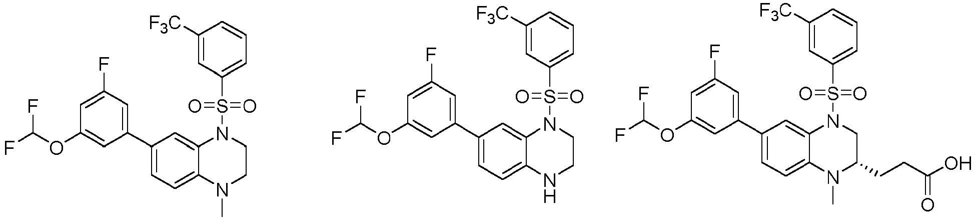

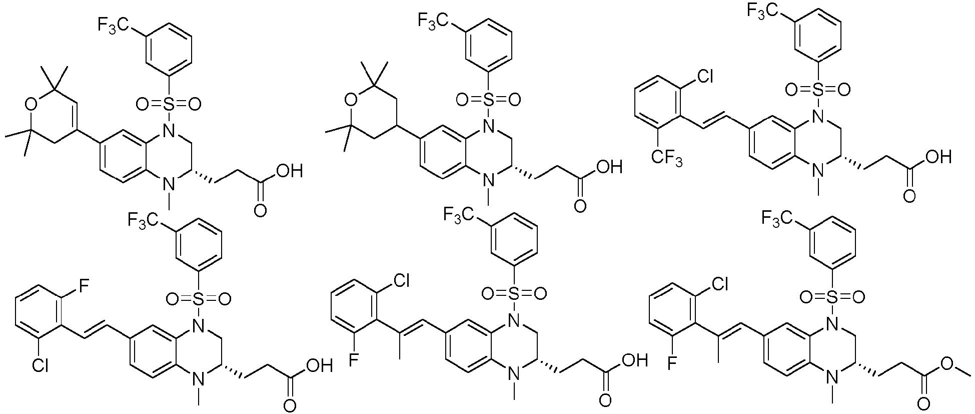

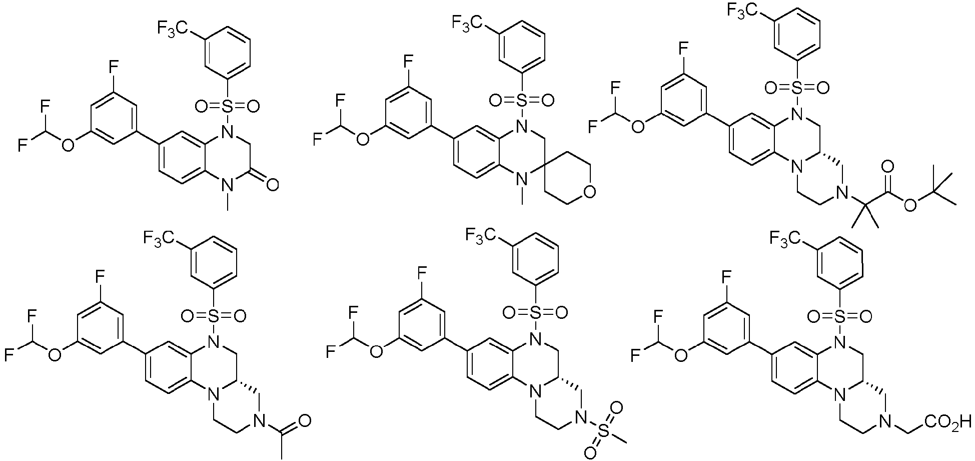

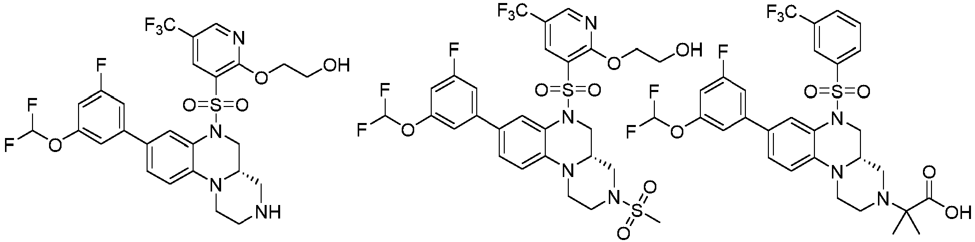

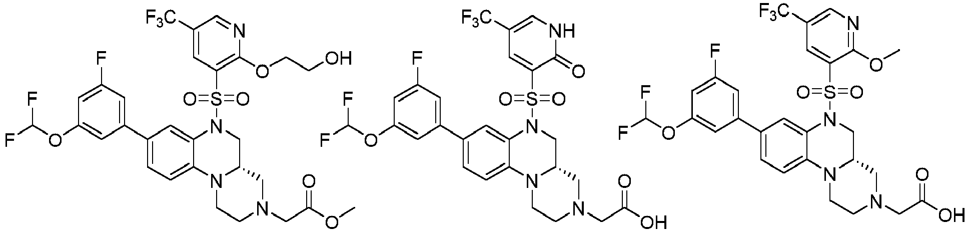

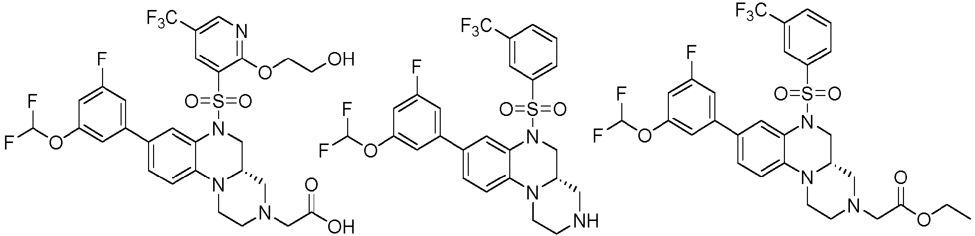

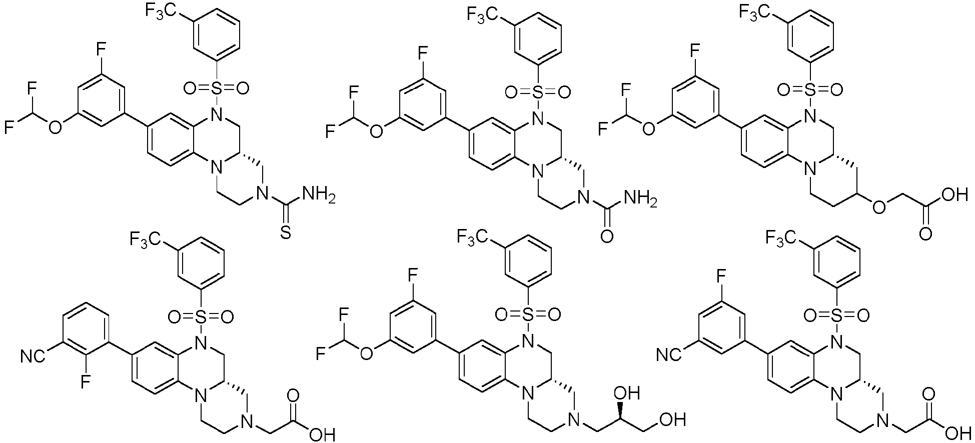

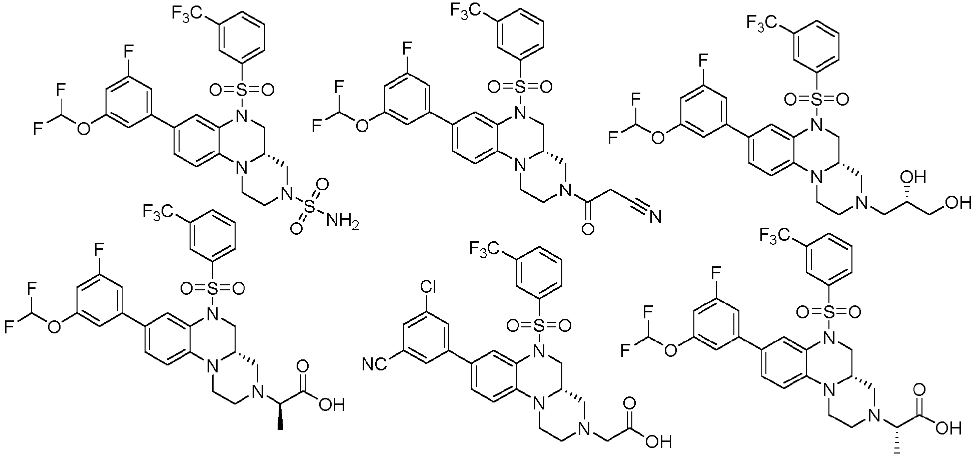

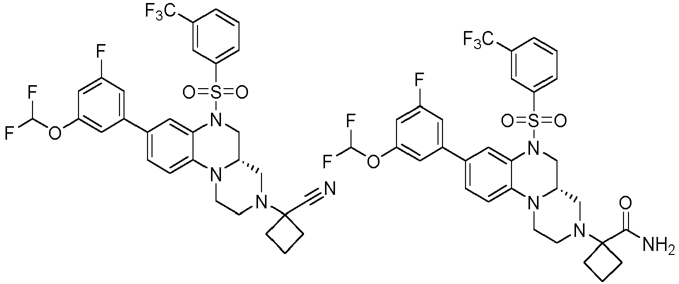

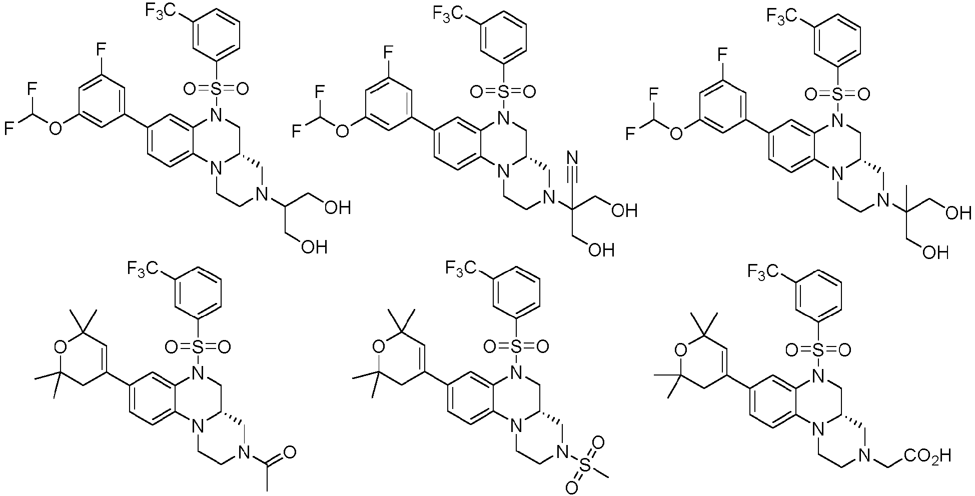

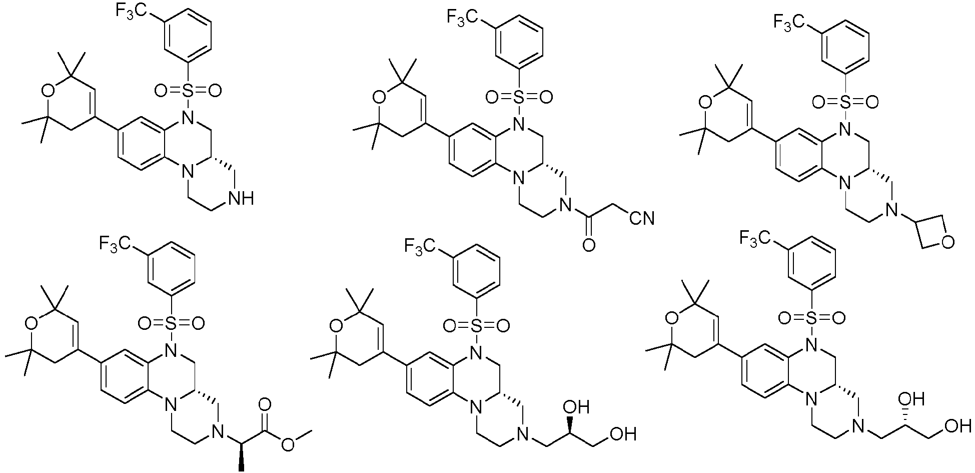

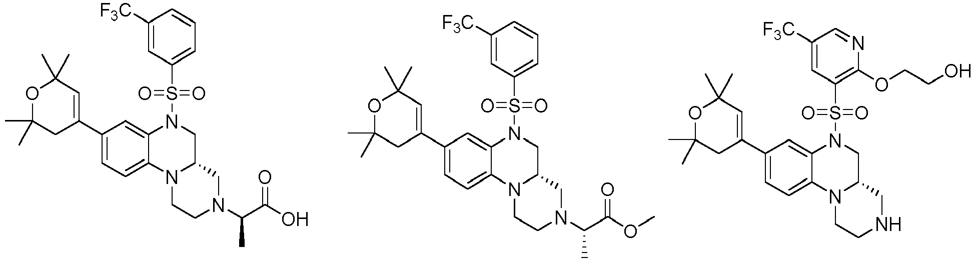

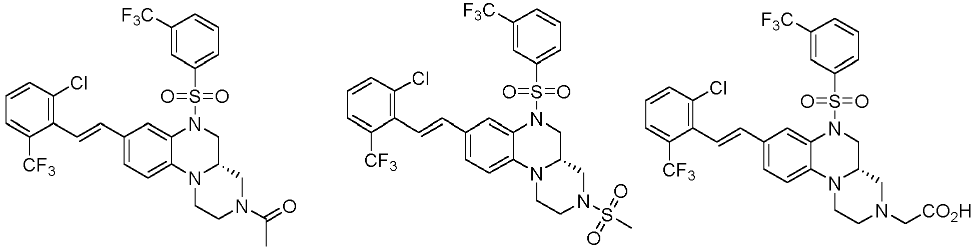

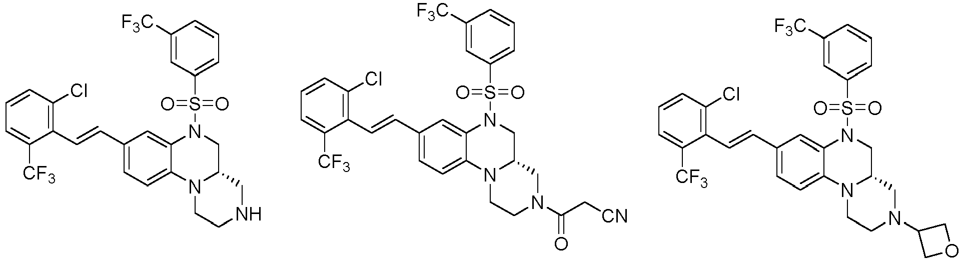

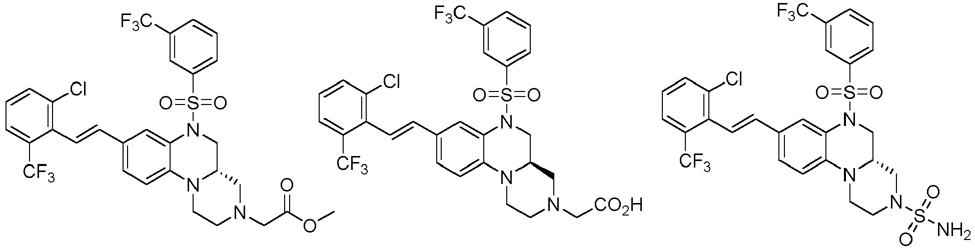

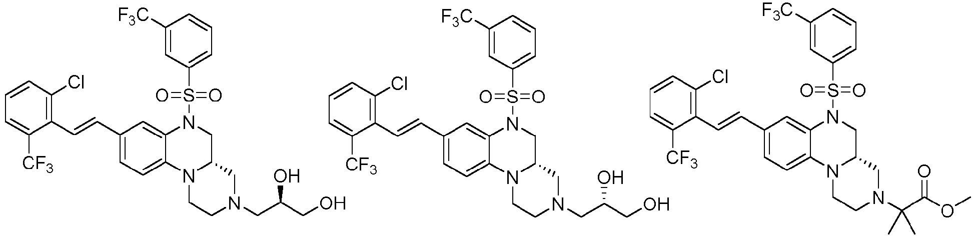

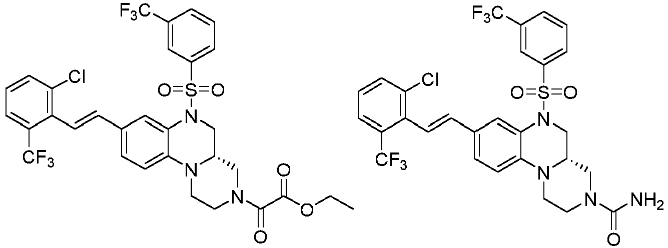

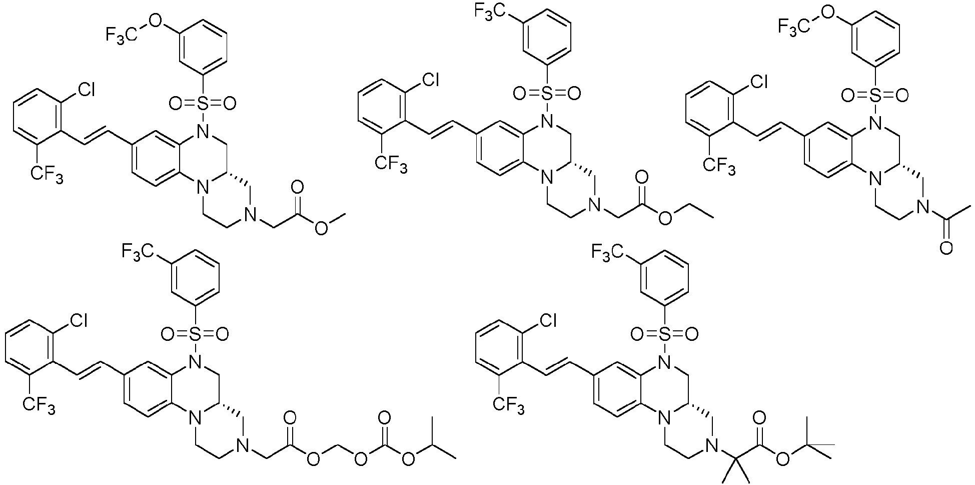

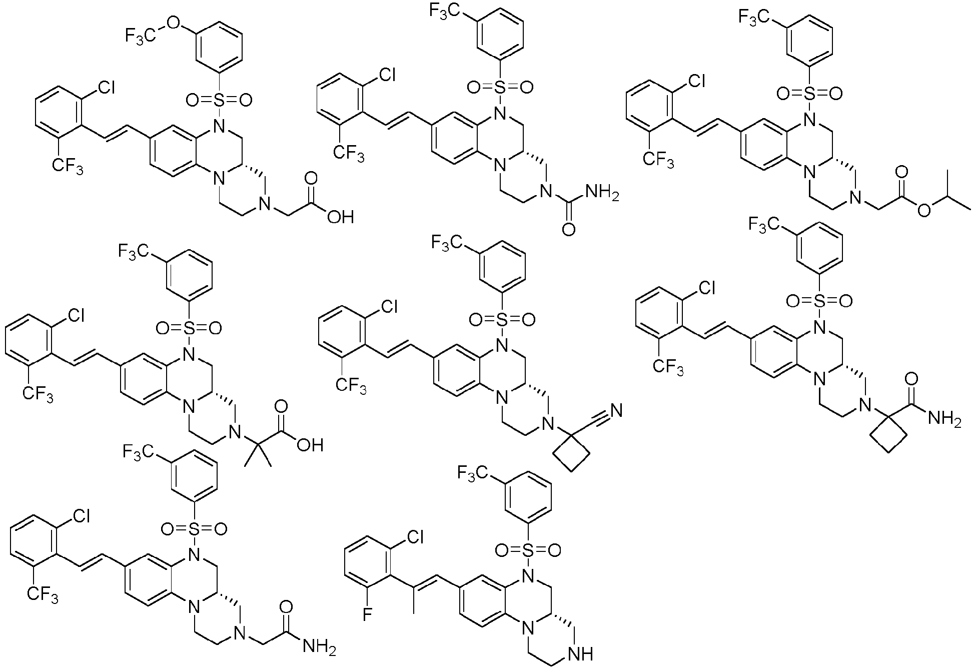

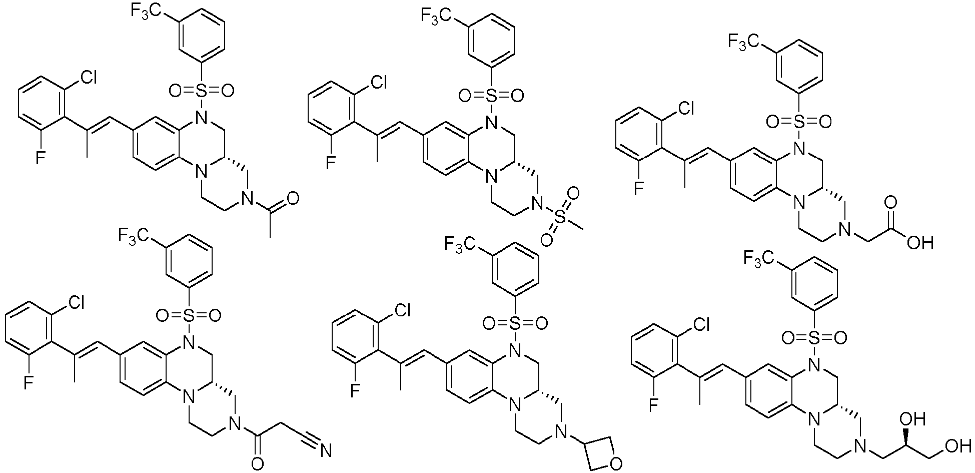

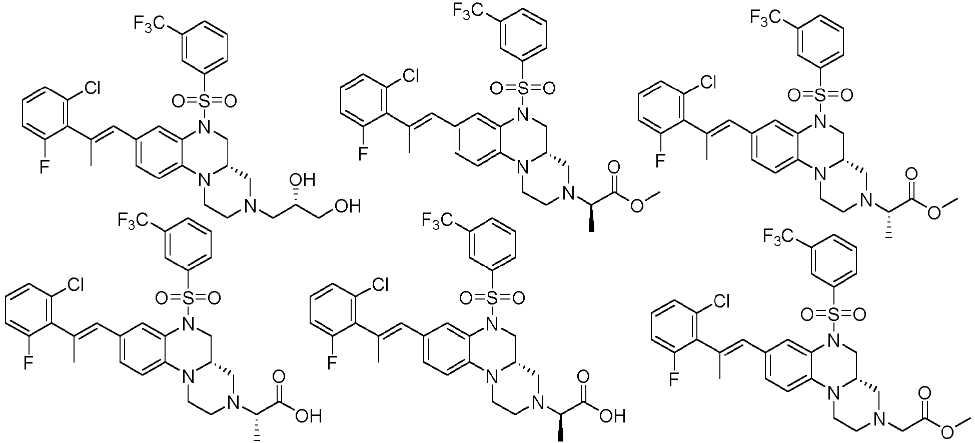

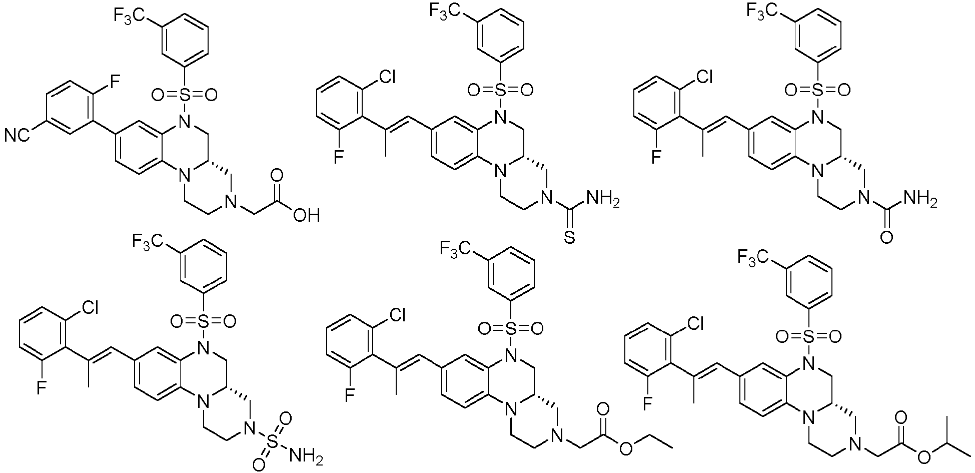

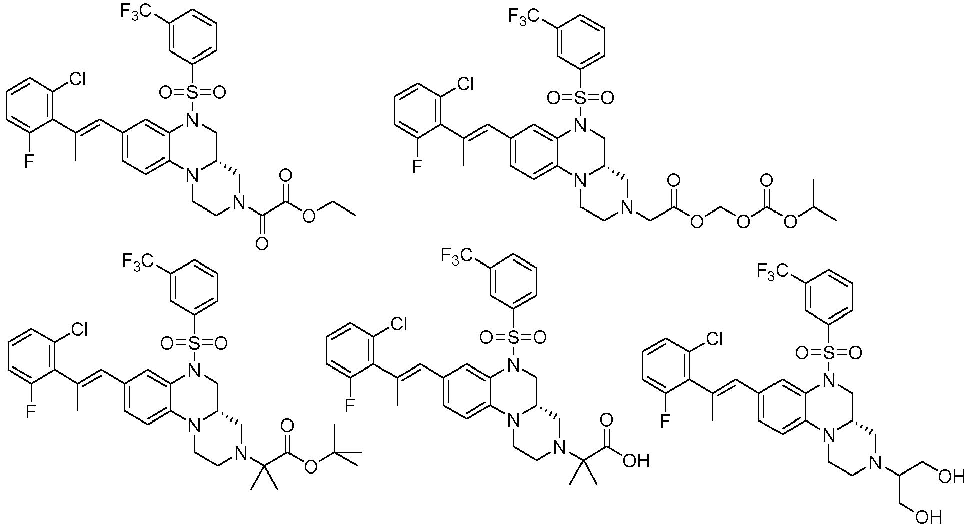

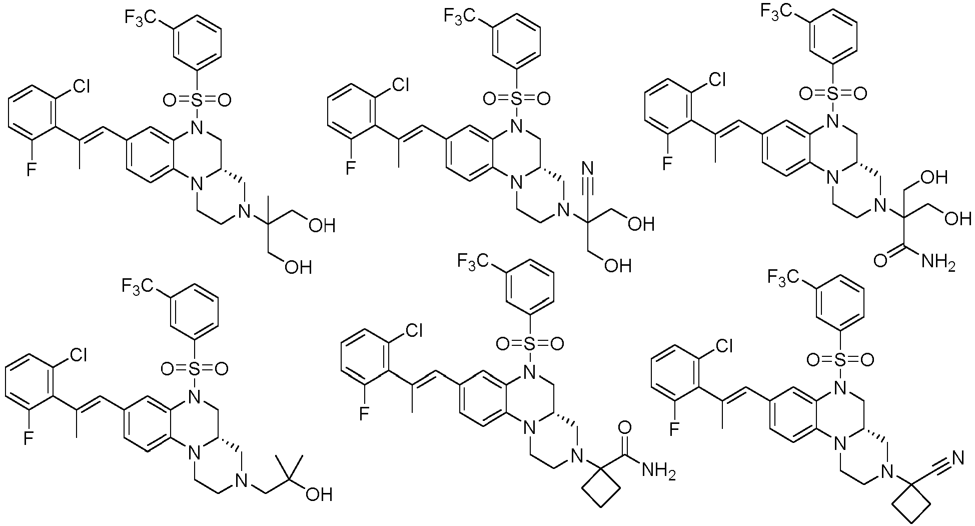

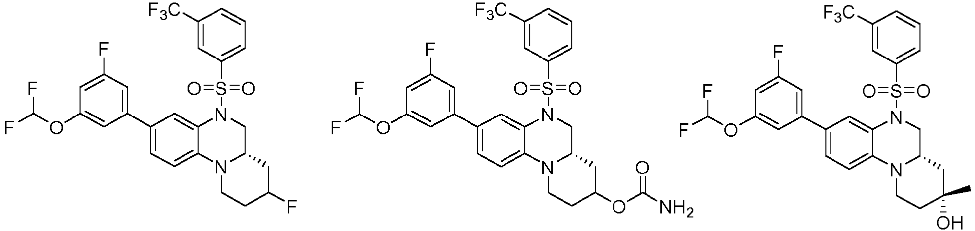

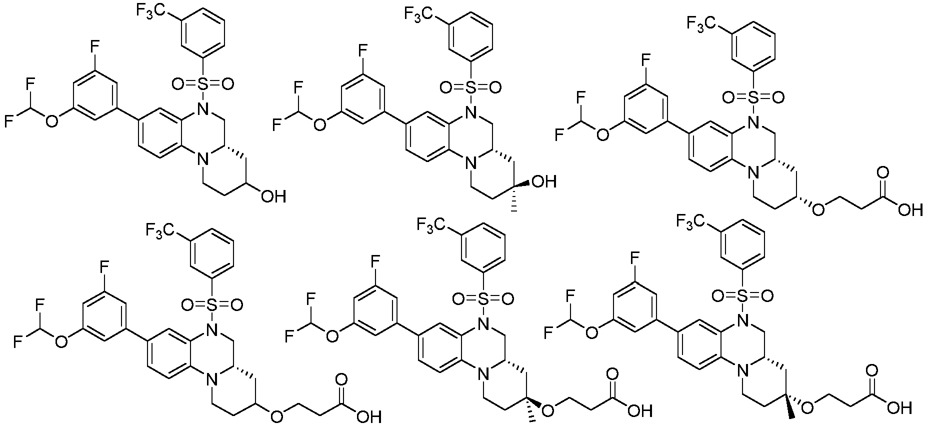

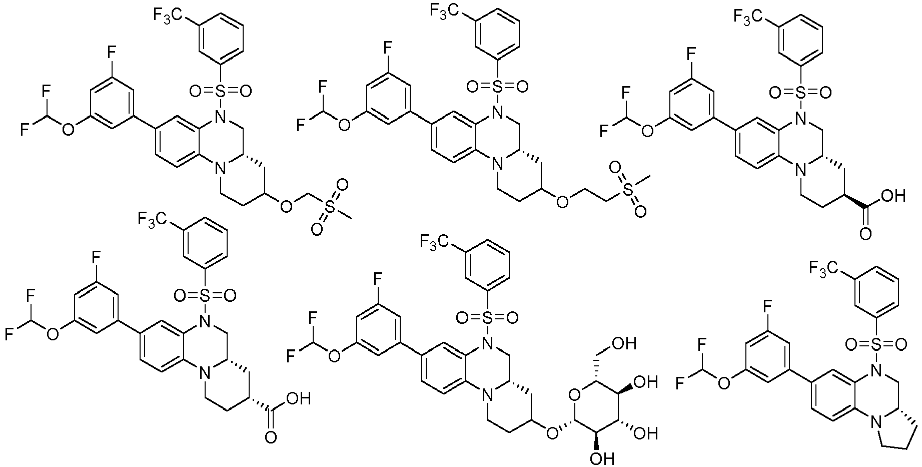

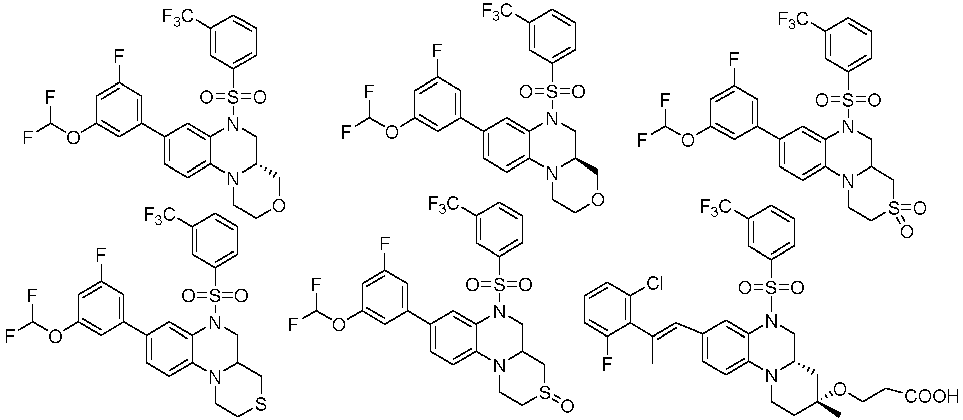

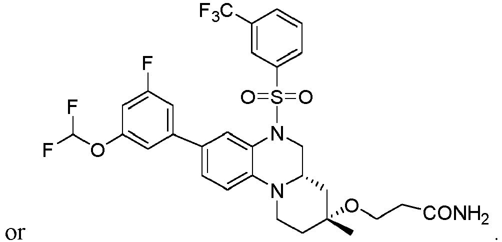

The second aspect of the present invention provides a method for preparing the aforementioned compound of formula (I), the stereoisomer, prodrug or pharmaceutically acceptable salt thereof, comprising the following step: 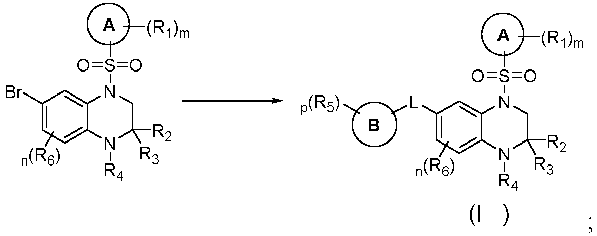 or, 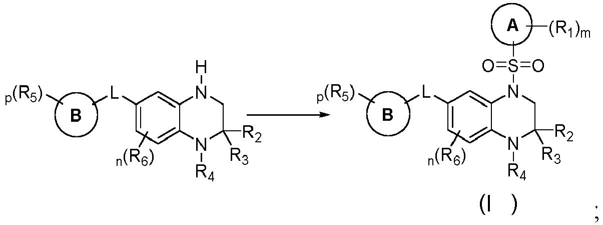
optionally, the compound of formula (I) can be obtained by further substitution reaction according to the definitions of substituents R₂, R₃ and R₄;
wherein, ring A, ring B, L, R₁, R₂, R₃, R₄, R₅, R₆, m, n and p are defined as those in the compound of formula (I).

The third aspect of the present invention provides a pharmaceutical composition, comprising the aforementioned compound of formula (I), the stereoisomer, prodrug or pharmaceutically acceptable salt thereof, and pharmaceutically acceptable carrier.

The fourth aspect of the present invention provides uses of the above compound of formula (I), the stereoisomer, prodrug or pharmaceutically acceptable salt thereof in the preparation of medicaments for the treatment of one or more tumors, cancers, metabolic diseases, and autoimmune diseases or disorders.

As a preferred embodiment, the metabolic disease and autoimmune disease or disorder are selected from the group consisting of atopic dermatitis, contact dermatitis, allergic dermatitis, comedo, acne, cystic fibrosis, allograft rejection, multiple sclerosis, scleroderma, Systemic Lupus Erythematosus (SLE), psoriasis, Hashimoto's disease, arthritis, rheumatoid arthritis, psoriatic arthritis, juvenile idiopathic arthritis, juvenile rheumatoid arthritis, osteoarthritis, ankylosing spondylitis, Psoriatic Arthritis (PsA), autoimmune diabetes, diabetes mellitus type I, diabetes mellitus type II, obesity, fatty liver, adipose tissue-related inflammation, pancreatitis, thyroiditis, autoimmune thyroid disease, biliary cirrhosis, liver fibrosis, Non-alcoholic Fatty Liver Disease (NAFLD), ulcerative colitis, Crohn's disease, regional enteritis,Inflammatory Bowel Disease (IBD), Inflammatory Bowel Syndrome (IBS), Jogging Syndrome (S Jogging Syndrome), original sclerosing cholangitis, autoimmune polyendocrine syndrome type I, autoimmune polyendocrine syndrome type II, celiac disease, neuritis, systemic sclerosis, endometriosis, Behcet's syndrome, myocarditis, dermatomyositis, polymyositis, graft-versus-host disease, sarcoidosis, myocardial infarction, pulmonary hypertension, cutaneous leishmaniasis, Crohn's disease, autoimmune ocular disease, optic neuritis, neuromyelitis optica, xerophthalmia, uveitis, insulin resistance, myasthenia gravis, age-related macular degeneration, Guillain-Barre syndrome, glomerulonephritis, scleritis, major depressive disorder, seasonal affective disorder, Post-Traumatic Stress (Mental) Disorder (PTSD), bipolar disorder, autism, epilepsy, Alzheimer's disease, asthma, Chronic Obstructive Pulmonary Disease (COPD), bronchitis, allergic rhinitis, anaphylactic rhinitis, steroid-resistant asthma, toxic diffuse goiter, Obstructive Sleep Apnea Syndrome (OSAS), sinus polyps and a central nervous system disorder associated with changes in sleep and/or circadian rhythm.

As a preferred embodiment, the tumor or cancer is selected from the group consisting of fallopian tube tumor, ovarian tumor, peritoneal tumor, stage IV melanoma, solid tumor, glioma, glioblastoma, papillary renal carcinoma, head and neck tumor, lymphoma, myeloma, non-Hodgkin's lymphoma, diffuse large B-cell lymphoma, follicular lymphoma, synovial sarcoma, hepatocellular carcinoma, breast cancer, uterine cancer, colon cancer, lung cancer, gastric cancer, rectal cancer, pancreatic cancer, brain cancer, skin cancer, oral cancer, prostate cancer, bone cancer, renal cancer, ovarian cancer, bladder cancer, liver cancer, leukemia and non-small cell lung cancer.

The fifth aspect of the present invention provides the above compound of formula (I), the stereoisomer, prodrug or pharmaceutically acceptable salt thereof for use as medicaments for treating one or more tumors, cancers, metabolic diseases, autoimmune diseases or disorders.

### DETAILED DESCRIPTION OF THE INVENTION

After an extensive and intensive research, the inventors of the present invention develop a 1,2,3,4-tetrahydroquinoxaline derivative with the structure of formula (I) as well as preparation method therefor and application thereof for the first time. The compound of the present invention has a strong inhibition effect on the activity of RORyt kinase, can be widely applied to preparing therapeutic drugs and is expected to be developed into a new generation of RORyt agonist drugs. The present invention is achieved on this basis.

Detailed description: unless otherwise stated, the following terms used in the specification and claims have the following meanings.

"Alkyl" refers to linear or branched saturated aliphatic alkyl groups, for example, "C₁₋₈ alkyl" refers to a linear or branched alkyl containing 1 to 8 carbon atoms, which includes, but is not limited to methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, *etc.*

Alkyl may be optionally substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Cycloalkyl" refers to monocyclic or polycyclic hydrocarbon substituents that are saturated or partially unsaturated, for example, "C₃₋₁₀ cycloalkyl" refers to a cycloalkyl containing 3 to 10 carbon atoms, which may be monocyclic cycloalkyl and polycyclic cycloalkyl, wherein,
monocyclic cycloalkyl includes, but is not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl, *etc*;
polycyclic cycloalkyl includes spirocycloalkyl, fused cycloalkyl and bridged cycloalkyl. "Spirocycloalkyl" refers to a polycyclic group in which a carbon atom (called spiro-atom) is shared among monocyclic rings, wherein those rings may contain one or more (preferably, 1, 2 or 3) double bonds, but none of them has a fully conjugated π-electron system. According to the number of the spiro-atoms shared among the rings, the spirocycloalkyl may be monospirocycloalkyl, bispirocycloalkyl or polyspirocycloalkyl, including but not limited to:

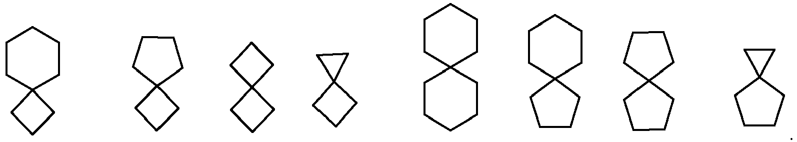

"Fused cycloalkyl" refers to an all-carbon polycyclic group in which each ring shares a pair of adjacent carbon atoms with the other rings in the system, wherein one or more (preferably, 1 or 2) of the rings may contain one or more (preferably, 1, 2 or 3) double bonds, but none of them has a fully conjugated π-electron system. According to the number of formed rings, the fused cycloalkyl may be bicyclic, tricyclic, tetracyclic or polycyclic, including but not limited to:

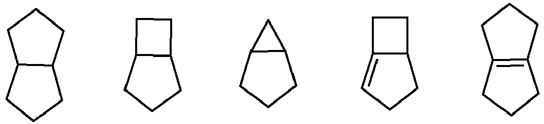

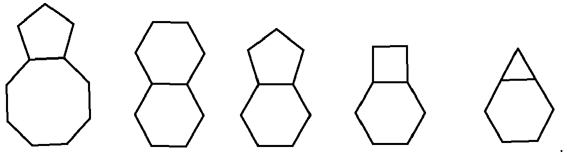

"Bridged cycloalkyl" refers to an all-carbon polycyclic group in which any two rings share two carbon atoms that are not directly connected to each other, wherein these rings may contain one or more (preferably, 1, 2 or 3) double bonds, but none of them has a fully conjugated π-electron system. According to the number of formed rings, the bridged cycloalkyl may be bicyclic, tricyclic, tetracyclic or polycyclic, including but not limited to:

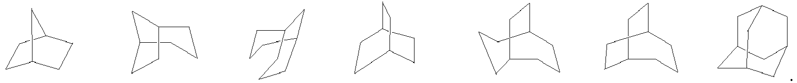

The cycloalkyl ring can be fused to an aryl, heteroaryl or heterocycloalkyl ring, wherein the ring attached to the parent structure is cycloalkyl, which includes, but is not limited to, indanyl, tetrahydronaphthyl, benzocycloheptyl, *etc.*

Cycloalkyl may be optionally substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Heterocyclyl" refers to a monocyclic or polycyclic hydrocarbon substituent that is saturated or partially unsaturated, wherein one or more (preferably, 1, 2, 3 or 4) of the ring atoms are heteroatoms selected from nitrogen, oxygen or S(O)ᵣ (wherein r is an integer of 0, 1 or 2), excluding ring portions of -O-O-, -O-S- or -S-S-, and the remaining ring atoms are carbon atoms. For example, "5-10 membered heterocyclyl" refers to a cyclic group containing 5 to 10 ring atoms, and "3-10 membered heterocyclyl" refers to a cyclic group containing 3 to 10 ring atoms.

Monocyclic heterocyclyl includes, but is not limited to, pyrrolidinyl, piperidinyl, piperazinyl, morpholinyl, thiomorpholinyl, homopiperazinyl, *etc.*

Polycyclic heterocyclyl includes spiroheterocyclyl, fused heterocyclyl, and bridged heterocyclyl. "Spiroheterocyclyl" refers to a polycyclic heterocyclyl group in which an atom (called spiro-atom) is shared among monocyclic rings, wherein one or more ring atoms are heteroatoms selected from nitrogen, oxygen or S(O)ᵣ (wherein r is an integer of 0, 1 or 2), and the remaining ring atoms are carbon atoms. These rings may contain one or more (preferably, 1, 2, 3 or 4) double bonds, but none of them has a fully conjugated π-electron system. According to the number of spiro-atoms shared among the rings, spiroheterocyclyl may be monospiroheterocyclyl, bispiroheterocyclyl or polyspiroheterocyclyl. Spiroheterocyclyl includes, but is not limited to:

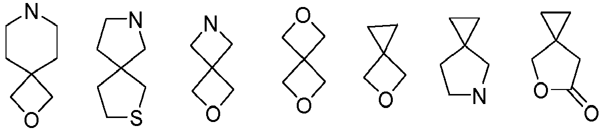

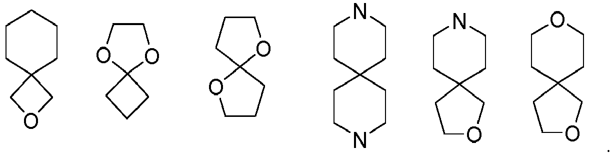

"Fused heterocyclyl" refers to a polycyclic heterocyclyl in which each ring shares a pair of adjacent atoms with the other rings in the system, wherein one or more (preferably, 1 or 2) of the rings may contain one or more (preferably, 1, 2 or 3) double bonds, but none of them has a fully conjugated π-electron system, wherein one or more (preferably, 1, 2, 3 or 4) of the ring atoms are heteroatoms selected from nitrogen, oxygen or S(O)ᵣ (wherein r is an integer of 0, 1 or 2), and the remaining ring atoms are carbon atoms. According to the number of formed rings, the fused heterocyclyl may be bicyclic, tricyclic, tetracyclic or polycyclic, including, but not limited to:

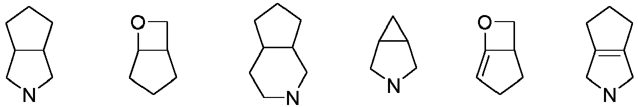

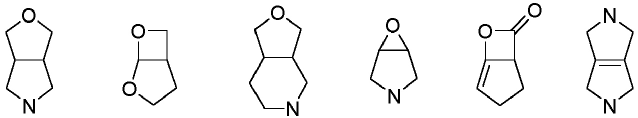

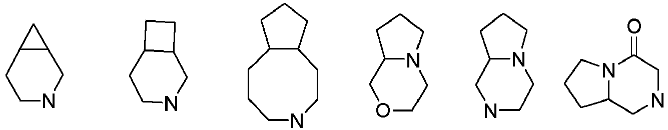

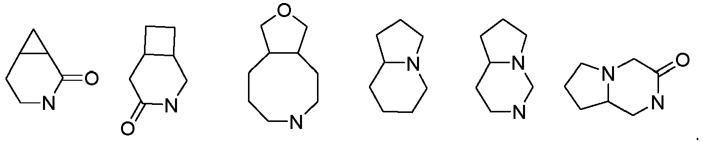

"Bridged heterocyclyl" refers to a polycyclic heterocyclyl in which any two rings share two carbon atoms that are not directly attached to each other, wherein these rings may contain one or more (preferably, 1, 2 or 3) double bonds, but none of them has a fully conjugated π-electron system, wherein one or more (preferably, 1, 2, 3 or 4) of the ring atoms are heteroatoms selected from nitrogen, oxygen or S(O)ᵣ (wherein r is an integer of 0, 1 or 2), and the remaining ring atoms are carbon atoms. According to the number of formed rings, the bridged heterocyclyl may be bicyclic, tricyclic, tetracyclic or polycyclic, including, but not limited to:

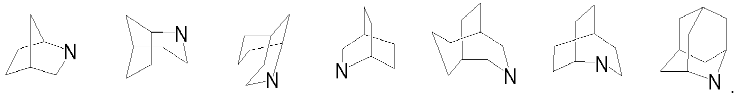

The heterocyclyl ring may be fused to an aryl, heteroaryl or cycloalkyl ring, wherein the ring attached to the parent structure is heterocyclyl, including, but not limited to:

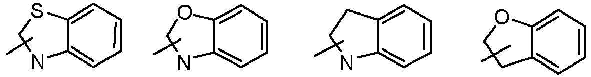

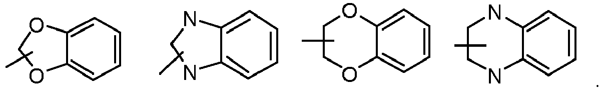

Heterocyclyl may be optionally substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Aryl" refers to an all-carbon monocyclic or fused-polycyclic group (i.e., rings that share a pair of adjacent carbon atoms) and a polycyclic group having a conjugated π-electron system (i.e., rings with adjacent pairs of carbon atoms), for example, "C₅₋₁₀ aryl" refers to an all-carbon aryl containing 5 to 10 carbon atoms, and "5-10 membered aryl" refers to an all-carbon aryl containing 5 to 10 carbon atoms, including but not limited to phenyl and naphthyl. The aryl ring can be fused to a heteroaryl, heterocyclyl or cycloalkyl ring, wherein the ring attached to the parent structure is the aryl ring, including, but not limited to:

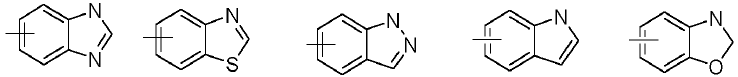

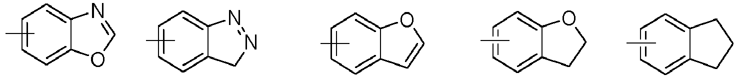

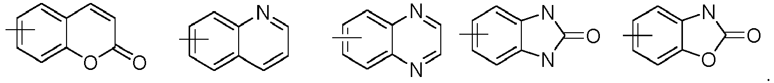

Aryl may be substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Heteroaryl" refers to a heteroaromatic system containing 1 to 4 heteroatoms, and the heteroatoms include heteroatoms selected from nitrogen, oxygen or S(O)ᵣ (wherein r is an integer of 0, 1 or 2), for example, 5-8 membered heteroaryl refers to a heteroaromatic system containing 5 to 8 ring atoms, and 5-10 membered heteroaryl refers to a heteroaromatic system containing 5 to 10 ring atoms, including but not limited to furyl, thiophenyl, pyridyl, pyrrolyl, *N*-alkylpyrrolyl, pyrimidinyl, pyrazinyl, imidazolyl, tetrazolyl, *etc.* The heteroaryl ring can be fused to an aryl, heterocyclyl or cycloalkyl ring, wherein the ring attached to the parent structure is the heteroaryl ring, including, but not limited to:

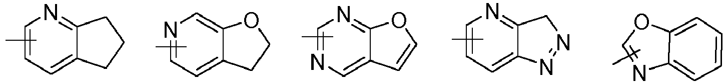

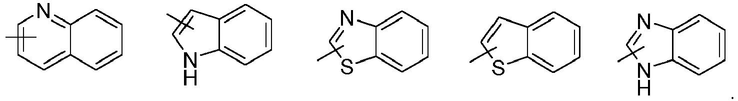

Heteroaryl may be optionally substituted or unsubstituted, and when it is substituted, the substituent is preferably one or more of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Alkenyl" refers to an alkyl defined as above consisting of at least two carbon atoms and at least one carbon-carbon double bond, for example, C₂₋₈ alkenyl refers to a linear or branched alkenyl containing 2 to 8 carbon atoms. The alkenyl includes, but is not limited to, vinyl, 1-propenyl, 2-propenyl, 1-, 2- or 3-butenyl, *etc.*

Alkenyl may be substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Alkynyl" refers to an alkyl defined as above consisting of at least two carbon atoms and at least one carbon-carbon triple bond, for example, C₂₋₈ alkynyl refers to a linear or branched alkynyl containing 2 to 8 carbon atoms. The alkynyl includes, but is not limited to, ethynyl, 1-propynyl, 2-propynyl, 1-, 2- or 3-butynyl, *etc.*

Alkynyl may be substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Alkoxy" refers to -O-(alkyl), wherein the alkyl is defined as above, for example, "C₁₋₈ alkoxy" refers to an alkoxy containing 1 to 8 carbons atoms, including but not limited to methoxy, ethoxy, propoxy, butoxy, *etc.*

Alkoxy may be optionally substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"Cycloalkyloxy" refers to -O-(unsubstituted cycloalkyl), wherein the cycloalkyl is defined as above, for example, "C₃₋₁₀ cycloalkyloxy" refers to a cycloalkyloxy containing 3 to 10 carbon atoms, including but not limited to cyclopropyloxy, cyclobutyloxy, cyclopentyloxy, cyclohexyloxy, *etc.*

Cycloalkyloxy may be optionally substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)P₃₀.

"3-10 membered heterocyclyloxy" refers to -O-(unsubstituted 3-10 membered heterocyclyl), wherein 3-10 membered heterocyclyl is defined as above. 3-10 membered heterocyclyloxy may be optionally substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, - C₀₋₈-C(O)NR₃₁R₃₂ and -C₀₋₈-N(R₃₁)-C(O)R₃₀.

"C₅₋₁₀ aryloxy" refers to -O-(unsubstituted C₅₋₁₀ aryl), wherein C₅₋₁₀ aryl is defined as above. C₅₋₁₀ aryloxy may be optionally substituted or unsubstituted, and when it is substituted, the substituent is preferably one or more of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀.

"5-10 membered heteroaryloxy" refers to -O-(unsubstituted 5-10 membered heteroaryl), wherein the 5-10 membered heteroaryl is defined as above. 5-10 membered heteroaryloxy may be optionally substituted or unsubstituted, and when it is substituted, the substituent is preferably one or more (preferably, 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, - C₀₋₈-C(O)NR₃₁R₃₂ and -C₀₋₈-N(R₃₁)-C(O)R₃₀.

"C₁₋₈ alkanoyl" refers to a monovalent atomic group which is obtained after a hydroxy is removed from the C₁₋₈ alkyl acid, and is also generally referred to as "C₀₋₇-C(O)-", for example, "C₁₋C(O)-" refers to an acetyl; "C₂-C(O)-" refers to a propionyl; and "C₃-C(O)-" refers to a butyryl or isobutyryl.

"-C₀₋₈-S(O)rR₂₈" means that the sulfur atom in -S(O)rR₂₈ is attached to C₀₋₈ alkyl, wherein Co alkyl refers to a bond, and C₁₋₈ alkyl is defined as above.

"-C₀₋₈-O-R₂₉" means that the oxygen atom in -O-R₂₉ is attached to C₀₋₈ alkyl, wherein Co alkyl refers to a bond, and C₁₋₈ alkyl is defined as above.

"-C₀₋₈-C(O)OR₂₉" means that the carbonyl group in -C(O)OR₂₉ is attached to C₀₋₈ alkyl, wherein Co alkyl refers to a bond, and C₁₋₈ alkyl is defined as above.

"-C₀₋₈-C(O)R₃₀" means that the carbonyl group in -C(O)R₃₀ is attached to C₀₋₈ alkyl, wherein Co alkyl refers to a bond, and C₁₋₈ alkyl is defined as above.

"-C₀₋₈-O-C(O)R₃₀" means that the oxygen atom in -O-C(O)R₃₀ is attached to C₀₋₈ alkyl, wherein Co alkyl refers to a bond, and C₁₋₈ alkyl is defined as above.

"-C₀₋₈-NR₃₁R₃₂" means that the nitrogen atom in -NR₃₁R₃₂ is attached to C₀₋₈ alkyl, wherein Co alkyl refers to a bond, and C₁₋₈ alkyl is defined as above.

"-C₀₋₈-C(O)NR₃₁R₃₂" means that the carbonyl group in -C(O)NR₃₁R₃₂ is attached to C₀₋₈ alkyl, wherein Co alkyl refers to a bond, and C₁₋₈ alkyl is defined as above.

"-C₀₋₈-N(R₃₁)-C(O)R₃₀" means that the nitrogen atom in -N(R₃₁)-C(O)R₃₀ is attached to C₀₋₈ alkyl, wherein Co alkyl refers to a bond, and C₁₋₈ alkyl is defined as above.

"C₁₋₈ haloalkyl" refers to an alkyl having 1 to 8 carbon atoms in which hydrogens on the alkyl are optionally substituted by a fluorine, chlorine, bromine or iodine atom, including but not limited to difluoromethyl, dichloromethyl, dibromomethyl, trifluoromethyl, trichloromethyl, tribromomethyl, *etc.*

"C₁₋₈ haloalkoxy" refers to an alkoxy having 1 to 8 carbon atoms in which hydrogens on the alkyl are optionally substituted by a fluorine, chlorine, bromine or iodine atom, including, but not limited to, difluoromethoxy, dichloromethoxy, dibromomethoxy, trifluoromethoxy, trichloromethoxy, tribromomethoxy, *etc.*

"Halogen" refers to fluorine, chlorine, bromine or iodine. "DCM" refers to dichloromethane. "PE" refers to petroleum ether. "EA/EtOAc" refers to ethyl acetate. "THF" refers to tetrahydrofuran. "PE" refers to petroleum ether. "DMSO" refers to dimethylsulfoxide. "MeCN" refers to acetonitrile. "DME" refers to dimethyl ether. "Pd(dppf)Cl₂" refers to palladium[1,1'-dikis(diphenylphosphorus)ferrocene ]chloride.

The term "optional" or "optionally" means that the event or circumstance subsequently described may, but not necessarily, occur, and that the description includes instances where the event or circumstance occurs or does not occur. For example, "heterocyclyl group optionally substituted by alkyl" means that alkyl may be, but not necessarily, present, and that the description includes instances where the heterocyclyl group is or is not substituted by alkyl.

The term "substituted" means that one or more hydrogen atoms in the group are each independently substituted by a corresponding number of substituents. It goes without saying that a substituent is only in its possible chemical position, and those skilled in the art will be able to determine (by experiments or theories) possible or impossible substitution without undue efforts. For example, it may be unstable when an amino or hydroxy having a free hydrogen is bound to a carbon atom having an unsaturated bond (such as olefin).

"Pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

### The present invention is further explained in detail below with reference to examples, which are not intended to limit the present invention, and the present invention is not merely limited to the contents of the examples.

The compound structure of the present invention is determined by nuclear magnetic resonance (NMR) and/or liquid chromatography-mass spectrometry (LC-MS). The NMR chemical shift (δ) is given in parts per million (ppm). The NMR determination is conducted by using a Bruker AVANCE-400 or a Bruker AVANCE-500 nuclear magnetic resonance apparatus, with deuterated dimethyl sulfoxide (DMSO-*d₆*), deuterated methanol (CD₃OD), and deuterated chloroform (CDCl₃) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatograph (Sunfire C18 150 * 4.6 mm chromatographic column) and a Waters 2695-2996 high pressure liquid chromatograph (Gimini C18 150 * 4.6 mm chromatographic column).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation and purification of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, wherein the solvent is a dry solvent, and the reaction temperature is in degree centigrade (°C).

### Preparation of Intermediates

### 1. Preparation of 6-bromo-1-methyl-4-((3-(trifluoromethyl)phenyl)sulfonyl)-1,2,3,4-tetrahydroquinoxaline

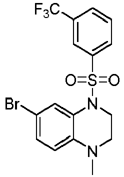

### Step 1: synthesis of 2-((4-bromo-2-nitrophenyl)(methyl)amino)ethan-1-ol

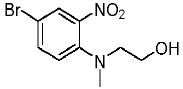

4-bromo-1-fluoro-2-nitrobenzene (2.50 g, 11.4 mmol), 2-(methylamino)ethane-1-ol (2.13 g, 28.4 mmol), potassium carbonate (4.70 g, 34.1 mmol) and *N,N*-dimethylformamide (10 mL) were added into a 250 mL single-necked flask, the reaction mixture was stirred at 60 °C for 2 hrs. The reaction mixture was cooled, and then diluted with water (50 mL), an d extracted with ethyl acetate (50 mL * 2). The organic phases were washed with brine (50 mL), dried over anhydrous sodium sulfate, and then filtered and concentrated to obtain the product 2-((4-bromo-2-nitrophenyl)(methyl)amino) ethan-1-ol (3.20 g, yield 100%). ESI -MS: 275.0 [M+1]⁺.

### Step 2: synthesis of 2-((2-amino-4-bromophenyl)(methyl)amino)ethan-1-ol

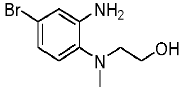

2-((4-bromo-2-nitrophenyl)(methyl)amino)ethan-1-ol (3.20 g, 99%, 11.6 mmol), water (50 mL), iron powder (2.35 g, 41.9 mmol), and ammonium chloride (626 mg, 11.6 mmol) wer e added into a 100 mL single-necked flask. The reaction mixture was reacted overnight at 105 °C, cooled to 40-50 °C and filtered through celite, and the filter cake was rinsed with ethyl acetate (20 mL * 4). The filtrate was separated into layers and the aqueous la yer was further extracted with ethyl acetate (50 mL). The organic phases were combined a nd dried over anhydrous sodium sulfate, and then filtered and concentrated. The crude pro duct was separated by a silica gel column [eluent: petroleum ether : ethyl acetate = 2:1-1: 3] to obtain 2-((2-amino-4-bromophenyl)(methyl)amino)ethan-1-ol (2.30 g, yield 76.9%). ESI -MS: 245.0 [M+1]⁺.

### Step 3: synthesis of 4-bromo-N¹-(2-chloroethyl)-N¹-methylbenzene-1,2-diamine

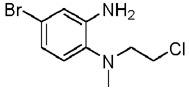

2-((2-amino-4-bromophenyl)(methyl)amino)ethan-1-ol (2.30 g, 95%, 8.91 mmol) and dic hloromethane (50 mL) were added into a 250mL single-necked flask, then thionyl chloride (1.27 g, 10.7 mmol) was added dropwise under ice bath, then two drops of *N,N*-dimethylfo rmamide were added. The reaction mixture was warmed to room temperature and stirred for 1 hr, and then warmed to 35 °C and stirred for 2.5 hrs. After concentration, sodium hydrox ide aqueous solution (IN) was added thereto, the mixture solution was extracted with dichl oromethane. The organic phases were washed with brine, dried over anhydrous sodium sulf ate, filtered and concentrated. The residue was separated by a rapid silica gel column [elu ent: petroleum ether : ethyl acetate = 0:100-15:85] to obtain 4-bromo-*N*¹-(2-chloroethyl)-*N*¹-methylbenzene-1,2-diamine (1.84 g, yield 74.4%). ESI-MS: 263.0 [M+1]⁺.

### Step 4: synthesis of 6-bromo-1-methyl-1,2,3,4-tetrahydroquinoxaline

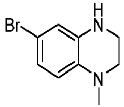

4-bromo-*N*¹-(2-chloroethyl)-*N*¹-methylbenzene-1,2-diamine (1.84 g, 95%, 6.63 mmol) wa s dissolved in *N,N*-dimethylformamide (20 mL) and potassium carbonate (1.83 g, 13.3 mm ol) was added thereto. The reaction mixture was stirred at 80 °C for 1 hr, then warmed t o 100 °C and stirred for 1.5 hrs. The mixture solution was cooled to room temperature, d iluted with water (50 mL), and extracted twice with ethyl acetate (50 mL). The organic p hases were washed once with saturated sodium chloride (80 mL). The organic phases were dried and filtered, the filtrate was concentrated; the residue was separated by a rapid silic a gel column [eluent: petroleum ether : ethyl acetate = 0:100-70:30] to obtain 6-bromo-1-methyl-1,2,3,4-tetrahydroquinoxaline (750 mg, yield 47.3%). ESI-MS: 227.0 [M+1]⁺.

### Step 5: synthesis of 6-bromo-l-methyl-4-((3-(trifluoromethyl)phenyl)sulfonyl)-1,2,3,4-tetra hydroquinoxaline

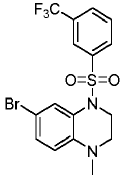

6-bromo-1-methyl-1,2,3,4-tetrahydroquinoxaline (375 mg, 95%, 1.57 mmol), 3-(trifluorometh yl) benzenesulfonyl chloride (422 mg, 1.73 mmol) was dissolved in dichloromethane (15 m L), and 4-dimethylaminopyridine (375 mg, 95%, 1.57 mmol) was added thereto. The reacti on mixture was stirred at room temperature overnight; LCMS showed the reaction was co mpleted. The reaction mixture was concentrated to dryness, and the residue was separated by a rapid silica gel column [eluent: EtOAc : PE = 0-80%] to obtain 6-bromo-1-methyl-4-((3-(trifluoromethyl)phenyl)sulfonyl)-1,2,3,4-tetrahydroquinoxaline (480 mg, yield 66.7%). ES I-MS: 435.0 [M+1]⁺.

### 2. Preparation of tert-butyl 6-bromo-4-((3-(trifluoromethyl)phenyl)sulfonyl)-3,4-dihydroquinoxaline-1(2H)-carboxytate

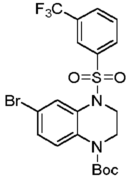

### Step 1: synthesis of methyl (4-bromo-2-nitrophenyl)glycinate

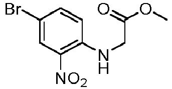

4-bromo-1-fluoro-2-nitrobenzene (2.0 g, 9.09 mmol), glycine methyl ester hydrochloride (1.26 g, 10.0 mmol), diisopropylethylamine (2.5 mL, 14.5 mmol) and acetonitrile (30 mL) were added into a 250 mL single-necked flask, and the reaction mixture was stirred at 8 0 °C for 2 hrs. The reaction mixture was cooled, and then diluted with water (40 mL), a nd extracted with ethyl acetate (70 mL * 2). The organic phases were washed with brine (50 mL), dried over anhydrous sodium sulfate, filtered, and concentrated to obtain methyl (4-bromo-2-nitrophenyl)glycinate (2.1 g, yield 71.9%). ESI-MS: 289.0 [M+1]⁺.

### Step 2: synthesis of 7-bromo-3,4-dihydroquinoxalin-2(1H)-one

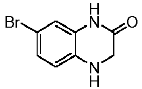

Methyl (4-bromo-2-nitrophenyl)glycinate (1.8 g, 90%, 5.6 mmol), acetic acid (20 mL) and iron powder (1.57 g, 28.0 mmol) were added into a 100 mL single-necked flask. The reaction mixture was then reacted at 60 °C for 2.5 hrs. The mixture solution was cooled t o 40-50 °C and filtered through celite, and the filter cake was rinsed with ethyl acetate (2 0 mL * 4). The filtrate was separated into layers and the aqueous layer was further extrac ted with ethyl acetate (50 mL). The organic phases were combined, washed successively w ith brine (50 mL * 2) and saturated sodium bicarbonate solution (50 mL * 2), dried over anhydrous sodium sulfate, filtered and concentrated. The residue was separated by a silica gel column [eluent: petroleum ether : ethyl acetate = 95:5-0:100] to obtain 7-bromo-3,4-dih ydroquinoxalin-2(1*H*)-one (1.0 g, yield 74.7%). ESI-MS: 227.0 [M+1]⁺.

### Step 3: synthesis of tert-butyl 6-bromo-3-oxo-3,4-dihydroquinoxaline-1(2H)-carboxylate

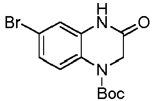

7-bromo-3,4-dihydroquinoxalin-2(1*H*)-one (500 mg, 95%, 2.1 mmol), dichloromethane (15 mL), di-*tert*-butyl dicarbonate (684 mg, 3.14 mmol) and 4-dimethylaminopyridine (684 mg, 3.14 mmol) were added into a 100 mL single-necked flask. The reaction mixture was heat ed to 40 °C and stirred for 2 hrs. The mixture solution was concentrated, and the residue was separated by a rapid silica gel column [eluent: petroleum ether : ethyl acetate = 0:100 -40:60] to obtain tert-butyl 6-bromo-3-oxo-3,4-dihydroquinoxaline-1(2*H*)-carboxylate (580 m g, yield 80.0%). ESI-MS: 349.0 [M+23]⁺, 271.0 [M-56]⁺.

### Step 4: synthesis of tert-butyl 6-bromo-3,4-dihydroquinoxaline-1(2H)-carboxylate

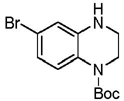

*Tert*-butyl 6-bromo-3-oxo-3,4-dihydroquinoxaline-1(2*H*)-carboxylate (260 mg, 95%, 0.75 mmol) was dissolved in anhydrous tetrahydrofuran (6 mL), and borane dimethylsulfide com plex (2M tetrahydrofuran solution, 1.13 mL, 2.26 mmol) was added thereto. The reaction mixture was stirred at 50 °C for 1.5 hrs. LCMS showed the reaction was completed; the r eaction was quenched with methanol (10 mL) and stirred at 40 °C for 1 hr. The mixture was concentrated, and the residue was separated by a rapid silica gel column [eluent: petr oleum ether : ethyl acetate = 0:100-50:50] to obtain tert-butyl 6-bromo-3, 4-dihydroquinoxa line-1(2*H*)-carboxylate (210 mg, yield 84.9%). ESI-MS: 313.0 [M+1]⁺.

### Step 5: synthesis of tert-butyl 6-bromo-4-((3-(trifluoromethyl)phenyl)sulfonyl)-3,4-dihydroquino xaline-1(2H)-carboxylate

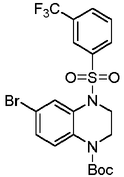

Tert-butyl 6-bromo-3,4-dihydroquinoxaline-1(2*H*)-carboxylate (210 mg, 95%, 0.64 mmo 1), 3-(trifluoromethyl)benzenesulfonyl chloride (171 mg, 0.70 mmol) was dissolved in dichlo romethane (10 mL) and 4-dimethylaminopyridine (78 mg, 0.64 mmol) was added thereto. The reaction mixture was stirred at room temperature overnight; LCMS showed the reactio n was completed. The reaction mixture was concentrated to dryness, and the residue was s eparated by a rapid silica gel column [eluent: EtOAc : PE = 0-60%)] to obtain a pale yel low oily tert-butyl 6-bromo-4-((3-(trifluoromethyl)phenyl)sulfonyl)-3,4-dihydroquinoxaline-1(2 *H*)-carboxylate (277 mg, yield 79.2%). ESI-MS: 421.0 [M+1]⁺.

### 3. Preparation of methyl (S)-3-(6-bromo-1-methyl-4-((3-(trifluoromethyl)phenyl)sulfonyl)-1,2,3,4 -tetrahydroquinoxalin-2-yl)propanoate

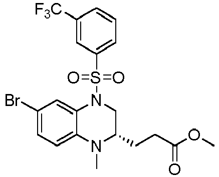

### Step 1: synthesis of dimethyl (4-bromo-2-nitrophenyl)-L-glutamate

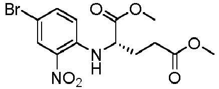

4-bromo-1-fluoro-2-nitrobenzene (10.0 g, 45.4 mmol), dimethyl *D*-glutamate hydrochlori de (11.5 g, 54.5 mmol), potassium carbonate (25.1 g, 182 mmol), *N,N*-dimethylformamide (50 mL) were added into a 250 mL single-necked flask, and the reaction mixture was stirred at 80 °C for 18 hrs. The reaction mixture was cooled, and then diluted with water (100 m L), and extracted with ethyl acetate (150 mL * 2). The organic phases were washed with brine (200 mL * 2), separated, dried over anhydrous sodium sulfate and filtered, and the r esidue was separated by a silica gel column [eluent: petroleum ether : ethyl acetate = 100:0-20:80] to obtain dimethyl (4-bromo-2-nitrophenyl)-*L*-glutamate (6.0 g, yield 32.8%). ESI-MS: 375.0 [M+1]⁺.

### Step 2: synthesis of methyl (S)-3-(6-bromo-3-oxo-1,2,3,4-tetrahydroquinoxalin-2-yl)propanoat e and (S)-7-bromo-3,3a-dihydropyrrolo[1,2-a]quinoxaline-1,4(2H,5H)-dione

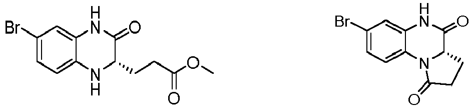

Dimethyl (4-bromo-2-nitrobenzene)-L-glutamate (6.0 g, 93%, 14.9 mmol), acetic acid (30 mL) and iron powder (4.17 g, 74.5 mmol) were added into a 250 mL single-necked f lask, then reacted at 60 °C for 2 hrs, cooled to 40-50 °C and filtered through celite, and the filter cake was rinsed with ethyl acetate (30 mL * 5). The filtrate was separated into layers and the aqueous layer was further extracted with ethyl acetate (50 mL). The combin ed organic phases were washed successively with water (100 mL), brine (100 mL) and sat urated sodium bicarbonate (100 mL * 3), and then dried over anhydrous sodium sulfate, fi ltered and concentrated. The residue was separated by a silica gel column [eluent: petroleu m ether : ethyl acetate = 100:0-0:100] to obtain methyl (*S*)-3-(6-bromo-3-oxo-1,2,3,4-tetrahy droquinoxalin-2-yl)propanoate (1.7 g) (ESI-MS: 313.0 [M+1]⁺) and (*S*)-7-bromo-3,3a-dihydro pyrrolo[1,2-*a*]quinoxaline-1,4(2*H*, 5*H*)-dione (500 mg) (ESI-MS: 281.0 [M+1]⁺).

### Step 3: synthesis of methyl (S)-3-(6-bromo-1-methyl-3-oxo-1,2,3,4-tetrahydroquinoxalin-2 -yl) propanoate

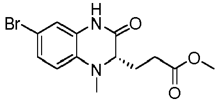

Methyl (*S*)-3-(6-bromo-3-oxo-1,2,3,4-tetrahydroquinoxalin-2-yl)propanoate (1.70 g, 5.16 mm ol) and *N,N*-dimethylformamide (10 mL) were added into a 250 mL single-necked flask. A queous formaldehyde (3.9 g, 40%, 51.6 mmol) and a few drops of acetic acid were added thereto, and the reaction mixture was stirred at room temperature for 1.5 hrs. Sodium cya noborohydride (1.62 g, 25.8 mmol) was added thereto and stirred for 18 hrs. After dilutio n with ethyl acetate (150 mL), the mixture solution was washed with brine (100 mL * 2), dried over anhydrous sodium sulfate, filtered and concentrated. The residue was separated by a rapid silica gel column [eluent: petroleum ether : ethyl acetate = 0:100-15:85] to obt ain methyl (*S*)-3-(6-bromo-1-methyl-3-oxo-1, 2,3,4-tetrahydroquinoxalin-2-yl)propanoate (660 mg, yield 34.4%). ESI-MS: 263.0 [M+1]⁺.

### Step 4: synthesis of methyl (S)-3-(6-bromo-1-methyl-1,2,3,4-tetrahydroquinoxalin-2-yl)propano ate

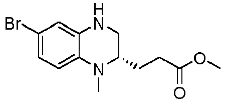

Methyl (*S*)-3-(6-bromo-1-methyl-3-oxo-1,2,3,4-tetrahydroquinoxalin-2-yl)propanoate (660 mg, 1.78 mmol) was dissolved in anhydrous tetrahydrofuran (10 mL), and borane dimethyl sulf ide complex (2M tetrahydrofuran solution, 2.2 mL, 4.44 mmol) was added thereto. The rea ction mixture was stirred at 40 °C for 3 hrs; LCMS and TLC plate showed the reaction was completed; the reaction was quenched with methanol (10 mL), and the reaction mixtur e was heated to 50 °C and stirred for 1 hr. The mixture was concentrated, and the residu e was separated by a rapid silica gel column [eluent: petroleum ether : ethyl acetate = 0:1 00-40:60] to obtain methyl (*S*)-3-(6-bromo-1-methyl-1,2,3,4-tetrahydroquinoxalin-2-yl)propano ate (360 mg, yield 61.4%). ESI-MS: 313.0 [M+1]⁺.

### Step 5: synthesis of methyl (S)-3-(6-bromo-1-methyl-4-((3-(trifluoromethyl)phenyl)sulfony 1)-1,2,3,4-tetrahydroquinoxalin-2-yl)propanoate

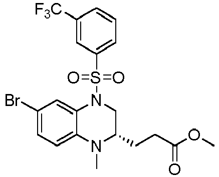

Methyl (*S*)-3-(6-bromo-1-methyl-1,2,3,4-tetrahydroquinoxalin-2-yl)propanoate (360 m g, 1.09 mmol) and 3-(trifluoromethyl)benzenesulfonyl chloride (538 mg, 2.19 mmol) were dissolved in pyridine (8 mL), and 4-dimethylaminopyridine (199 mg, 1.64 mmol) was adde d thereto. The reaction mixture was stirred overnight at 50 °C. LCMS showed the reaction was completed. The reaction mixture was concentrated to dryness, and the residue was se parated by a rapid silica gel column (0-80% EtOAc : PE) to obtain methyl (*S*)-3-(6-bromo -1-methyl-4-((3-(trifluoromethyl)phenyl)sulfonyl)-1,2,3,4-tetrahydroquinoxalin-2-yl)propanoate (560 mg, yield 93.6%). ESI-MS: 521.2 [M+1]⁺.

### 4. Preparation of (S)-7-bromo-5-((3-(trifluoromethyl)phenyl)sulfonyl)-1,2,3,3a,4,5-hexahyd ropyrrolo [1,2-a] quinoxaline

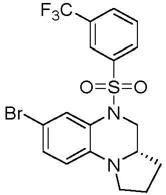

### Step 1: synthesis of (S)-7-bromo-1,2,3,3a,4,5-hexahydropyrroto[1,2-a]quinoxaline

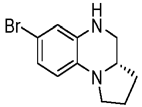

(*S*)-7-bromo-3,3a-dihydropyrrolo[1,2-*a*]quinoxaline-1,4(2*H*, 5*H*)-dione (170 mg, 0.57 mmo 1) was dissolved in anhydrous tetrahydrofuran (3 mL), and borane dimethylsulfide complex (2M tetrahydrofuran solution, 0.86 mL, 1.72 mmol) was added thereto. The reaction mixtur e was stirred at 50 °C for 2 hrs. LCMS showed the reaction was completed; the reaction was quenched with methanol (10 mL) and stirred at 50 °C for 1 hr. The mixture was con centrated, and the residue was separated by a rapid silica gel column [eluent: petroleum eth er : ethyl acetate = 0:100-50:50] to obtain (*S*)-7-bromo-1,2,3,3a,4,5-hexahydropyrrolo [1,2-*a*]quino xaline (100 mg, yield 65.9%). ESI-MS: 253.0 [M+1]⁺.

### Step 2: synthesis of (S)-7-bromo-5-((3-(trifluoromethyl)phenyl)sulfonyl)-1,2,3,3a,4,5-hexah ydropyrrolo [1,2-a] quinoxaline

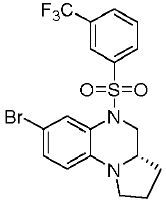

(*S*)-7-bromo-1,2,3,3a,4,5-hexahydropyrrolo[1,2-*a*]quinoxaline (100 mg, 0.375 mmol) and 3-(trifluoromethyl)benzenesulfonyl chloride (137 mg, 0.563 mmol) were dissolved in dichlor omethane (5 mL), and 4-dimethylaminopyridine (46 mg, 0.375 mmol) was added thereto. The reaction mixture was stirred at room temperature overnight. LCMS showed the reactio n was essentially completed. The reaction was concentrated to dryness, and the residue wa s separated by a rapid silica gel column (0-30% EtOAc : PE) to obtain (*S*)-7-bromo-5-((3-(trifluoromethyl)phenyl)sulfonyl)-1,2,3,3a,4,5-hexahydropyrrolo[1,2-*a*]quinoxaline (160 mg, yie 1d 93%). ESI-MS: 461.0 [M+1]⁺. **5. Preparation of *tert-butyl* (*S*)-8-bromo-6-((3-(trifluoromethyl)phenyl)sulfonyl)-1,2,4,4a,5,**

### 6-hexahydro-3H-pyrazino[1,2-a]quinoxaline-3-carboxylate

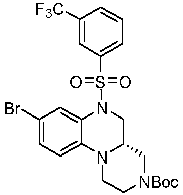

### Step 1: synthesis of (S)-1-(4-bromo-2-nitrophenyl)-4-(tert-butoxycarbonyl)piperazine-2-car boxylic acid

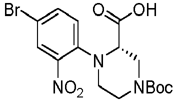

4-bromo-1-fluoro-2-nitrobenzene (2.2 g, 10 mmol) was dissolved in *N,N*-dimethylforma mide (20 mL), and cesium carbonate (9.75 g, 30 mmol) and 1-(*tert*-butyl)3-methyl (*S*)-pipe razine-1,3-dicarboxylate (2.44 mg, 10 mmol) were added into the solution. The reaction mi xture was stirred at 70 °C for 16 hrs. The reaction mixture was concentrated to remove t he solvent. The residue was separated by a rapid silica gel column to obtain (*S*)-1-(4-brom o-2-nitrophenyl)-4-(*tert*-butoxycarbonyl)piperazine-2-carboxylic acid (310 mg, 7.2%). ESI-M S: 374 [M-55]⁺.

### Step 2: synthesis of tert-butyl (S)-8-bromo-5-oxo-1,2,4,4a,5,6-hexahydro-3H-pyrazino[1,2-a] quinoxaline-3-carboxylate

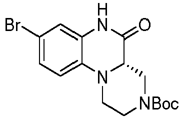

(*S*)-1-(4-bromo-2-nitrophenyl)-4-(*tert*-butoxycarbonyl)piperazine-2-carboxylic acid (890 m g, 2.0 mmol) was dissolved in acetic acid (10 mL), and iron powder (560 mg, 10 mmol) was added thereto. The mixture solution was stirred at 70 °C for 2 hrs, filtered, and conc entrated to remove the solvent. The residue was washed with saturated sodium bicarbonate, extracted with ethyl acetate, dried, concentrated and then separated by a rapid silica gel c olumn to obtain tert-butyl (*S*)-8-bromo-5-oxo-1,2,4,4a,5,6-hexahydro-3*H*-pyrazino[1,2-*a*]quinoxa line-3-carboxylate (325 mg, 43%). ESI-MS: 326 [M-55]⁺.

### Step 3: synthesis of tert-butyl (R)-8-bromo-1,2,4,4a,5,6-hexahydro-3H-pyrazino[1,2-a]quin oxaline-3-carboxylate

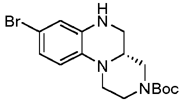

*Tert*-butyl (*S*)-8-bromo-5-oxo-1,2,4,4a,5,6-hexahydro-3*H*-pyrazino[1,2-*a*]quinoxaline-3-carb oxylate (325 mg, 0.85 mmol) was dissolved in tetrahydrofuran (15 mL), and a solution of borane dimethylsulfide in tetrahydrofuran (1.3 mL, 2M) was added thereto. The reaction m ixture was stirred at 50 °C for 16 hrs, then cooled to 0 °C, quenched with methanol and concentrated to remove the solvent. The residue was separated by a rapid silica gel colum n to obtain tert-butyl (*R*)-8-bromo-1,2,4,4a,5,6-hexahydro-3*H*-pyrazino[1,2-*a*]quinoxaline-3-carb oxylate (250 mg, 80%). ESI-MS: 312 [M-55]⁺.

### Step 4: synthesis of tert-butyl (S)-8-bromo-6-((3-(trifluoromethyl)phenyl)sulfonyl)-1,2,4,4 a,5,6-hexahydro-3H-pyrazino [1,2-a] quinoxaline-3-carboxylate

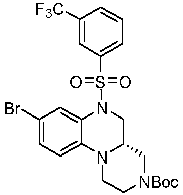

Tert-butyl (*R*)-8-bromo-1,2,4,4a,5,6-hexahydro-3*H*-pyrazino[1,2-*a*]quinoxaline-3-carboxylat e (250 mg, 0.68 mmol) was dissolved in pyridine (4 mL), and 3-(trifluoromethyl)benzenesu lfonyl chloride (332 mg, 1.36 mmol) was added thereto. The reaction mixture was stirred at 70 °C for 5 hrs. The reaction mixture was concentrated to remove the solvent. The resi due was separated by a rapid silica gel column to obtain tert-butyl (*S*)-8-bromo-6-((3-(triflu oromethyl)phenyl)sulfonyl)-1,2,4,4a,5,6-hexahydro-3*H*-pyrazino[1,2-*a*]quinoxaline-3-carboxylate (340 mg, 96%). ESI-MS: 520 [M-55]⁺.

### 6. Preparation of 2-(3-(difluoromethoxy)-5-fluorophenyl)-4,4,5,5-tetramethyl-1,3,2-dioxaborolan e

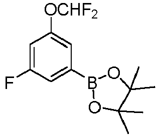

### Step 1: synthesis of 1-bromo-3-(difluoromethoxy)-5-fluorobenzene

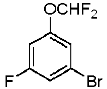

3-fluoro-5-bromophenol (8.69 g, 45.5 mmol) was dissolved in *N,N*-dimethylfonnamide (30 mL); potassium carbonate (16.00 g, 115.8 mmol) was added into the above solution, and t he mixture solution was stirred at room temperature for 30 mins. Water (8.2 mL) and sod ium difluorochloroacetate (11.98 g, 78.6 mmol) were added into the reaction mixture, and the reaction mixture was stirred at 100 °C under nitrogen protection for 3 days; the reacti on mixture was cooled to room temperature, and then diluted with ethyl acetate (30 mL) and washed with brine (100 mL * 3). The organic phases were dried over anhydrous mag nesium sulfate. The reaction mixture was filtered, concentrated, and separated by column c hromatography [eluent: EA : PE = 2%] to obtain 1-bromo-3-(difluoromethoxy)-5-fluorobenz ene (4.624 g, 42%), which was directly used in the next step.

### Step 2: synthesis of 2-(3-(difluoromethoxy)-5-fluorophenyl)-4,4,5,5-tetramethyl-1,3,2-dioxa borolane

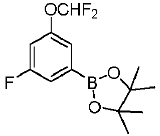

1-bromo-3-(difluoromethoxy)-5-fluorobenzene (4.62 g, 19.1 mmol), pinacol diboron (9.7 9 g, 38.6 mM), potassium acetate (7.56 g, 77.0 mmol) and 1,1'-bis(diphenylphosphino)ferro cene palladium chloride (1.51 g, 2.1 mmol) were dissolved in dioxane (45 mL), and the r eaction mixture was reacted under nitrogen protection at 80 °C for 17 hrs. The reaction m ixture was cooled to room temperature, directly concentrated and separated by column chro matography [eluent: EA : PE = 0%-10%] to obtain 2-(3-(difluoromethoxy)-5-fluorophenyl)-4,4,5,5-tetramethyl-1,3,2-dioxaborolane (3.63 g, 66%), which was directly used in the next step.

### 7. Preparation of (E)-2-(2-(2-chloro-6-fluorophenyl)prop-1-en-1-yl)-4,4,5,5-tetramethyl-1,3, 2-dioxaborolane

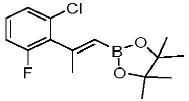

### Step 1: synthesis of 1-chloro-2-ethynyl-3-fluorobenzene

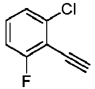

2-chloro-6-fluorobenzaldehyde (1.0 g, 6.3 mmol) was dissolved in methanol (40 mL), and dimethyl (1-diazo-2-oxopropyl) phosphonate (1.2 mL, 7.9 mmol) and potassium carbon ate (2.16 g, 15.75 mmol) were added thereto. The reaction mixture was stirred at room te mperature overnight, concentrated, added with methyl t-butyl ether (50 mL), extracted with water (50 mL * 3), washed with brine (50 mL). The organic phase was dried over anhydr ous sodium sulfate and concentrated to obtain 1-chloro-2-ethynyl-3-fluorobenzene (0.8 g, 8 2%), which was directly used in the next step.

### Step 2: synthesis of (E)-2-(2-(2-chloro-6-fluorophenyl)prop-1-en-1-yl)-4,4,5,5-tetramethyl-1, 3,2-dioxaborolane

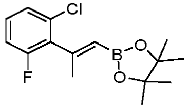

Pinacol diboron (1.45 g, 5.7 mmol), CuCl (0.05 g, 0.5 mmol) and 4,5-bis(di-*tert*-butyl phosphino)-9,9-dimethylxanthene (0.30 g, 0.5 mmol) were mixed in tetrahydrofuran (50 m L) and reacted under nitrogen protection for 5 mins. Sodium *tert*-butoxide (0.55 g, 5.7 m mol) was dissolved in tetrahydrofuran (5 mL) and added into the reaction mixture, and stir red for 5 mins. 1-chloro-2-ethynyl-3-fluorobenzene (0.80 g, 5.2 mmol) and iodomethane (2.9 6 g, 20.8 mmol) were added into the reaction mixture, the reaction mixture was reacted o vernight at room temperature, and then concentrated and separated by column chromatogra phy [eluent: Petroleum ether to petroleum ether/ethyl acetate (98:2)] to obtain (E)-2-(2-(2-c hloro-6-fluorophenyl)prop-1-en-1-yl)-4,4,5,5-tetramethyl-1,3,2-dioxaborolane (0.5 g, 33%), whi ch was directly used in the next step.

### 8. Preparation of intermediate B3: (E)-2-(2-chloro-6-(trifluoromethyl)styryl)-4,4,5,5-tetramethyl -1,3,2-dioxaborolane

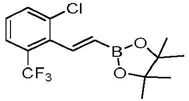

### Step 1: synthesis of 1-chloro-2-ethynyl-3-trifluoromethylbenzene

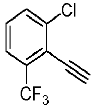

2-chloro-6-trifluoromethylbenzaldehyde (3.65 g, 17.4 mmol) and dimethyl(1-diazo-2-oxopropy 1) phosphonate (2.85 g, 14.9 mmol) were dissolved in methanol (30 mL). Potassium carbon ate (8.28 g, 59.9 mM) was added thereto, and the reaction mixture was stirred at room te mperature overnight. The reaction mixture was diluted with methyl tert-butyl ether (100 m L) and washed with saturated aqueous sodium chloride (30 mL * 3). The organic phases were combined, dried over anhydrous magnesium sulfate, filtered and concentrated to obtai n crude 1-chloro-2-ethynyl-3-trifluoromethylbenzene (3.471 g, 98%) which was used directl y in the next step.

### Step 2: synthesis of (E)-2-(2-chloro-6-(trifluoromethyl)styryl)-4,4,5,5-tetramethyl-1,3,2-dio xaborolane

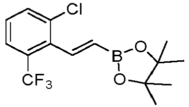

Crude product 1-chloro-2-ethynyl-3-trifluoromethylbenzene (3.47 g, 17.0 mmol) and 4, 4,5,5-tetramethyl-1,3,2-dioxaborolane (6.75 g, 52.7 mmol) were dissolved in toluene (26 m L), and carbonyl chlorotris (triphenylphosphine) ruthenium (0.96 g, 1.0 mmol) was added t hereto, the mixture solution was stirred under nitrogen protection at 50 °C for overnight. The reaction mixture was cooled to room temperature, diluted with ethyl acetate (80 mL) and washed with brine (30 mL * 3). The organic phases were combined, dried over anhy drous magnesium sulfate, filtered, concentrated and separated by column chromatography [e luent: EA : PE = 0%-10% ] to obtain (*E*)-2-(2-chloro-6-(trifluoromethyl)styryl)-4,4,5,5-tetra methyl-1,3,2-dioxaborolane (3.096 g, 55%), which was directly used in the next step.

### 9. Preparation of 4,4,5,5-tetramethyl-2-(2,2,6,6-tetramethyl-3,6-dihydro-2H-pyran-4-yl)-1, 3, 2-dioxaborolane

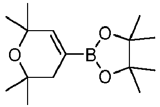

### Step 1: synthesis of 2,2,6,6-tetramethyl-3,6-dihydro-2H-pyran-4-yl-4,4,4,4,4,4,4,4,4-nonafluoro -4λ¹²-but-1,3-diyne-1-sulfonate

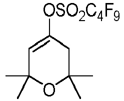

LDA (4.1 g, 2N, 38.4 mmol) was added into the solution under nitrogen protection. The solution was cooled to -78 °C; a solution of 2,2,6,6-tetramethyltetrahydro-4*H*-pyran-4-o ne (5.0 g, 32.0 mmol) in THF (80 mL) was slowly added dropwise thereto. The reaction mixture was stirred at-78 °C for 1 hr; 1,1,2,2,3,3,4,4,4-nonafluorobutane-1-sulfonyl fluoride (11.6 g, 38.4 mmol) was added thereto. The reaction mixture was stirred at -78∼0 °C for 16 hrs. After the reaction was completed, the reaction mixture was quenched with saturat ed NaHCO₃ (100 mL). The mixture solution was extracted with ethyl acetate (3 * 50 mL). The organic phases were combined, washed with brine (50 mL), dried over magnesium s ulfate and filtered. The filtrate was concentrated, and the residue was separated by a rapid silica gel column (PE : EA = 0-20%) to obtain 2,2,6,6-tetramethyl-3,6-dihydro-2*H*-pyran-4-yl-4,4,4,4,4,4,4,4,4-nonafluoro-**4λ¹²**-but-1,3-diyne-1-sulfonate (10 g, 71%), which was directly used in the next step.

### Step 2: synthesis of 4,4,5,5-tetramethyl-2-(2,2,6,6-tetramethyl-3,6-dihydro-2H-pyran-4-yl)-1, 3,2-dioxaborolane

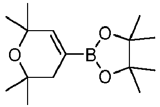

2,2,6,6-tetramethyl-3,6-dihydro-2*H*-pyran-4-yl-4,4,4,4,4,4,4,4,4-nonafluoro-**4λ¹²**-but-1,3-diyn e-1-sulfonate (10.0 g, 22.8 mmol), bis(pinacolato) biboronate (6.3 g, 25.1 mmol), Pd(dp pf)Cl₂ (930 mg, 1.14 mmol) and potassium acetate (6.7 g, 68.4 mmol) were mixed in D ME (100 mL). The reaction mixture was stirred at 80 °C under nitrogen protection for 16 hrs. After the reaction was completed, the reaction mixture was filtered through celite. The filtrate was concentrated, and the residue was separated by a rapid silica gel column [elu ent: PE : EA = 0-5%] to obtain the product 4,4,5,5-tetramethyl-2-(2,2,6,6-tetramethyl-3,6-di hydro-2*H*-pyran-4-yl)-1,3,2-dioxaborolane (2.3 g, 38%), which was directly used in the next step.

### 10. Preparation of tert-butyl (R)-8-bromo-6-((3-(trifluoromethyl)phenyl)sulfonyl)-1,2,4,4a, 5,6-hexahydro-3H-pyrazino[1,2-a]quinoxaline-3-carboxylate

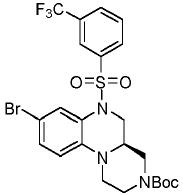

Reference was made to the preparation of tert-butyl (*S*)-8-bromo-6-((3-(trifluoromethyl) phenyl)sulfonyl)-1,2,4,4a,5,6-hexahydro-3*H*-pyrazino[1,2-*a*]quinoxaline-3-carboxylate.

### 11. Preparation of (S,E)-8-(2-chloro-6-(trifluoromethyl)styryl)-6-((3-(trifluoromethoxy)phenyl) sulfonyl)-2,3,4,4a,5,6-hexahydro-1H-pyrazino[1,2-a]quinoxaline

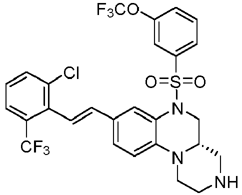

### Step 1: synthesis of tert-butyl (S)-8-bromo-6-((3-(trifluoromethoxy)phenyl)sulfonyl)-1,2,4, 4a,5,6-hexahydro-3H-pyrazino [1,2-a] quinoxaline-3-carboxylate

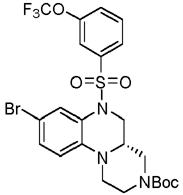

*Tert*-butyl (*R*)-8-bromo-1,2,4,4a,5,6-hexahydro-3*H*-pyrazino[1,2-*a*]quinoxaline-3-carboxylat e (1.2 g, 3.2 mmol) was dissolved in pyridine (10 mL), and 3-(trifluoromethoxy)benzenesul fonyl chloride (1.3 g, 4.8 mmol) was added thereto. The reaction mixture was stirred at 5 0 °C for 20 hrs. The solvent was removed by concentration to obtain crude *tert-butyl* (*S*)-8-bromo-6-((3-(trifluoromethoxy)phenyl)sulfonyl)-1,2,4,4a,5,6-hexahydro-3*H*-pyrazino[1,2-*a*]qui noxaline-3-carboxylate, which was directly used as a raw material in the next step.

### Step 2: synthesis of tert-butyl(S,E)-8-(2-chloro-6-(trifluoromethyl)styryl)-6-((3-(trifluoromethox y)phenyl)sulfonyl)-1,2,4,4a,5,6-hexahydro-3H-pyrazino[1,2-a]quinoxaline-3-carboxylate

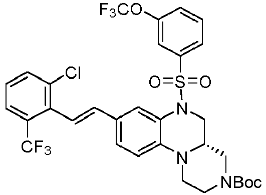

Crude *tert-*butyl (*S*)-8-bromo-6-((3-(trifluoromethoxy)phenyl)sulfonyl)-1,2,4,4a,5,6-hexahydro-3*H*-pyrazino[1,2-*a*]quinoxaline-3-carboxylate, tetrakis (triphenylphosphine)palladium (0.67 g,0. 58 mmol), sodium carbonate (1.1 g, 16.8 mmol) and (*E*)-2-(2-chloro-6-(trifluoromethyl)styry l)-4,4,5,5-tetramethyl-1,3,2-dioxaborolane (1.6 g, 5.0 mmol) were mixed; toluene (18 mL), e thanol (12 mL) and water (6 mL) were added thereto. The nitrogen was charged to replac e three times by evacuation. The mixture solution was heated to 90 °C and reacted for 20 hrs. After the reaction was completed, the reaction mixture was concentrated to remove the solvent, washed with brine (60 mL), and extracted with ethyl acetate (30 mL * 3). The org anic phases were combined, dried, filtered and concentrated, the residue was separated by a rapid silica gel column to obtain tert-butyl (*S,E*)-8-(2-chloro-6-(trifluoromethyl)styryl)-6-((3 -(trifluoromethoxy)phenyl)sulfonyl)-1,2,4,4a,5,6-hexahydro-3*H*-pyrazino[1,2-*a*]quinoxaline-3-carbo xylate (235 mg, 10%). ESI-MS: 618 [M-Boc+H]⁺.

### Step 3: synthesis of (S,E)-8-(2-chloro-6-(trifluoromethyl)styryl)-6-((3-(trifluoromethoxy)pheny l)sulfonyl)-2,3,4,4a,5,6-hexahydro-1H-pyrazino[1,2-a]quinoxaline

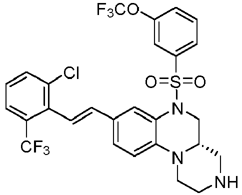

*Tert-*butyl (*S,E*)-8-(2-chloro-6-(trifluoromethyl)styryl)-6-((3-(trifluoromethoxy)phenyl)sulfo nyl)-1,2,4,4a,5,6-hexahydro-3*H*-pyrazino[1,2-*a*]quinoxaline-3-carboxylate (235 mg, 0.33 mmol) was dissolved in a solution of HCl in dioxane (6 mL, 4M). The reaction mixture was sti rred at room temperature for 5 hrs. The solvent was removed by concentration to obtain (*S,E*)-8-(2-chloro-6-(trifluoromethy)styry)-6-((3-(trifluoromethoxy)pheny)sulfonyl)-2,3,4,4a,5,6-hexahydro-1*H*-pyrazino[1,2-*a*]quinoxaline (219 mg, 100%), which was directly used in the next step.

### 12. Preparation of tert-butyl (S)-8-(3-(difluoromethoxy)-5-fluorophenyl)-6-((2-(2-hydroxyethoxy) -5-(trifluoromethyl)pyridin-3-yl)sulfonyl)-1,2,4,4a,5,6-hexahydro-3H-pyrazino[1,2-a]quinoxa line-3-carboxylate

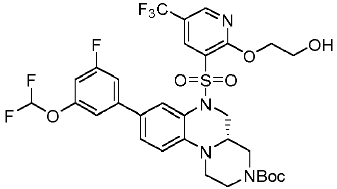

### Step 1: synthesis of 3-bromo-2-(2-((tert-butyldimethylsilyl)oxy)ethoxy)-5-(trifluoromethyl)pyridi ne

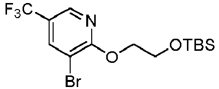

2-((*tert*-Butyldimethylsilyl)oxy)ethanol (9.7 g, 55.0 mmol) was dissolved in anhydrous THF (60 mL), and NaH (3.0 g, 60% in oil, 75.0 mmol) was added portionwise. The react ion mixture was stirred at 24 °C under nitrogen protection for 30 mins. A solution of 3-b romo-2-chloro-5-(trifluoromethyl)pyridine (13.0 g, 50.0 mmol) in anhydrous THF (20 mL) was added thereto, and the reaction mixture was stirred at 80 °C for 3 hrs; LCMS showe d the reaction was completed. The reaction mixture was quenched with saturated ammoniu m chloride (100 mL). The mixture was extracted with ethyl acetate (3 * 100 mL). The or ganic phases were combined, dried over magnesium sulfate and filtered. The filtrate was c oncentrated and separated by a rapid silica gel column [eluent: PE : EA = 0-50%] to obta in 3-bromo-2-(2-((*tert*-butyldimethylsilyl)oxy)ethoxy)-5-(trifluoromethyl)pyridine (9.8 g, 49%).
¹H NMR (500 MHz, CDCl₃) δ 8.25 (dd, *J* = 2.5, 1.3 Hz, 1H), 7.92 (d, *J* = 2.4 Hz, 1H), 4.42 (t, *J* = 5.8 Hz, 2H), 3.91 (t, *J* = 5.8 Hz, 2H), 0.80 (s, 9H), 0.00 (s, 6H). ESI-MS: 400 [M+H]⁺.

### Step 2: synthesis of 3-(benzylthio)-2-(2-((tert-butyldimethylsilyl)oxy)ethoxy)-5-(trifluoromethyl) pyridine

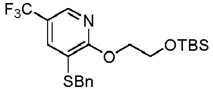

3-bromo-2-(2-((*tert*-butyldimethylsilyl)oxy)ethoxy)-5-(trifluoromethyl)pyridine (3.0 g, 7.5 mmo 1), benzylthiol (930 mg, 7.5 mmol), Pd₂(dba)₃ (300 mg), Xant-phos (300 mg) and diisoprop ylethylamine (1.9 g, 15.0 mmol) were mixed in 1,4-dioxane (50 mL). The reaction mixture was stirred at 100 °C under nitrogen protection for 16 hrs; LCMS showed the reaction w as completed. The reaction mixture was concentrated and the residue was separated by a r apid silica gel column to obtain 3-(benzylthio)-2-(2-((*tert*-butyldimethylsilyl)oxy)ethoxy)-5-(tr ifluoromethyl)pyridine (2.8 g, 84%) (PE : EA = 0-5%) ESI-MS: 444 [M+H]⁺.

### Step 3: synthesis of 2-(2-hydroxyethoxy)-5-(trifluoromethyl)pyridine-3-sulfonyl chloride

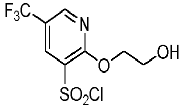

3 -(benzylthio)-2-(2-((*tert*-butyldimethylsilyl)oxy)ethoxy)-5 -(trifluoromethyl)pyridine (2.8 g, 6.32 mmol) and dichlorohydantoin (2.7 g, 13.9 mmol) were dissolved in acetic acid (50 mL) and H₂O (15 mL). The reaction mixture was stirred at room temperature for 16 hrs; LCMS showed the reaction was completed. The reaction mixture was concentrated. The re sidue was dissolved in DCM (100 mL), successively washed with saturated NaHCO₃ (50 mL), H₂O (50 mL) and brine (50 mL). The organic phases were dried over magnesium su lfate and filtered. The filtrate was concentrated, the residue was separated by a rapid silica gel column to obtain 2-(2-hydroxyethoxy)-5-(trifluoromethyl)pyridine-3-sulfonyl chloride (9 30 mg, 48%) (DCM : MeOH = 0-5%).
¹H NMR (500 MHz, CDCl₃) δ 8.68 (d, *J* = 2.3 Hz, 1H), 8.40 (d, *J* = 2.3 Hz, 1H), 4.70 (t, *J* = 5.8 Hz, 2H), 4.00 (t, *J* = 5.8 Hz, 2H), 2.14 (br, 1H). ESI-MS: 306 [M+H]⁺.

### Step 4: synthesis of tert-butyl (S)-8-bromo-6-((2-(2-hydroxyethoxy)-5-(trifluoromethyl)pyridin -3-yl)sulfonyl)-1,2,4,4a,5,6-hexahydro-3H-pyrazino[1,2-a]quinoxaline-3-carboxylate

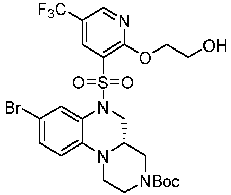

2-(2-hydroxyethoxy)-5-(trifluoromethyl)pyridine-3-sulfonyl chloride (170 mg, 0.56 mmo 1), *tert-butyl* (*R*)-8-bromo-1,2,4,4a,5,6-hexahydro-3*H*-pyrazino[1,2-*a*]quinoxaline-3-carboxylate (205 mg, 0.56 mmol) and diisopropylethylamine (145 mg, 1.11 mmol) were dissovled in a cetonitrile (10 mL). The reaction mixture was stirred at 50 °C for 16 hrs; LCMS showed the reaction was completed. The reaction mixture was concentrated, and the residue was se parated by a rapid silica gel column to obtain tert-butyl (*S*)-8-bromo-6-((2-(2-hydroxyethox y)-5-(trifluoromethyl)pyridin-3-yl)sulfonyl)-1,2,4,4a,5,6-hexahydro-3*H*-pyrazino[1,2-*a*]quinoxalin e-3-carboxylate (350 mg, 90%) (PE : EA = 0-25%) ESI-MS: 637 [M+H]⁺.

### Step 5: synthesis of tert-butyl (S)-8-(3-(difluoromethoxy)-5-fluorophenyl)-6-((2-(2-hydroxy ethoxy)-5-(trifluoromethyl)pyridin-3-yl)sulfonyl)-1,2,4,4a,5,6-hexahydro-3H-pyrazino[1,2-a] quinoxaline-3-carboxylate

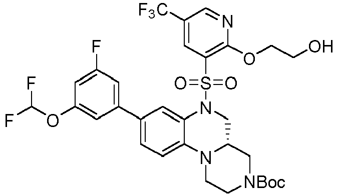

Tert-butyl (*S*)-8-bromo-6-((2-(2-hydroxyethoxy)-5-(trifluoromethyl)pyridin-3-yl)sulfonyl)-1, 2,4,4a,5,6-hexahydro-3*H*-pyrazino [1,2-*a*] quinoxaline-3-carboxylate (350 mg, 0.55 mmol), pin acol 3-difluoromethoxy-5-fluorobenzeneborate (237 mg, 0.82 mmol) and Pd(dppf)Cl₂ (50 m g) and potassium carbonate (228 mg, 1.65 mmol) were added to 1,4-dioxane (15 mL) and H₂O (5 mL). The reaction mixture was stirred at 100 °C under nitrogen protection for 16 hrs; LCMS showed the reaction was completed. The reaction mixture was concentrated. Th e residue was dissolved in DCM (50 mL) and H₂O (50 mL). The organic phases were dri ed over magnesium sulfate and filtered. The filtrate was concentrated, the residue was sepa rated by a rapid silica gel column [eluent: PE : EA = 0-25%] to obtain tert-butyl (*S*)-8-(3 -(difluoromethoxy)-5-fluorophenyl)-6-((2-(2-hydroxyethoxy)-5-(trifluoromethyl)pyridin-3-yl)sulf onyl)-1,2,4,4a,5,6-hexahydro-3*H*-pyrazino[1,2-*a*]quinoxaline-3-carboxylate (280 mg, 71%). ESI -MS: 719 [M+H]⁺.

### 13. Tert-butyl (S)-6-((2-(2-hydroxyethoxy)-5-(trifluoromethyl)pyridin-3-yl)sulfonyl)-8-(2,2,6, 6-tetramethyl-3,6-dihydro-2H-pyran-4-yl)-1,2,4,4a,5,6-hexahydro-3H-pyrazino[1,2-a]quino xaline-3-carboxylate

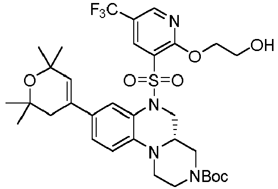

Reference was made to the preparation of tert-butyl (*S*)-8-(3-(difluoromethoxy)-5-fluoro phenyl)-6-((2-(2-hydroxyethoxy)-5-(trifluoromethyl)pyridin-3-yl)sulfonyl)-1,2,4,4a,5,6-hexahydro -3*H*-pyrazino[1,2-*a*]quinoxaline-3-carboxylate. ESI-MS: 697 [M+H]⁺.

### 14. Preparation of (S)-2-(8-bromo-6-((3-(trifluoromethyl)phenyl)sulfonyl)-1,2,4,4a,5,6-hexahyd ro-3H-pyrazino[1,2-a]quinoxalin-3-yl)acetic acid

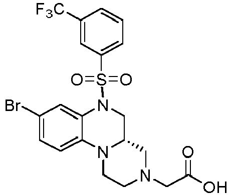

### Step 1: synthesis of (S)-8-bromo-6-((3-(trifluoromethyl)phenyl)sulfonyl)-2,3,4,4a,5,6-hexahydro-1H-pyrazino [1,2-a] quinoxaline

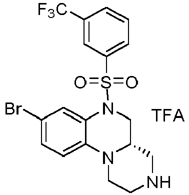

Tert-butyl (*S*)-8-bromo-6-((3-(trifluoromethyl)phenyl)sulfonyl)-1,2,4,4a,5,6-hexahydro-3*H*-p yrazino[1,2-*a*]quinoxaline-3-carboxylate (350 mg, 0.6 mmol) was dissolved in dichlorometha ne (3 mL). Trifluoroacetic acid (1 mL) was added thereto. The reaction mixture was stirre d at room temperature for 2 hrs. The solvent was removed by concentration to obtain cru de (*S*)-8-bromo-6-((3-(trifluoromethyl)phenyl)sulfonyl)-2,3,4,4a,5,6-hexahydro-1*H*-pyrazino[1,2-*a*]quinoxaline (360 mg, crude). ESI-MS: 476.0 [M+H]⁺.

### Step 2: synthesis of methyl (S)-2-(8-bromo-6-((3-(trifluoromethyl)phenyl)sulfonyl)-1,2,4,4a, 5,6-hexahydro-3H-pyrazino[1,2-a]quinoxalin-3-yl)acetate

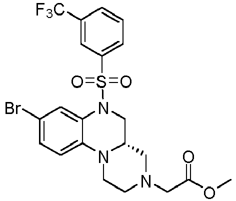

(*S*)-8-bromo-6-((3-(trifluoromethyl)phenyl)sulfonyl)-2,3,4,4a,5,6-hexahydro-1*H*-pyrazino[1,2 -*a*]quinoxaline (300 mg, 0.52 mmol) was dissolved in dimethylsulfoxide (3 mL). Potassium carbonate (215 mg, 1.56 mmol) and methyl bromoacetate (159 mg, 1.04 mmol) were add ed thereto. The reaction mixture was stirred at 50 °C for 2 hrs. After the reaction was co mpleted, the reaction mixture was added with water (10 mL) and extracted with ethyl acet ate (10 mL * 3). The organic phases were combined, washed three times with water (20 mL * 3), dried over anhydrous sodium sulfate and concentrated. The residue was separated by a rapid silica gel column [eluent: EtOAc : PE = 0-30%] to obtain methyl (*S*)-2-(8-bro mo-6-((3-(trifluoromethyl)phenyl)sulfonyl)-1,2,4,4a,5,6-hexahydro-3H*-*pyrazino[1,2-*a*]quinoxalin -3-yl)acetate (210 mg, 73%). ESI-MS: 548.2 [M+H]⁺.

### Step 3: synthesis of (S)-2-(8-bromo-6-((3-(trifluoromethyl)phenyl)sulfonyl)-1,2,4,4a,5,6-hex ahydro-3H-pyrazino[1,2-a]quinoxalin-3-yl)acetic acid

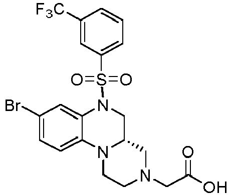

Methyl (*S*)-2-(8-bromo-6-((3-(trifluoromethyl)phenyl)sulfonyl)-1,2,4,4a,5,6-hexahydro-3*H*-p yrazino[1,2-*a*]quinoxalin-3-yl)acetate (210 mg, 0.38 mmol) was dissolved in tetrahydrofuran (6 mL) and water (2 mL). Lithium hydroxide monohydrate (92 mg, 3.8 mmol) was added thereto. The mixture solution was stirred at room temperature for 16 hrs, concentrated to r emove the solvent and acidified with dilute hydrochloric acid. The residue was separated b y a reversed-phase column chromatography [eluent: H₂O : MeCN = 0-50%] to obtain (S)-2 -(8-bromo-6-((3-(trifluoromethyl)phenyl)sulfony)-1,2,4,4a,5,6-hexahydro-3*H*-pyrazino[1,2-*a*]qui noxalin-3-yl)acetic acid (130 mg, 63 %). ESI-MS: 534.2 [M+H]⁺.

### 15. Preparation of 2-fluoro-3-(4,4,5,5-tetramethyl-1,3,2-dioxaborolan-2-yl)benzonitrile

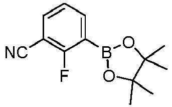

3-bromo-2-fluorobenzonitrile (500 mg, 2.5 mmol), bis(pinacolato)borate (950 mg, 3.5 mmol), [1,1'-bis(diphenylphosphino)ferrocene]palladium chloride (185 mg, 0.25 mmol) and p otassium acetate (491 mg, 5.0 mmol) were mixed in 1,4-dioxane (6 mL). The nitrogen wa s charged to replace three times by evacuation. The mixture solution was reacted at a tem perature of 100 °C for 4 hrs. After the reaction was completed, the reaction mixture was filtered. The filtrate was concentrated, and the residue was separated by a rapid silica gel column [eluent: EtOAc : PE = 0-20%] to obtain 2-fluoro-3-(4,4,5,5-tetramethyl-1,3,2-dioxab orolan-2-yl)benzonitrile (450 mg, 73%).
¹H NMR (400 MHz, CDCl₃) δ 7.98 (ddd, *J* = 7.5, 5.6, 1.9 Hz, 1H), 7.71 (ddd, *J =* 7.9, 6.3, 1.9 Hz, 1H), 7.26 (d, *J* = 7.6 Hz, 1H), 1.37 (s, 12H).

### 16. Preparation of 3-fluoro-5-(4,4,5,5-tetramethyl-1,3,2-dioxaborolan-2-yl)benzonitrile

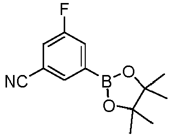

Reference was made to the preparation of 2-fluoro-3-(4,4,5,5-tetramethyl-1,3,2-dioxabor olan- 2-yl)benzonitrile.
¹H NMR (400 MHz, CDCl₃) δ 7.88 (t, *J* = 1.1 Hz, 1H), 7.71 (dd, *J* = 8.6, 2.7 Hz, 1H), 7.55 - 7.45 (m, 1H), 1.35 (s, 12H).

### 17. Preparation of 4-fluoro-3-(4,4,5,5-tetramethyl-1,3,2-dioxaborolan-2-yl)benzonitrile

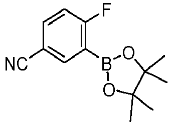

Reference was made to the preparation of 2-fluoro-3-(4,4,5,5-tetramethyl-1,3,2-dioxabor olan- 2-yl)benzonitrile.
¹H NMR (400 MHz, CDCl₃) δ 8.08 (dd, *J* = 5.3, 2.2 Hz, 1H), 7.35 (s, 1H), 7.14 (t, *J* = 8.6 Hz, 1H), 1.37 (s, 12H).

### 18. Preparation of 3-chloro-5-(4,4,5,5-tetramethyl-1,3,2-dioxaborolan-2-yl)benzonitrile

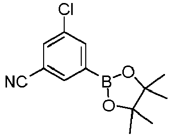

Reference was made to the preparation of 2-fluoro-3-(4,4,5,5-tetramethyl-1,3,2-dioxabor olan- 2-yl)benzonitrile.
¹H NMR (400 MHz, CDCl₃) δ 7.97 (dt, *J* = 7.3, 1.1 Hz, 1H), 7.79 - 7.66 (m, 2H), 1.35 (s, 6H), 1.26 (s, 6H).

### 19. Preparation of (R)-8-bromo-6-((3-(trifluoromethyl)phenyl)sulfonyl)-1,2,4,4a,5,6-hexahydro-[1,4] oxazino[4,3-a] quinoxaline

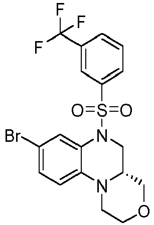

### Step 1: synthesis of (S)-4-(4-bromo-2-nitrophenyl)-morpholine-3-carboxylic acid

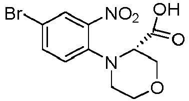

4-bromo-1-fluoro-2-nitrobenzene (2.2 g, 10.3 mmol) was dissolved in dimethyl sulfoxide (20 mL). Cesium carbonate (6.6 g, 20.4 mmol) and (*S*)-morpholine-3-carboxylic acid (0.9 g, 6.8 mmol) were added thereto. The reaction mixture was stirred at 110 °C for 2 hrs. The re action mixture was cooled to room temperature, and then diluted with 40 mL of ethyl ace tate, washed with brine (20 mL * 3). The organic layer was dried and concentrated to re move the solvent. The residue was separated by a rapid silica gel column to obtain (S)-4-(4-bromo-2-nitrophenyl)-morpholine-3-carboxylic acid (2.0 g, yield 88.8%). ESI-MS: 329, 33 1[M-H]⁻.

### Step 2: synthesis of (S)-8-bromo-1,2,4,4a-tetrahydro-[1,4]oxazino[4,3-a]quinoxalin-5(6H)-one

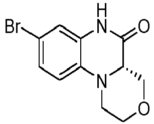

(*S*)-4-(4-bromo-2-nitrophenyl)-morpholine-3-carboxylic acid (2.0 g, 6.0 mmol) was disso lved in ethanol (20 mL). Iron powder (1.7 g, 30 mmol) was added thereto. The mixture s olution was stirred at 80 °C for 2 hrs, filtered, and concentrated to remove the solvent. T he residue was separated by a rapid silica gel column to obtain (*S*)-8-bromo-1,2,4,4a-tetrah ydro- [1, 4] oxazino [4,3-*a*] quinoxalin-5(6*H*)-one (230 mg, yield 13.5%). ESI-MS: 283, 285 [M +H]⁺.

### Step 3: synthesis of (R)-8-bromo-1,2,4,4a,5,6-hexahydro-[1,4]oxazino[4,3-a]quinoxaline

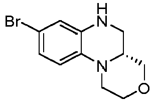

(*S*)-8-bromo-1,2,4,4a-tetrahydro-[1,4]oxazino[4,3-*a*]quinoxalin-5(6*H*)-one (230 mg, 0.81 mmol) was dissolved in tetrahydrofuran (15 mL). A solution of borane dimethylsulfide in t etrahydrofuran (1.3 mL, 2M in THF) was added thereto. The reaction mixture was stirred at 30 °C for 2 hrs, cooled to 0 °C, quenched with methanol, and concentrated to remove the solvent. The residue was separated by a rapid silica gel column to obtain (*R*)-8-bromo-1,2,4,4a,5,6-hexahydro-[1,4]oxazino[4,3-*a*]quinoxaline (190 mg, yield 87.6%). ESI-MS: 269, 271 [M+H]⁺.

### Step 4: synthesis of (R)-8-bromo-6-((3-(trifluoromethyl)phenyl)sulfonyl)-1,2,4,4a,5,6-hexahydr o- [1,4] oxazino [4,3-a] quinoxaline

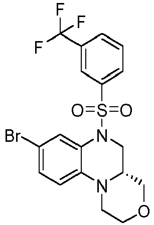

(*R*)-8-bromo-1,2,4,4a,5,6-hexahydro-[1,4]oxazino[4,3-*a*]quinoxaline (190 mg, 0.71 mmol) was dissolved in dichloromethane (5 mL). 0.5 mL of pyridine and 3-(trifluoromethyl) benz enesulfonyl chloride (259 mg, 1.06 mmol) were added into the solution. The reaction mixt ure was stirred at room temperature for 16 hrs. The reaction mixture was concentrated to remove the solvent. The residue was separated by a rapid silica gel column to obtain (R)-8-bromo-6-((3-(trifluoromethyl)phenyl)sulfonyl)-1,2,4,4a,5,6-hexahydro-[1,4]oxazino[4,3-*a*]quino xaline (270 mg, yield 79.5%). ESI-MS: 477, 479 [M+H]⁺.

### 20. Preparation of (S)-8-bromo-6-((3-(trifluoromethyl)phenyl)sulfonyl)-1,2,4,4a,5,6-hexahydro [1,4] oxazino[ 4,3-a] quinoxaline

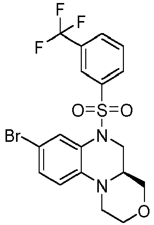

Reference was made to the preparation of (*R*)-8-bromo-6-((3-(trifluoromethyl)phenyl)sul fonyl)-1,2,4,4a,5,6-hexahydro-[1,4]oxazino[4,3-*a*]quinoxaline. ESI-MS: 477, 479 [M+H]⁺.

### 21. Preparation of 8-bromo-6-((3-(trifluoromethyl)phenyl)sulfonyl)-1,2,4,4a,5,6-hexahydro-[1,4]thiazino[4,3-a]quinoxaline

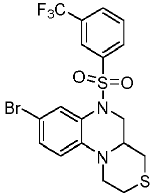

Reference was made to the preparation of (*R*)-8-bromo-6-((3-(trifluoromethyl)phenyl)sul fonyl)-1,2,4,4a,5,6-hexahydro-[1,4]oxazino[4,3-*a*]quinoxaline. ESI-MS: 493, 495 [M+H]⁺.

### 22. Preparation of (6aS)-3-bromo-5-((3-(trifluoromethyl)phenyl)sulfonyl)-6,6a,7,8,9,10-hex ahydro-5H-pyrido[1,2-a]quinoxalin-8-ol

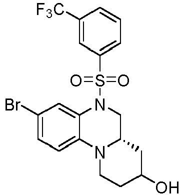

### Step 1: synthesis of (S)-1-(4-bromo-2-nitrophenyl)-4-oxopiperidine-2-carboxylic acid

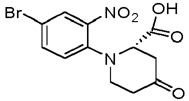

(*S*)-1-(*tert*-Butoxycarbonyl)-4-oxopiperidine-2-carboxylic acid (5.0 g, 20.5 mmol) was di ssolved in a 4M solution of hydrogen chloride in dioxane (20 mL) and stirred at 25 °C f or 1 hr. The solvent was removed by concentration. The residue was dissolved in dimethy 1 sulfoxide (20 mL). Cesium carbonate (16.7 g, 51.2 mmol) and 4-bromo-1-fluoro-2-nitrobe nzene were added thereto. The reaction was stopped after the mixture solution was stirred at 100 °C for 2 hrs, cooled to room temperature, diluted with 40 mL of ethyl acetate, wa shed with water (20 mL * 2), brine (20 mL * 2), dried over anhydrous sodium sulfate an d concentrated to remove the solvent. The residue was separated by a rapid silica gel colu mn to obtain (*S*)-1-(4-bromo-2-nitrophenyl)-4-oxopiperidine-2-carboxylic acid (4.0 g, yield 5 6.9%). ESI-MS: 341, 343 [M-H]⁻.

### Step 2: synthesis of (2S)-1-(4-bromo-2-nitrophenyl)-4-hydroxypiperidine-2-carboxylic aci d

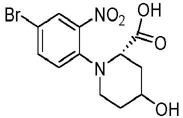

(*S*)-1-(4-bromo-2-nitrophenyl)-4-oxopiperidine-2-carboxylic acid (4.0 g, 11.6 mmol) was dissolved in methanol (20 mL). Sodium borohydride (1.3 g, 34.8 mmol) was added theret o. The mixture solution was stirred at 25 °C for 2 hrs, and concentrated to remove the so lvent. The residue was separated by a rapid silica gel column to obtain (2*S*)-1-(4-bromo-2-nitr ophenyl)-4-hydroxypiperidine-2-carboxylic acid (3.4 g, yield 85.0%). ESI-MS: 343, 345 [M-H]⁻.

### Step 3: synthesis of (6aS)-3-bromo-8-hydroxy-7,8,9,10-tetrahydro-5H-pyrido[1,2-a]quinoxalin -6(6aH)-one

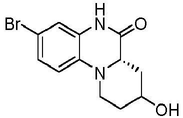

(2*S*)-1-(4-bromo-2-nitrophenyl)-4-hydroxypiperidine-2-carboxylic acid (3.4 g, 9.8 mmol) was dissolved in ethanol (20 mL). Iron powder (2.7 g, 49.0 mmol) and ammonium chlorid e (2.6 g, 49.0 mmol) were added thereto. The mixture solution was stirred at 80 °C for 2 hrs, filtered, and concentrated to remove the solvent. The residue was separated by a rapi d silica gel column to obtain (6a*S*)-3-bromo-8-hydroxy-7,8,9,10-tetrahydro-5*H*-pyiido[1,2-*a*]q uinoxalin-6(6a*H*)-one (2.0 g, yield 68.9%). ESI-MS: 297, 299 [M+H]⁺.

### Step 4: synthesis of (6aS)-3-bromo-6,6a,7,8,9,10-hexahydro-5H-pyrido[1,2-a]quinoxalin-8-ol

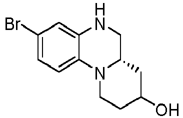

(6a*S*)-3-bromo-8-hydroxy-7,8,9,10-tetrahydro-5*H*-pyrido[1,2-*a*]quinoxalin-6(6a*H*)-one (2.0 g, 6.7 mmol) was dissolved in 4M borane-tetrahydrofuran (20 mL). The reaction mixture was stirred at 30 °C for 2 hrs. Methanol was added dropwise to quench the reaction. The reaction mixture was concentrated to remove the solvent. The residue was separated by a r apid silica gel column to obtain (6a*S*)-3-bromo-6,6a,7,8,9,10-hexahydro-5*H*-pyrido[1,2-*a*]quin oxaline-8-ol (1.7 g, yield 89.5%). ESI-MS: 283, 285 [M+H]⁺.

### Step 5: synthesis of (6aS)-3-bromo-5-((3-(trifluoromethyl)phenyl)sulfonyl)-6,6a,7,8,9,10-he xahydro-5H-pyrido [1,2-a] quinoxalin-8-ol

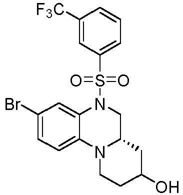

(6a*S*)-3-bromo-6,6a,7,8,9,10-hexahydro-5*H*-pyrido[1,2-*a*]quinoxalin-8-ol (1.7 mg, 6.0 mm ol) was dissolved in dichloromethane (20 mL). Pyridine (1.0 mL), dimethylaminopyridine (0.15 g, 1.2 mmol) and 3-(trifluoromethyl)benzenesulfonyl chloride (1.47 g, 6.0 mmol) wer e added thereto. The mixture solution was reacted at 25 °C for 4 hrs. The solvent was re moved by concentration. The residue was dissolved in ethyl acetate (30 mL), successively washed with water (20 mL), a saturated aqueous solution of sodium hydrogencarbonate (20 mL) and brine (20 mL), dried over anhydrous sodium sulfate and then concentrated to re move the solvent. The residue was separated by a rapid silica gel column to obtain (6*aS*)-3-bromo-5-((3-(trifluoromethyl)phenyl)sulfonyl)-6,6a,7,8,9,10-hexahydro-5*H*-pyrido[1,2-*a*]quinox alin-8-ol (1.6 g, yield 54.2%). ESI-MS: 491, 493 [M+1]⁺.

### 23. Preparation of (S)-3-bromo-5-((3-(trifluoromethyl)phenyl)sulfonyl)-5,6,6a,7,9,10-hexah ydro-8H-pyrido[1,2-a]quinoxalin-8-one

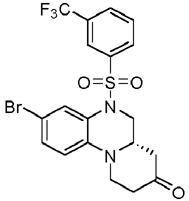

(6a*S*)-3-bromo-5-((3-(trifluoromethyl)phenyl)sulfonyl)-6,6a,7,8,9,10-hexahydro-5*H*-pyrido[1, 2-*a*]quinoxalin-8-ol (250 mg, 0.5 mmol) was dissolved in dichloromethane (10 mL). Dess-Martin oxidant (318 mg, 0.75 mmol) was added thereto. The mixture solution was reacted at 25 °C for 4 hrs. The reaction mixture was filtered to remove insoluble material. The so lution was washed with sodium bicarbonate solution (15 mL), and concentrated to remove solvent. The residue was separated by a rapid silica gel column to obtain (*S*)-3-bromo-5-((3-(trifluoromethyl)phenyl)sulfonyl)-5,6,6a,7,9,10-hexahydro-8*H*-pyrido[1,2-*a*]quinoxalin-8-one (240 mg, yield 98.3%). ESI-MS: 489, 491 [M+1]⁺.

### Preparation of Specific Examples

### Example 1: preparation of 6-(3-(difluoromethoxy)-5-fluorophenyl)-1-methyl-4-((3-(trifluor omethyl)phenyl)sulfonyl)-1,2,3,4-tetrahydroquinoxaline

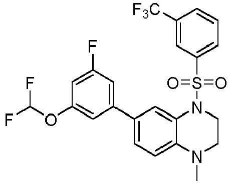

6-bromo-1-methyl-4-((3-(trifluoromethyl)phenyl)sulfonyl)-1,2,3,4-tetrahydroquinoxaline (10 0 mg, 95%, 0.23 mmol) and 2-(3-(difluoromethoxy)-5-fluorophenyl)-4,4,5,5-tetramethyl-1,3,2-dioxaborolane (90 mg, 95%, 0.30 mmol) were dissolved in a mixture solvent of toluene : ethanol : water = 3:2:2 (14 mL). Sodium carbonate (66 mg, 0.62 mmol) and tetrakis(triph enylphosphine) palladium (27 mg, 0.023 mmol) were added thereto. The reaction mixture was heated to 90 °C overnight; the reaction mixture was cooled, and then diluted with eth yl acetate and washed with brine (30 mL). The organic phases were dried over anhydrous sodium sulfate, filtered and concentrated. The residue was separated by a rapid silica gel c olumn [eluent: EA: PE = 0-50%] to obtain 6-(3-(difluoromethoxy)-5-fluorophenyl)-1-methyl - 4-((3-(trifluoromethyl)phenyl)sulfonyl)-1,2,3,4-tetrahydroquinoxaline (5.1 mg, 95%). ESI-MS: 517.2 [M+1]⁺.
¹H NMR (400 MHz, CDCl₃) δ 7.81-7.78 (m, 3H), 7.73(d, J = 8.0 Hz, 1H), 7.56(t, J = 8.0 Hz, 1H), 7.35 (dd, *J* = 8.8, 2.0 Hz, 1H), 7.16-7.13 (m, 1H), 7.11 (s, 1H), 6.79-6.7 6 (m, 1H),6.63 (d, *J* = 8.8 Hz, 1H), 6.57 (t, J =73.2Hz, 1H), 3.90 (t, *J* = 5.6 Hz, 1H), 2.92 (t, *J* = 5.6 Hz, 1H), 2.66 (s, 3H).

### Example 2: preparation of 7-(3-(difluoromethoxy)-5-fluorophenyl)-1-((3-(trifluoromethyl) phenyl)sulfonyl)-1,2,3,4-tetrahydroquinoxaline

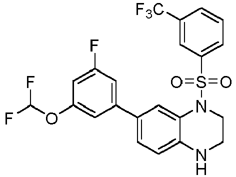

### Step 1: synthesis of tert-butyl 6-(3-(difluoromethoxy)-5-fluorophenyl)-4-((3-(trifluorometh yl)phenyl)sulfonyl)-3,4-dihydroquinoxaline-1(2H)-carboxylate

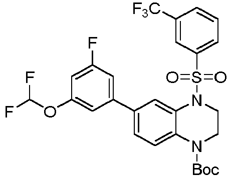

Tert-butyl 6-bromo-4-((3 -(trifluoromethyl)phenyl)sulfonyl)-3,4-dihydroquinoxaline-1 (2H)-c arboxylate (277 mg, 95%, 0.50 mmol) and 2-(3-(difluoromethoxy)-5-fluorophenyl)-4,4,5,5-tet ramethyl-1,3,2-dioxaborolane (229 mg, 95%, 0.75 mmol) were dissolved in a mixture solve nt of toluene : ethanol : water = 3:2:2 (14 mL). Sodium carbonate (106 mg, 1.0 mmol) a nd tetrakis(triphenylphosphine) palladium (60 mg, 0.05 mmol) were added thereto. The reac tion mixture was heated to 90 °C overnight; the reaction mixture was cooled, and then dil uted with ethyl acetate and washed with brine (30 mL). The organic phases were dried ov er anhydrous sodium sulfate, filtered and concentrated. The residue was separated by a rap id silica gel column [eluent: EA: PE = 0-60%] to obtain tert-butyl 6-(3-(difluoromethoxy)-5-fluorophenyl)-4-((3-(trifluoromethyl)phenyl)sulfonyl)-3,4-dihydroquinoxaline-1(2*H*)-carboxylat e (100 mg, 88%). ESI-MS: 503.2 [M-100]⁺.

### Step 2: synthesis of 7-(3-(difluoromethoxy)-5-fluorophenyl)-1-((3-(trifluoromethyl)phenyl) sulfonyl)-1,2,3,4-tetrahydroquinoxaline

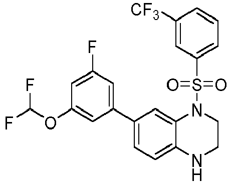

Tert-butyl 6-(3-(difluoromethoxy)-5-fluorophenyl)-4-((3-(trifluoromethyl)phenyl)sulfonyl)-3,4-dihydroquinoxaline-1(2*H*)-carboxylate (100 mg, 88%, 0.146 mmol) was dissolved in dic hloromethane (5 mL). A solution of hydrogen chloride (4N in 1,4-dioxane) (0.12 mL, 0.43 8 mmol) was added thereto. The reaction was allowed to proceed overnight at room tempe rature. The reaction mixture was concentrated; ethyl acetate (30 mL) was added thereto. T he solution was washed with saturated sodium bicarbonate (10 mL * 2). The organic phas es were dried over anhydrous sodium sulfate, filtered and concentrated. The residue was se parated by a reversed-phase column [C18, 45 g, 0-100% acetonitrile : 0.01 mM trifluoroac etic acid/water] to obtain 7-(3-(difluoromethoxy)-5-fluorophenyl)-1-((3-(trifluoromethyl)pheny 1)sulfonyl)-1,2,3,4-tetrahydroquinoxaline (8.2 mg, 95%). ESI-MS: 503.2 [M+1]⁺.
¹H NMR (400 MHz, Methanol-*d₄*) δ 7.92 (d, *J* = 7.6 Hz, 1H), 7.84(d, J = 8.0 Hz, 1 H), 7.82 (d, *J* = 2.4 Hz, 1H), 7.76 (s, 1H), 7.71 (t, *J* = 8.0 Hz, 1H), 7.30 (dd, *J* = 8.4, 2.0 Hz, 1H), 7.18-7.11 (m, 2H), 6.93 (t, *J* =74Hz, 1H), 6.84-6.81 (m, 1H), 6.59 (d, *J* =8. 4Hz, 1H), 3.83 (t, *J* = 5.2 Hz, 2H), 2.93 (t, *J* = 5.2 Hz, 2H).

### Example 3: preparation of (S,E)-3-(6-(2-(2-chloro-6-fluorophenyl)prop-1-en-1-yl)-1-methy 1-4-((3-(trifluoromethyl)phenyl)sulfonyl)-1,2,3,4-tetrahydroquinoxalin-2-yl)propanoic acid

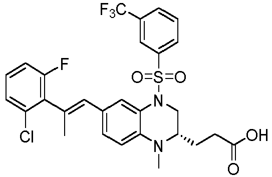

### Step 1: synthesis of methyl (S,E)-3-(6-(2-(2-chloro-6-fluorophenyl)prop-1-en-1-yl)-1-meth yl-4-((3-(trifluoromethyl)phenyl)sulfonyl)-1,2,3,4-tetrahydroquinoxalin-2-yl)propanoate

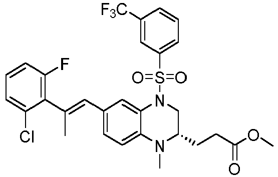

Methyl (*S*)-3-(6-bromo-1-methyl-4-((3-(trifluoromethyl)phenyl)sulfonyl)-1,2,3,4-tetrah ydroquinoxalin-2-yl)propanoate (60 mg, 95%, 0.11 mmol) and (*E*)-2-(2-(2-chloro-6-fluorophe nyl)prop-1-en-1-yl)-4,4,5,5-tetramethyl-1,3,2-dioxaborolane (52 mg, 95%, 0.16 mmol) were d issolved in the mixture solvent of toluene : ethanol : water = 3:2:2 (7 mL). Sodium carbo nate (18 mg, 0.17 mmol) and tetrakis(triphenylphosphine) palladium (10 mg, 0.01 mmol) were then added thereto. The reaction mixture was heated to 90 °C overnight. The reactio n mixture was cooled. A few drops of dilute hydrochloric acid (IN) were added to adjust the pH of the reaction mixture to about 7. The organic phases were separated and concent rated. The residue was separated by a rapid silica gel column [eluent: EA : PE = 0-80%] to obtain methyl (*S,E*)-3-(6-(2-(2-chloro-6-fluorophenyl)prop-1-en-1-yl)-1-methyl-4-((3-(trifluor omethyl)phenyl)sulfonyl)-1,2,3,4-tetrahydroquinoxalin-2-yl)propanoate (15 mg, yield 21.2%). ESI-MS: 611.3 [M+1]⁺.

### Step 2: synthesis of (S,E)-3-(6-(2-(2-chloro-6-fluorophenyl)prop-1-en-1-yl)-1-methyl-4-((3-(trifluoromethyl)phenyl)sulfonyl)-1,2,3,4-tetrahydroquinoxalin-2-yl)propanoic acid

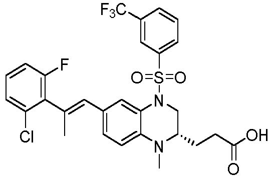

Methyl (*S,E*)-3-(6-(2-(2-chloro-6-fluorophenyl)prop-1-en-1-yl)-1-methyl-4-((3-(trifluoromethyl) phenyl)sulfonyl)-1,2,3,4-tetrahydroquinoxalin-2-yl)propanoate (15 mg, 0.023 mmol) was disso lved in the mixture solvent of tetrahydrofuran-water (3:1, 2 mL). Lithium hydroxide mono hydrate (10 mg, 0.23 mmol) was added thereto. The reaction mixture was stirred at room temperature for 3 hrs. The reaction mixture was cooled to 0 °C. A few drops of dilute hy drochloric acid (IN) were added to adjust the pH of the reaction mixture to about 6. The organic phases were separated and concentrated. The residue was separated by a preparativ e TLC[eluent: MeOH : DCM = 10%] to obtain (*S,E*)-3-(6-(2-(2-chloro-6-fluorophenyl)prop-1-en-1-yl)-1-methyl-4-((3-(trifluoromethyl)phenyl)sulfonyl)-1 ,2,3,4-tetrahydroquinoxalin-2-yl)pro panoic acid (5.1 mg, yield 35.3%). ESI-MS: 597.3 [M+1]⁺.
¹H NMR (400 MHz, DMSO-*d₆*) δ 8.15-8.09 (m, 2H), 7.91-7.87(m, 2H), 7.37-7.32(m, 2H), 7.27-7.23(m, 1H),7.18(d, J = 1.6 Hz, 1H), 7.06(dd, *J* = 8.8, 1.6 Hz, 1H), 6.66 (d, *J* = 8.8 Hz, 1H), 6.23 (s, 1H), 4.01-3.96 (m, 1H), 3.81-3.77 (m, 1H), 3.35-3.24 (m, 2H),2.7 2 (s, 3H), 2.27-2.21 (m, 2H), 1.97 (s, 3H), 1.84-1.81 (m, 1H), 1.58-1.53 (m, 1H).

### Examples 4 to 6 were prepared according to the synthesis method of Example 1 or 3.

| **Example No.** | **Structural formula** | **Chemical name** | **ESI-MS:** [M+1]^{÷} |
|---|---|---|---|
| 4 | 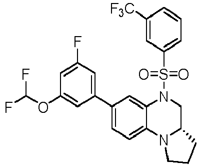 | (*S*)-7-(3 -(difluoromethox y)-5 -fluorophenyl)-5 -((3 - (trifluoromethyl)phenyl )s ulfonyl)-1,2,3,3a,4,5-hexa hydropyrrolo[ 1,2-*a*] quino xaline | 543.2 |
| **5** | 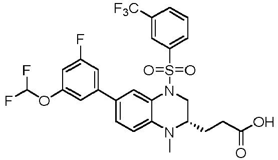 | (*S*)-3 -(6-(3 -(difluorometh oxy)-5-fluorophenyl)-1-m ethyl-4-((3 -(trifluorometh yl)phenyl)sulfonyl)-1,2,3, 4-tetrahydroquinoxalin - 2-yl)propanoic acid | 589.3 |
| **6** | 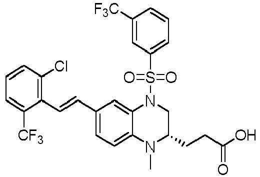 | (*S,E*)-3-(6-(2-chloro-6-(tri fluoromethyl)styryl)-1-me thyl-4-((3 -(trifluoromethy 1)phenyl)sulfonyl)-1,2,3,4 -tetrahydroquinoxalin -2-y 1)propanoic acid | 633.3 |

### ¹H NMR data of the compound prepared in Examples 4 to 6 were as follows:

**Example 4:** ¹H NMR (400 MHz, DMSO-*d₆*) δ7.91-7.86(m, 3H), 7.80(s, 1H), 7.69(t, *J*= 8.0 Hz, 1H), 7.42(dd, J= 8.0,2.0 Hz, 1H), 7.18-7.11(m, 2H), 7.10-6.74(m, 2H), 6.60(d, J= 8.8 Hz, 1H), 4.62-4.58 (m, 1H), 3.35-3.31 (m, 1H), 2.95-2.87(m, 1H), 2.72-2.66 (m, 1H), 2.07-1.99 (m, 2H), 1.81-1.68 (m, 1H), 1.38-1.28 (m, 1H).
**Example 5:** ¹H NMR (400 MHz, DMSO-*d₆*) δ 8.1 1(t, *J*= 8.0 Hz, 2H), 7.87(t, *J*= 8.0 Hz, 2H), 7.54-7.18(m, 3H), 7.16-7.14(m, 1H), 7.06(s, 1H), 7.02-7.00(m, 1H),6.71(d, J= 8.8 Hz, 1H), 3.95-3.89(m, 1H), 3.84-3.79(m, 1H), 3.25-3.21(m, 2H),2.69 (s, 3H), 2.25-2.21 (m, 2H), 1.84-1.81 (m, 1H), 1.58-1.52 (m,1H).
**Example 6:** ¹H (400 MHz, DMSO-*d₆*) δ 8.17(d, J= 8.0 Hz, 1H), 8.09(d, J= 8.0 Hz, 1H), 7.93(s, 1H), 7.90-7.80(m, 2H), 7.78-7.75(m, 1H), 7.51-7.48(m, 1H),7.29(d, J= 2.0 Hz, 1H), 7.23(d, *J =* 8.4 Hz, 1H), 6.70 (s, 2H), 6.63(d, *J =* 8.8 Hz, 1H), 4.03-3.99 (m, 1H), 3.32-3.30 (m, 1H), 2.74 (s, 3H), 2.21-2.15 (m, 2H), 1.85-1.82 (m, 1H), 1.56-1.52 (m, 1H).

### Example 7: preparation of (S)-1-(8-(3-(difluoromethoxy)-5-fluorophenyl)-6-((3-(trifluorom ethyl)phenyl)sulfonyl)-1,2,4,4a,5,6-hexahydro-3H-pyrazino[1,2-a]quinoxalin-3-yl)ethan-1-on e

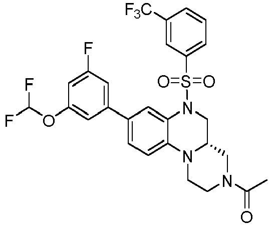

### Step 1: synthesis of tert-butyl (S)-8-(3-(difluoromethoxy)-5-fluorophenyl)-6-((3-(trifluoromethyl) phenyl)sulfonyl)-1,2,4,4a,5,6-hexahydro-3H-pyrazino[1,2-a]quinoxaline-3-carboxylate

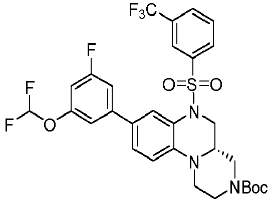

*Tert*-butyl (*S*)-8-bromo-6-((3-(trifluoromethyl)phenyl)sulfonyl)-1,2,4,4a,5,6-hexahydro-3*H*-p yrazino[1,2-*a*]quinoxaline-3-carboxylate (340 mg, 0.59 mmol), [1,1'-bis(diphenylphosphino)fer rocene]palladium chloride (50 mg, 0.059 mmol), potassium carbonate (244 mg, 1.77 mmol) and 2-(3-(difluoromethoxy)-5-fluorophenyl)-4,4,5,5-tetramethyl-1,3-dioxalane (221 mg, 0.77 mmol) were placed in a microwave tube; 1,4-dioxane (4 mL) and water (1 mL) were add ed thereto. The nitrogen was charged to replace three times by evacuation in a microwave reactor. The mixture solution was heated to 100 °C and reacted for half an hour. After th e reaction was completed, the reaction mixture was concentrated to remove the solvent. Th e residue was separated by a rapid silica gel column to obtain tert-butyl (*S*)-8-(3-(difluoro methoxy)-5-fluorophenyl)-6-((3-(trifluoromethyl)phenyl)sulfonyl)-1,2,4,4a,5,6-hexahydro-3*H*-pyr azino [1, 2-*a*] quinoxaline-3-carboxylate (300 mg, 77%).

### Step 2: synthesis of (S)-8-(3-(difluoromethoxy)-5-fluorophenyl)-6-((3-(trifluoromethyl)phe nyl)sulfonyl)-2,3,4,4a,5,6-hexahydro-1H-pyrazino [1,2-a] q uinoxaline

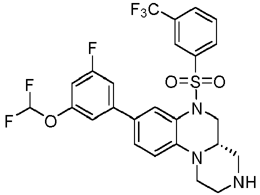

*Tert*-butyl (*S*)-8-(3-(difluoromethoxy)-5-fluoropheny)-6-((3-(trifluoromethyl)pheny)sulfony 1)-1,2,4,4a,5,6-hexahydro-3*H*-pyrazino [1, 2-*a*] quinoxaline-3-carboxylate (300 mg, 0.46 mmol) was dissolved in dichloromethane (5 mL). Trifluoroacetic acid (1 mL) was added thereto. The reaction mixture was stirred at room temperature for 1 hr. The reaction mixture was concentrated to remove the solvent. The residue was washed with a saturated solution of s odium hydrogencarbonate, extracted with ethyl acetate, dried, and concentrated to obtain (*S*)-8-(3-(difluoromethoxy)-5-fluorophenyl)-6-((3-(trifluoromethyl)phenyl)sulfonyl)-2,3,4,4a,5,6-h exahydro-1*H*-pyrazino[1,2-*a*]quinoxaline (240 mg, 93%).

### Step 3: synthesis of (S)-1-(8-(3-(difluoromethoxy)-5-fluorophenyl)-6-((3-(trifluoromethyl)p henyl)sutfonyl)-1,2,4,4a,5,6-hexahydro-3H-pyrazino[1,2-a]quinoxatin-3-yl)ethan-1-one

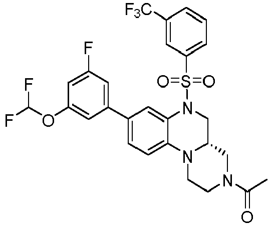

(*S*)-8-(3-(difluoromethoxy)-5-fluorophenyl)-6-((3-(trifluoromethyl)phenyl)sulfonyl)-2,3,4,4a, 5,6-hexahydro-1*H*-pyrazino[1,2-*a*]quinoxaline (50 mg, 0.09 mmol) was dissolved in pyridine (1 mL), and the mixture solution was cooled to 0 °C. Acetyl chloride (0.2 mL) was adde d thereto. The reaction mixture was stirred at 0 °C for half an hour, quenched with metha nol and concentrated to remove the solvent. The residue was separated by a rapid silica g el column to obtain (*S*)-1-(8-(3-(difluoromethoxy)-5-fluorophenyl)-6-((3-(trifluoromethyl)pheny 1)sulfonyl)-1,2,4,4a,5,6-hexahydro-3*H*-pyrazino[1,2-*a*]quinoxalin-3-yl)ethan-1-one (24 mg, 4 4%). ESI-MS: 600 [M+1]⁺.
¹H NMR (500 MHz, CDCl₃) δ 7.92-7.56 (m, 5H), 7.38 (dd, J = 8.8, 2.2 Hz, 1H), 7. 16-7.09(m, 2H), 6.84-6.74 (m, 2H), 6.57 (t, J = 73.3 Hz, 1H), 4.48 (s, 1H), 4.28 (dd, J = 14.3, 4.4 Hz, 1H), 3.78-3.57 (m, 2H), 3.34 (m, 1H), 3.15 (m, 0.5H), 2.72-2.82 (m, 1H), 2.59 (m, 1H), 2.08-2.38 (m,1.5H), 2.08 (s, 3H).

### Example 8: preparation of (S)-8-(3-(difluoromethoxy)-5-fluorophenyl)-3-(methylsulfonyl)-6-((3-(trifluoromethyl)phenyl)sulfonyl)-2,3,4,4a,5,6-hexahydro-1H-pyrazino[1,2-a]quinoxaline

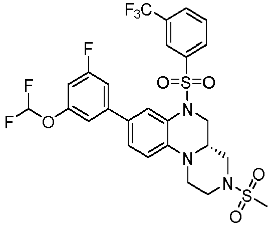

(*S*)-8-(3-(difluoromethoxy)-5-fluorophenyl)-6-((3-(trifluoromethyl)phenyl)sulfonyl)-2,3,4,4a, 5,6-hexahydro-1*H*-pyrazino[1,2-*a*]quinoxaline (50 mg, 0.09 mmol) was dissolved in pyridine (1 mL), and the mixture solution was cooled to 0 °C. Methanesulfonyl chloride (0.2 mL) was added thereto. The reaction mixture was stirred at 0 °C for half an hour, quenched w ith methanol and concentrated to remove the solvent. The residue was separated by a rapi d silica gel column to obtain (*S*)-8-(3-(difluoromethoxy)-5-fluorophenyl)-3-(methylsulfonyl)-6 -((3-(trifluoromethyl)phenyl)sulfonyl)-2,3,4,4a,5,6-hexahydro-1*H*-pyrazino[1,2-*a*]quinoxaline (20 mg, 35%). ESI-MS: 636 [M+1]⁺.
¹H NMR (500 MHz, CDCl₃) δ 7.89-7.77 (m, 3H), 7.70-7.61 (m, 2H), 7.38 (dd, *J =* 8.8, 2.2 Hz, 1H), 7.17-7.08 (m, 2H), 6.81 (dd, *J* = 11.0, 8.9 Hz, 2H), 6.57 (t, *J* = 73.3 Hz, 1H), 4.26 (dd, *J* = 14.4, 4.6 Hz, 1H), 3.76-3.59 (m, 3H), 3.39 (dd, *J* = 14.4, 9.8 Hz, 1H), 2.80-2.65 (m, 5H), 2.49 (td, *J* = 12.4, 3.3 Hz, 1H), 2.37 (t, *J* = 10.8 Hz, 1H).

### Example 9: preparation of (S)-2-(8-(3-(difluoromethoxy)-5-fluorophenyl)-6-((3-(trifluorom ethyl)phenyl)sulfonyl)-1,2,4,4a,5,6-hexahydro-3H-pyrazino[1,2-a]quinoxalin-3-yl)acetic acid

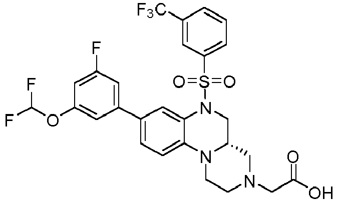

### Step 1: synthesis of methyl (S)-2-(8-(3-(difluoromethoxy)-5-fluorophenyl)-6-((3-(trifluoromethy 1)phenyl)sulfonyl)-1,2,4,4a,5,6-hexahydro-3H-pyrazino[1,2-a]quinoxalin-3-yl)acetate

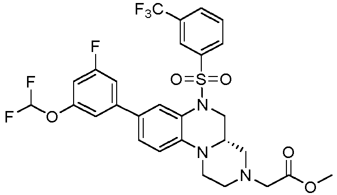

(*S*)-8-(3-(difluoromethoxy)-5-fluorophenyl)-6-((3-(trifluoromethyl)phenyl)sulfonyl)-2,3,4,4a, 5,6-hexahydro-1*H*-pyrazino[1,2-*a*]quinoxaline (50 mg, 0.09 mmol) was dissolved in *N,N-*dim ethylformamide (2 mL). Cesium carbonate (88 mg, 0.27 mmol) and methyl bromoacetate (14 mg, 0.45 mmol) were added thereto. The reaction mixture was stirred at 95 °C for 5 hrs. The reaction mixture was concentrated to remove the solvent. The residue was separat ed by a rapid silica gel column to obtain crude methyl (*S*)-2-(8-(3-(difluoromethoxy)-5-fluo rophenyl)-6-((3-(trifluoromethyl)phenyl)sulfonyl)-1,2,4,4a,5,6-hexahydro-3*H*-pyrazino[1,2-*a*]quin oxalin-3-yl)acetate.

### Step 2: synthesis of (S)-2-(8-(3-(difluoromethoxy)-5-fluorophenyl)-6-((3-(trifluoromethyl)p henyl)sulfonyl)-1,2,4,4a,5,6-hexahydro-3H-pyrazino[1,2-a]quinoxalin-3-yl)acetic acid

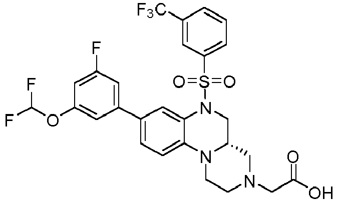

Crude methyl (*S*)-2-(8-(3-(difluoromethoxy)-5-fluorophenyl)-6-((3-(trifluoromethyl)phenyl) sulfonyl)-1,2,4,4a,5,6-hexahydro-3*H*-pyrazino[1,2-*a*]quinoxalin-3-yl) acetate was dissolved in methanol (2 mL). Lithium hydroxide monohydrate (38 mg) and water (1 mL) were added thereto. The mixture solution was stirred at room temperature for 4 hrs. The reaction mixt ure was concentrated to remove the solvent, and acidified with dilute hydrochloric acid. T he residue was separated by a rapid silica gel column to obtain (*S*)-2-(8-(3-(difluoromethox y)-5-fluorophenyl)-6-((3-(trifluoromethyl)phenyl)sulfonyl)-1,2,4,4a,5,6-hexahydro-3*H*-pyrazino[1, 2-*a*]quinoxalin-3-yl)acetic acid (5 mg, 9%). ESI-MS: 616 [M+1]⁺.
¹H NMR (500 MHz, CDCl₃) δ 7.88 (s, 1H), 7.80 (d, *J* = 9.1 Hz, 2H), 7.64 (s, 2H), 7.04 (d, *J* = 13.5 Hz, 2H), 6.79 (d, *J* = 10.1 Hz, 2H), 6.54 (m, 1H), 4.24 (s, 1H), 3.41 (m, 7H), 2.78 (m, 1H), 2.57 (s, 1H), 2.29 (s, 1H).

### Example 10: preparation of (S,E)-8-(2-(2-chloro-6-fluorophenyl)prop-1-en-1-yl)-6-((3-(trifl uoromethyl)phenyl)sulfonyl)-2,3,4,4a,5,6-hexahydro-1H-pyrazino[1,2-a]quinoxaline

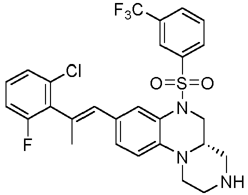

### Step 1: synthesis of tert-butyl (S,E)-8-(2-(2-chloro-6-fluorophenyl)prop-1-en-1-yl)-6-((3-(tr ifluoromethyl)phenyl)sulfonyl)-1,2,4,4a,5,6-hexahydro-3H-pyrazino[1,2-a]quinoxaline-3-carb oxylate

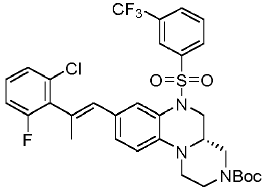

Tert-butyl (*S*)-8-bromo-6-((3-(trifluoromethyl)phenyl)sulfonyl)-1,2,4,4a,5,6-hexahydro-3*H*-p yrazino[1,2-*a*]quinoxaline-3-carboxylate (340 mg, 0.59 mmol), [1,1'-bis(diphenylphosphino)fer rocene]palladium chloride (98 mg,0.12 mmol), potassium carbonate (244 mg, 1.77 mmol) a nd (*E*)-2-(2-(2-chloro-6-fluorophenyl)prop-1-en-1-yl)-4,4,5,5-tetramethyl-1,3,2-dioxaborolane (2 09 mg, 0.71 mmol) were placed in a reaction flask. Toluene (4 mL), ethanol (2 mL) and water (2 mL) were added thereto. The nitrogen was charged to replace three times by eva cuation. The mixture solution was heated to 90 °C for 2 hrs. After the reaction was comp leted, the reaction mixture was concentrated to remove the solvent. The residue was separa ted by a rapid silica gel column to obtain tert-butyl (*S,E*)-8-(2-(2-chloro-6-fluorophenyl)prop -1-en-1-yl)-6-((3-(trifluoromethyl)phenyl)sulfonyl)-1,2,4,4a,5,6-hexahydro-3*H*-pyrazino[1,2-*a*]qui noxaline-3-carboxylate (285 mg, 73%).

### Step 2: synthesis of (S,E)-8-(2-(2-chloro-6-fluorophenyl)prop-1-en-1-yl)-6-((3-(trifluoromethyl) phenyl)sulfonyl)-2,3,4,4a,5,6-hexahydro-1H-pyrazino[1,2-a]quinoxaline

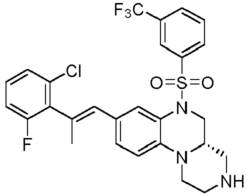

Tert-butyl (*S,E*)-8-(2-(2-chloro-6-fluorophenyl)prop-1-en-1-yl)-6-((3-(trifluoromethyl)phenyl) sul fonyl)-1,2,4,4a,5,6-hexahydro-3*H*-pyrazino[1,2-*a*]quinoxaline-3-carboxylate (250 mg, 0.38 mm ol) was dissolved in dichloromethane (5 mL). Trifluoroacetic acid (1 mL) was added theret o. The reaction mixture was stirred at room temperature for 1 hr. The reaction mixture wa s concentrated to remove the solvent. The residue was washed with a saturated solution of sodium hydrogencarbonate, extracted with ethyl acetate, dried, and concentrated to obtain (*S,E*)-8-(2-(2-chloro-6-fluorophenyl)prop-1-en-1-yl)-6-((3-(trifluoromethyl)phenyl)sulfonyl)-2,3,4, 4a,5,6-hexahydro-1*H*-pyrazino[1,2-*a*]quinoxaline (200 mg, 93%). ESI-MS: 566 [M+H]⁺.
¹H NMR (500 MHz, CDCl₃) δ 8.22 (s, 1H), 7.80-7.84 (m, 2H), 7.75 (s, 1H), 7.62-7.67 (m, 2H), 7.17-7.26 (m, 3H), 7.02 (t, *J=* 8.4 Hz, 1H), 6.71 (d, *J=* 8.7 Hz, 1H), 6.35 (s, 1H), 4.21-4.23 (m,, 1H), 3.61 (d*, J=* 12.6 Hz, 1H), 3.36 (t*, J=* 12.1 Hz, 1H), 3.06-3.15 m, 2H), 2.78-2.81 (m, 2H), 2.46-2.54(m,, 2H), 2.19 (s, 3H).

### Examples 11, 12 and 13 were prepared according to the synthesis method of Example 10.

| **Example No.** | **Structural formula** | **Chemical name** | **ESI-MS:** [M+1]^{÷} |
|---|---|---|---|
| **11** | 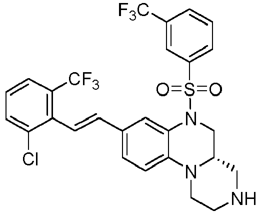 | (*S,E*)-8-(2-chloro-6-(trifl uoromethyl )styryl )-6-((3-(trifluoromethyl)phenyl)s ulfonyl)-2,3,4,4a,5,6-hex ahydro-1*H*-pyrazino[1,2-*a*]quinoxaline | 602 |
| **12** | 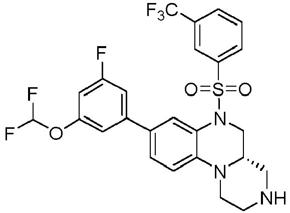 | (*S*)-8-(3-(difluoromethox y)-5 -fluorophenyl)-6-((3 - (trifluoromethyl)phenyl)s ulfonyl)-2,3,4,4a,5,6-hex ahydro-1*H*-pyrazino[ 1,2-*a* ]quinoxaline | 558 |
| **13** | 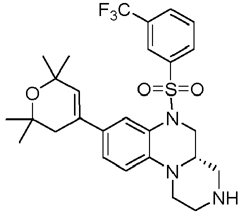 | (*S*)-8-(2,2, 6, 6-tetramethy 1-3,6-dihydro-2*H*-pyran-4-yl)-6-((3 -(trifluorometh yl)phenyl)sulfonyl)-2,3, 4,4a,5,6-hexahydro-1*H*-p yrazino [ 1,2-*a*] quinoxalin e | 536 |

### ¹H NMR data of the compound prepared in Examples 11 to 13 were as follows:

**Example 11:** ¹H NMR (500 MHz, CDCl₃) δ 7.873-7.82 (m, 4H), 7.63-7.61 (m , 3H), 7.33-7.26 (m , 2H), 6.97 (d, *J=* 16.6 Hz, 1H), 6.80 (d, *J=* 16.6 Hz, 1H), 6.71 (d, *J=* 8.5 Hz, 1H), 4.23 (dd, *J* = 14.2, 4.3 Hz, 1H), 3.58 (d, *J=* 12.6 Hz, 1H), 3.30-3.36 (m , 1H), 3.08-3.00 (m , 2H), 2.75 (t, *J*= 12.5 Hz, 2H), 2.55 - 2.32 (m, 2H).
**Example 12:** ¹H (400 MHz, Methanol-*d₄*) δ 8.04 (d, *J=* 7.9 Hz, 1H), 7.99 (d, *J=* 7.9 Hz, 1H), 7.87-7.77 (m, 2H), 7.65 (s, 1H), 7.51 (dd, *J*= 8.7, 2.2 Hz, 1H), 7.22-7.14 (m, 2H), 6.95 (t, 1H), 7.04 (d, *J =* 8.7 Hz, 1H), 6.90 (dt, *J =* 9.6, 2.3 Hz, 1H), 4.44 (dd, *J =* 14.5, 4.4 Hz, 1H), 4.03 (d, *J =* 14.4 Hz, 1H), 3.49-3.34 (m, 3H), 3.05 (qd, *J*= 14.8, 13.7, 3.5 Hz, 2H), 2.78 (t, *J =* 11.9 Hz, 1H), 2.68-2.56 (m, 1H).
**Example 13:** ¹H NMR (500 MHz, CDCl₃) δ 7.81 (t, *J*= 7.4 Hz, 2H), 7.72 (s, 1H), 7.65-7.57 (m, 2H), 7.20 (d, *J*= 8.6 Hz, 1H), 6.67 (d, *J*= 8.8 Hz, 1H), 5.96 (s, 1H), 4.19 (dd, *J*= 14.2, 4.3 Hz, 1H), 3.53 (d, *J*= 12.6 Hz, 1H), 3.32 (dd, *J*= 14.2, 9.9 Hz, 1H), 3.05 (d, *J*= 12.5 Hz, 1H), 2.98 (d, *J* = 12.0 Hz, 2H), 2.76-2.63 (m, 2H), 2.46-2.35 (m, 2H), 2.31 (s, 2H), 1.32 (d, *J=* 11.6 Hz, 12H).

### Examples 14, 15 and 16 were prepared according to the synthesis method of Example 7.

| **Example No.** | **Structural formula** | **Chemical name** | **ESI-MS:** [M+1]^{÷} |
|---|---|---|---|
| **14** | 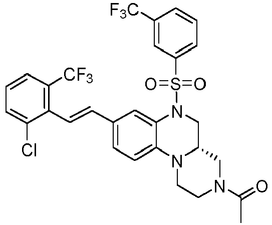 | (*S,E*)-1-(8-(2-chloro-6-(trifluoromethyl)styryl)-6-((3-(trifluoromethyl )phenyl)sulfonyl)-1,2,4,4a,5,6-hexahydro-3*H*-pyrazino[1,2-*a*]quinoxalin-3 -yl)ethan-1-one | 644 |
| **15** | 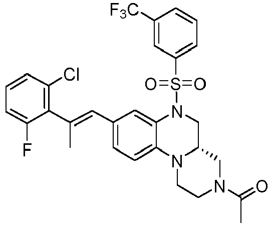 | (*S*,*E*)-1-(8-(2-(2-chloro-6-fluorophenyl)prop-1-en-1-yl)-6-((3-trifluoromethyl)phenyl)sulfonyl)-1,2,4,4a,5,6-hexahydro-3*H*-pyrazino[1,2-*a*]quinoxalin-3-yl)ethan-1-one | 608 |
| **16** | 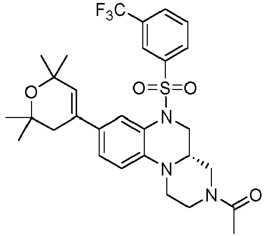 | (*S*)-1-(8-(2,2,6,6-tetramethyl-3,6-dihydro-2*H*-pyran-4-yl)-6-((3-trifluoromethyl)phenyl)sulfonyl)-1,2,4,4a,5,6-hexahydro-3*H*-pyrazino[1,2-*a*]quinoxalin-3-yl)ethan-1-one | 578 |

### ¹H NMR data of the compound prepared in Examples 14 to 16 were as follows:

**Example 14:** ¹H NMR (500 MHz, CDCl₃) δ 7.92-7.71 (m, 4H), 7.68-7.59 (m, 3H), 7.39-7.30 (m, 2H), 7.02-6.98 (m, 1H), 6.89-6.67 (m, 2H), 4.59-4.41 (m, 1H), 4.30 (dd, *J=* 14.3, 4.5 Hz, 1H), 3.81-3.55 (m, 2H), 3.36 (dt, *J*= 14.2, 10.3 Hz, 1H), 3.18-3.12 (m, 0.5H), 2.88-2.83 (m, 0.5H), 2.81-2.58 (m, 1.5H), 2.49-2.25 (m, 1.5H), 2.11 (d, *J*= 4.0 Hz, 3H).
**Example 15:** ¹H NMR (500 MHz, CDCl₃) δ 7.83-7.69 (m, 2H), 7.68-7.59 (m, 2H), 7.56-7.52 (m, 1H), 7.18-7.08 (m, 3H), 6.99-6.90 (m, 1H), 6.66 (dd, *J=* 22.1, 8.6 Hz, 1H), 6.29 (d, *J=* 1.7 Hz, 1H), 4.40 (t*, J=* 14.3 Hz, 1H), 4.19 (dd, *J*= 14.2, 4.5 Hz, 1H), 3.69-3.45 (m, 2H), 3.28 (dt*, J=* 14.4, 9.2 Hz, 1H), 3.06 (t, *J*= 10.8 Hz, 0.5H), 2.82-2.67 (m, 0.5H), 2.67-2.43 (m, 1.5H), 2.37-2.16 (m, 1.5H), 2.14 (d, *J=* 1.6Hz, 3H), 2.01 (d, *J=* 5.0 Hz, 3H).
**Example 16:** ¹H NMR (500 MHz, CDCl₃) δ 7.92-7.78 (m, 2H), 7.76-7.67 (m, 1H), 7.67-7.58 (m, 2H), 7.24 (td, *J*= 5.9, 2.9 Hz, 1H), 6.71 (dd, *J*= 22.1, 8.7 Hz, 1H), 6.03-5.95 (m, 1H), 4.55-4.43 (m, 1H),4.32-4.20 (m, 1H), 3.76-3.54 (m, 2H), 3.36 (ddd, *J=* 14.3, 9.9, 7.5 Hz, 1H), 3.19-3.05 (m, 0.5H), 2.82(dd, *J*= 12.8, 10.9 Hz, 0.5H), 2.73-2.50 (m, 1.5H), 2.42-2.23 (m, 3.5H), 2.09 (d, *J*= 4.7 Hz, 3H), 1.35 (d*, J =* 11.8 Hz, 12H).

### Examples 17, 18 and 19 were prepared according to the synthesis method of Example 8.

| **Example No.** | **Structural formula** | **Chemical name** | **ESI-MS:** [M+1]^{÷} |
|---|---|---|---|
| **17** | 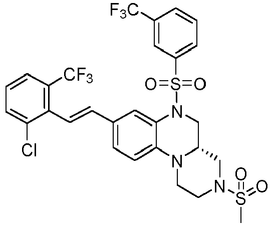 | (*S,E*)-8-(2-chloro-6-(trifluoromethyl)styryl)-3-(methylsulfonyl)-6-((3- (trifluoromethyl)phenyl)sulfonyl)-2,3,4,4a,5,6-hexahydro-1*H*-pyrazino[1,2-*a*]quinoxaline | 680 |
| **18** | 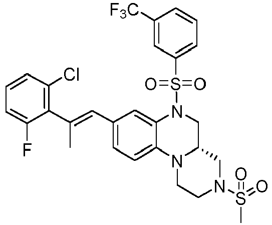 | (*S,E*)-8-(2-(2-chloro-6-fluorophenyl)prop-1-en-1-yl)-3-(methylsulfonyl)-6-((3-(trifluoromethyl)phenyl)sulfonyl)-2,3,4,4a,5,6-hexahydro-1*H*-pyrazino[1,2-*a*]quinoxaline | 644 |
| **19** | 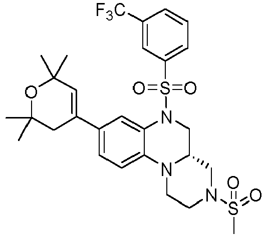 | (*S*)-3 -(methylsulfonyl)-8-(2,2,6,6-tetramethyl-3,6-dihydro-2*H*-pyran-4-yl)-6-((3-(trifluoromethyl)phenyl)sulfonyl)-2,3,4,4a,5,6-hexahydro-1*H-*pyrazino[1,2-*a*]quinoxaline | 614 |

### ¹H NMR data of the compound prepared in Examples 17 to 19 were as follows:

**Example 17:** ¹H (500 MHz, CDCl₃) δ 7.90-7.81 (m, 2H), 7.80-7.72 (m, 2H), 7.69-7.61 (m, 3H), 7.39-7.30 (m, 2H), 7.05-6.96 (m, 1H), 6.85-6.73 (m, 2H), 4.27 (dd, *J=* 14.3, 4.7 Hz, 1H), 3.77-3.63(m, 3H), 3.42 (dd, *J*= 14.3, 9.5 Hz, 1H), 2.86-2.81 (m, 1H), 2.80(s, 3H), 2.70 (td, *J*= 11.7, 3.2Hz, 1H), 2.54 (td, *J*= 12.3, 3.3 Hz, 1H), 2.40 (t, *J*= 10.9 Hz, 1H).
**Example 18:** ¹H NMR (500 MHz, CDCl₃) δ 7.87-7.79 (m, 2H), 7.75 (d, *J=* 1.8 Hz, 1H), 7.68 (d*, J =* 2.1 Hz, 1H), 7.63 (t*, J=* 7.8 Hz, 1H), 7.25-7.15 (m, 3H), 7.04-7.02 (m, 1H), 6.73 (d*, J =* 8.7Hz, 1H), 6.36 (d, *J*= 1.9 Hz, 1H), 4.23 (dd, *J =* 14.2, 4.7 Hz, 1H), 3.66 (dddd, *J*= 19.2, 9.2, 4.9, 2.4 Hz,3H), 3.41 (dd, *J*= 14.3, 9.6 Hz, 1H), 2.77 (s, 4H), 2.66 (td, *J*= 11.6, 2.8 Hz, 1H), 2.49 (td, *J =* 12.2, 3.0Hz, 1H), 2.37 (dd, *J=* 11.3, 10.4 Hz, 1H), 2.20 (d*, J =* 1.5 Hz, 3H).
**Example 19:** ¹H (500 MHz, DMSO-*d₆*) δ 8.09 (d, *J*= 7.7 Hz, 1H), 7.97 (d, *J*= 8.0 Hz, 1H), 7.86 (t, *J=* 7.9 Hz, 1H), 7.66 (d, *J=* 1.8 Hz, 1H), 7.42 (d, *J=* 2.2 Hz, 1H), 7.26 (dd, *J=* 8.7, 2.2 Hz, 1H), 6.90 (d, *J=* 8.8 Hz, 1H), 6.02 (d, *J=* 1.4 Hz, 1H), 4.38 *(dd, J=* 14.4, 4.6 Hz, 1H), 3.89-3.75 (m, 1H), 3.57 (dt, J= 11.4, 2.5Hz, 1H), 3.34 (s, 1H), 3.32-3.28 (m, 1H), 2.85 (s, 3H), 2.65 (td, *J =* 11.8, 3.1 Hz, 1H), 2.60-2.52 (m, 1H), 2.36 (t*, J=* 10.9 Hz, 1H), 2.30-2.15 (m, 3H), 1.24 (d*, J=* 16.2 Hz, 12H).

### Examples 20, 21 and 22 were prepared according to the synthesis method of Example 9.

| **Example No.** | **Structural formula** | **Chemical name** | **ESI-MS:** [M+1 ]^{÷} |
|---|---|---|---|
| **20** | 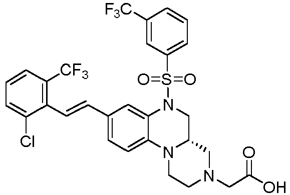 | (*S,E*)-2-(8-(2-chloro-6-(triflu oromethyl)styryl)-6-((3-(trifl uoromethyl)phenyl)sulfonyl)-1,2,4,4a,5,6-hexahydro-3 *H*-pyrazino[ 1,2-*a*]quinoxalin-3-yl)acetic acid | 660 |
| **21** | 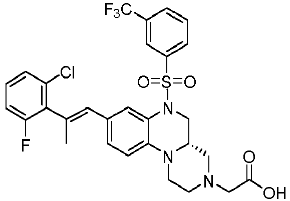 | (*S,E*)- 2-(8-(2-(2-chloro-6-fluorophenyl)prop-1-en-1-yl)-6-((3-(trifluoromethyl)phenyl)sulfonyl)-1,2,4,4a,5,6-hexahydro-3*H*-pyrazino[1,2-*a*]quinoxalin-3-yl)acetic acid | 624 |
| **22** | 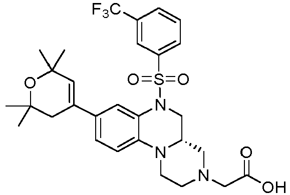 | (*S*)-2-(8-(2,2,6,6-tetramethyl-3,6-dihydro-2*H*-pyran-4-yl)-6-((3-trifluoromethyl)phenyl)sulfonyl)-1,2,4,4a,5,6-hexahydro-3*H*-pyrazino[1,2-*a*]quinoxalin-3-yl)acetic acid | 594 |

### ¹H NMR data of the compound prepared in Examples 20 to 22 were as follows:

**Example 20:** ¹H (500 MHz, CDCl₃) δ 7.78 (d, *J*= 8.0 Hz, 1H), 7.73 (s, 1H), 7.68 (d, *J* = 8.0 Hz, 1H),7.55-7.47 (m, 4H), 7.22-7.14 (m, 2H), 6.80 *(dd, J=* 16.7, 1.9 Hz, 1H), 6.68-6.58 (m, 2H), 4.14 *(dd, J=* 14.0, 4.2 Hz, 1H), 3.56 (d*, J=* 12.9 Hz, 1H), 3.39-3.09 (m, 6H), 2.74 (q, *J*= 15.1, 13.8 Hz, 1H), 2.51 (s, 1H), 2.25 (s, 1H).
**Example 21:** ¹H NMR (500 MHz, CDCl₃) δ 7.79-7.81 (m, 3H), 7.55-7.64 (m,, 2H), 7.15-7.23 (m, 3H), 7.02-6.99 (m, 1H), 6.72 (s, 1H), 6.30 (s, 1H), 4.24 (s, 1H), 3.67 (s, 2H), 3.43 (s, 3H), 3.20 (s, 3H), 2.63 (s, 1H), 2.35 (s, 1H), 2.14 (s, 3H).
**Example 22:** ¹H NMR (500 MHz, CDCl₃) δ 7.80 (s, 3H), 7.62 (s, 2H), 7.18 (d, *J =* 8.0 Hz, 1H), 6.67 (s, 1H),5.93 (s, 1H), 4.20 (s, 1H), 3.57 (s, 1H), 3.30 (d, *J=* 62.0 Hz, 3H), 2.94 (s, 3H), 2.65-2.63 (m, 3H), 2.39 (s, 1H), 2.12 (s, 1H),1.31 (d*, J =* 12.6 Hz, 12H).

### Example 23: preparation of methyl 2-((S)-8-((E)-2-(2-chloro-6-fluorophenyl)prop-l-en-1-yl)-6-((3-(trifluoromethyl)phenyl)sulfonyl)-1,2,4,4a,5,6-hexahydro-3H-pyrazino[1,2-a] quinoxalin-3-yl)propanoate

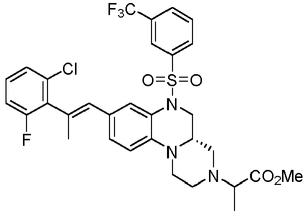

(*S,E*)-8-(2-(2-chloro-6-fluorophenyl)prop-1-en-1-yl)-6-((3-(trifluoromethyl)phenyl)sulfonyl)-2,3,4,4a,5,6-hexahydro-1*H*-pyrazino[1,2-*a*]quinoxaline(60 mg, 0.106 mmol), methyl (*S*)-2-chlo ropropanoate (115 mg, 1.06 mmol) and potassium carbonate (44 mg, 0.318 mmol) were m ixed in acetonitrile (2 mL), and the reaction mixture was reacted under microwave at 60 ° C for 12 hrs. The reaction mixture was separated by a rapid reversed-phase column to obt ain methyl 2-((*S*)-8-((*E*)-2-(2-chloro-6-fluorophenyl)prop-1-en-1-yl)-6-((3-(trifluoromethyl)phen yl)sulfonyl)-1,2,4,4a,5,6-hexahydro-3*H*-pyrazino[1,2-a]quinoxalin-3-yl)propanoate (15 mg, 2 2%). ESI-MS: 652 [M+H]⁺.
¹H NMR (500 MHz, CDCl₃): δ 7.82-7.77 (m, 2H), 7.74 (d, *J* = 7.9 Hz, 1H),7.71 (d, *J* = 2.1 Hz, 1H), 7.59 (t, *J* = 7.7 Hz, 1H), 7.25-7.12 (m, 3H), 7.07-6.97 (m, 1H), 6.69 (d, *J* = 8.6 Hz, 1H), 6.35 (d, *J* = 1.8 Hz, 1H), 4.20 (dd, *J* = 14.1, 4.5 Hz, 1H), 3.70 (s, 3H), 3.53-3.44 (m, 1H), 3.37-3.22 (m, 2H), 2.78-2.74 (m, 2H), 2.44-2.40 (m, 1H), 2.37-2.26 (m, 2H), 2.21 (d, *J* = 1.5 Hz, 3H), 1.99 (t, *J* = 10.6 Hz, 1H), 1.26 (d, *J* = 6.8 Hz, 3H).

### Example 24 was prepared according to the synthesis method of Example 23.

| **Example No.** | **Structural formula** | **Chemical name** | **ESI-MS:** [M+1]^{÷} |
|---|---|---|---|
| **24** | 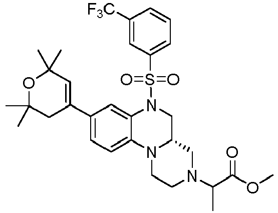 | methyl 2-((*S*)-8-(2,2,6,6-tetramethyl-3,6-dihydro-2*H*-pyran-4-yl)-6-((3-(trifluoromethyl)phenyl)sulfonyl)-1,2,4,4a,5,6-hexahydro-3*H*-pyrazino[1,2-*a*]quinoxalin-3-yl)propanoate | 622 |

¹H NMR (500 MHz, CDCl₃) δ 7.76 (t, *J*= 11.7 Hz, 3H), 7.63 (d, *J* = 2.2Hz, 1H), 7.58 (t, *J* = 7.8 Hz, 1H), 7.18 (dd, *J* = 8.6, 2.2 Hz, 1H), 6.65 (d, *J* = 8.8 Hz, 1H), 5.95 (d, *J* = 1.4 Hz, 1H), 4.18 (dd, *J* = 14.1, 4.4 Hz, 1H), 3.69 (s, 3H), 3.46 (d, *J* = 11.5 Hz, 1H), 3.36-3.30 (m, 1H), 3.28 (d, *J* = 7.8 Hz, 1H), 2.75 (t, *J* = 9.5 Hz, 2H), 2.56 (s, 1H), 2.39 (dt, *J* = 37.2, 10.5 Hz, 2H), 2.31 (d, *J* = 1.6 Hz, 2H), 1.97 (t, *J* = 10.6 Hz, 1H), 1.32 (d, *J* = 11.2 Hz, 12H), 1.27 (d, *J* = 7.1 Hz, 3H).

### Example 25: preparation of 2-((S)-8-((E)-2-(2-chloro-6-fluorophenyl)prop-1-en-1-yl)-6-((3-(trifluoromethyl)phenyl)sulfonyl)-1,2,4,4a,5,6-hexahydro-3H-pyrazino[1,2-a]quinoxalin-3-yl) propanoic acid

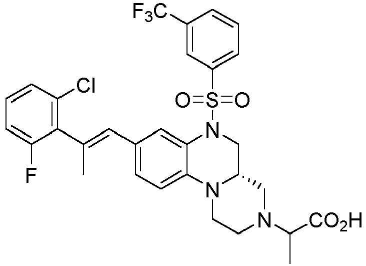

Methyl 2-((*S*)-8-((*E*)-2-(2-chloro-6-fluorophenyl)prop-1-en-1-yl)-6-((3-(trifluoromethyl)phen yl)sulfonyl)-1,2,4,4a,5,6-hexahydro-3*H*-pyrazino[1,2-*a*]quinoxalin-3-yl)propanoate (13 mg, 0.02 mmol) and lithium hydroxide (5 mg, 0.1 mmol) were mixed in methanol (2 mL). Water was added thereto dropwise. The reaction mixture was stirred at 40 °C overnight. The pH of the reaction mixture was adjusted to about 5 with dilute hydrochloric acid. The resultin g mixture was separated by a rapid reversed-phase column to obtain 2-((*S*)-8-((*E*)-2-(2-chlo ro-6-fluorophenyl) prop-1-en-1-yl)-6-((3-(trifluoromethyl)phenyl)sulfonyl)-1,2,4,4a,5,6-hexahydr o-3*H*-pyrazino[1,2-*a*]quinoxalin-3-yl)propanoic acid (7.5 mg, 58%). ESI-MS: 638 [M+H]⁺.
¹H NMR (500 MHz, CDCl₃): δ 7.86-7.76 (m, 3H), 7.62-7.60 (m, 2H), 7.22-7.15 (m, 3H), 7.01 (t, *J =* 8.3 Hz, 1H), 6.71 (d, *J* = 8.4 Hz, 1H), 6.32 (s, 1H), 4.19 (d, *J* = 13.3 Hz, 1H), 3.58 (d, *J* = 10.5Hz 1H), 3.44-3.18 (m, 2H), 2.80-2.20 (m, 5H), 2.17 (s, 3H), 2.07 (brs, 1H), 1.39-1.18 (m, 3H).

### Examples 26 to 27 were prepared according to the synthesis method of Example 25.

| **Example No.** | **Structural formula** | **Chemical name** | **ESI-MS:** [M+1]⁺ |
|---|---|---|---|
| **26** | 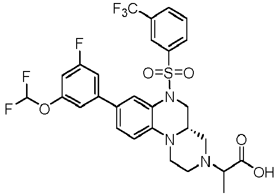 | 2-((*S*)-8-(3-(difluorometh oxy)-5-fluorophenyl)-6-((3 -(trifluoromethyl)phen yl)sulfonyl)-1,2,4,4a,5,6-h exahydro-3*H*-pyrazino[1, 2-*a*]quinoxalin-3-yl)propa noic acid | 630 |
| **27** | 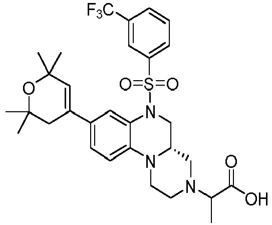 | 2-((*S*)-8-(2,2,6,6-tetramet hyl-3,6-dihydro-2*H*-pyran -4-yl)-6-((3-(trifluorometh yl)phenyl)sulfonyl)-1,2,4, 4a, 5, 6-hexahydro-3*H*-pyr azino[1,2-*a*]quinoxalin-3 - yl)propanoic acid | 608 |

### ¹H NMR data of the compound prepared in Examples 26 to 27 were as follows:

**Example 26:** ¹H NMR (400 MHz, Methanol-*d*₄) δ 8.02 (dd, *J* = 14.5, 7.8 Hz, 2H), 7.85-7.79 (m, 2H), 7.69 (d, *J* = 4.6 Hz, 1H), 7.51 (dd, *J* = 8.7, 2.2 Hz, 1H), 7.22-7.15 (m, 2H), 6.95 (t, 1H), 7.04 (dd, *J* = 8.9, 2.5 Hz, 1H), 6.93-6.88 (m, 1H), 4.47 (ddd, *J=* 14.2, 8.5, 4.3 Hz, 1H), 4.10 (d, *J* = 9.6 Hz, 2H), 3.66-3.51 (m, 2H), 3.42 (ddd, *J* = 14.6, 9.9, 5.4 Hz, 1H), 3.24-3.05 (m, 2H), 3.00-2.70 (m, 2H), 1.61 (d, *J* = 7.3 Hz, 3H).
**Example 27:** ¹H NMR (400 MHz, CDCl₃) δ 7.82 (d, *J=* 15.2 Hz, 3H), 7.61 (d, *J* = 8.1 Hz, 2H), 7.17 (d, *J* = 9.4 Hz, 1H), 6.65 (s, 1H), 5.93 (s, 1H), 4.27-4.12 (m, 1H), 3.56 (ddd, *J* = 16.1, 8.2, 4.6 Hz, 1H), 3.44-3.27 (m, 2H), 2.97-2.68 (m, 3H), 2.49 (d, *J* = 32.6 Hz, 2H), 2.31-2.22 (m, 3H), 2.19-2.03 (m, 3H), 1.32 (s, 6H), 1.30 (s, 6H).

### Example 28: preparation of (S)-3-(8-(3-(difluoromethoxy)-5-fluorophenyl)-6-((3-(trifluoro methyl)phenyl)sulfonyl)-1,2,4,4a,5,6-hexahydro-3H-pyrazino[1,2-a]quinoxalin-3-yl)-3-oxopro panenitrile

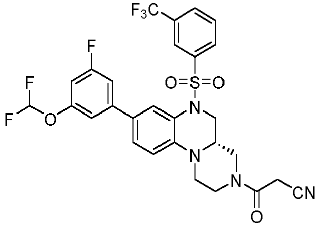

(*S*)-8-(3-(difluoromethoxy)-5-fluorophenyl)-6-((3-(trifluoromethyl)phenyl)sulfonyl)-2,3,4,4a, 5,6-hexahydro-1*H*-pyrazino[1,2-*a*]quinoxaline (50 mg, 0.09 mmol) was dissolved in acetonitrile (2 mL); 2-cyanoacetic acid (96 mg, 1.13 mmol), 2-(7-oxybenzotriazole)-*N*,*N*,*N*',*N*'-tetramethylur onium hexafluorophosphate (108 mg, 0.28 mmol) and *N*,*N*-diisopropylethylamine (148 mg, 1.15 mmol) were added to the solution. The reaction mixture was stirred at 50 °C for 5 h rs. The reaction mixture was concentrated to remove the solvent. The residue was separate d by TLC [solvent: methanol : dichloromethane =1:20], then separated by a reversed-phase column [eluent: acetonitrile : water (0.5% HCl) = 0%-60%] to obtain (*S*)-3-(8-(3-(difluoro methoxy)-5-fluorophenyl)-6-((3-(trifluoromethyl)phenyl)sulfonyl)-1,2,4,4a,5,6-hexahydro-3*H*-pyr azino[1,2-*a*]quinoxalin-3-yl)-3-oxopropanenitrile (27.4 mg, 49%). ESI-MS: 625 [M+H]⁺.
¹H NMR (400 MHz, Methanol-*d*₄) δ 7.92 (t, *J* = 8.4 Hz, 2H), 7.83 (d, *J* = 2.3 Hz, 1H), 7.74 (t, *J* = 7.9 Hz, 1H), 7.65 (d, *J* = 6.2 Hz, 1H), 7.48 (dd, *J* = 8.7, 2.3 Hz, 1H), 7.23-7.15 (m, 2H), 6.94 (t, 1H), 6.94 (d, *J* = 8.7 Hz, 1H), 6.88 (dt, *J* = 9.5, 2.3 Hz, 1H), 4.47-4.24 (m, 2H), 3.91 (dd, *J* = 18.6, 2.9 Hz, 1H), 3.81 (d, *J* = 18.6 Hz, 1H), 3.78-3.65 (m, 2H), 3.37 (td, *J* = 10.5, 5.1 Hz, 1H), 2.90-2.60 (m, 2H), 2.47-2.31 (m, 1H), 2.03 (s, 1H).

### Examples 29, 30 and 31 were prepared according to the synthesis method of Example 28.

| **Example No.** | **Structural formula** | **Chemical name** | **ESI-MS:** [M+1]⁺ |
|---|---|---|---|
| **29** | 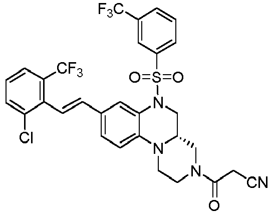 | (*S*,*E*)-3-(8-(2-chloro-6-(tri fluoromethyl)styryl)-6-((3 -(trifluoromethyl)phenyl)s ulfonyl)-1,2,4,4a,5,6-hexa hydro-3H-pyrazino[1,2-*a*] quinoxalin-3-yl)-3-oxopro panenitrile | 669 |
| **30** | 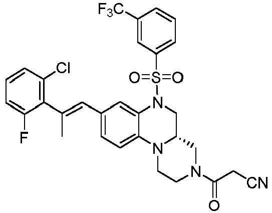 | (*S*,*E*)-3-(8-(2-(2-chloro-6-fluorophenyl)prop-1-en-1-yl)-6-((3 -(trifluoromethyl) phenyl)sulfonyl)-1,2,4,4a, 5,6-hexahydro-3*H*-pyrazi no[1,2-*a*]quinoxalin-3-yl) -3-oxopropanenitrile | 633 |
| **31** | 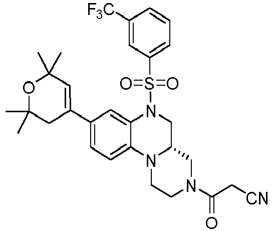 | (*S*)-3-oxo-3-(8-(2,2,6,6-tet ramethyl-3,6-dihydro-2*H-*pyran-4-yl)-6-((3-(trifluor omethyl)phenyl)sulfonyl)-1,2,4,4a,5,6-hexahydro-3 *H*-pyrazino[1,2-*a*]quinoxa lin-3 -yl)propanenitrile | 603 |

### ¹H NMR data of the compound prepared in Examples 29 to 31 were as follows:

**Example 29:** ¹H NMR (500 MHz, CDCl₃) δ 7.79 (d, *J* = 7.8 Hz, 1H), 7.75 (d, *J* = 7.9 Hz, 1H), 7.70-7.69 (m, 1H), 7.63-7.62(m, 1H), 7.55-7.58 (m, 3H), 7.30-7.21 (m, 2H), 6.93-6.89 (m, 1H), 6.74-6.64 (m, 2H), 4.39-4.34 (m, 1H), 4.27-4.19 (m, 1H), 3.65-3.51 (m, 2H), 3.48-3.37 (m, 2H), 3.34-3.16 (m, 1.5H), 2.91-2.86 (m, 0.5H), 2.76-2.70(m , 1H), 2.63-2.60 (m, 0.5H), 2.50-2.40 (m, 0.5H), 2.39-2.28 (m, 1H).
**Example 30:** ¹H NMR (500 MHz, CDCl₃): δ 7.85-7.75 (m, 2H), 7.68 (s, 1H), 7.64-7.52 (m, 2H), 7.18-7.08 (m, 3H), 7.01-6.91 (m, 1H), 6.67 (dd, *J* = 16.5, 8.6 Hz, 1H), 6.29 (s, 1H), 4.36 (d, J = 13.0 Hz, 1H), 4.38-4.19 (m, 1H), 3.60-3.45 (m, 2H), 3.45-3.23 (m, 3H), 3.19-3.14 (m, 0.5H), 2.95-2.84 (m, 0.5H), 2.76-2.52 (m, 1.5H), 2.46-2.23 (m, 1.5H), 2.13 (d, *J* = 1.8 Hz, 3H).
**Example 31:** ¹H NMR (500 MHz, CDCl₃) δ 7.85 (d, *J* = 6.8 Hz, 1H), 7.81 (d, *J* = 7.8 Hz, 1H), 7.71 (s, 1H), 7.63 (t, *J* = 7.9 Hz, 1H), 7.60-7.56 (m, 1H), 7.24-7.19 (m, 1H), 6.70 (dd, *J* = 16.0, 8.7 Hz, 1H), 5.97 (s, 1H), 4.42 (d, *J* = 13.1 Hz, 1H), 4.27 (ddd, *J* = 19.3, 14.3, 4.4 Hz, 1H), 3.60 (dd, *J* = 26.2, 12.3 Hz, 2H), 3.52-3.40 (m, 2H), 3.39-3.31 (m, 1H), 3.24 (t, *J* = 11.7 Hz, 1H), 2.97-2.89 (m, 1H), 2.80-2.69 (m, 1H), 2.61 (s, 1H), 2.47 (t, *J* = 11.1 Hz, 1H), 2.40-2.34 (m, 1H), 2.30 (s, 2H), 1.32 (d, *J* = 12.1 Hz, 12H).

### Example 32: preparation of (S,E)-8-(2-chloro-6-(trifluoromethyl)styryl)-3-(oxetan-3-yl)-6-((3-(trifluoromethyl)phenyl)sulfonyl)-2,3,4,4a,5,6-hexahydro-1H-pyrazino[1,2-a]quinoxaline

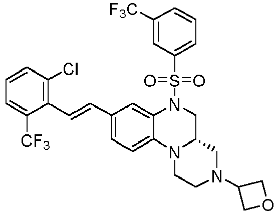

(*S*,*E*)-8-(2-chloro-6-(trifluoromethyl)styryl)-6-((3-(trifluoromethyl)phenyl)sulfonyl)-2,3,4,4 a,5,6-hexahydro-1*H*-pyrazino[1,2-*a*]quinoxaline (50 mg, 0.083 mmol) was dissolved in *N,N-*d imethylformamide (2 mL). Oxetan -3-one (12 mg, 0.166 mmol) and one drop of acetic aci d were added to the solution. The reaction mixture was stirred at room temperature for 0. 5 hrs, then sodium cyanoborohydride (10 mg, 0.166 mmol) was added. The reaction mixtu re was stirred at room temperature for 2 hrs. The residue was separated by a rapid revers ed-phase column to obtain (*S*,*E*)-8-(2-chloro-6-(trifluoromethyl)styryl)-3-(oxetan-3-yl)-6-((3-(trifl uoromethyl)phenyl)sulfonyl)-2,3,4,4a,5,6-hexahydro-1*H*-pyrazino[1,2-*a*]quinoxaline (14.7 mg, 2 7%). ESI-MS: 658 [M+1]^{÷}.
¹H NMR (500 MHz, CDCl₃) δ 7.83-7.77 (m, 3H), 7.75 (d, J = 2.1 Hz, 1H), 7.65-7.59 (m, 3H),7.34-7.28 (m, 2H), 6.97 (dd, J = 16.7, 1.9 Hz, 1H), 6.80 (d, J = 16.6 Hz, 1H), 6.71 (d, J = 8.6 Hz, 1H),4.65 (q, J = 6.2 Hz, 2H), 4.56 (td, J = 6.0, 3.7 Hz, 2H), 4.21 (dd, J = 14.1, 4.4 Hz, 1H), 3.59-3.49 (m,1H), 3.45 (q, J = 6.3 Hz, 1H), 3.34 (dd, *J* = 14.1, 9.9 Hz, 1H), 2.74 (dd, J = 4.5, 2.8 Hz, 1H), 2.67 (d, J =11.1 Hz, 1H), 2.61 (dt, *J* = 10.5, 2.5 Hz, 1H), 2.52 (td, J = 12.0, 3.1 Hz, 1H), 1.88 (td, J = 11.5, 3.2 Hz,1H), 1.60 (d, *J* = 10.5 Hz, 1H).

### Examples 33 to 34 were prepared according to the synthesis method of Example 32.

| **Example No.** | **Structural formula** | **Chemical name** | **ESI-MS:** [M+1]⁺ |
|---|---|---|---|
| **33** | 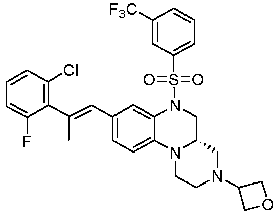 | (*S*,*E*)-8-(2-(2-chloro-6-fluor ophenyl)prop-1-en-1-yl)-3-(oxetan-3-yl)-6-((3-(trifluoro methyl)phenyl)sulfonyl)-2,3, 4,4a,5,6-hexahydro-1*H*-pyra zino[1,2-*a*]quinoxaline | 622 |
| **34** | 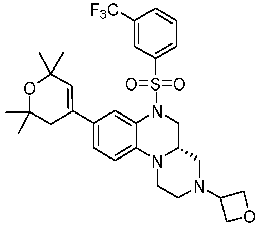 | (*S*)-3-(oxetan-3-yl)-8-(2,2,6, 6-tetramethyl-3,6-dihydro-2 *H*-pyran-4-yl)-6-((3-(trifluor omethyl)phenyl)sulfonyl)-2, 3,4,4a,5,6-hexahydro-1*H*-py razino[1,2-*a*]quinoxaline | 592 |

### ¹H NMR data of the compound prepared in Examples 33 to 34 were as follows:

**Example 33:** ¹H NMR (500 MHz, CDCl₃): 7.83-7.73 (m, 3H), 7.72 (d, J = 2.1 Hz, 1H), 7.60 (t, J = 7.8 Hz, 1H), 7.24-7.14 (m, 3H), 7.07-6.97 (m, 1H), 6.71 (d, J = 8.7 Hz, 1H), 6.36 (d, J = 1.6 Hz, 1H), 4.65 (q, J = 6.3 Hz, 2H), 4.55 (q, J = 5.9 Hz, 2H), 4.18 (dd, J = 14.2, 4.4 Hz, 1H), 3.52 (dt, J = 12.2, 2.8 Hz, 1H), 3.47-3.38 (m, 1H), 3.36 (dd, J = 13.5, 10.0 Hz, 1H), 2.67-2.60 (m, 2H), 2.60 (dt, J = 10.5, 2.5 Hz, 1H), 2.49 (td, J = 12.0, 3.1 Hz, 1H), 2.21 (d, J = 1.5 Hz, 3H), 1.86 (td, J = 11.4, 3.2 Hz, 1H), 1.62-1.50 (m, 1H).
**Example 34:** ¹H NMR (500 MHz, CDCl₃) δ 7.80 (d, *J* = 7.8 Hz, 1H), 7.77 (d, *J* = 9.3 Hz, 2H), 7.63 (d, *J* = 2.2 Hz, 1H), 7.60 (t, *J* = 7.8 Hz, 1H), 7.19 (dd, *J =* 8.7, 2.2 Hz, 1H), 6.67 (d, *J* = 8.8 Hz, 1H), 5.96 (t, *J* = 1.4 Hz, 1H), 4.64 (q, *J* = 6.2 Hz, 2H), 4.55 (td, *J* = 6.2, 4.3 Hz, 2H), 4.17 (dd, *J* = 14.1, 4.5 Hz, 1H), 3.49 (d, *J* = 12.1 Hz, 1H), 3.42 (t, *J* = 6.3 Hz, 1H), 3.34 (dd, *J* = 14.1, 9.8 Hz, 1H), 2.72-2.62 (m, 2H), 2.59 (d, *J* = 10.6 Hz, 1H), 2.48 (td, *J* = 11.9, 3.0 Hz, 1H), 2.31 (d, *J* = 1.4 Hz, 2H), 1.85 (td, *J* = 11.4, 3.2 Hz, 1H), 1.55 (d, *J* = 10.4 Hz, 1H), 1.32 (d, *J* = 11.4 Hz, 12H).

### Example 35: preparation of (R)-3-((S)-8-((E)-2-chloro-6-(trifluoromethyl)styryl)-6-((3-(trif luoromethyl)phenyl)sulfonyl)-1,2,4,4a,5,6-hexahydro-3H-pyrazino[1,2-a]quinoxalin-3-yl)pro pane-1,2-diol

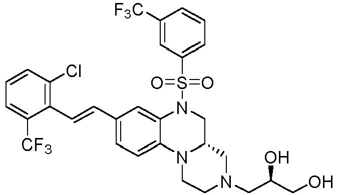

(*S*,*E*)-8-(2-chloro-6-(trifluoromethyl)styryl)-6-((3-(trifluoromethyl)phenyl)sulfonyl)-2,3,4,4 a,5,6-hexahydro-1*H*-pyrazino[1,2-*a*]quinoxaline (40 mg, 0.067 mmol) was dissolved in aceto nitrile (2 mL). Potassium carbonate (27 mg, 0.201 mmol) and sodium iodide (30 mg, 0.20 1 mmol) were added thereto. The reaction mixture was stirred at 60 °C for 0.5 hrs, and (*S*)-3-chloropropane-1,2-diol (15 mg, 0.133 mmol) was added. The reaction mixture was sti rred at 60 °C for a further 16 hrs. The residue was separated by a rapid column to obtai n (*R*)-3-((*S*)-8-((*E*)-2-chloro-6-(trifluoromethyl)styryl)-6-((3-(trifluoromethyl)phenyl)sulfonyl)-1, 2,4,4a,5,6-hexahydro-3*H*-pyrazino[1,2-*a*]quinoxalin-3-yl)propane-1,2-diol (14.7 mg, 27%). ESI -MS: 676 [M+1]⁺.
¹H NMR (500 MHz, CDCl₃) δ 7.77 (s, 1H), 7.73 (d, *J* = 7.9 Hz, 1H), 7.70-7.64 (m, 2H), 7.58 -7.48 (m, 3H), 7.25-7.20 (m, 2H), 6.90 (d, *J* = 16.6 Hz, 1H), 6.73 (d, *J* = 16.6 Hz, 1H), 6.64 (d, *J* = 8.7 Hz, 1H), 4.13 (dd, *J* = 14.2, 4.2 Hz, 1H), 3.70 (d, *J* = 11.9 Hz, 2H), 3.52-3.36 (m, 2H), 3.27 (dd, *J* = 14.1, 10.0 Hz, 1H), 2.82 (d, *J* = 10.9 Hz, 1H), 2.71 (d, *J* = 11.0 Hz, 1H), 2.60 (s, 1H), 2.51 (t, *J* = 11.1 Hz, 1H), 2.40 (t, *J* = 11.9 Hz, 1H), 2.31-2.17 (m, 2H), 1.67 (t, *J* = 10.7 Hz, 1H).

### Examples 36 to 42 were prepared according to the synthesis method of Example 35.

| **Example No.** | **Structural formula** | **Chemical name** | **ESI-MS:** [M+1]⁺ |
|---|---|---|---|
| **36** | 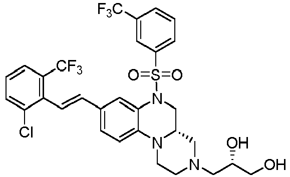 | (*S*)-3-((*S*)-8-((*E*)-2-chloro-6-(t rifluoromethyl)styryl)-6-((3-(t rifluoromethyl)phenyl)sulfony l)-1,2,4,4a,5,6-hexahydro-3*H-*pyrazino[1,2-*a*]quinoxalin-3-y l)propane-1,2-diol | 676 |
| **37** | 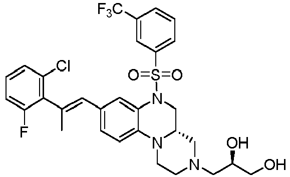 | (*R*)-3-((*S*)-8-((*E*)-2-(2-chloro-6-fluorophenyl)prop-1-en-1-y 1)-6-((3 -(trifluoromethyl)phen yl)sulfonyl)-1,2,4,4a,5,6-hexa hydro-3*H*-pyrazino[1,2-*a*]quin oxalin-3-yl)propane-1,2-diol | 640 |
| **38** | 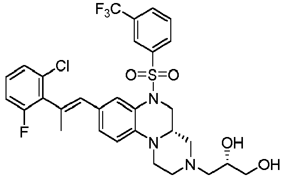 | (*S*)-3-((*S*)-8-((*E*)-2-(2-chloro-6-fluorophenyl)prop-1-en-1-y 1)-6-((3 -(trifluoromethyl)phen yl)sulfonyl)-1,2,4,4a,5,6-hexa hydro-3*H*-pyrazino[1,2-*a*]quin oxalin-3-yl)propane-1,2-diol | 640 |
| **39** | 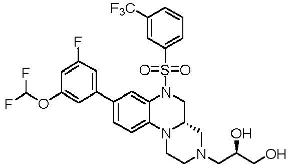 | (*R*)-3-((*S*)-8-(3-(difluorometh oxy)-5-fluorophenyl)-6-((3-(tr ifluoromethyl)phenyl)sulfony l)-1,2,4,4a,5,6-hexahydro-3*H-*pyrazino[1,2-*a*] quinoxalin-3-y l)propane-1,2-diol | 632 |
| **40** | 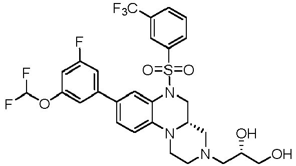 | (*S*)-3-((*S*)-8-(3-(difluorometho xy)-5-fluorophenyl)-6-((3-(trif luoromethyl)phenyl)sulfonyl)-1,2,4,4a,5,6-hexahydro-3*H*-py razino[1,2-*a*]quinoxalin-3-yl) propane-1,2-diol | 632 |
| **41** | 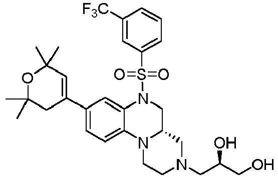 | (*R*)-3-((*S*)-8-(2,2,6,6-tetramet hyl-3,6-dihydro-2*H*-pyran-4-y 1)-6-((3 -(trifluoromethyl)phen yl)sulfonyl)-1,2,4,4a,5,6-hexa hydro-3*H*-pyrazino[1,2-*a*]quin oxalin-3-yl)propane-1,2-diol | 610 |
| **42** | 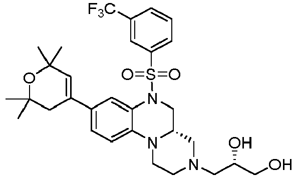 | (*S*)-3-((*S*)-8-(2,2,6,6-tetrameth yl-3,6-dihydro-2*H*-pyran-4-yl) -6-((3 -(trifluoromethyl)pheny l)sulfonyl)-1,2,4,4a,5,6-hexah ydro-3*H*-pyrazino[1,2-*a*]quino xalin-3-yl)propane-1,2-diol | 610 |

### ¹H NMR data of the compound prepared in Examples 36 to 42 were as follows:

**Example 36:** ¹H NMR (500 MHz, CDCl₃) δ 7.86 (s, 1H), 7.83 (d, *J* = 7.9 Hz, 1H), 7.79-7.74 (m, 2H), 7.67-7.58 (m, 3H), 7.36-7.29 (m, 2H), 7.00 (d, *J* = 16.4 Hz, 1H), 6.83 (d, *J* = 16.6 Hz, 1H), 6.73 (d, *J* = 8.6 Hz, 1H), 4.23 (dd, *J* = 14.2, 4.1 Hz, 1H), 3.84-3.76 (m , 2H), 3.57 (d, *J* = 12.2 Hz, 1H), 3.52 (dd, *J* = 11.4, 4.2 Hz, 1H), 3.36 (dd, *J* = 14.1, 10.1 Hz, 1H), 2.97 (d, *J* = 11.3 Hz, 1H), 2.77 (d, 7 = 11.6 Hz, 2H), 2.60 (t, *J* = 11.1 Hz, 1H), 2.45 (t, *J* = 11.4 Hz, 1H), 2.39-2.34 (m, 1H), 2.05 (dt, *J* = 21.2, 11.0 Hz, 2H).
**Example 37:** ¹H NMR (500 MHz, CDCl₃): δ 7.83 (s, 1H), 7.80 (d, J = 8.0 Hz, 1H), 7.76-7.66 (m, 2H), 7.58 (t, J = 7.8 Hz, 1H), 7.25-7.10 (m, 3H), 7.07-6.97 (m, 1H), 6.71 (d, J = 8.5 Hz, 1H), 6.36 (d, J = 1.6 Hz, 1H), 4.19 (dd, J = 14.2, 4.3 Hz, 1H), 3.83-3.71 (m, 2H), 3.61-3.44 (m, 2H), 3.35 (dd, J = 14.2, 9.9 Hz, 1H), 2.94 (d, J = 11.4 Hz, 1H), 2.78-2.60 (m, 2H), 2.57 (dd, J = 12.5, 9.7 Hz, 1H), 2.45-2.26 (m, 2H), 2.21 (d, J = 1.5 Hz, 3H), 2.08-1.92 (m, 2H).
**Example 38:** ¹H NMR (500 MHz, CDCl₃): δ 7.83 (s, 1H), 7.80 (d, *J* = 8.0 Hz, 1H), 7.76-7.70 (m, 2H), 7.58 (t, *J* = 7.8 Hz, 1H), 7.25-7.14 (m, 3H), 7.07-6.97 (m, 1H), 6.71 (d, *J* = 8.9 Hz, 1H), 6.36 (d, *J* = 1.7 Hz, 1H), 4.18 (dd, *J* = 14.1, 4.4 Hz, 1H), 3.84 - 3.68 (m, 2H), 3.57-3.43 (m, 2H), 3.35 (dd, *J* = 14.2, 10.0 Hz, 1H), 2.85 (d, *J* = 11.1 Hz,1H), 2.76 (d, *J* = 11.1 Hz, 1H), 2.65-2.50 (m, 2H), 2.43 (td, *J* = 12.0, 3.0 Hz, 1H), 2.36-2.23 (m, 2H), 2.21 (d, *J* = 1.5 Hz, 3H), 1.72 (t, *J* = 10.6 Hz, 1H).
**Example 39:** ¹H NMR (400 MHz, Methanol-*d*₄) δ 8.07 (d, *J* = 7.9 Hz, 1H), 8.01 (d, *J* = 7.9 Hz, 1H), 7.89-7.80 (m, 2H), 7.69 (d, *J* = 6.3 Hz, 1H), 7.52 (dd, *J* = 8.7, 2.2 Hz, 1H), 7.21 (d, *J* = 9.7 Hz, 1H), 7.17 (s, 1H), 6.97 (t, 1H), 7.06 (d, *J* = 8.7 Hz, 1H), 6.93 (dt, *J* = 9.5, 2.3 Hz, 1H), 4.48 (ddd, *J* = 14.4, 6.9, 4.3 Hz, 1H), 4.09 (td, *J* = 12.1, 10.8, 6.0 Hz, 2H), 3.74-3.63 (m, 2H), 3.58 (tt, *J* = 11.3, 6.1 Hz, 2H), 3.50-3.41 (m, 1H), 3.31-3.23 (m, 2H), 3.23-3.07 (m, 2H), 2.94-2.82 (m, 1H), 2.82-2.69 (m, 1H).
**Example 40:** ¹H NMR (400 MHz, Methanol-*d*₄) δ 8.06 (d, *J* = 7.9 Hz, 1H), 7.99 (d, *J* = 7.9 Hz, 1H), 7.87-7.79 (m, 2H), 7.64 (d, *J* = 5.3 Hz, 1H), 7.50 (dd, *J* = 8.7, 2.3 Hz, 1H), 7.21-7.14 (m, 2H), 7.05 (d, *J* = 8.8 Hz, 1H), 6.95 (t, 1H), 6.90 (dt, *J* = 9.5, 2.3 Hz, 1H), 4.47 (ddd, *J* = 13.2, 8.4, 4.3 Hz, 1H), 4.13-4.02 (m, 2H), 3.69 (t, *J* = 14.1 Hz, 2H), 3.56 (qd, *J* = 11.3, 5.0 Hz, 2H), 3.43 (ddd, *J* = 13.2, 9.9, 2.9 Hz, 1H), 3.24 (dd, *J =* 13.6, 3.6 Hz, 2H), 3.20-3.06 (m, 2H), 2.93-2.69 (m, 2H).
**Example 41:** ¹H NMR (500 MHz, CDCl₃) δ 7.83-7.69 (m, 3H), 7.65-7.55 (m, 2H), 7.20 (dd, *J* = 8.7, 2.2 Hz, 1H), 6.67 (d, *J* = 8.8 Hz, 1H), 5.96 (s, 1H), 4.16 (dd, *J* = 14.2, 4.4 Hz, 1H), 3.84-3.73 (m, 2H), 3.53-3.45 (m, 2H), 3.34 (dd, *J* = 14.2, 9.9 Hz, 1H), 2.81 (dd, *J* = 51.6, 10.9 Hz, 2H), 2.64-2.51 (m, 2H), 2.42 (d, *J* = 12.2 Hz, 1H), 2.29 (d, *J* = 19.8 Hz, 3H), 1.71 (t, *J* = 10.5 Hz, 2H), 1.32 (d, *J* = 11.6 Hz, 12H).
**Example 42:** ¹H NMR (500 MHz, CDCl₃) δ 7.79 (d, *J* = 8.0 Hz, 1H), 7.74 (d, *J* = 7.9 Hz, 1H), 7.68 (s, 1H), 7.60 (t, *J* = 7.9 Hz, 1H), 7.49 (d, *J* = 2.2 Hz, 1H), 7.12 (dd, *J* = 8.6, 2.2 Hz, 1H), 6.60 (d, *J* = 8.8 Hz, 1H), 5.88 (d, *J* = 1.4 Hz, 1H), 4.13 (dd, *J* = 14.1, 4.2 Hz, 1H), 3.96 (s, 1H), 3.68 (dd, *J* = 11.5, 4.1 Hz, 1H), 3.58-3.44 (m, 2H), 3.35 (dd, *J* = 14.2, 9.0 Hz, 1H), 3.23-2.93 (m, 3H), 2.84-2.48 (m, 3H), 2.33-2.13 (m, 4H), 1.25 (d, *J* = 12.0 Hz, 12H).

### Example 43: preparation of ethyl (S,E)-2-(8-(2-(2-chloro-6-fluorophenyl)prop-1-en-1-yl)-6 -((3-(trifluoromethyl)phenyl)sulfonyl)-1,2,4,4a,5,6-hexahydro-3H-pyrazino[1,2-a]quinoxalin-3-yl)acetate

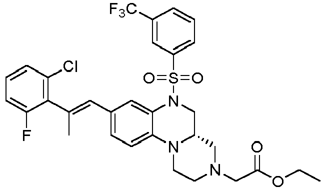

(*S*,*E*)-8-(2-(2-chloro-6-fluorophenyl)prop-1-en-1-yl)-6-((3-(trifluoromethyl)phenyl)sulfonyl)-2,3,4,4a,5,6-hexahydro-1*H*-pyrazino[1,2-a]quinoxaline (40 mg, 0.71 mmol) was dissolved in acetonitrile (3 mL). Potassium carbonate (29 mg, 0.212 mmol) and ethyl bromoacetate (18 mg, 0.106 mmol) were added thereto. The reaction mixture was reacted in a microwave re actor at 60 °C for 12 hrs. The reaction mixture was concentrated and separated by a rapi d silica gel column to obtain ethyl (*S*,*E*)-2-(8-(2-(2-chloro-6-fluorophenyl)prop-1-en-1-yl)-6-((3-(trifluoromethyl)phenyl)sulfonyl)-1,2,4,4a,5,6-hexahydro-3*H*-pyrazino[1,2-*a*]quinoxalin-3-yl)a cetate (10.5 mg, 23%). ESI-MS: 652 [M+H]⁺.
¹H NMR (400 MHz, CDCl₃) δ 7.74 -7.69 (m, 2H), 7.69 -7.63 (m, 2H), 7.52 (t, *J* = 7.8 Hz, 1H), 7.11 (ddd, *J* = 13.7, 9.5, 5.5 Hz, 3H), 6.95 (t, *J* = 8.2 Hz, 1H), 6.63 (d, *J* = 8.6 Hz, 1H), 6.29 (s, 1H), 4.11(q, *J* = 7.4 Hz, 3H), 3.42 (d, *J* = 12.2 Hz, 1H), 3.29 (dd, *J* = 14.2, 9.8 Hz, 1H), 3.11 (d, *J* = 4.2 Hz, 2H),2.75 (q, *J* = 16.0, 13.3 Hz, 3H), 2.53-2.42 (m, 1H),2.18-2.14 (m, 4H), 1.86 (s, 1H), 1.20 (d, *J* = 7.2 Hz, 3H).

### Examples 44 to 48 were prepared according to the synthesis method of Example 43.

| **Example No.** | **Structural formula** | **Chemical name** | **ESI-MS:** [M+1]⁺ |
|---|---|---|---|
| **44** | 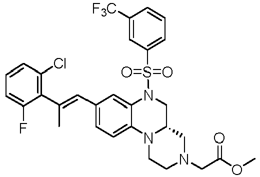 | methyl (*S*,*E*)-2-(8-(2-(2-chloro-6-fluorophenyl)prop-1-en-1-yl)-6-((3-(trifluoromethyl)phenyl)s ulfonyl)-1,2,4,4a,5,6-hexahydro -3*H*-pyrazino[1,2-*a*]quinoxalin-3-yl)acetate | 638 |
| **45** | 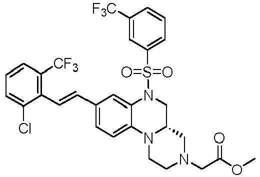 | methyl (S,*E*)-2-(8-(2-chloro-6-(trifluoromethyl)styryl)-6-((3-(t rifluoromethyl)phenyl)sulfonyl) -1,2,4,4a,5,6-hexahydro-3*H*-pyr azino[1,2-*a*] quinoxalin-3-yl)ace tate | 674 |
| **46** | 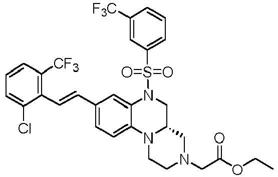 | ethyl (*S*,*E*)-2-(8-(2-chloro-6-(tr ifluoromethyl)styryl)-6-((3-(trifl uoromethyl)phenyl)sulfonyl)-1, 2,4,4a,5,6-hexahydro-3*H*-pyrazi no[1,2-*a*]quinoxalin-3-yl)acetat e | 688 |
| **47** | 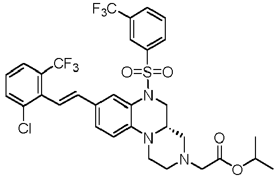 | isopropyl (*S*,*E*)-2-(8-(2-chloro-6-(trifluoromethyl)styryl)-6-((3-(trifluoromethyl)phenyl)sulfony l)-1,2,4,4a,5,6-hexahydro-3*H*-p yrazino[1,2-*a*]quinoxalin-3-yl)a cetate | 702 |
| **48** | 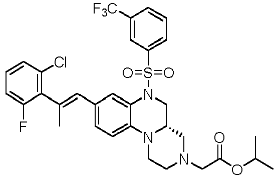 | isopropyl (*S*,*E*)-2-(8-(2-(2-chlo ro-6-fluorophenyl)prop-1-en-1-yl)-6-((3 -(trifluoromethyl)phen yl)sulfonyl)-1,2,4,4a,5,6-hexah ydro-3*H*-pyrazino[1,2-*a*]quinox alin-3-yl)acetate | 666 |

### ¹H NMR data of the compound prepared in Examples 44 to 48 were as follows:

**Example 44:** ¹H NMR¹H NMR (400 MHz, CDCl₃) δ7.76-7.62 (m, 4H), 7.52 (t, *J* = 7.8 Hz, 1H), 7.18-7.06 (m, 3H), 6.99-6.89 (m, 1H), 6.63 (d, *J* = 8.7 Hz, 1H), 6.29 (s, 1H), 4.12 (dd, *J* = 14.1, 4.5 Hz, 1H), 3.65 (s, 3H), 3.42 (d, *J* = 12.3 Hz, 1H), 3.29 (dd, *J* = 14.2, 9.7 Hz, 1H), 3.21-3.04 (m, 2H), 2.83-2.60 (m, 3H), 2.48 (td, *J* = 11.9, 2.8 Hz, 1H), 2.21-2.10 (m, 4H), 1.85 (t, *J* = 10.3 Hz, 1H).
**Example 45:** ¹H NMR (400 MHz, CDCl₃) δ 7.86-7.75 (m, 4H), 7.67-7.57 (m, 3H), 7.35-7.29 (m, 2H), 6.99 (d, *J* = 16.9 Hz, 1H), 6.82 (d, *J* = 16.5 Hz, 1H), 6.71 (d, *J* = 8.7 Hz, 1H), 4.22 (dd, *J* = 14.2, 4.4 Hz, 1H), 3.74 (s, 3H), 3.53 (d, *J* = 12.2 Hz, 1H), 3.35 (dd, *J* = 14.1, 9.9 Hz, 1H), 3.29 - 3.14 (m, 2H), 2.85 (t, *J* = 9.8 Hz, 3H), 2.64-2.52 (m, 1H), 2.32-2.19 (m, 1H), 2.02-1.87 (m, 1H).
**Example 46:** ¹H NMR (400 MHz, CDCl₃) δ 7.85-7.73 (m, 4H), 7.65-7.56 (m, 3H), 7.34-7.28 (m, 2H), 7.02-6.92 (m, 1H), 6.80 (d, *J* = 16.5 Hz, 1H), 6.70 (d, *J* = 8.7 Hz, 1H), 4.27-4.12 (m, 3H), 3.51 (d, *J* = 12.4 Hz, 1H), 3.34 (dd, *J* = 14.2, 9.9 Hz, 1H), 3.18 (d, *J* = 3.6 Hz, 2H), 2.85 (dd, *J* = 10.5, 8.1 Hz, 3H), 2.64-2.51 (m, 1H), 2.26 (dd, *J* = 11.5, 3.2 Hz, 1H), 2.02-1.87 (m, 1H), 1.28 (t, *J* = 7.1 Hz, 3H).
**Example 47:** ¹H NMR (400 MHz, CDCl₃) δ 7.85-7.72 (m, 4H), 7.64-7.56 (m, 3H), 7.31 (dd, *J* = 10.3, 3.0Hz, 2H), 6.80 (d, *J* = 16.5 Hz, 1H), 6.70 (d, *J* = 8.6 Hz, 1H), 5.05 (m, 1H), 4.21 (dd, *J* = 14.2,4.3 Hz, 1H), 3.51 (d, *J* = 12.2 Hz, 1H), 3.34 (dd, *J* = 14.2, 100 Hz, 1H), 3.15 (d, *J* = 3.3 Hz, 2H), 2.84 (s,3H), 2.64-2.50 (m, 1H), 2.24 (t, *J* = 10.6 Hz, 1H), 1.94 (t, *J* = 10.5 Hz, 1H), 1.25 (d, *J* = 6.2 Hz, 6H).
**Example 48: ¹H** NMR (400 MHz, CDCl₃) δ 7.72 (t, J = 6.8 Hz, 2H), 7.66 (s, 2H), 7.52 (t, J = 7.9 Hz, 1H), 7.17-7.07 (m, 3H), 6.95 (t, J = 8.3 Hz, 1H), 6.63 (d, J = 8.6 Hz, 1H), 6.29 (s, 1H), 4.98 (m, 1H), 4.12 (dd, *J* = 14.2, 4.6 Hz, 1H), 3.41 (d, J = 12.2 Hz, 1H), 3.28 (dd, *J* = 14.2, 9.8 Hz, 1H), 3.15-3.00 (m, 2H), 2.81-2.65 (m, 3H), 2.53-2.42 (m, 1H), 2.18-2.14 (m, 4H), 1.85 (t, J = 10.4 Hz, 1H), 1.18 (d, J = 6.2 Hz, 6H).

### Example 49: preparation of ((isopropoxycarbonyl)oxy)methyl (S,E)-2-(8-(2-(2-chloro-6-fl uorophenyl)prop-1-en-1-yl)-6-((3-(trifluoromethyl)phenyl)sulfonyl)-1,2,4,4a,5,6-hexahydro-3 H-pyrazino[1,2-a]quinoxalin-3-yl)acetate

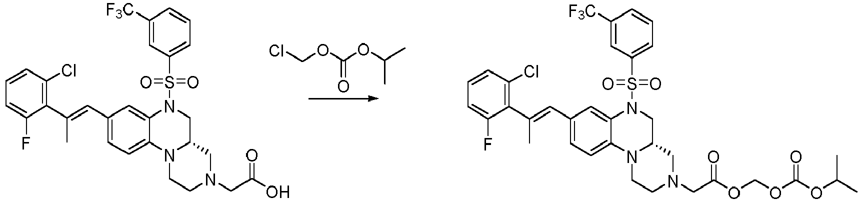

(*S*,*E*)-2-(8-(2-(2-chloro-6-fluorophenyl)prop-1-en-1-yl)-6-((3-(trifluoromethyl)phenyl)sulfon yl)-1,2,4,4a,5,6-hexahydro-3*H*-pyrazino[1,2-a]quinoxalin-3-yl)acetic acid (15 mg, 0.024 mmol) was dissolved in *N*,*N*-dimethylformamide (2 mL). Triethylamine (12 mg, 0.12 mmol) and chloromethyl isopropyl carbonate (18 mg, 0.12 mmol) were added thereto. The reaction mi xture was stirred at 60 °C for 3 hrs. The reaction mixture was separated by a rapid silica gel column to obtain ((isopropoxycarbonyl)oxy)methyl (*S*,*E*)-2-(8-(2-(2-chloro-6-fluorophenyl) prop-1-en-1-yl)-6-((3-(trifluoromethyl)phenyl)sulfonyl)-1,2,4,4a,5,6-hexahydro-3*H*-pyrazino[1,2-*a*]quinoxalin-3-yl)acetate (39.2 mg, 56%). ESI-MS: 740 [M+1]^{÷}.
¹H NMR (400 MHz, CDCl₃) δ 7.83-7.80 (m, 2H), 7.75 (d, *J* = 5.2 Hz, 2H), 7.63-7. 61 (m, 1H), 7.27-7.17 (m, 3H), 7.04 (t, *J* = 8.3 Hz, 1H), 6.72 (d, *J* = 8.6 Hz, 1H), 6.38 (s, 1H), 5.80 (s, 2H),4.98-4.92(m, 2H), 4.20 (dd, *J* = 14.2, 4.5 Hz, 1H), 3.51 (d, *J* = 12. 2 Hz, 1H), 3.37 (dd, *J* = 14.3, 9.9 Hz, 1H), 3.29 (s, 2H), 2.83 (t, *J* = 11.4 Hz, 2H), 2.7 5 (s, 1H), 2.53 (t, *J* = 11.5 Hz, 1H), 2.31 (dd, *J* = 13.5, 10.5 Hz, 1H), 2.23 (s, 3H), 2.0 1 (t, *J* = 10.3 Hz, 1H), 1.38-1.32 (d, *J* = 10 Hz, 6H).

### Example 50: preparation of ((isopropoxycarbonyl)oxy)methyl (S,E)-2-(8-(2-chloro-6-(trifl uoromethyl)styryl)-6-((3-(trifluoromethyl)phenyl)sulfonyl)-1,2,4,4a,5,6-hexahydro-3H-pyrazi no[1,2-a] quinoxalin-3-yl)acetate

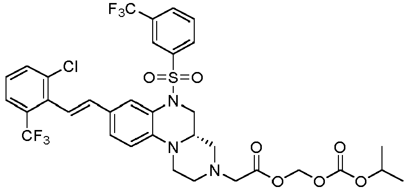

### Example 50 was prepared according to the synthesis method of Example 49: ES I-MS: 776 [M+1]^{÷}.

¹H NMR (400 MHz, CDCl₃) δ 7.84-7.73 (m, 4H), 7.62 (dd, *J* = 8.2, 3.0 Hz, 3H), 7.33-7.28 (m, 2H), 7.02-6.93 (m, 1H), 6.80 (d, *J* = 16.5 Hz, 1H), 6.69 (d, *J* = 8.7 Hz, 1 H), 5.78 (s, 2H), 4.93 (m, 1H), 4.20 (dd, *J* = 14.1, 4.4 Hz, 1H), 3.51 (d, *J* = 12.3 Hz, 1 H), 3.33 (dd, *J* = 14.1, 10.0 Hz, 1H), 3.30-3.27 (m, 2H), 2.82 (q, *J* = 10.0, 9.2 Hz, 3H), 2.54 (td, *J* = 12.0, 3.0 Hz, 1H), 2.37-2.25 (m, 1H), 2.01 (t, *J* = 10.4 Hz, 1H), 1.33 (d, *J* = 6.3 Hz, 6H).

### Example 51: preparation of tert-butyl (S)-2-(8-(3-(difluoromethoxy)-5-fluorophenyl)-6-((3-(trifluoromethyl)phenyl)sulfonyl)-1,2,4,4a,5,6-hexahydro-3H-pyrazino[1,2-a]quinoxalin-3-yl) -2-methylpropanoate

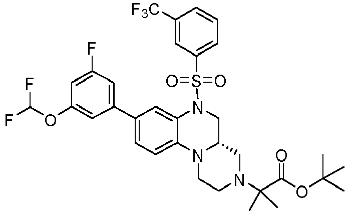

(*S*)-8-(3-(difluoromethoxy)-5-fluorophenyl)-6-((3-(trifluoromethyl)phenyl)sulfonyl)-2,3,4,4a, 5,6-hexahydro-1*H*-pyrazino[1,2-*a*]quinoxaline (110 mg, 0.20 mmol) was dissolved in acetoni trile (5 mL). Sodium iodide (120 mg, 0.80 mmol), *tert*-butyl 2-bromo-2-methylpropanoate (362 mg, 1.6 mmol) and *N*,*N*-diisopropylethylamine (80.8 mg, 0.63 mmol) were added ther eto. The reaction mixture was reacted in a microwave reactor at 120 °C for 2.5 hrs. The reaction mixture was concentrated to remove the solvent. The residue was separated by a r eversed-phase column [eluent: acetonitrile : water (0.5% HCl) = 0%-49%] to obtain *tert*-bu tyl (*S*)-2-(8-(3-(difluoromethoxy)-5-fluorophenyl)-6-((3-(trifluoromethyl)phenyl)sulfonyl)-1,2,4,4 a,5,6-hexahydro-3*H*-pyrazino[1,2-*a*]quinoxalin-3-yl)-2-methylpropanoate (26 mg, 83%). ESI-M S: 700 [M+H]⁺.
¹H NMR (400 MHz, CDCl₃) δ 8.16 (s, 1H), 7.90 (s, 1H), 7.84 (d, *J* = 7.4 Hz, 1H), 7.77 (s, 1H), 7.53 (d, *J* = 10.2 Hz, 1H), 7.38-7.34 (m, 1H), 7.14-7.07 (m, 2H), 6.84 (d, *J* = 9.3 Hz, 1H), 6.77 (s, 1H), 6.59 (t, 1H), 4.29 (d, *J* = 13.8 Hz, 1H), 4.08 (s, 1H), 3.69 (d, *J* = 10.0 Hz,1H), 3.50 (m, 5H), 3.23 (s, 1H), 1.85 (s, 6H), 1.59 (d, *J* = 16.7 Hz, 9H).

### Examples 52 to 53 were prepared according to the synthesis method of Example 51.

| **Example No.** | **Structural formula** | **Chemical name** | **ESI-MS:** [M+1]⁺ |
|---|---|---|---|
| **52** | 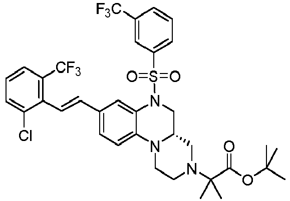 | *tert*-butyl (*S*,*E*)-2-(8-(2-chloro -6-(trifluoromethyl)styryl)-6-((3-(trifluoromethyl)phenyl)sul fonyl)-1,2,4,4a,5,6-hexahydro-3*H*-pyrazino[1,2-*a*]quinoxalin-3-yl)-2-methylpropanoate | 744 |
| **53** | 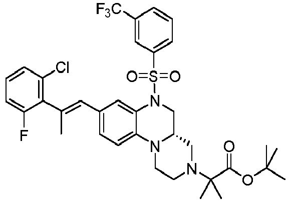 | *tert*-butyl (*S*,*E*)-2-(8-(2-(2-chl oro-6-fluorophenyl)prop-1-en-1-yl)-6-((3-(trifluoromethyl)ph enyl)sulfonyl)-1,2,4,4a,5,6-hex ahydro-3*H*-pyrazino[1,2-*a*]qui noxalin-3-yl)-2-methylpropano ate | 708 |

### ¹H NMR data of the compound prepared in Examples 52 to 53 were as follows:

**Example 52:** ¹H NMR (400 MHz, CDCl₃) δ 7.87 (s, 1H), 7.83-7.74 (m, 3H), 7.67-7.57 (m, 3H), 7.32 (dd, *J* = 8.5, 2.3 Hz, 2H), 6.99 (d, *J* = 16.6 Hz, 1H), 6.82 (d, *J* = 16.6 Hz, 1H), 6.71 (d, *J* = 8.6 Hz, 1H), 4.24 (dd, *J =* 14.0, 4.1 Hz, 1H), 3.54 (d, *J* = 11.7 Hz, 1H), 3.32 (dd, *J* = 14.0, 10.4 Hz, 1H), 2.99-2.87 (m, 2H), 2.66-2.56 (m, 1H), 2.47-2.37 (m, 1H), 2.34-2.25 (m, 1H), 1.98 (t, *J* = 10.5 Hz, 1H), 1.46 (s, 9H), 1.26 (s, 6H).
**Example 53:** ¹H NMR (400 MHz, CDCl₃) δ 7.86 (s, 1H), 7.81 (d, *J* = 7.8 Hz, 1H), 7.76-7.71 (m, 2H), 7.59 (t, *J* = 7.9 Hz, 1H), 7.26-7.15 (m, 3H), 7.07-7.01 (m, 1H), 6.72 (d, *J* = 8.6 Hz, 1H), 6.37 (s, 1H), 4.21 (dd, *J* = 14.1, 4.2 Hz, 1H), 3.52 (d, *J* = 11.5 Hz, 1H), 3.34 (dd, *J* = 14.1, 10.3 Hz, 1H), 2.91 (dd, *J* = 19.6, 11.1 Hz, 2H), 2.62-2.50 (m, 1H), 2.39 (td, *J* = 11.6, 2.8 Hz, 1H), 2.34-2.26 (m, 1H), 2.23 (d, *J* = 1.4 Hz, 3H), 1.97 (t, *J* = 10.4 Hz, 1H), 1.46 (s, 9H), 1.25 (s, 6H).

### Example 54: preparation of (S)-2-(8-(3-(difluoromethoxy)-5-fluorophenyl)-6-((3-(trifluoromethy l)phenyl)sulfonyl)-1,2,4,4a,5,6-hexahydro-3H-pyrazino[1,2-a]quinoxalin-3-yl)-2-methylpropa noic acid

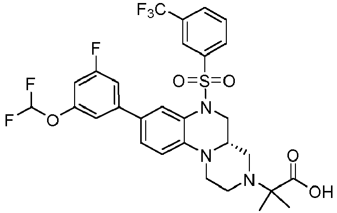

*Tert*-butyl (*S*)-2-(8-(3-(difluoromethoxy)-5-fluorophenyl)-6-((3-(trifluoromethyl)phenyl)sulf onyl)-1,2,4,4a,5,6-hexahydro-3*H*-pyrazino[1,2-*a*]quinoxalin-3-yl)-2-methylpropanoate (21 mg, 0.03 mmol) was dissolved in a solution of hydrogen chloride in dioxane (3 mL, 4M). The reaction solution was stirred at room temperature for 12 hr. The reaction mixture was co ncentrated to remove the solvent. The residue was separated by a reversed-phase column [eluent: acetonitrile : water (0.5% HCl) = 0%-46%] to obtain (*S*)-2-(8-(3-(difluoromethoxy)-5-fluorophenyl)-6-((3-(trifluoromethyl)phenyl)sulfonyl)-1,2,4,4a,5,6-hexahydro-3*H*-pyrazino[1,2-*a*]quinoxalin-3-yl)-2-methylpropanoic acid (10 mg, 52%). ESI-MS: 644 [M+H]⁺.
¹H NMR (400 MHz, Methanol-*d*₄) δ 8.02 (dd, *J* = 15.4, 7.9 Hz, 2H), 7.85-7.78 (m, 2H), 7.69 (s, 1H), 7.51 (dd, *J* = 8.7, 2.2 Hz, 1H), 7.22-7.15 (m, 2H), 6.95 (t, 1H), 7.04 (d, *J* = 8.8 Hz, 1H), 6.91 (d, *J* = 9.5 Hz, 1H), 4.47 (dd, *J* = 14.4, 4.2 Hz, 1H), 4.09 (d, *J* = 13.9 Hz, 1H), 3.53 (t, *J* = 9.7 Hz, 2H), 3.42 (dd, *J* = 14.4, 10.0 Hz, 1H), 3.15 (d, *J* = 13.2 Hz, 2H), 2.90 (t, *J* = 11.4 Hz, 1H), 2.82 (t, *J* = 12.7 Hz, 1H), 1.60 (s, 6H).

### Examples 55 to 56 were prepared according to the synthesis method of Example 54.

| **Example No.** | **Structural formula** | **Chemical name** | **ESI-MS:** [M+1]⁺ |
|---|---|---|---|
| **55** | 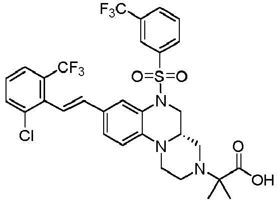 | (*S*,*E*)-2-(8-(2-chloro-6-(trifluo romethyl)styryl)-6-((3-(trifluo romethyl)phenyl)sulfonyl)-1, 2,4,4a,5, 6-hexahydro-3*H*-pyra zino[1 ,2-*a*]quinoxalin-3-yl)-2-methylpropanoic acid | 688 |
| **56** | 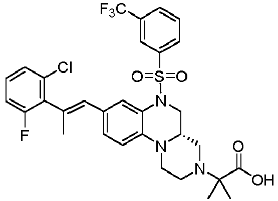 | (*S*,*E*)-2-(8-(2-(2-chloro-6-fluor ophenyl)prop-1-en-1-yl)-6-((3 -(trifluoromethyl)phenyl)sulfo nyl)-1,2,4,4a,5,6-hexahydro-3 *H*-pyrazino[1,2-*a*]quinoxalin-3-yl)-2-methylpropanoic acid | 652 |

### ¹H NMR data of the compound prepared in Examples 55 to 56 were as follows:

**Example 55:** ¹H NMR (400 MHz, Methanol-*d*₄) δ 7.92 (d, *J* = 7.9 Hz, 1H), 7.86 (d, *J* = 7.9 Hz, 1H), 7.72-7.68 (m, 1H), 7.67-7.59 (m, 3H), 7.56 (s, 1H), 7.35-7.31 (m, 1H), 7.25 (dd, *J* = 8.6, 2.0 Hz, 1H), 6.90 (dd, *J* = 16.6, 2.1 Hz, 1H), 6.81 (d, *J* = 8.7 Hz, 1H), 6.67 (d, *J* = 16.5 Hz, 1H), 4.32 (dd, *J* = 14.3, 4.3 Hz, 1H), 3.81 (d, *J=* 13.2 Hz, 1H), 3.29 (dd, *J=* 14.3, 10.0 Hz, 2H), 3.12 (d, *J* = 12.1 Hz, 1H), 2.95 (s, 1H), 2.77-2.69 (m, 1H), 2.62-2.54 (m, 1H), 2.45 (t, *J* = 11.1 Hz, 1H), 1.31 (s, 6H).
**Example 56:** ¹H NMR (400 MHz, Methanol-*d*₄) δ 7.96 (d, *J* = 7.9 Hz, 1H), 7.90 (d, *J* = 7.9 Hz, 1H), 7.73 (t, *J* = 7.9 Hz, 1H), 7.62-7.55 (m, 2H), 7.19 (q, *J* = 3.6 Hz, 2H), 7.11 (dd, *J* = 8.6, 2.0 Hz, 1H), 7.02 (td, *J* = 5.9, 3.0 Hz, 1H), 6.86 (d, *J* = 8.7 Hz, 1H), 6.23 (s, 1H), 4.37 (dd, *J* = 14.3, 4.2 Hz, 1H), 3.96 (d, *J* = 14.4 Hz, 1H), 3.43 (t, *J* = 10.5 Hz, 2H), 3.34 (dd, *J* = 14.4, 9.9 Hz, 1H), 3.14-3.01 (m, 2H), 2.85-2.66 (m, 2H), 2.06 (d, *J* = 1.4 Hz, 3H), 1.51 (d, *J* = 2.2 Hz, 6H).

### Example 57: preparation of ethyl (S,E)-2-(8-(2-(2-chloro-6-fluorophenyl)prop-1-en-1-yl)-6 -((3-(trifluoromethyl)phenyl)sulfonyl)-1,2,4,4a,5,6-hexahydro-3H-pyrazino[1,2-a]quinoxalin-3-yl)-2-oxoacetate

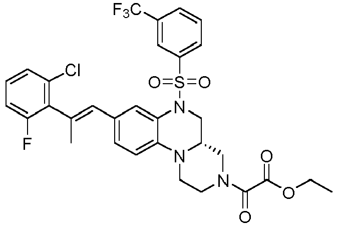

(*S*,*E*)-8-(2-(2-chloro-6-fluorophenyl)prop-1-en-1-yl)-6-((3-(trifluoromethyl)phenyl)sulfonyl)-2,3,4,4a,5,6-hexahydro-1*H*-pyrazino[1,2-*a*]quinoxaline (40 mg, 0.071 mmol) was dissolved in dichloromethane (3 mL). Triethylamine (22 mg, 0.212 mmol) and oxalyl chloride monoeth yl ester (15 mg, 0.106 mmol) were added to the solution at 0 °C. The reaction mixture w as stirred to room temperature and reacted overnight. The mixture solution was concentrate d and separated by a reversed-phase column to obtain ethyl (*S*,*E*)-2-(8-(2-(2-chloro-6-fluoro phenyl)prop-1-en-1-yl)-6-((3-(trifluoromethyl)phenyl)sulfonyl)-1,2,4,4a,5,6-hexahydro-3*H*-pyrazi no[1,2-*a*]quinoxalin-3-yl)-2-oxoacetate (24.6 mg, 52%). ESI-MS: 666.2 [M+H]⁺.
¹H NMR (400 MHz, CDCl₃) δ 7.92-7.58 (m, 5H), 7.24-7.15 (m, 3H), 7.02 (t, J = 8.6 Hz, 1H), 6.73 (dd, J = 19.3, 8.6 Hz, 1H), 6.36 (s, 1H), 4.44-4.19 (m, 4H), 3.64 (dt, J = 21.8, 11.8 Hz, 2H), 3.37 (td, J = 13.9, 9.7 Hz, 1H), 3.17 (t, J = 12.0 Hz, 1H), 2.88-2.60 (m, 2H), 2.39 (dt, J = 33.4, 11.4 Hz, 2H), 2.20 (s, 3H), 1.39 (dt, J = 14.8, 7.0 Hz, 3H).

### Example 58: preparation of ethyl (S,E)-2-(8-(2-chloro-6-(trifluoromethyl)styryl)-6-((3-(trif luoromethyl)phenyl)sulfonyl)-1,2,4,4a,5,6-hexahydro-3H-pyrazino[1,2-a]quinoxalin-3-yl)-2-o xoacetate

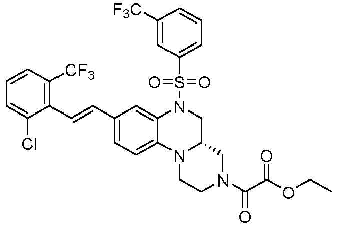

**Example 58 was prepared according to the synthesis method of Example 57:** ESI-MS: 702.2 [M+H]⁺.
¹H NMR (400 MHz, CDCl₃) δ 7.90 (d, *J* = 8.0 Hz, 0.5H), 7.86-7.79 (m, 2H), 7.77-7.70 (m, 1.5H), 7.67-7.61 (m, 3H), 7.38-7.31 (m, 2H), 6.99 (d, *J=* 13.7 Hz, 1H), 6.85-6.70 (m, 2H), 4.46-4.24 (m, 4H), 3.73-3.60 (m, 2H), 3.43-3.30 (m, 1H), 3.19 (d, *J* = 11.1 Hz, 0.5H), 2.91-2.69 (m, 2H), 2.44 (dd, *J* = 24.2, 11.7 Hz, 1.5H), 1.41 (dt, *J* = 15.5, 7.2 Hz, 3H).

### Example 59: preparation of (S,E)-2-(8-(2-chloro-6-(trifluoromethyl)styryl)-6-((3-(trifluoro methyl)phenyl)sulfonyl)-1,2,4,4a,5,6-hexahydro-3H-pyrazino[1,2-a]quinoxalin-3-yl)acetamid e

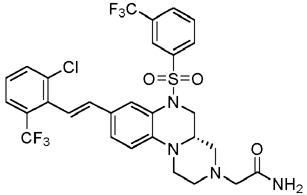

Methyl (*S*,*E*)-2-(8-(2-chloro-6-(trifluoromethyl)styryl)-6-((3-(trifluoromethyl)phenyl)sulfon yl)-1,2,4,4a,5,6-hexahydro-3*H*-pyrazino[1,2-*a*]quinoxalin-3-yl)acetate (150 mg, 0.23 mmol) wa s dissolved in a solution of ammonia in methanol (3 mL, 7N). The reaction mixture was heated to 85 °C in a sealed vessel and stirred for 48 hrs. The reaction mixture was conce ntrated to remove the solvent. The residue was separated by a rapid silica gel column to obtain (*S*,*E*)-2-(8-(2-chloro-6-(trifluoromethyl)styryl)-6-((3-(trifluoromethyl)phenyl)sulfonyl)-1,2, 4,4a,5,6-hexahydro-3*H*-pyrazino[1,2-*a*]quinoxalin-3-yl)acetamide (38.5 mg, 84%). ESI-MS: 65 9 [M+1]⁺.
¹HNMR (400 MHz, Methanol-*d*₄) δ 7.95 (d, *J=* 7.8 Hz, 1H), 7.92-7.87 (m, 1H), 7.79-7.70 (m, 5H), 7.46-7.42 (m , 1H), 7.34 (dd, *J* = 8.7, 2.1 Hz, 1H), 7.01 (dd, *J* = 16.5, 2.0 Hz, 1H), 6.86 (d, *J* = 8.7 Hz, 1H), 6.79 (d, *J* = 16.7 Hz, 1H), 4.31 (dd, *J* = 14.3, 4.5 Hz, 1H), 3.64 (d, *J* = 12.5 Hz, 1H), 3.41-3.34 (m, 1H), 3.01 (d, *J* = 2.5 Hz, 2H), 2.85 (ddt, *J* = 9.7, 5.1, 2.2 Hz, 2H), 2.77 (ddt, *J* = 10.2, 5.7, 2.8 Hz, 1H), 2.46 (td, *J* = 12.2, 3.0 Hz, 1H), 2.16 (td, *J* = 11.6, 3.1 Hz, 1H), 1.87 (t, *J* = 10.6 Hz, 1H).

### Example 60: preparation of (S,E)-8-(2-chloro-6-(trifluoromethyl)styryl)-6-((3-(trifluoromethy l)phenyl)sulfonyl)-1,2,4,4a,5,6-hexahydro-3H-pyrazino[1,2-a]quinoxalin-3-carboxamide

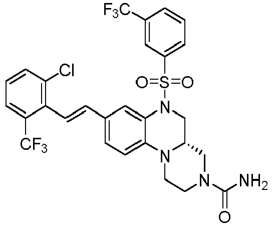

(*S*,*E*)-8-(2-chloro-6-(trifluoromethyl)styryl)-6-((3-(trifluoromethyl)phenyl)sulfonyl)-2,3,4,4a, 5,6-hexahydro-1*H*-pyrazino[1,2-*a*]quinoxaline (50 mg, 0.083 mmol) and isocyanatotrimethylsi lane (50 mg, 0.083 mmol) were heated to 90 °C and stirred for 2 hrs. The reaction was quenched with methanol. The mixture solution was separated by a rapid reversed-phase col umn to obtain (*S*,*E*)-8-(2-chloro-6-(trifluoromethyl)styryl)-6-((3-(trifluoromethyl)phenyl)sulfony l)-1,2,4,4a,5,6-hexahydro-3*H*-pyrazino[1,2-*a*]quinoxaline-3-carboxamide (24.2 mg, 14%). ESI-MS: 645 [M+1]⁺.
¹H NMR (400 MHz, CDCl₃) δ 7.82-7.64 (m, 3H), 7.72 (d, *J* = 2.0 Hz, 1H), 7.64 (dd,J = 8.1, 2.5 Hz, 3H), 7.37-7.28 (m, 2H), 7.03-6.94 (m, 1H), 6.81 (d, *J* = 16.5 Hz, 1H), 6.72 (d, *J* = 8.7Hz, 1H), 4.50 (s, 2H), 4.28 (dd, *J* = 14.2, 4.5 Hz, 1H), 3.79 (dd, *J* = 27.1, 12.6 Hz, 2H), 3.64-3.53 (m,1H), 3.33 (dd, *J* = 14.3, 10.0 Hz, 1H), 3.08-2.93 (m, 1H), 2.75 (dd, *J* = 8.5, 5.1 Hz, 1H), 2.59 (dd, *J* = 12.4, 10.7 Hz, 1H), 2.48 (td, *J* = 11.8, 3.4 Hz, 1H).

### Examples 61 to 62 were prepared according to the synthesis method of Example 60.

| **Example No.** | **Structural formula** | **Chemical name** | **ESI-MS:** [M+1]⁺ |
|---|---|---|---|
| **61** | 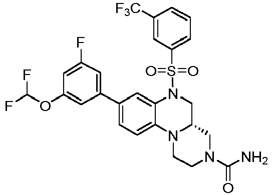 | (*S*)-8-(3-(difluoromethoxy)-5 -fluorophenyl)-6-((3 -(trifluor omethyl)phenyl)sulfonyl)-1, 2,4,4a, 5, 6-hexahydro-3*H*-pyr azino[1,2-*a*] quinoxaline-3 -ca rboxamide | 601 |
| **62** | 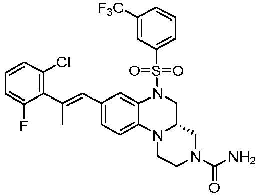 | (*S*,*E*)-8-(2-(2-chloro-6-fluoro phenyl)prop-1-en-1-yl)-6-((3 -(trifluoromethyl)phenyl)sulf onyl)-1,2,4,4a,5,6-hexahydro -3*H*-pyrazino[1,2-*a*]quinoxal ine-3-carboxamide | 609 |

### ¹H NMR data of the compound prepared in Examples 61 to 62 were as follows:

**Example 61**: ¹H NMR (400 MHz, Methanol-*d*₄) δ 7.93 (d, *J=* 7.9 Hz, 1H), 7.87 (d, *J=* 8.0 Hz, 1H), 7.83 (d, *J* = 2.3 Hz, 1H), 7.74 (t, *J* = 7.9 Hz, 1H), 7.64 (s, 1H), 7.48 (dd, *J* = 8.7, 2.3 Hz, 1H), 7.21 (dt, *J* = 9.8, 1.9 Hz, 1H), 7.18 (d, *J* = 1.9 Hz, 1H), 6.95 (t, 1H), 6.93 (d, *J* = 8.8 Hz, 1H), 6.88 (dt, *J* = 9.5, 2.3 Hz, 1H), 4.36 (dd, *J=* 14.5, 3.6 Hz, 1H), 3.92-3.82 (m, 2H), 3.68 (dt, *J=* 12.6, 3.1 Hz, 1H), 3.38-3.32 (m, 1H), 2.89 (td, *J* = 13.0, 12.4, 3.4 Hz, 1H), 2.60-2.49 (m, 2H), 2.26 (td, *J* = 12.0, 3.3 Hz, 1H).
**Example 62**: ¹HNMR (400 MHz, CDCl₃) δ 7.81 (t, *J* = 6.2 Hz, 2H), 7.76 (s, 1H), 7.68 (d, *J* = 1.9 Hz, 1H), 7.61 (t, *J* = 7.9 Hz, 1H), 7.25-7.14 (m, 3H), 7.02 (t, *J* = 8.5 Hz, 1H), 6.72 (d, *J* = 8.6 Hz, 1H), 6.36 (s, 1H), 4.50 (s, 2H), 4.25 (dd, *J* = 14.3, 4.5 Hz, 1H), 3.77 (dd, *J* = 23.3, 12.6 Hz, 2H), 3.56 (dt, *J* = 12.5, 3.3 Hz, 1H), 3.33 (dd, *J* = 14.3, 9.8 Hz, 1H), 3.04-2.90 (m, 1H), 2.68 (d, *J* = 10.0 Hz, 1H), 2.56 (t, *J* = 11.5 Hz, 1H), 2.43 (td, *J* = 11.8, 3.3 Hz, 1H), 2.20 (s, 3H).

### Example 63: preparation of (S,E)-8-(2-(2-chloro-6-fluorophenyl)prop-1-en-1-yl)-6-((3-(trifl uoromethyl)phenyl)sulfonyl)-1,2,4,4a,5,6-hexahydro-3H-pyrazino[1,2-a]quinoxaline-3-carbot hioamide

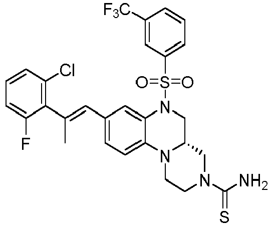

(*S*,*E*)-8-(2-(2-chloro-6-fluorophenyl)prop-1-en-1-yl)-6-((3-(trifluoromethyl)phenyl)sulfonyl)-2,3,4,4 a,5,6-hexahydro-1*H*-pyrazino[1,2-*a*]quinoxaline (50 mg, 0.088 mmol), isothiocyanatotrimethylsilane (0.5 mL) were heated to 90 °C and stirred for 2 hrs, and the reaction was quenched with methanol. The mixture solution was separated by a rapid reversed-phase column to obtain (*S*,*E*)-8-(2-(2-chloro-6-fluorophenyl)prop-1-en-1-yl)-6-((3-(trifluoromethyl)phenyl)sulfonyl)-1,2,4, 4a,5,6-hexahydro-3*H*-pyrazino[1,2-*a*]quinoxaline-3-carbothioamide (10.3 mg, 18.7%). ESI-MS: 625 [M+1]⁺.
¹HNMR (400 MHz, CDCl₃) δ 7.84 (dd, *J* = 19.9, 7.9 Hz, 2H), 7.76 (s, 1H), 7.65-7.62 (m, 2H), 7.26-7.21 (m, 3H), 7.02 (t, *J* = 8.5 Hz, 1H), 6.69 (d, *J* = 8.6 Hz, 1H), 6.36 (s, 1H), 5.75 (s, 2H), 4.38 (d, *J* = 11.8 Hz, 1H), 4.29 (dd, *J* = 14.2, 4.3 Hz, 1H), 4.16 (d, *J* = 13.3 Hz, 1H), 3.59 (dd, *J* = 10.7, 6.2 Hz, 1H), 3.47 (t, *J* = 12.2 Hz, 1H), 3.32 (dd, *J* = 14.2, 9.5 Hz, 1H), 3.01-2.84 (m, 2H), 2.75-2.61 (m, 1H), 2.19 (s, 3H).

### Examples 64 to 65 were prepared according to the synthesis method of Example 63.

| **Example No.** | **Structural formula** | **Chemical name** | **ESI-MS:** [M+1]⁺ |
|---|---|---|---|
| **64** | 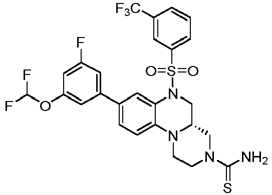 | (*S*)-8-(3-(difluoromethoxy)-5 - fluorophenyl)-6-((3-(trifluoro methyl)phenyl)sulfonyl)-1,2,4, 4a,5,6-hexahydro-3*H*-pyrazin o[1,2-*a*]quinoxaline-3-carboth ioamide | 617 |
| **65** | 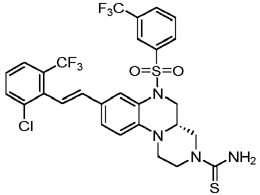 | (*S*,*E*)-8-(2-chloro-6-(trifluoro methyl)styryl)-6-((3-(trifluoro methyl)phenyl)sulfonyl)-1,2,4, 4a,5,6-hexahydro-3*H*-pyrazin o[1,2-*a*]quinoxaline-3-carboth ioamide | 661 |

### ¹H NMR data of the compound prepared in Examples 64 to 65 were as follows:

**Example 64**: ¹H NMR (400 MHz, Methanol-*d*₄) δ 7.92 (t, *J* = 8.1 Hz, 2H), 7.84 (d, *J* = 2.3 Hz, 1H), 7.75 (t, *J* = 7.9 Hz, 1H), 7.60 (s, 1H), 7.49 (dd, *J* = 8.7, 2.3 Hz, 1H), 7.24-7.16 (m, 2H), 6.95 (t, 1H), 6.94-6.85 (m, 2H), 4.49 (d, *J* = 20.2 Hz, 1H), 4.37 (dd, *J* = 14.4, 4.3 Hz, 2H), 3.67 (dt, *J* = 12.5, 3.7 Hz, 1H), 3.35 (d, *J* = 10.3 Hz, 1H), 3.28-3.21 (m, 1H), 2.83-2.74 (m, 1H), 2.69 (tt, *J* = 10.8, 3.7 Hz, 1H), 2.41 (ddd, *J* = 13.3, 10.6, 3.4 Hz, 1H).
**Example 65:** ¹HNMR (400 MHz, CDCl₃) δ 7.81 (d, *J=* 7.9 Hz, 1H), 7.78-7.69 (m, 2H), 7.63-7.52 (m, 4H), 7.31-7.21 (m, 2H), 6.94-6.84 (m, 1H), 6.73 (d, *J* = 16.5 Hz, 1H), 6.61 (d, *J* = 8.6 Hz, 1H), 5.66 (s, 2H), 4.31 (d, *J* = 12.4 Hz, 1H), 4.24 (dd, *J* = 14.2, 4.4 Hz, 1H), 4.08 (d, *J* = 13.3 Hz, 1H), 3.54 (dt, *J* = 12.3, 4.4 Hz, 1H), 3.45 (t, *J* = 11.3 Hz, 1H), 3.23 (dd, *J* = 14.2, 9.8 Hz, 1H), 3.01-2.90 (m, 1H), 2.84 (t, *J* = 11.6 Hz, 1H), 2.71-2.59 (m, 1H).

### Example 66: preparation of (R,E)-8-(2-(2-chloro-6-fluorophenyl)prop-1-en-1-yl)-6-((3-(trif luoromethyl)phenyl)sulfonyl)-1,2,4,4a,5,6-hexahydro-3H-pyrazino[1,2-a]quinoxaline-3-sulfo namide

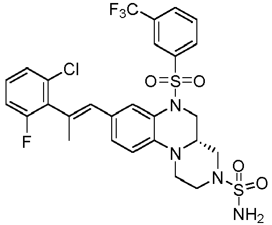

(*S*,*E*)-8-(2-(2-chloro-6-fluorophenyl)prop-1-en-1-yl)-6-((3-(trifluoromethyl)phenyl)sulfonyl) -2,3,4,4a,5,6-hexahydro-1*H*-pyrazino[1,2-*a*]quinoxaline (40 mg, 0.071 mmol) was dissolved i n ethylene glycol dimethyl ether (3 mL). Sulfonamide (40 mg, 0.417 mmol) was added th ereto. The reaction mixture was heated to 85 °C and stirred overnight, then concentrated t o remove the solvent. The residue was separated by a rapid silica gel column to obtain (*R*,*E*)-8-(2-(2-chloro-6-fluorophenyl)prop-1-en-1-yl)-6-((3-(trifluoromethyl)phenyl)sulfonyl)-1,2, 4,4a,5,6-hexahydro-3*H*-pyrazino[1,2-*a*]quinoxaline-3-sulfonamide (8.8 mg, 19%). ESI-MS: 64 5.2 [M+1]⁺.
¹H NMR (400 MHz, CDCl₃) δ 7.82 (d, *J* = 7.9 Hz, 2H), 7.75 (s, 1H), 7.69 (s, 1H), 7.63 (s, 1H), 7.20 (dd, *J* = 12.3, 4.5 Hz, 3H), 7.02 (t, *J* = 8.7 Hz, 1H), 6.74 (d, *J* = 8.6 Hz, 1H), 6.36 (s, 1H), 4.36 (s, 2H), 4.23 (dd, *J* = 14.3, 4.6 Hz, 1H), 3.71-3.50 (m, 3H), 3.40 (dd, *J* = 14.3, 9.6 Hz, 1H), 2.80-2.70 (m, 1H), 2.65 (t, *J* = 10.7 Hz, 1H), 2.57-2.44 (m, 1H), 2.35 (t, *J* = 10.8 Hz, 1H), 2.20 (s, 3H).

### Examples 67 to 68 were prepared according to the synthesis method of Example 66.

| **Example No.** | **Structural formula** | **Chemical name** | **ESI-MS: [M+1]⁺** |
|---|---|---|---|
| **67** | 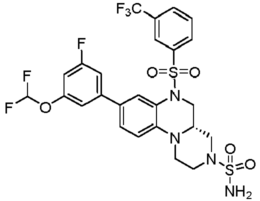 | (*R*)-8-(3-(difluoromethoxy)-5-fl uorophenyl)-6-((3-(trifluoromet hyl)phenyl)sulfonyl)-1,2,4,4a,5, 6-hexahydro-3*H*-pyrazino[1,2-*a*]quinoxaline-3-sulfonamide | ND |
| **68** | 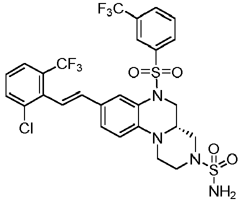 | (*R*,*E*)-8-(2-chloro-6-(trifluorom ethyl)styryl)-6-((3-(trifluoromet hyl)phenyl)sulfonyl)-1,2,4,4a,5, 6-hexahydro-3*H*-pyrazino[1,2-*a*]quinoxaline-3-sulfonamide | 681 |

### ¹H NMR data of the compound prepared in Examples 67 to 68 were as follows:

**Example 67**: ¹H NMR (400 MHz, Methanol-*d*₄) δ 8.00 (s, 1H), 7.90-7.81 (m, 2H), 7.77-7.65 (m, 2H), 7.49 (dd, *J* = 8.7, 2.3 Hz, 1H), 7.24-7.16 (m, 2H), 6.95 (t, 1H), 6.98 (dd, *J* = 8.8, 2.8 Hz, 1H), 6.89 (dt, *J* = 9.4, 2.3 Hz, 1H),4.42 (dt, *J* = 14.7, 4.0 Hz, 1H), 4.22 (t, *J* = 10.9 Hz, 1H), 3.80 (t, *J* = 11.4 Hz, 1H), 3.67 (dd, *J* = 21.4, 12.9 Hz, 1H), 3.43-3.34 (m, 3H), 2.86-2.67 (m, 1H), 2.47-2.36 (m, 1H).
**Example 68**: ¹H NMR (400 MHz, CDCl₃) δ 7.82 (d, *J* = 8.9 Hz, 2H), 7.79-7.70 (m, 2H), 7.66 -7.59 (m, 3H), 7.36-7.29 (m, 2H), 6.98 (dd, *J* = 15.2, 3.0 Hz, 1H), 6.84-6.70 (m, 2H), 4.36 (s, 2H), 4.25 (dd, *J* = 14.3, 4.6 Hz, 1H), 3.72-3.53 (m, 3H), 3.39 (dd, *J* = 14.3, 9.6 Hz, 1H), 2.82 (t, *J* = 3.9 Hz, 1H), 2.74-2.62 (m, 1H), 2.59-2.49 (m, 1H), 2.36 (t, *J* = 10.9 Hz, 1H).

### Example 69: preparation of (S,E)-2-(8-(2-(2-chloro-6-fluorophenyl)prop-1-en-1-yl)-6-((3-(t rifluoromethyl)phenyl)sulfonyl)-1,2,4,4a,5,6-hexahydro-3H-pyrazino[1,2-a]quinoxalin-3-yl)p ropane-1,3-diol

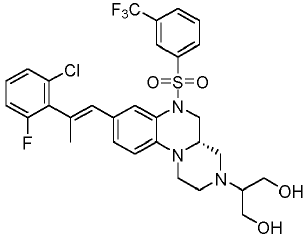

### Step 1: synthesis of (S,E)-8-(2-(2-chloro-6-fluorophenyl)prop-1-en-1-yl)-3-(2,2-dimethyl-1, 3-dioxan-5-yl)-6-((3-(trifluoromethyl)phenyl)sulfonyl)-2,3,4,4a,5,6-hexahydro-1H-pyrazino[1, 2-a]quinoxaline

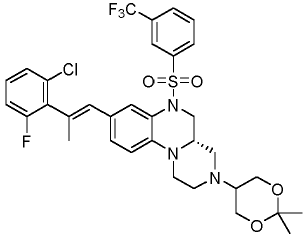

(*S*,*E*)-8-(2-chloro-6-(trifluoromethyl)styryl)-6-((3-(trifluoromethyl)phenyl)sulfonyl)-2,3,4,4a, 5,6-hexahydro-1*H*-pyrazino[1,2-*a*]quinoxaline (56 mg, 0.1 mmol) was dissolved in *N,N*-dime thylformamide (2 mL); 2,2-dimethyl-1,3-dioxan-5-one (26 mg, 0.2 mmol) and one drop of acetic acid were added to the solution. The reaction mixture was stirred at room temperatu re for 3 hrs, then sodium cyanoborohydride (7 mg, 0.12 mmol) was added. The reaction mixture was stirred at room temperature for 16 hrs. The residue was separated by a rapid reversed-phase column to obtain (*S*,*E*)-8-(2-(2-chloro-6-fluorophenyl)prop-1-en-1-yl)-3-(2,2-di methyl-1,3-dioxan-5-yl)-6-((3-(trifluoromethyl)phenyl)sulfonyl)-2,3,4,4a,5,6-hexahydro-1*H*-pyraz ino[1,2-a]quinoxaline (30 mg, 44%), which was directly used in the next step.

### Step 2: synthesis of (S,E)-2-(8-(2-(2-chloro-6-fluorophenyl)prop-1-en-1-yl)-6-((3-(trifluoro methyl)phenyl)sulfonyl)-1,2,4,4a,5,6-hexahydro-3H-pyrazino[1,2-a]quinoxalin-3-yl)propane-1,3-diol

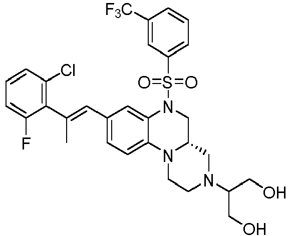

(*S*,*E*)-8-(2-(2-chloro-6-fluorophenyl)prop-1-en-1-yl)-3-(2,2-dimethyl-1,3-dioxan-5-yl)-6-((3-(trifluoromethyl)phenyl)sulfonyl)-2,3,4,4a,5,6-hexahydro-1*H*-pyrazino[1,2-*a*]quinoxaline (30 m g, 0.044 mmol) and a solution of trifluoroacetic acid (1 mL) in water (1 mL) were stirred at room temperature for 2 hrs. The reaction mixture was neutralized with a 15% sodium hydroxide aqueous solution, and separated by a rapid reversed-phase column to obtain (*S*, *E*)-2-(8-(2-(2-chloro-6-fluorophenyl)prop-1-en-1-yl)-6-((3-(trifluoromethyl)phenyl)sulfonyl)-1,2, 4,4a,5,6-hexahydro-3*H*-pyrazino[1,2-*a*]quinoxalin-3-yl)propane-1,3-diol (4.5 mg, 16%). ESI-M S: 640 [M+l]⁺.
¹H NMR (400 MHz, Methanol-*d*₄) δ 7.94 (d, *J* = 7.8 Hz, 1H), 7.92-7.86 (m, 1H), 7.76 (t, *J* = 7.9 Hz, 1H), 7.71-7.66 (m, 2H), 7.34-7.26 (m, 2H), 7.20-7.10 (m, 2H), 6.84 (d, *J* = 8.7 Hz, 1H), 6.34 (d, *J* = 1.7 Hz, 1H), 4.29 (dd, *J* = 14.3, 4.4 Hz, 1H), 3.71-3.57 (m, 5H), 3.41-3.36 (m, 1H), 2.87 (ddd, *J* = 12.9, 6.3, 3.7 Hz, 2H), 2.66-2.50 (m, 3H), 2.36 (dd, *J* = 11.9, 3.1 Hz, 1H), 2.28-2.18 (m, 4H).

### Example 70 was prepared according to the synthesis method of Example 69.

| **Example No.** | **Structural formula** | **Chemical name** | **ESI-MS:** [M+1]⁺ |
|---|---|---|---|
| **70** | 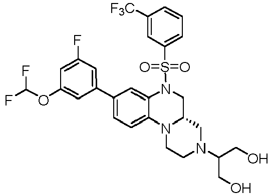 | (*S*)-2-(8-(3-(difluoromethoxy)-5 -fluorophenyl)-6-((3-(trifluorom ethyl)phenyl)sulfonyl)-1,2,4,4a, 5,6-hexahydro-3*H*-pyrazino[1,2 -*a*]quinoxalin-3-yl)propane-1,3-diol | 632 |

### ¹H NMR data of the compound prepared in Example 70 was as follows:

**Example 70**: ¹H NMR (400 MHz, Methanol-*d*₄) δ 8.05 (d, *J* = 7.9 Hz, 1H), 8.00 (d, *J* = 7.8 Hz, 1H), 7.87-7.77 (m, 2H), 7.68 (s, 1H), 7.52 (dd, *J* = 8.7, 2.3 Hz, 1H), 7.26-7.16 (m, 2H), 6.96 (t, 1H), 7.04 (d, *J* = 8.7 Hz, 1H), 6.92 (dd, *J* = 9.5, 2.3 Hz, 1H), 4.45 (dd, *J* = 14.4, 4.2 Hz, 1H), 4.02 (d, *J* = 13.8 Hz, 1H), 3.91 (d, *J* = 5.0 Hz, 4H), 3.60 (d, *J* = 11.8 Hz, 2H), 3.46 (dd, *J* = 14.4, 9.8 Hz, 1H), 3.37 (s, 1H), 3.28 (s, 1H), 3.21-3.10 (m, 1H), 3.03 (d, *J* = 11.8 Hz, 1H), 2.77 (t, *J* = 13.1 Hz, 1H).

### Example 71: preparation of (S,E)-2-(8-(2-(2-chloro-6-fluorophenyl)prop-1-en-1-yl)-6-((3-(t rifluoromethyl)phenyl)sulfonyl)-1,2,4,4a,5,6-hexahydro-3H-pyrazino[1,2-a]quinoxalin-3-yl)-2 -methylpropane-1,3-diol

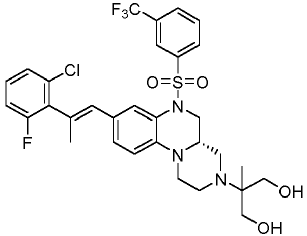

### Step 1: synthesis of dimethyl (S,E)-2-(8-(2-(2-chloro-6-fluorophenyl)prop-1-en-1-yl)-6-((3-(trifluoromethyl)phenyl)sulfonyl)-1,2,4,4a,5,6-hexahydro-3H-pyrazino[1,2-a]quinoxalin-3-yl) -2-methylmalonate

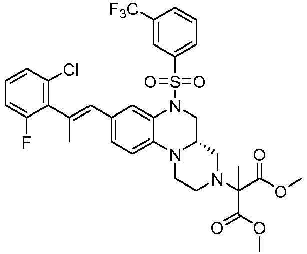

(*S*,*E*)-8-(2-(2-chloro-6-fluorophenyl)prop-1-en-1-yl)-6-((3-(trifluoromethyl)phenyl)sulfonyl)-2,3,4,4a,5,6-hexahydro-1*H*-pyrazino[1,2-*a*]quinoxaline (150 mg, 0.27 mmol) was dissolved in dimethylsulfoxide (2 mL). Diisopropylethylamine (70 mg, 0.53 mmol) and dimethyl 2-bro mo-2-methylmalonate (280 mg, 0.53 mmol) were added to the solution. The reaction mixtu re was stirred in a microwave reactor at 60 °C for 8 hrs, and then separated by a rapid reverse d-phase column to obtain dimethyl (*S*,*E*)-2-(8-(2-(2-chloro-6-fluorophenyl)prop-1-en-1-yl)-6-((3-(trifl uoromethyl)phenyl)sulfonyl)-1,2,4,4a,5,6-hexahydro-3*H*-pyrazino[1,2-*a*]quinoxalin-3-yl)-2-methyl malonate (120 mg, 60%), which was directly used in the next step.

### Step 2: synthesis of (S,E)-2-(8-(2-(2-chloro-6-fluorophenyl)prop-1-en-1-yl)-6-((3-(trifluoro methyl)phenyl)sulfonyl)-1,2,4,4a,5,6-hexahydro-3H-pyrazino[1,2-a]quinoxalin-3-yl)-2-methyl propane-1,3-diol

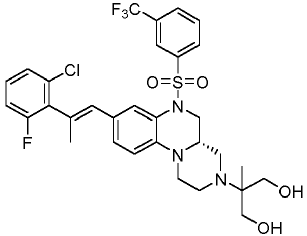

Dimethyl (*S*,*E*)-2-(8-(2-(2-chloro-6-fluorophenyl)prop-1-en-1-yl)-6-((3-(trifluoromethyl)phe nyl)sulfonyl)-1,2,4,4a,5,6-hexahydro-3*H*-pyrazino[1,2-*a*]quinoxalin-3-yl)-2-methylmalonate (120 mg, 0.16 mmol) was dissolved in tetrahydrofuran (4 mL). Lithium borohydride (30 mg, 1. 6 mmol) was added thereto. The reaction mixture was stirred at room temperature for 3 h rs. The reaction mixture was washed with water, extracted with ethyl acetate, dried, and c oncentrated. The residue was separated by a rapid reversed-phase column to obtain (*S*,*E*)-2-(8-(2-(2-chloro-6-fluorophenyl)prop-1-en-1-yl)-6-((3-(trifluoromethyl)phenyl)sulfonyl)-1,2,4,4a,5, 6-hexahydro-3*H*-pyrazino[1,2-*a*]quinoxalin-3-yl)-2-methylpropane-1,3-diol (10.8 mg, 10%). ES I-MS: 654 [M+l]⁺.
¹HNMR (400 MHz, Methanol-*d*₄) δ 8.01-7.80 (m, 2H), 7.82-7.64 (m, 3H), 7.38-7.23 (m, 2H), 7.23-7.08 (m, 2H), 6.83 (d, *J* = 8.7 Hz, 1H), 6.34 (d, *J* = 1.7 Hz, 1H), 4.28 (dd, *J* = 14.2, 4.4 Hz, 1H), 3.63-3.50 (m, 5H), 3.44-3.35 (m, 1H), 3.00 (d, *J* = 10.6 Hz, 2H), 2.58 (s, 1H), 2.52-2.29 (m, 2H), 2.21 (d, *J* = 1.5 Hz, 3H), 2.11 (t, *J* = 10.7 Hz, 1H), 0.98 (s, 3H).

### Example 72 was prepared according to the synthesis method of Example 71.

| **Example No.** | **Structural formula** | **Chemical name** | **ESI-MS:** [M+1]⁺ |
|---|---|---|---|
| **72** | 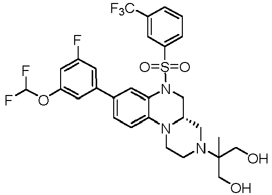 | (*S*)-2-(8-(3-(difluoromethoxy) -5-fluorophenyl)-6-((3 -(trifluo romethyl)phenyl)sulfonyl)-1, 2,4,4a,5,6-hexahydro-3*H*-pyra zino[1,2-*a* ]quinoxalin-3-yl)-2-methylpropane-1,3-diol | 646 |

### ¹H NMR data of the compound prepared in Example 72 was as follows:

**Example 72**: ¹H NMR (400 MHz, Methanol-*d*₄) δ 7.91 (dd, *J* = 12.9, 7.9 Hz, 2H), 7.83 (d, *J* = 2.2 Hz, 1H), 7.74 (t, *J=* 7.9 Hz, 1H), 7.65 (s, 1H), 7.45 (dd, *J=* 8.6, 2.3 Hz, 1H), 7.22-7.14 (m, 2H), 6.94 (t, 1H), 6.92-6.84 (m, 2H), 4.31 (dd, *J* = 14.3, 4.2 Hz, 1H), 3.68 (d, *J* = 11.9 Hz, 1H), 3.58 (qd, *J* = 11.6, 2.3 Hz, 4H), 3.36 (dd, *J* = 14.4, 10.1 Hz, 1H), 3.11 (d, *J* = 11.2 Hz, 2H), 2.71 (t, *J* = *9.7* Hz, 1H), 2.52 (dt, *J* = 46.4, 11.7 Hz, 2H), 2.26 (t, *J* = 10.8 Hz, 1H), 1.01 (s, 3H).

### Example 73: preparation of (S,E)-2-(8-(2-(2-chloro-6-fluorophenyl)prop-1-en-1-yl)-6-((3-(t rifluoromethyl)phenyl)sulfonyl)-1,2,4,4a,5,6-hexahydro-3H-pyrazino[1,2-a]quinoxalin-3-yl)-3 -hydroxy-2-(hydroxymethyl)propanenitrile

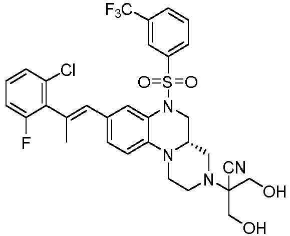

### Step 1: synthesis of (S,E)-5-(8-(2-(2-chloro-6-fluorophenyl)prop-1-en-1-yl)-6-((3-(trifluoro methyl)phenyl)sulfonyl)-1,2,4,4a,5,6-hexahydro-3H-pyrazino[1,2-a]quinoxalin-3-yl)-2,2-dime thyl-1,3-dioxane-5-carbonitrile

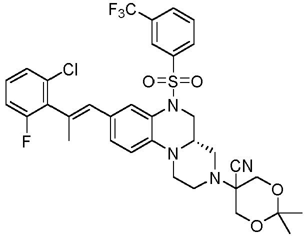

(*S*,*E*)-8-(2-chloro-6-(trifluoromethyl)styryl)-6-((3-(trifluoromethyl)phenyl)sulfonyl)-2,3,4,4a, 5,6-hexahydro-1*H*-pyrazino[1,2-*a*]quinoxaline(100 mg, 0.18 mmol) was dissolved in acetic a cid (5 mL), and the mixture solution was cooled to 0 °C; 2,2-dimethyl-1,3-dioxan-5-one (6 9 mg, 0.53 mmol) and trimethylsilanecarbonitrile (52 mg, 0.53 mmol) were added thereto and stirred at room temperature for 18 hrs. The reaction mixture was neutralized to pH>7, and then separated by a rapid reversed-phase column to obtain (*S*,*E*)-5-(8-(2-(2-chloro-6-fl uorophenyl)prop-1-en-1-yl)-6-((3-(trifluoromethyl)phenyl)sulfonyl)-1,2,4,4a,5,6-hexahydro-3*H*-py razino[1,2-*a*]quinoxalin-3-yl)-2,2-dimethyl-1,3-dioxane-5-carbonitrile (90 mg, 71%), which wa s directly used in the next step.

### Step 2: synthesis of (S,E)-2-(8-(2-(2-chloro-6-fluorophenyl)prop-1-en-1-yl)-6-((3-(trifluoro methyl)phenyl)sulfonyl)-1,2,4,4a,5,6-hexahydro-3H-pyrazino[1,2-a]quinoxalin-3-yl)-3-hydro xy-2-(hydroxymethyl)propanenitrile

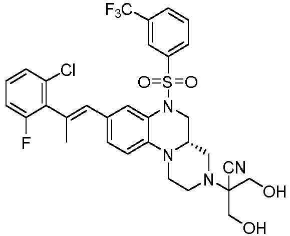

(*S*,*E*)-5-(8-(2-(2-chloro-6-fluorophenyl)prop-1-en-1-yl)-6-((3-(trifluoromethyl)phenyl)sulfony l)-1,2,4,4a, 5,6-hexahydro-3*H*-pyrazino[1,2-*a*]quinoxalin-3 -yl)-2,2-dimethyl-1,3 -dioxane-5 -carboni trile (90 mg, 0.128 mmol) and a solution of trifluoroacetic acid (2 mL) in water (2 mL) were stirred at room temperature for 24 hrs. The reaction mixture was neutralized with a 15% sodium hydroxide aqueous solution, and then separated by a rapid reversed-phase col umn to obtain (*S*,*E*)-2-(8-(2-(2-chloro-6-fluorophenyl)prop-1-en-1-yl)-6-((3-(trifluoromethyl)phe nyl)sulfonyl)-1,2,4,4a,5,6-hexahydro-3*H*-pyrazino[1,2-*a*]quinoxalin-3-yl)-3-hydroxy-2-(hydroxym ethyl)propanenitrile (50 mg, 58.8%). ESI-MS: 665 [M+l]⁺.
¹H NMR (400 MHz, Methanol-*d*₄) δ 8.01-7.87 (m, 2H), 7.83-7.63 (m, 3H), 7.36-7.27 (m, 2H), 7.24-7.09 (m, 2H), 6.86 (d, *J* = 8.7 Hz, 1H), 6.35 (d, *J* = 1.7 Hz, 1H), 4.31 (d d, *J* = 14.3, 4.4 Hz, 1H), 3.93-3.75 (m, 4H), 3.65 (dt, *J* = 11.6, 2.5 Hz, 1H), 3.41 (dd, *J* = 14.4, 10.1 Hz, 1H), 3.07 (dd, *J* = 10.7, 2.3 Hz, 2H), 2.63 (tdd, *J* = 10.1, 4.4, 2.5 Hz, 1H), 2.35 (dtd, *J* = 38.3, 11.7, 2.7 Hz, 2H), 2.21 (d, *J* = 1.5 Hz, 3H), 2.00 (t, *J* = 10.6 Hz, 1H).

### Example 74: preparation of (S)-2-(8-(3-(difluoromethoxy)-5-fluorophenyl)-6-((3-(trifluoro methyl)phenyl)sulfonyl)-1,2,4,4a,5,6-hexahydro-3H-pyrazino[1,2-a]quinoxalin-3-yl)-3-hydro xy-2-(hydroxymethyl)propanenitrile

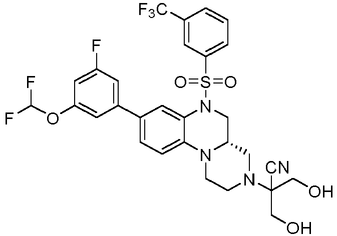

**Example 74 was prepared according to the synthesis method of Example 73:** ESI-MS: 657 [M+l]⁺.
¹H NMR (400 MHz, Methanol-*d*₄) δ 7.91 (t, *J* = 8.1 Hz, 2H), 7.85 (d, *J* = 2.2 Hz, 1H), 7.74 (t, *J=* 7.9 Hz, 1H), 7.61 (s, 1H), 7.46 (dd, *J* = 8.7, 2.2 Hz, 1H), 7.23-7.15 (m, 2H), 6.94 (t, 1H), 6.89 (dd, *J* = 16.2, 9.0 Hz, 2H), 4.31 (dd, *J* = 14.4, 4.5 Hz, 1H), 3.84 (dd, *J* = 11.5, 8.7 Hz, 2H), 3.76 (dd, *J* = 11.5, 4.7 Hz, 2H), 3.67 (d, *J* = 11.9 Hz, 1H), 3.37 (dd, *J* = 14.4, 10.2 Hz, 1H), 3.06 (d, *J* = 10.8 Hz, 2H), 2.64 (d, *J* = 11.2 Hz, 1H), 2.45-2.34 (m, 1H), 2.29 (dd, *J* = 12.7, 9.9 Hz, 1H), 1.99 (q, *J* = 11.3, 10.5 Hz, 1H).

### Example 75: preparation of (S,E)-2-(8-(2-(2-chloro-6-fluorophenyl)prop-1-en-1-yl)-6-((3-(t rifluoromethyl)phenyl)sulfonyl)-1,2,4,4a,5,6-hexahydro-3H-pyrazino[1,2-a]quinoxalin-3-yl)-3 -hydroxy-2-(hydroxymethyl)propanamide

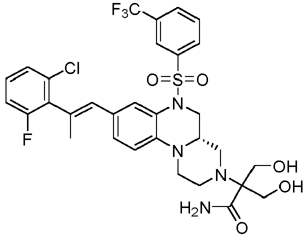

(*S*,*E*)-2-(8-(2-(2-chloro-6-fluorophenyl)prop-1-en-1-yl)-6-((3-(trifluoromethyl)phenyl)sulfon yl)-1,2,4,4a,5,6-hexahydro-3*H*-pyrazino[1,2-*a*]quinoxalin-3-yl)-3-hydroxy-2-(hydroxymethyl)prop anenitrile (30 mg, 0.045 mmol) was dissolved in dimethylsulfoxide (2 mL), and the mixtur e solution was cooled to 0 °C. Potassium carbonate (100 mg) and hydrogen peroxide (0.5 mL, 30 wt%) were added thereto and stirred at room temperature for 18 hrs. The reaction mixture was separated by a rapid reversed-phase column to obtain (*S*,*E*)-2µ-(8-(2-(2-chloro-6-fluorophenyl)prop-1-en-1-yl)-6-((3-(trifluoromethyl)phenyl)sulfonyl)-1,2,4,4a,5,6-hexahydro-3*H-*pyrazino[1,2-*a*]quinoxalin-3-yl)-3-hydroxy-2-(hydroxymethyl)propanamide (27.5 mg, 92%). ES I-MS: 683 [M+l]⁺.
¹H NMR (400 MHz, Methanol-*d*₄) δ 7.95 (d, *J* = 7.8 Hz, 1H), 7.92-7.85 (m, 1H), 7.77 (t, *J* = 7.9 Hz, 1H), 7.73-7.66 (m, 2H), 7.34-7.26 (m, 2H), 7.21-7.09 (m, 2H), 6.84 (d, *J=* 8.7 Hz, 1H), 6.34 (d, *J* = 1.7 Hz, 1H), 4.28 (dd, *J* = 14.2, 4.3 Hz, 1H), 3.91 (dd, *J* = 11.9, 6.4 Hz, 2H), 3.81 (d, *J* = 11.8 Hz, 2H), 3.66-3.55 (m, 1H), 3.42-3.34 (m, 1H), 2.95 (dt, *J* = 9.0, 3.0 Hz, 2H), 2.74-2.64 (m, 1H), 2.58 (t, *J* = 3.4 Hz, 1H), 2.44-2.30 (m, 2H), 2.21 (d, *J* = 1.4 Hz, 3H).

### Example 76: preparation of (S,E)-1-(8-(2-(2-chloro-6-fluorophenyl)prop-1-en-1-yl)-6-((3-(t rifluoromethyl)phenyl)sulfonyl)-1,2,4,4a,5,6-hexahydro-3H-pyrazino[1,2-a]quinoxalin-3-yl)c yclobutane-1-carbonitrile

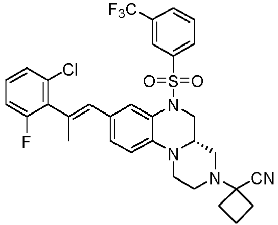

(*S*,*E*)-8-(2-(2-chloro-6-fluorophenyl)prop-1-en-1-y1)-6-((3-(trifluoromethyl)phenyl)sulfonyl) -2,3,4,4a,5,6-hexahydro-1*H*-pyrazino[1,2-*a*]quinoxaline (300 mg, 0.53 mmol) was dissolved i n acetic acid (5 mL), and the mixture solution was cooled to 0 °C. Cyclobutanone (186 mg, 2.65 mmol) and trimethylsilanecarbonitrile (262 mg, 2.65 mmol) were added thereto a nd stirred at room temperature for 18 hrs. The reaction mixture was neutralized to pH>7, and then separated by a rapid reversed-phase column to obtain (*S*,*E*)-1-(8-(2-(2-chloro-6-flu orophenyl)prop-1-en-1-yl)-6-((3-(trifluoromethyl)phenyl)sulfonyl)-1,2,4,4a,5,6-hexahydro-3*H*-pyr azino[1,2-*a*]quinoxalin-3-yl)cyclobutane-1-carbonitrile (315 mg, 92%). ESI-MS: 645 [M+l]⁺.
¹H NMR (400 MHz, Methanol-*d*₄) δ 7.94 (d, *J* = 7.9 Hz, 1H), 7.86 (d, *J* = 7.9 Hz, 1H), 7.76-7.74 (m, 1H), 7.70-7.68 (m, 2H), 7.29-7.27 (m, 2H), 7.18 (dd, *J* = 8.6, 2.1 Hz, 1H), 7.14-7.07 (m, 1H), 6.86 (d, *J* = 8.6 Hz, 1H), 6.33 (s, 1H), 4.30 (dd, *J* = 14.5, 4.4 Hz, 1H), 3.69 (dt, *J* = 12.1, 2.6 Hz, 1H), 3.43-3.34 (m, 1H), 2.82-2.68 (m, 2H), 2.53-2.48 (m, 1H), 2.42-2.17 (m, 8H), 2.14-1.85 (m, 3H), 1.68 (t, *J* = 10.4 Hz, 1H).

### Examples 77 to 78 were prepared according to the synthesis method of Example 76.

| **Example No.** | **Structural formula** | **Chemical name** | **ESI-MS:** [M+1]⁺ |
|---|---|---|---|
| 77 | 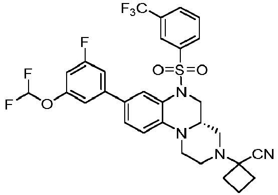 | (*S*)-1-(8-(3-(difluoromethoxy) -5 -fluorophenyl)-6-((3 -(trifluo romethyl)phenyl)sulfonyl)-1, 2,4,4a,5,6-hexahydro-3*H*-pyra zino[1,2-*a*]quinoxalin-3-yl)cy clobutane-1 -carbonitrile | 637 |
| **78** | 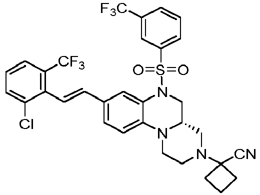 | (*S*,*E*)-1-(8-(2-chloro-6-(trifluo romethyl)styryl)-6-((3-(trifluo romethyl)phenyl)sulfonyl)-1, 2,4,4a,5,6-hexahydro-3*H*-pyra zino[1,2-*a*]quinoxalin-3-yl)cy clobutane-1 -carbonitrile | 681 |

### ¹H NMR data of the compound prepared in Examples 77 to 78 were as follows:

**Example 77**: ¹HNMR (400 MHz, Methanol-*d*₄) δ 7.94 (d, *J* = 7.8 Hz, 1H), 7.88-7.83 (m, 2H), 7.75 (t, *J=* 7.8 Hz, 1H), 7.67 (d, *J=* 1.8 Hz, 1H), 7.50-7.44 (m, 1H), 7.24-7.17 (m, 2H), 6.95 (t, 1H), 6.95-6.85 (m, 2H), 4.36-4.29 (m, 1H), 3.73 (d, *J=* 12.2 Hz, 1H), 3.40-3.32 (m, 1H), 2.75 (t, *J=* 10.3 Hz, 2H), 2.50 (ddt, *J* = 10.3, 7.3, 3.4 Hz, 1H), 2.42-2.32 (m, 2H), 2.32-1.86 (m, 6H), 1.67 (t, *J* = 10.5 Hz, 1H).
**Example 78:** ¹H NMR (400 MHz, CDCl₃) δ 7.84-7.82 (m, 3H), 7.74 (d, *J* = 2.1 Hz, 1H), 7.67-7.63 (m, 3H), 7.36-7.29 (m, 2H), 7.00 (dd, *J* = 16.6, 2.0 Hz, 1H), 6.82 (d, *J* = 16.5 Hz, 1H), 6.74 (d, *J* = 8.7 Hz, 1H), 4.25 (dd, *J* = 14.1, 4.3 Hz, 1H), 3.70-3.51 (m, 1H), 3.40-3.34 (m , 1H), 2.80-2.59 (m, 3H), 2.47-2.40 (m , 3H), 2.28-2.07 (m, 4H), 1.96 (d, *J* = 2.4 Hz, 1H), 1.81 (t, *J* = 10.4 Hz, 1H).

### Example 79: preparation of (S,E)-1-(8-(2-(2-chloro-6-fluorophenyl)prop-1-en-1-yl)-6-((3-(t rifluoromethyl)phenyl)sulfonyl)-1,2,4,4a,5,6-hexahydro-3H-pyrazino[1,2-a]quinoxalin-3-yl)c yclobutane-1-carboxamide

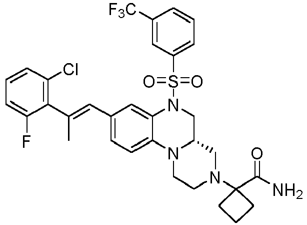

(*S*,*E*)-1-(8-(2-(2-chloro-6-fluorophenyl)prop-1-en-1-yl)-6-((3-(trifluoromethyl)phenyl)sulfon yl)-1,2,4,4a,5,6-hexahydro-3*H*-pyrazino[1,2-*a*]quinoxalin-3-yl)cyclobutane-1-carbonitrile (300 m g, 0.465 mmol) was dissolved in dimethylsulfoxide (5 mL), and the mixture solution was cooled to 0 °C. Potassium carbonate (12.8 mg, 0.093 mmol) and hydrogen peroxide (0.5 mL, 30% wt%) were added thereto and stirred at room temperature for 72 hrs. The reacti on mixture was separated by a rapid reversed-phase column to obtain (*S*,*E*)-1-(8-(2-(2-chlor o-6-fluorophenyl)prop-1-en-1-yl)-6-((3-(trifluoromethyl)phenyl)sulfonyl)-1,2,4,4a,5,6-hexahydro-3*H*-pyrazino[1,2-*a*]quinoxalin-3-yl)cyclobutane-1-carboxamide (179.7 mg, 58%). ESI-MS: 663 [M+1]⁺.
¹H NMR (400 MHz, Methanol-*d*₄) δ 7.93 (d, *J* = 7.8 Hz, 1H), 7.83 (d, *J* = 7.9 Hz, 1H), 7.79-7.66 (m, 3H), 7.33-7.25 (m, 2H), 7.20-7.07 (m, 2H), 6.83 (d, *J* = 8.6 Hz, 1H), 6.32 (s, 1H), 4.25 (dd, *J* = 14.3, 4.4 Hz, 1H), 3.60 (d, *J* = 12.0 Hz, 1H), 3.41-3.33 (m, 1H), 2.76 (d, *J* = 11.0 Hz, 2H), 2.55 - 2.47 (m, 1H), 2.34-2.03 (m, 9H), 1.83-1.72 (m, 3H).

### Examples 80 to 81 were prepared according to the synthesis method of Example 79.

| **Example No.** | **Structural formula** | **Chemical name** | **ESI-MS:** [M+1]⁺ |
|---|---|---|---|
| **80** | 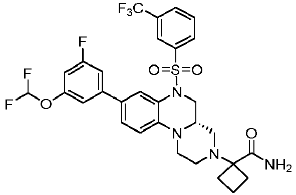 | (*S*)-1-(8-(3-(difluoromethoxy) -5-fluorophenyl)-6-((3-(triflu oromethyl)phenyl)sulfonyl)-1,2,4,4a,5,6-hexahydro-3*H*-p yrazino [1,2-*a*]quinoxalin-3-y l)cyclobutane-1-carboxamide | 655 |
| **81** | 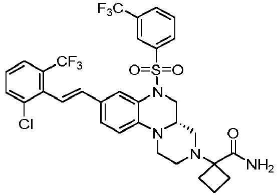 | (*S*,*E*)-1-(8-(2-chloro-6-(trifluo romethyl)styryl)-6-((3-(trifluo romethyl)phenyl)sulfonyl)-1, 2,4,4a,5,6-hexahydro-3*H*-pyr azino[1,2-*a*]quinoxalin-3 -yl)c yclobutane-1 -carboxamide | 699 |

### ¹H NMR data of the compound prepared in Examples 80 to 81 were as follows:

**Example 80**: ¹HNMR (400 MHz, Methanol-*d*₄) δ 7.94 (d, *J=* 7.7 Hz, 1H), 7.86-7.80 (m, 2H), 7.74 (t, *J* = 7.9 Hz, 1H), 7.68 (d, *J* = 1.9 Hz, 1H), 7.45 (dd, *J* = 8.7, 2.3 Hz, 1H), 7.23-7.16 (m, 2H), 6.94 (t, 1H), 6.92-6.83 (m, 2H), 4.27 (dd, *J* = 14.4, 4.4 Hz, 1H), 3.67-3.59 (m, 1H), 3.39-3.32 (m, 1H), 2.81-2.74 (m, 2H), 2.54-2.44 (m, 1H), 2.32 (td, *J* = 11.9, 2.9 Hz, 1H), 2.27-2.19 (m, 2H), 2.17-2.12 (m, 1H), 2.12-2.03 (m, 2H), 1.81-1.70 (m, 3H).
**Example 81:** ¹HNMR (400 MHz, Methanol-*d*₄) δ 7.95 (d, *J=* 7.8 Hz, 1H), 7.88-7.83 (m, 1H), 7.79-7.70 (m, 5H), 7.44 (t, *J* = 8.0 Hz, 1H), 7.35 (dd, *J* = 8.7, 2.1 Hz, 1H), 7.01 (dd, *J* = 16.6, 2.0 Hz, 1H), 6.88-6.75 (m, 2H), 4.29 (dd, *J=* 14.3, 4.4 Hz, 1H), 3.69-3.61 (m, 1H), 3.40-3.35 (m, 1H), 2.85-2.75 (m, 2H), 2.56 (dd, *J* = 4.4, 2.7 Hz, 1H), 2.39-2.31 (m, 1H), 2.30-2.21 (m, 2H), 2.20-2.08 (m, 3H), 1.84-1.74 (m, 3H).

### Example 82: preparation of (S,E)-1-(8-(2-(2-chloro-6-fluorophenyl)prop-1-en-1-yl)-6-((3-(t rifluoromethyl)phenyl)sulfonyl)-1,2,4,4a,5,6-hexahydro-3H-pyrazino[1,2-a]quinoxalin-3-yl)-2 -methylpropan-2-ol

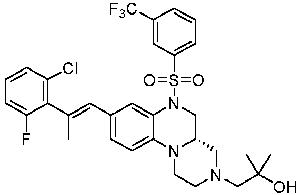

(*S*,*E*)-8-(2-(2-chloro-6-fluorophenyl)prop-1-en-1-yl)-6-((3-(trifluoromethyl)phenyl)sulfonyl) -2,3,4,4a,5,6-hexahydro-1*H*-pyrazino[1,2-*a*]quinoxaline (100 mg, 0.18 mmol) was dissolved i n dimethylsulfoxide (2 mL). Cesium carbonate (172 mg, 0.531 mmol), potassium iodide (8 8 mg, 0.531 mmol) and 1-bromo-2-methylpropan-2-ol (134 mg, 0.88 mmol) were added th ereto. The reaction mixture were stirred in a microwave reactor at 80 °C for 15 hrs, and separated by a rapid reversed-phase column to obtain (*S*,*E*)-1-(8-(2-(2-chloro-6-fluorophenyl) prop-1-en-1-yl)-6-((3-(trifluoromethyl)phenyl)sulfonyl)-1,2,4,4a,5,6-hexahydro-3*H*-pyrazino[1,2-*a*]quinoxalin-3-yl)-2-methylpropan-2-ol (20.5 mg, 18.2%). ESI-MS: 638 [M+l]⁺.
¹HNMR (400 MHz, CDCl₃) δ 7.89 (s, 1H), 7.83 (d, *J=* 7.9 Hz, 1H), 7.75 (d, *J=* 2.0 Hz, 1H), 7.71 (d, *J* = 7.9 Hz, 1H), 7.59 (t, *J* = 7.8 Hz, 1H), 7.25 (d, *J* = 8.0 Hz, 1H), 7.29-7.17 (m , 2H), 7.04 (t, *J* = 8.3 Hz, 1H), 6.72 (d, *J* = 8.6 Hz, 1H), 6.38 (s, 1H), 4.17 (dd, *J* = 14.2, 4.3 Hz, 1H), 3.48 (d, *J* = 11.9 Hz, 1H), 3.35 (dd, *J* = 14.1, 10.1 Hz, 1H), 2.83 (dd, *J* = 23.1, 11.3 Hz, 2H), 2.67-2.60 (m, 1H), 2.48-2.41 (m, 1H), 2.35 (dd, *J* = 11.4, 2.8 Hz, 1H), 2.31 (s, 2H), 2.24 (d, *J* = 1.4 Hz, 3H), 2.04 (t, *J* = 10.6 Hz, 1H), 1.17 (s, 6H).

### Example 83: preparation of (R,E)-2-(8-(2-chloro-6-(trifluoromethyl)styryl)-6-((3-(trifluoro methyl)phenyl)sulfonyl)-1,2,4,4a,5,6-hexahydro-3H-pyrazino[1,2-a]quinoxalin-3-yl)acetic aci d

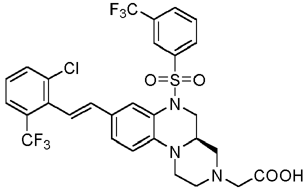

### Step 1: synthesis of tert-butyl (R,E)-8-(2-chloro-6-(trifluoromethyl)styryl)-6-((3-(trifluoro methyl)phenyl)sulfonyl)-1,2,4,4a,5,6-hexahydro-3H-pyrazino[1,2-a]quinoxaline-3-carboxylat e

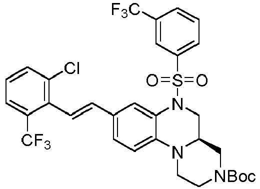

*Tert*-butyl (*R*)-8-bromo-6-((3-(trifluoromethyl)phenyl)sulfonyl)-1,2,4,4a,5,6-hexahydro-3*H*-p yrazino[1,2-*a*]quinoxaline-3-carboxylate (100 mg, 0.17 mmol), (*E*)-2-(3-chloro-5-(trifluoromet hyl)styryl)-4,4,5-trimethyl-1,3,2-dioxaborolane (67 mg, 0.21 mmol), [1,1'-bis(diphenylphosphi no)ferrocene]palladium chloride (13 mg, 0.017 mmol) and potassium carbonate (47 mg, 0.3 4 mmol) were mixed in 1,4-dioxane (6 mL) and water (3 mL). The nitrogen was charged to replace three times by evacuation. The mixture solution was reacted at a temperature of 90 °C for 2 hrs. After the reaction was completed, the reaction mixture was added with water (20 mL) and extracted three times with ethyl acetate (20 mL * 3). The organic pha ses were combined, washed with brine (30 mL), dried over anhydrous sodium sulfate, filte red and concentrated. The residue was separated by a rapid silica gel column [eluent: EtO Ac : PE = 0-20%] to obtain *tert*-butyl (*R*,*E*)-8-(2-chloro-6-(trifluoromethyl)styryl)-6-((3-(trifl uoromethyl)phenyl)sulfonyl)-1,2,4,4a,5,6-hexahydro-3H-pyrazino[1,2-*a*]quinoxaline-3-carboxylat e (105 mg, 86%). ESI-MS: 702.3 [M+H]⁺.

### Step 2: synthesis of (R,E)-8-(2-chloro-6-(trifluoromethyl)styryl)-6-((3-(trifluoromethyl)phe nyl)sulfonyl)-2,3,4,4a,5,6-hexahydro-1H-pyrazino[1,2-a]quinoxaline

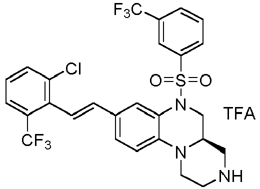

Tert-butyl (*R*,*E*)-8-(2-chloro-6-(trifluoromethyl)styryl)-6-((3-(trifluoromethyl)phenyl)sulfony l)-1,2,4,4a,5,6-hexahydro-3*H*-pyrazino[1,2-*a*]quinoxaline-3-carboxylate (210 mg, 0.3 mmol) w as dissolved in dichloromethane (5 mL). Trifluoroacetic acid (2 mL) was added thereto. T he reaction mixture was stirred at room temperature for 2 hrs. The solvent was removed b y concentration to obtain crude (*R*,*E*)-8-(2-chloro-6-(trifluoromethyl)styryl)-6-((3-(trifluoromet hyl)phenyl)sulfonyl)-2,3,4,4a,5,6-hexahydro-1*H*-pyrazino[1,2-*a*]quinoxaline (300 mg, crude). E SI-MS: 602.2 [M+H]⁺.

### Step 3: synthesis of methyl (R,E)-2-(8-(2-chloro-6-(trifluoromethyl)styryl)-6-((3-(trifluoro methyl)phenyl)sulfonyl)-1,2,4,4a,5,6-hexahydro-3H-pyrazino[1,2-a]quinoxalin-3-yl)acetate

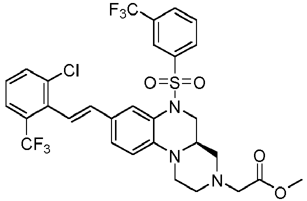

(*R*,*E*)-8-(2-chloro-6-(trifluoromethyl)styryl)-6-((3-(trifluoromethyl)phenyl)sulfonyl)-2,3,4,4a, 5,6-hexahydro-1*H*-pyrazino[1,2-a]quinoxaline (300 mg, 0.75 mmol) was dissolved in dimeth ylsulfoxide (5 mL). Potassium carbonate (311 mg, 2.25 mmol) and methyl bromoacetate (2 30 mg, 1.5 mmol) were added thereto. The reaction mixture was stirred at 50 °C for 2 hr s. After the reaction was completed, the reaction mixture was added with water (20 mL) a nd extracted three times with ethyl acetate (20 mL * 3), organic phases were combined, washed three times with water (30 mL * 3), dried over anhydrous sodium sulfate, filtered and concentrated. The residue was separated by a rapid silica gel column [eluent: EtOAc : PE = 0-20%] to obtain methyl **(*R*,*E*)-2-(8-(2-chloro-6-(trifluoromethyl)styryl)-6-((3-(trifluoro** methyl)phenyl)sulfonyl)-1,2,4,4a,5,6-hexahydro-3*H*-pyrazino[1,2-*a*]quinoxalin-3-yl)acetate (250 mg, 86%). ESI-MS: 674.2 [M+H]⁺.

### Step 4: synthesis of (R,E)-2-(8-(2-chloro-6-(trifluoromethyl)styryl)-6-((3-(trifluoromethyl)p henyl)sulfonyl)-1,2,4,4a,5,6-hexahydro-3H-pyrazino[1,2-a]quinoxalin-3-yl)acetic acid

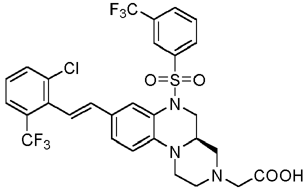

Methyl (*R*,*E*)-2-(8-(2-chloro-6-(trifluoromethyl)styryl)-6-((3-(trifluoromethyl)phenyl)sulfony l)-1,2,4,4a,5,6-hexahydro-3*H*-pyrazino[1,2-*a*]quinoxalin-3-yl)acetate (250 mg, 0.37 mmol) was dissolved in methanol (5 mL) and water (1 mL). Lithium hydroxide monohydrate (36 m g, 1.5 mmol) was added thereto. The mixture solution was stirred at room temperature for 2 hrs, then concentrated to remove the solvent and acidified with dilute hydrochloric acid. The residue was separated by a reversed-phase column chromatography [eluent: H₂O : Me CN = 0-70%] to obtain (*R*,*E*)-2-(8-(2-chloro-6-(trifluoromethyl)styryl)-6-((3-(trifluoromethyl)p henyl)sulfonyl)-1,2,4,4a,5,6-hexahydro-3*H*-pyrazino[1,2-*a*]quinoxalin-3-yl)acetic acid (80 mg, 33%). ESI-MS: 660.3 [M+H]⁺.
¹H NMR (400 MHz, CDCl₃) δ 7.92-7.74 (m, 3H), 7.70-7.55 (m, 4H), 7.29 (t, *J* = 7. 8 Hz, 2H), 6.94 (d, *J* = 16.5 Hz, 1H), 6.84-6.64 (m, 2H), 4.23 (d, *J* = 14.3 Hz, 1H), 3.6 3 (s, 1H), 3.35 (s, 3H), 3.09 (s, 3H), 2.73 (s, 1H), 2.49 (s, 1H).

### Example 84: preparation of (S,E)-1-(8-(2-chloro-6-(trifluoromethyl)styryl)-6-((3-(trifluoro methoxy)phenyl)sulfonyl)-1,2,4,4a,5,6-hexahydro-3H-pyrazino[1,2-a]quinoxalin-3-yl)ethan-1 -one

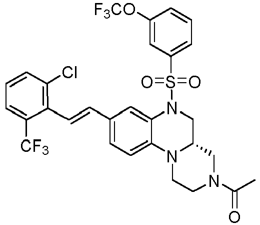

**Example 84 was prepared according to the synthesis method of Example 7:** ESI-MS: 660 [M+H]⁺.
¹H NMR (400 MHz, Methanol-*d*₄) δ 7.75 (d, *J* = 2.1 Hz, 1H), 7.73-7.68 (m, 2H), 7. 61 (t, *J* = 8.5 Hz, 1H), 7.55 (d, *J* = 8.0 Hz, 1H), 7.46 (s, 1H), 7.44-7.32 (m, 2H), 7.20 (d, *J* = 14.7 Hz, 1H), 7.00 (d, *J* = 16.7 Hz, 1H), 6.89 (d, *J* = 8.9 Hz, 1H), 6.79-6.72 (m, 1H), 4.45-4.36 (m, 1H), 4.28 (d, *J* = 22.3 Hz, 1H), 3.76 (t, *J* = 11.1 Hz, 1H), 3.48 (s, 1H), 3.16 (d, *J* = 21.2 Hz, 1H), 2.69 (s, 3H), 2.08 (d, *J* = 6.4 Hz, 2H), 2.04 (d, *J =* 7.9 Hz, 2H).

### Example 85: preparation of methyl (S,E)-2-(8-(2-chloro-6-(trifluoromethyl)styryl)-6-((3-(t rifluoromethoxy)phenyl)sulfonyl)-1,2,4,4a,5,6-hexahydro-3H-pyrazino[1,2-a]quinoxalin-3-yl) acetate

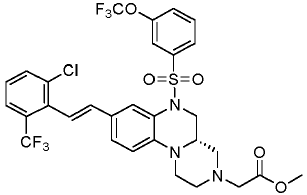

**Example 85 was prepared according to the synthesis method of Example 44:** ESI-MS: 690 [M+H]⁺.
¹H NMR (400 MHz, CDCl₃) δ 7.78 (d, *J* = 2.1 Hz, 1H), 7.63 (d, *J* = 2.3 Hz, 1H), 7.61 (d, *J* = 2.2 Hz, 1H), 7.57 (dt, *J* = 7.9, 1.4 Hz, 1H), 7.53-7.49 (m, 1H), 7.40-7.36 (m, 2H), 7.31 (d, *J* = 2.0 Hz, 1H), 7.29 (d, *J* = 1.6 Hz, 1H), 7.01-6.94 (m, 1H), 6.80 (d, *J* = 16.5 Hz, 1H), 6.71 (d, *J* = 8.7 Hz, 1H), 4.23 (dd, *J* = 14.1, 4.1 Hz, 1H), 3.73 (s, 3H), 3.56 (d, *J* = 12.3 Hz, 1H), 3.33-3.26 (m, 1H), 3.22 (d, *J* = 3.1 Hz, 2H), 2.89-2.76 (m, 3H), 2.59 (td, *J* = 12.1, 3.1 Hz, 1H), 2.29 (td, *J* = 11.5, 3.2 Hz, 1H), 2.01-1.91 (m, 1H).

### Example 86: preparation of (S,E)-2-(8-(2-chloro-6-(trifluoromethyl)styryl)-6-((3-(trifluoro methoxy)phenyl)sulfonyl)-1,2,4,4a,5,6-hexahydro-3H-pyrazino[1,2-a]quinoxalin-3-yl)acetic a cid

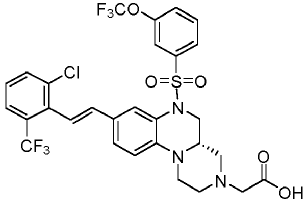

**Example 86 was prepared according to the synthesis method of Example 9:** ESI-MS: 676 [M+H]⁺.
¹H NMR (400 MHz, Methanol-*d*₄) δ 7.78-7.62 (m, 5H), 7.59 (d, *J* = 8.7 Hz, 1H), 7. 51-7.19 (m, 3H), 7.15-6.92 (m, 2H), 6.76 (d, *J* = 16.6 Hz, 1H), 4.53-4.36 (m, 1H), 4.00 (d, *J* = 13.8 Hz, 1H), 3.74 (d, *J* = 22.8 Hz, 2H), 3.50 (d, *J* = 12.4 Hz, 2H), 3.41-3.37 (m, 1H), 3.16-3.02 (m, 1H), 2.96 (t, *J* = 12.2 Hz, 1H), 2.67 (q, *J* = 11.2, 10.1 Hz, 2H).

### Example 87: preparation of (S)-2-((3-((8-(3-(difluoromethoxy)-5-fluorophenyl)-1,2,3,4,4a,5 -hexahydro-6H-pyrazino[1,2-a]quinoxalin-6-yl)sulfonyl)-5-(trifluoromethyl)pyridin-2-yl)oxy) ethan-1-ol

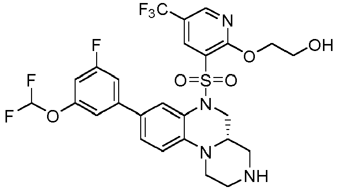

Tert-butyl (*S*)-8-(3-(difluoromethoxy)-5-fluorophenyl)-6-((2-(2-hydroxyethoxy)-5-(trifluoro methyl)pyridin-3-yl)sulfonyl)-1,2,4,4a,5,6-hexahydro-3*H*-pyrazino[1,2-*a*]quinoxaline-3-carboxyla te (280 mg, 0.39 mmol) was dissolved in HCl/dioxane (10 mL, 4M). The reaction mixture was stirred at room temperature for 1 hr. After the reaction was completed, the reaction mixture was concentrated to obtain a crude product (230 mg, 82%) used directly in the ne xt step. The crude product (30 mg) was separated by a reversed-phase column [eluent: H₂ O : MeCN = 0-80%] to obtain (*S*)-2-((3-((8-(3-(difluoromethoxy)-5-fluorophenyl)-1,2,3,4,4a,5 -hexahydro-6*H*-pyrazino[1,2-*a*]quinoxalin-6-yl)sulfonyl)-5-(trifluoromethyl)pyridin-2-yl)oxy)ethan-1-ol (7.6 mg, 25%). ESI-MS: 619 [M+H]⁺.
¹H NMR (400 MHz, CDCl₃) δ 9.63 (s, 1H), 8.53 (d, *J* = 28.1 Hz, 2H), 7.27 (s, 1 H), 7.14 (s, 1H), 6.88 (s, 2H), 6.78 (d, *J* = 9.1 Hz, 1H), 6.51 (t, *J* = 73.2 Hz, 1H), 4.66 (d, *J* = 30.7 Hz, 3H), 4.02 (s, 4H), 3.80 (s, 2H), 3.49 (s, 2H), 3.03 (s, 2H).

### Example 88: (S)-2-((3-((8-(2,2,6,6-tetramethyl-3,6-dihydro-2H-pyran-4-yl)-1,2,3,4,4a,5-hexa hydro-6H-pyrazino[1,2-a]quinoxalin-6-yl)sulfonyl)-5-(trifluoromethyl)pyridin-2-yl)oxy)etha n-1-ol

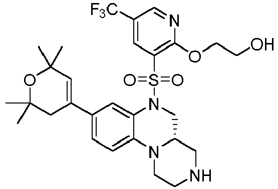

**Example 88 was prepared according to the synthesis method of Example 87.**
¹H NMR (400 MHz, Methanol-*d*₄) δ 8.76 (d, *J* = 2.3 Hz, 1H), 8.51 (d, *J* = 2.3 Hz, 1H), 7.17 (dd, *J* = 8.7, 2.1 Hz, 1H), 7.12 (d, *J* = 2.0 Hz, 1H), 6.99 (d, *J* = 8.7 Hz, 1 H), 5.86 (s, 1H), 4.68 (dt, *J* = 10.4, 4.9 Hz, 1H), 4.57 (dt, *J* = 11.7, 4.5 Hz, 1H), 4.43 (dd, *J* = 13.9, 3.5 Hz, 1H), 4.17 (d, *J* = 13.5 Hz, 1H), 3.85 (t, *J* = 4.8 Hz, 2H), 3.77 (d d, *J* = 13.9, 8.2 Hz, 1H), 3.58 - 3.44 (m, 3H), 3.20 (td, *J* = 12.5, 3.0 Hz, 1H), 3.15-3.0 4 (m, 1H), 2.97 (t, *J* = 12.3 Hz, 1H), 2.17 (s, 2H), 1.27 (s, 6H), 1.25 (s, 6H). ESI-MS: 597 [M+H]⁺.

### Example 89: preparation of (S)-2-((3-((8-(3-(difluoromethoxy)-5-fluorophenyl)-3-(methyls ulfonyl)-1,2,3,4,4a,5-hexahydro-6H-pyrazino[1,2-a]quinoxalin-6-yl)sulfonyl)-5-(trifluorometh yl)pyridin-2-yl)oxy)ethan-1-ol

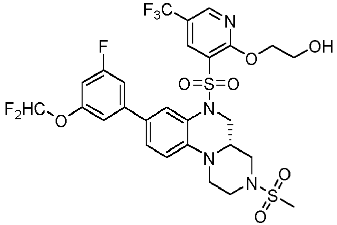

### Step 1: synthesis of (S)-6-((2-(2-((tert-butyldimethylsilyl)oxy)ethoxy)-5-(trifluoromethyl) p yridin-3-yl)sulfonyl)-8-(3-(difluoromethoxy)-5-fluorophenyl)-2,3,4,4a,5,6-hexahydro-1H-pyra zino[1,2-a] quinoxaline

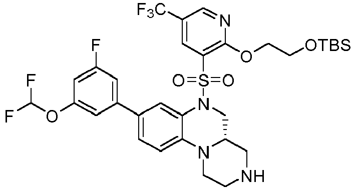

**(*S*)-2-((3-((8-(3-(difluoromethoxy)-5-fluorophenyl)-1,2,3,4,4a,5-hexahydro-6*H*-pyrazino[1,2-***a*]quinoxalin-6-yl)sulfonyl)-5-(trifluoromethyl)pyridin-2-yl)oxy)ethan-1-ol (200 mg, 0.32 mmo 1), TBSCl (150 mg, 0.97 mmol), imidazole (220 mg, 3.23 mmol) were mixed in anhydrou s dichloromethane (10 mL). The reaction mixture was stirred at room temperature for 2 hr s; after the reaction was completed, the reaction mixture was washed with saturated NaHC O₃ (10 mL), H₂O (10 mL) and brine (10 mL). The organic phases were dried over magne sium sulfate and filtered. The filtrate the was concentrated, the residue was separated by a rapid silica gel column to obtain (*S*)-6-((2-(2-((*tert*-butyldimethylsilyl)oxy)ethoxy)-5-(trifluoro methyl)pyridin-3-yl)sulfonyl)-8-(3-(difluoromethoxy)-5-fluorophenyl)-2,3,4,4a,5,6-hexahydro-1*H* -pyrazino[1,2-*a*]quinoxaline (180 mg, 77%) (PE : EA = 0-30%). ESI-MS: 733 [M+H]⁺.

### Step 2: synthesis of (S)-6-((2-(2-((tert-butyldimethylsilyl)oxy)ethoxy)-5-(trifluoromethyl)py ridin-3-yl)sulfonyl)-8-(3-(difluoromethoxy)-5-fluorophenyl)-3-(methylsulfonyl)-2,3,4,4a,5,6-h exahydro-1H-pyrazino[1,2-a]quinoxaline

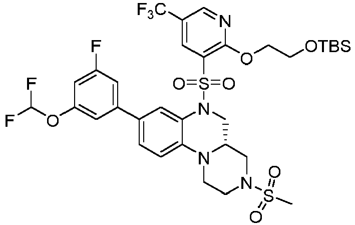

(*S*)-6-((2-(2-((*tert*-butyldimethylsilyl)oxy)ethoxy)-5-(trifluoromethyl)pyridin-3-yl)sulfonyl)-8 -(3-(difluoromethoxy)-5-fluorophenyl)-2,3,4,4a,5,6-hexahydro-1*H*-pyrazino[1,2-*a*]quinoxaline (4 0 mg, 5.46 * 10⁻² mmol), diisopropylethylamine (71 mg, 0.55 mmol) and methanesulfonyl chloride (31 mg, 0.27 mmol) were mixed in DCM (5 mL). The reaction mixture was stirr ed at room temperature for 1 hr. After the reaction was completed, the reaction mixture w as washed with saturated NaHCO₃ (5 mL), H₂O (5 mL) and brine (5 mL). The organic p hases were dried over magnesium sulfate and filtered. The filtrate was concentrated to obta in crude (*S*)-6-((2-(2-((*tert*-butyldimethylsilyl)oxy)ethoxy)-5-(trifluoromethyl)pyridin-3-yl)sulfon yl)-8-(3-(difluoromethoxy)-5-fluorophenyl)-3-(methylsulfonyl)-2,3,4,4a,5,6-hexahydro-1*H*-pyrazi no[1,2-a]quinoxaline (45 mg, 95%). ESI-MS: 811 [M+H]⁺. The crude product was used dir ectly in the next step.

### Step 3: synthesis of (S)-2-((3-((8-(3-(difluoromethoxy)-5-fluorophenyl)-3-(methylsulfonyl)-1,2, 3,4,4a,5-hexahydro-6H-pyrazino[1,2-a]quinoxalin-6-yl)sulfonyl)-5-(trifluoromethyl)pyridin-2 -yl)oxy)ethan-1-ol

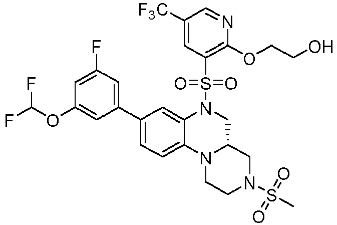

(*S*)-6-((2-(2-((*tert*-butyldimethylsilyl)oxy)ethoxy)-5-(trifluoromethyl)pyridin-3-yl)sulfonyl)-8 -(3-(difluoromethoxy)-5-fluorophenyl)-3-(methylsulfonyl)-2,3,4,4a,5,6-hexahydro-1*H*-pyrazino[1, 2-*a*]quinoxaline (45 mg, 5.46 * 10⁻² mmol) was dissolved in HCl/dioxane (5 mL, 4M). Th e reaction mixture was stirred at room temperature for 1 hr. After the reaction was compl eted, the reaction was concentrated to obtain a crude product, firstly separated by a prepar ative TLC (DCM : MeOH = 15:1), and then separated by a reversed-phase column [eluen t: H₂O : MeCN = 0-80%] to obtain (*S*)-2-((3-((8-(3-(difluoromethoxy)-5-fluorophenyl)-3-(me thylsulfonyl)-1,2,3,4,4a,5-hexahydro-6*H*-pyrazino[1,2-*a*]quinoxalin-6-yl)sulfonyl)-5-(trifluoromet hyl)pyridin-2-yl)oxy)ethan-1-ol (6.7 mg, 17%).
¹H NMR (400 MHz, CDCl₃) δ 8.53 (s, 1H), 8.40 (d, *J* = 2.3 Hz, 1H), 7.49 (d, *J =* 1.9 Hz, 1H), 7.22 (d, *J* = 2.0 Hz, 1H), 6.93 (dd, *J* = 8.2, 2.0 Hz, 2H), 6.82 (d, *J* = 8.6 Hz, 1H), 6.73 (d, *J* = 9.1 Hz, 1H), 6.47 (t, *J* = 73.3 Hz, 1H), 4.51 (s, 2H), 4.23 (d, *J =* 12.6 Hz, 1H), 3.79 (dd, *J* = 17.8, 10.9 Hz, 5H), 3.57 (s, 1H), 3.18 (s, 1H), 2.87 (q, *J* = 13.3, 12.3 Hz, 2H), 2.76 (s, 3H), 2.50 (s, 1H). ESI-MS: 697 [M+H]⁺.

### Example 90: preparation of methyl (S)-2-(8-(3-(difluoromethoxy)-5-fluorophenyl)-6-((2-(2 -hydroxyethoxy)-5-(trifluoromethyl)pyridin-3-yl)sulfonyl)-1,2,4,4a,5,6-hexahydro-3H-pyrazi no[1,2-a]quinoxalin-3-yl)acetate

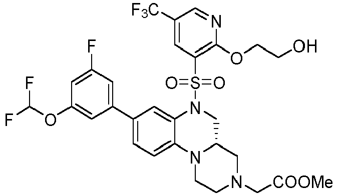

### Step 1: synthesis of methyl (S)-2-(6-((2-(2-((tert-butyldimethylsilyl)oxy)ethoxy)-5-(trifluoro methyl)pyridin-3-yl)sulfonyl)-8-(3-(difluoromethoxy)-5-fluorophenyl)-1,2,4,4a,5,6-hexahydro -3H-pyrazino[1,2-a] quinoxalin-3-yl)acetate

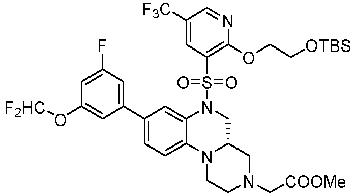

(*S*)-6-((2-(2-((*tert*-butyldimethylsilyl)oxy)ethoxy)-5-(trifluoromethyl)pyridin-3-yl)sulfonyl)-8 -(3-(difluoromethoxy)-5-fluorophenyl)-2,3,4,4a,5,6-hexahydro-1*H*-pyrazino[1,2-*a*]quinoxaline (8 5 mg, 0.12 mmol), methyl bromoacetate (88 mg, 0.58 mmol) and potassium carbonate (80 mg, 0.58 mmol) were mixed in acetonitrile (10 mL). The reaction mixture was stirred at 8 0 °C for 2 hrs; LCMS showed the reaction was completed, the reaction mixture was conc entrated. The residue was dissolved in DCM (10 mL), washed with saturated NaHCO₃ (5 m L), H₂O (5 mL) and brine (5 mL). The organic phases were dried over magnesium sulfate and filtered. The filtrate was concentrated to obtain crude methyl (*S*)-2-(6-((2-(2-((*tert*-butyl dimethylsilyl)oxy)ethoxy)-5-(trifluoromethyl)pyridin-3-yl)sulfonyl)-8-(3-(difluoromethoxy)-5-flu orophenyl)-1,2,4,4a,5,6-hexahydro-3*H*-pyrazino[1,2-*a*]quinoxalin-3-yl)acetate (90 mg, 95%). E SI-MS: 805 [M+H]⁺. The crude product was used directly in the next step.

### Step 2: synthesis of methyl (S)-2-(8-(3-(difluoromethoxy)-5-fluorophenyl)-6-((2-(2-hydrox yethoxy)-5-(trifluoromethyl)pyridin-3-yl)sulfonyl)-1,2,4,4a,5,6-hexahydro-3H-pyrazino[1,2-a]quinoxalin-3-yl)acetate

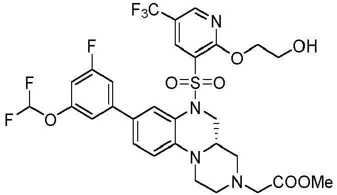

Methyl (*S*)-2-(6-((2-(2-((*tert*-butyldimethylsilyl)oxy)ethoxy)-5-(trifluoromethyl)pyridin-3-yl) sulfonyl)-8-(3-(difluoromethoxy)-5-fluorophenyl)-1,2,4,4a,5,6-hexahydro-3*H*-pyrazino[1,2-*a*]quin oxalin-3-yl)acetate (90 mg, 0.11 mmol) was dissolved in HCl/dioxane (5 mL, 4M). The re action mixture was stirred at room temperature for 1 hr. After the reaction was completed, the reaction mixture was concentrated to obtain a crude product (80 mg), the crude produ ct (50 mg) was used directly for the next step, and the remaining crude product (30 mg) was firstly separated by a preparative TLC [eluent: DCM : MeOH = 15:1], and then separ ated by a reversed-phase column [eluent: H₂O : MeCN = 0-80%] to obtain methyl (S)-2-(8-(3-(difluoromethoxy)-5-fluorophenyl)-6-((2-(2-hydroxyethoxy)-5-(trifluoromethyl)pyridin-3-y 1)sulfonyl)-1,2,4,4a,5,6-hexahydro-3*H*-pyrazino[1,2-*a*]quinoxalin-3-yl)acetate (5.7 mg, 7%). ES I-MS: 691 [M+H]⁺.
¹H NMR (400 MHz, CDCl₃) δ 8.57 (s, 1H), 8.44 (s, 1H), 7.66 (s, 1H), 7.22 (ddd, *J* = 6.9, 4.5, 1.7 Hz, 1H), 6.97 (s, 2H), 6.81 (d, *J* = 9.2 Hz, 2H), 6.54 (t, *J* = 73.2 Hz, 1 H), 4.82 (s, 1H), 4.66 (s, 2H), 4.19 (s, 1H), 4.02 (d, *J* = 31.0 Hz, 5H), 3.87 (s, 3H), 3.7 4-3.52 (m, 5H), 3.20 (s, 1H).

### Example 91: preparation of (S)-2-(8-(3-(difluoromethoxy)-5-fluorophenyl)-6-((2-(2-hydrox yethoxy)-5-(trifluoromethyl)pyridin-3-yl)sulfonyl)-1,2,4,4a,5,6-hexahydro-3H-pyrazino[1,2-a]quinoxalin-3-yl)acetic acid

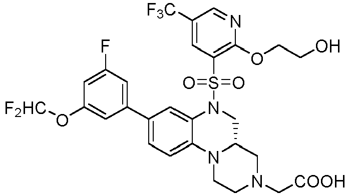

### Step 1: synthesis of tert-butyl (S)-2-(6-((2-(2-((tert-butyldimethylsilyl)oxy)ethoxy)-5-(triflu oromethyl)pyridin-3-yl)sulfonyl)-8-(3-(difluoromethoxy)-5-fluorophenyl)-1,2,4,4a,5,6-hexahy dro-3H-pyrazino[1,2-a]quinoxalin-3-yl)acetate

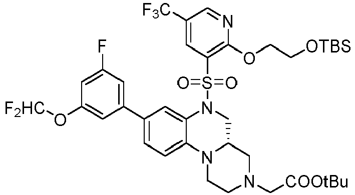

It was prepared according to the method of Example 90. ESI-MS: 847 [M+H]⁺.

### Step 2: synthesis of (S)-2-(8-(3-(difluoromethoxy)-5-fluorophenyl)-6-((2-(2-hydroxyethoxy) -5-(trifluoromethyl)pyridin-3-yl)sulfonyl)-1,2,4,4a,5,6-hexahydro-3H-pyrazino[1,2-a]quinoxa lin-3-yl)acetic acid

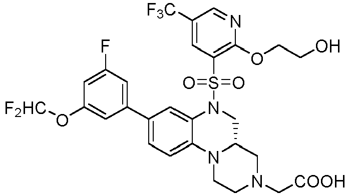

It was prepared according to the method of Example 90. ESI-MS: 677 [M+H]⁺.
¹H NMR (400 MHz, Methanol-*d*₄) δ 8.76 (d, *J* = 2.4 Hz, 1H), 8.55 (d, *J* = 2.4 Hz, 1H), 7.48-7.36 (m, 2H), 7.11 (d, *J* = 8.8 Hz, 1H), 7.07-7.02 (m, 2H), 6.90 (t, *J* = 73.2 Hz, 1H), 6.87 (d, *J* = 9.5 Hz, 1H), 4.67 (dt, *J* = 10.4, 4.9 Hz, 1H), 4.58-4.48 (m, 1H), 4.45 (dd, *J* = 14.0, 3.4 Hz, 1H), 4.25 (d, *J* = 11.5 Hz, 1H), 4.11 (s, 2H), 3.80 (t, *J* = 4. 8 Hz, 2H), 3.79-3.67 (m, 3H), 3.59 (s, 1H), 3.23 (d, *J* = 11.3 Hz, 2H), 3.01 (t, *J* = 11.7 Hz, 1H).

### Examples 92 and 93: preparation of 2-((4aS)-8-(3-(difluoromethoxy)-5-fluorophenyl)-6-((2-oxo-5-(trifluoromethyl)-2,3-dihydropyridin-3-yl)sulfonyl)-1,2,4,4a,5,6-hexahydro-3H-pyr azino[1,2-a]quinoxalin-3-yl)acetic acid and (S)-2-(8-(3-(difluoromethoxy)-5-fluorophenyl)-6 -((2-methoxy-5-(trifluoromethyl)pyridin-3-yl)sulfonyl)-1,2,4,4a,5,6-hexahydro-3H-pyrazino [1,2-a]quinoxalin-3-yl)acetic acid

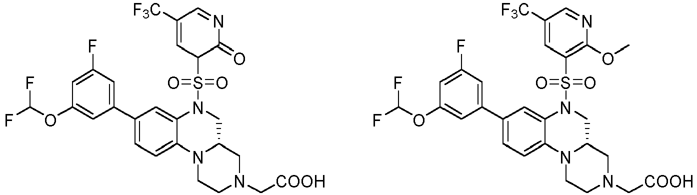

Methyl (*S*)-2-(8-(3-(difluoromethoxy)-5-fluorophenyl)-6-((2-(2-hydroxyethoxy)-5-(trifluoromethy 1)pyridin-3-yl)sulfonyl)-1,2,4,4a,5,6-hexahydro-3*H*-pyrazino[1,2-*a*]quinoxalin-3-yl)acetate (50 m g, 0.07 mmol) and lithium hydroxide monohydrate (30 mg, 0.7 mmol) were mixed in met hanol (5 mL) and H₂O (5 mL). The reaction mixture was stirred at 80 °C for 1hr; LCMS showed the reaction was completed, the reaction mixture was concentrated to dryness, an d the residue was added with DCM (10 mL) and H₂O (10 mL). The organic phases were separated and concentrated. The residue was firstly separated by a preparative TLC [eluent: DCM : MeOH = 15:1], and then separated by a reversed-phase column [eluent: H₂O : M eCN = 0-70%] to obtain 2-((4a*S*)-8-(3-(difluoromethoxy)-5-fluorophenyl)-6-((2-oxo-5-(trifluor omethyl)-2,3-dihydropyridin-3-yl)sulfonyl)-1,2,4,4a,5,6-hexahydro-3*H*-pyrazino[1,2-*a*]quinoxalin -3-yl)acetic acid (1.8 mg, 4%);
(Example 92): ¹H NMR (400 MHz, Methanol-*d*₄) δ 8.44 (d, *J* = 2.7 Hz, 1H), 8.21 (d d, *J* = 2.8, 1.3 Hz, 1H), 7.66 (d, *J* = 2.2 Hz, 1H), 7.36 (dd, *J* = 8.7, 2.2 Hz, 1H), 7.08 (dd, *J* = 11.4, 3.0 Hz, 3H), 6.91 (t, *J* = 73.2 Hz, 1H), 6.87 (d, *J* = 9.5 Hz, 1H), 4.41 (d d, *J* = 13.7, 3.5 Hz, 1H), 4.23 (d, *J* = 11.0 Hz, 1H), 4.14 (s, 2H), 3.77 (t, *J* = 10.1 Hz, 3H), 3.61 (dd, *J* = 13.8, 8.3 Hz, 1H), 3.30-3.20 (m, 2H), 3.07 (t, *J* = 11.6 Hz, 1H). ESI-MS: 633 [M+H]⁺, and
(*S*)-2-(8-(3-(difluoromethoxy)-5-fluorophenyl)-6-((2-methoxy-5-(trifluoromethyl)pyridin-3-y 1)sulfonyl)-1,2,4,4a,5,6-hexahydro-3*H*-pyrazino[1,2-*a*]quinoxalin-3-yl)acetic acid (3.5 mg, 8%).
(Example 93): ¹H NMR (400 MHz, Methanol-*d*₄) δ 8.80-8.72 (m, 1H), 8.56 (d, *J* = 2.4 Hz, 1H), 7.54 (d, *J* = 2.1 Hz, 1H), 7.42 (dd, *J* = 8.7, 2.2 Hz, 1H), 7.13 (d, *J* = 8.7 Hz, 1H), 7.10-7.04 (m, 2H), 6.91 (t, *J* = 73.2 Hz, 1H), 6.87 (d, *J* = 9.5 Hz, 1H), 4.34 (dd, *J* = 13.9, 3.5 Hz, 1H), 4.25 (d, *J* = 11.6 Hz, 1H), 4.05 (d, *J* = 3.7 Hz, 2H), 3.97 (s, 3H), 3.70 (t, *J* = 9.3 Hz, 2H), 3.58 (dd, *J* = 13.9, 8.8 Hz, 1H), 3.50-3.41 (m, 1H), 3. 24-3.11 (m, 2H), 2.94 (t, *J* = 11.6 Hz, 1H). ESI-MS: 647 [M+H]⁺.

### Example 94: preparation of (S)-2-(8-(3-cyano-2-fluorophenyl)-6-((3-(trifluoromethyl)phen yl)sulfonyl)-1,2,4,4a,5,6-hexahydro-3H-pyrazino[1,2-a]quinoxalin-3-yl)acetic acid

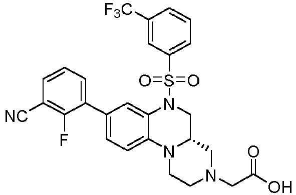

(*S*)-2-(8-bromo-6-((3-(trifluoromethyl)phenyl)sulfonyl)-1,2,4,4a,5,6-hexahydro-3*H*-pyrazino [1,2-*a*]quinoxalin-3-yl)acetic acid (30 mg, 0.056 mmol), 2-fluoro-3-(4,4,5,5-tetramethyl-1,3,2-dioxaborolan-2-yl)benzonitrile (17 mg, 0.067 mmol), [1,1'-bis(diphenylphosphino)ferrocene]pa lladium chloride (4 mg, 0.0056 mmol) and potassium carbonate (15 mg, 0.112 mmol) wer e mixed in 1,4-dioxane (3 mL) and water (1 mL). The nitrogen was charged to replace th ree times by evacuation. The mixture solution was reacted at a temperature of 90 °C for 2 hrs. After the reaction was completed, the reaction mixture was added with water (20 m L) and extracted three times with ethyl acetate (20 mL * 3), organic phases were combine d, washed with brine (30 mL), dried over anhydrous sodium sulfate, filtered and concentra ted. The residue was separated by a rapid silica gel column [eluent: MeOH : DCM = 0-4 0%] to obtain (*S*)-2-(8-(3-cyano-2-fluorophenyl)-6-((3-(trifluoromethyl)phenyl)sulfonyl)-1,2,4,4a, 5,6-hexahydro-3*H*-pyrazino[1,2-*a*]quinoxalin-3-yl) acetic acid, and then separated by a reversed -phase column chromatography [eluent: H₂O : MeCN = 0-60%, HCOOH] to obtain (*S*)-2-(8-(3-cyano-2-fluorophenyl)-6-((3-(trifluoromethyl)phenyl)sulfonyl)-1,2,4,4a,5,6-hexahydro-3*H*-p yrazino[1,2-*a*]quinoxalin-3-yl)acetic acid (3.5 mg, 11%). ESI-MS: 575.2 [M+H]⁺.
¹H NMR (400 MHz, Methanol-*d*₄) δ 7.98 (dd, *J* = 19.5, 7.9 Hz, 2H), 7.85-7.76 (m, 3H), 7.70 (td, *J* = 6.7, 5.7, 1.6 Hz, 1H), 7.62 (s, 1H), 7.48-7.36 (m, 2H), 6.99 (d, *J* = 8. 8 Hz, 1H), 4.39 (dd, *J* = 14.4, 4.2 Hz, 1H), 3.86 (d, *J* = 12.3 Hz, 1H), 3.41 (d, *J* = 12. 5 Hz, 2H), 3.36 (d, *J* = 9.6 Hz, 3H), 2.97 (d, *J* = 16.5 Hz, 1H), 2.70-2.56 (m, 2H), 2.36 (t, *J* = 11.2 Hz, 1H).

### Examples 95, 96 and 97 were prepared according to the synthesis method of Example 94.

| **Example No.** | **Structural formula** | **Chemical name** | **ESI-MS:** [M+1]⁺ |
|---|---|---|---|
| **95** | 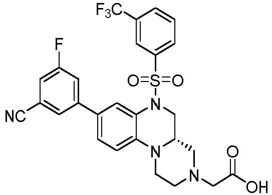 | (*S*)-2-(8-(3-cyano-5 -fluorophe nyl)-6-((3 -(trifluoromethyl)ph enyl)sulfonyl)-1,2,4,4a,5,6-he xahydro-3*H*-pyrazino[1,2-*a*]q uinoxalin-3-yl)acetic acid | 575.2 |
| **96** | 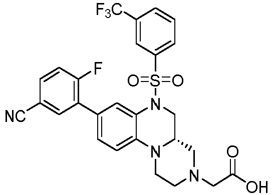 | (*S*)-2-(8-(5-cyano-2-fluorophe nyl)-6-((3 -(trifluoromethyl)ph enyl)sulfonyl)-1,2,4,4a,5,6-he xahydro-3*H*-pyrazino[1,2-*a*]q uinoxalin-3-yl)acetic acid | 575.2 |
| **97** | 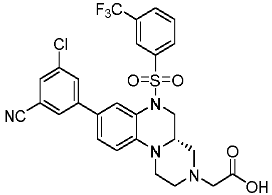 | (*S*)-2-(8-(3-chloro-5-cyanophe nyl)-6-((3 -(trifluoromethyl)ph enyl)sulfonyl)-1,2,4,4a,5,6-he xahydro-3*H*-pyrazino[1,2-*a*]q uinoxalin-3-yl)acetic acid | 591.2 |

### ¹H NMR data of the compound prepared in Examples 95, 96 and 97 were as follows:

**Example 95:** ¹H NMR (400 MHz, Methanol-*d*₄) δ 7.97 (t, *J* = 7.2 Hz, 2H), 7.87 (d, *J* = 2.3 Hz, 1H), 7.83-7.75 (m, 2H), 7.66 (dt, *J* = 10.1, 2.0 Hz, 1H), 7.60 (s, 1H), 7.53 (dd, *J* = 8.7, 2.3 Hz, 1H), 7.47 (dt, *J* = 8.1, 1.8 Hz, 1H), 6.99 (d, *J* = 8.8 Hz, 1H), 4.36 (dd, *J* = 14.5, 4.4 Hz, 1H), 3.92-3.73 (m, 1H), 3.36 (s, 3H), 3.22 (d, *J* = 10.0 Hz, 2H), 2.88 (s, 1H), 2.57 (d, *J* = 9.3 Hz, 2H), 2.28 (t, *J* = 11.1 Hz, 1H).
**Example 96:** ¹H NMR (400 MHz, Methanol-*d*₄) δ 8.00 (d, *J* = 7.9 Hz, 1H), 7.95 (d, *J* = 7.9 Hz, 1H), 7.87 (dd, *J* = 7.3, 2.1 Hz, 1H), 7.80 (d, *J* = 8.8 Hz, 2H), 7.73 (ddd, *J* = 8.6, 4.5, 2.1 Hz, 1H), 7.62 (s, 1H), 7.40 (dd, *J* = 10.4, 8.4 Hz, 2H), 6.97 (d, *J* = 8.8 Hz, 1H), 4.37 (dd, *J* = 14.9, 4.0 Hz, 1H), 3.81 (d, *J* = 9.4 Hz, 1H), 3.39-3.32 (m, 3H), 3.22 (d, *J* = 10.1 Hz, 2H), 2.94 (s, 1H), 2.57 (d, *J* = 8.7 Hz, 2H), 2.28 (d, *J* = 11.5 Hz, 1H).
**Example 97:** ¹H NMR (400 MHz, Methanol-*d*₄) δ 7.99 (t, *J* = 6.9 Hz, 2H), 7.90-7.86 (m, 2H), 7.84 (d, *J* = 2.3 Hz, 1H), 7.80 (t, *J* = 7.9 Hz, 1H), 7.72 (t, *J* = 1.6 Hz, 1H), 7.64 (s, 1H), 7.53 (dd, *J* = 8.7, 2.3 Hz, 1H), 7.02 (d, *J* = 8.8 Hz, 1H), 4.39 (dd, *J* = 14.5, 4.4 Hz, 1H), 3.92 (d, *J* = 13.3 Hz, 1H), 3.59 (s, 2H), 3.40-3.33 (m, 3H), 2.96 (s, 1H), 2.78 (dd, *J* = 13.4, 10.2 Hz, 1H), 2.63 (t, *J* = 12.5 Hz, 1H), 2.47 (t, *J* = 11.2 Hz, 1H).

### Example 98: preparation of (R)-8-(3-(difluoromethoxy)-5-fluorophenyl)-6-((3-(trifluoromethyl)p henyl)sulfonyl)-1,2,4,4a,5,6-hexahydro-[1,4]oxazino[4,3-a]quinoxaline

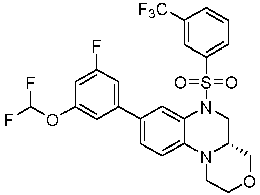

(*R*)-8-bromo-6-((3-(trifluoromethyl)phenyl)sulfonyl)-1,2,4,4a,5,6-hexahydro-[1,4]oxazino[4, 3-*a*]quinoxaline (80 mg, 0.16 mmol), [1,1'-bis(diphenylphosphino)ferrocene]palladium chlorid e (58 mg,0.08 mmol), potassium carbonate (58 mg, 0.42 mmol) and 2-(3-(difluoromethoxy) -5-fluorophenyl)-4,4,5,5-tetramethyl-1,3,2-pinacolborane (221 mg, 0.77 mmol) were placed in a three-necked flask. 1,4-dioxane (4 mL) and water (2 mL) were added thereto. The nitro gen was charged to replace three times by evacuation. The mixture solution was heated to 100 °C for 2 hrs. After the reaction was completed, the solvent was removed by concentr ation. The residue was separated by a rapid silica gel column to obtain (*R*)-8-(3-(difluorom ethoxy)-5-fluorophenyl)-6-((3-(trifluoromethyl)phenyl)sulfonyl)-1,2,4,4a,5,6-hexahydro-[1,4]oxaz ino[4,3-*a*]quinoxaline (60 mg, yield 64.5%). ESI-MS: 559.5 [M+H]⁺.
¹H NMR (500 MHz, Methanol-*d*₄) δ 7.95 (d, *J* = 7.8 Hz, 1H), 7.88 (d, *J* = 2.3 Hz, 1H), 7.86 (d, *J* = 8.1 Hz, 1H), 7.75 (t, *J* = 7.9 Hz, 1H), 7.65 (s, 1H), 7.47 (dd, *J* = 8.7, 2.3 Hz, 1H), 7.22 (dt, *J* = 9.9, 1.9 Hz, 1H), 7.19 (d, *J* = 2.0 Hz, 1H), 7.1-6.8 (t, 1H), 6.91-6.86 (m, 2H), 4.25 (dd, *J* = 14.4, 4.3 Hz, 1H), 3.77 (ddd, *J* = 31.8, 11.3, 3.3 Hz, 2H), 3.55 - 3.37 (m, 2H), 3.29-3.24 (m, 1H), 3.08-2.98 (m, 1H), 2.55-2.30 (m, 2H).

### Example 99 was prepared according to the synthesis method of Example 98.

| **Example No.** | **Structural formula** | **Chemical name** | **ESI-MS:** [M+1]⁺ |
|---|---|---|---|
| **99** | 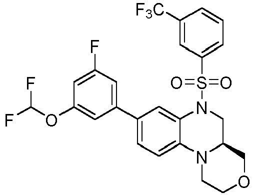 | (*S*)-8-(3-(difluoromethoxy) -5-fluorophenyl)-6-((3-(trifl uoromethyl)phenyl)sulfony 1)-1,2,4,4a,5,6-hexahydro-[1,4]oxazino[4,3-*a*]quinoxa line | 559.5 [M+H]⁺. |

### ¹H NMR data of the compound prepared in Example 99 was as follows:

**Example 99:** ¹H NMR (500 MHz, Methanol-*d*₄) δ 7.95 (d, *J* = 7.7 Hz, 1H), 7.88 (d, *J* = 2.2 Hz, 1H), 7.86 (d, *J* = 8.0 Hz, 1H), 7.75 (t, *J* = 7.9 Hz, 1H), 7.65 (s, 1H), 7.47 (dd, *J* = 8.7, 2.3 Hz, 1H), 7.22 (dt, *J* = 9.9, 1.9 Hz, 1H), 7.19 (d, *J* = 2.0 Hz, 1H), 7.1 - 6.81 (t, 1H),6.91 - 6.85 (m, 2H), 4.25 (dd, *J* = 14.4, 4.3 Hz, 1H), 3.77 (ddd, *J* = 32.0, 11.3, 3.4 Hz, 2H), 3.55-3.37 (m, 2H), 3.28-3.24 (m, 1H), 3.03 (t, *J* = 10.7 Hz, 1H), 2.58-2.31 (m, 2H).

### Example 100: preparation of 8-(3-(difluoromethoxy)-5-fluorophenyl)-6-((3-(trifluoromethy l)phenyl)sulfonyl)-1,2,4,4a,5,6-hexahydro-[1,4]thiazino[4,3-a]quinoxaline

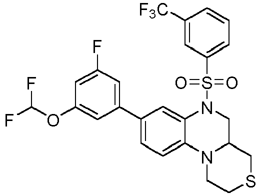

8-bromo-6-((3-(trifluoromethyl)phenyl)sulfonyl)-1,2,4,4a,5,6-hexahydro-[1,4]thiazino[4,3-*a*] quinoxaline (200 mg, 0.41 mmol), [1,1'-bis(diphenylphosphino)ferrocene]palladium chloride (58 mg,0.08 mmol), potassium carbonate (138 mg, 1.0 mmol) and 2-(3-(difluoromethoxy)-5 -fluorophenyl)-4,4,5,5-tetramethyl-1,3,2-pinacolborane (115 mg, 0.4 mmol) were placed in a three-necked flask; 1,4-dioxane (6 mL) and water (3 mL) were added thereto. The nitrogen was charged to replace three times by evacuation. The mixture solution was heated to 10 0°C for 2 hrs; after the reaction was completed, the solvent was removed by concentratio n. The residue was separated by a rapid silica gel column to obtain 8-(3-(difluoromethoxy) -5-fluorophenyl)-6-((3-(trifluoromethyl)phenyl)sulfonyl)-1,2,4,4a,5,6-hexahydro-[1,4]thiazino[4,3 -*a*]quinoxaline (230 mg, yield 98.7%). ESI-MS: 575.2 [M+H]⁺.
¹H NMR (500 MHz, CDCl₃) δ 7.84 (dd, *J* = 12.1, 7.8 Hz, 2H), 7.74-7.69 (m, 2H), 7.64 (t, *J*= 7.9 Hz, 1H), 7.36 (dd, J = 8.7, 2.3 Hz, 1H), 7.12 (dt, *J* = 9.5, 2.0 Hz, 1H), 7.09 (d, *J* = 2.0Hz, 1H), 6.80 (dt, *J* = 9.2, 2.3 Hz, 1H), 6.78-6.71 (m, 1H), 6.74-6.38 (t, 1H), 4.28 (dd, *J* = 14.4, 5.3 Hz, 1H), 3.93-3.80 (m, 1H), 3.41 (dd, *J* = 14.4, 10.0 Hz, 1H), 3.09-2.95 (m, 1H), 2.79-2.64 (m, 1H), 2.59-2.47 (m, 1H), 2.47-2.31 (m, 3H).

### Example 101: preparation of 8-(3-(difluoromethoxy)-5-fluorophenyl)-6-((3-(trifluoromethy l)phenyl)sulfonyl)-1,2,4,4a,5,6-hexahydro-[1,4]thiazino[4,3-a]quinoxaline 3-oxide

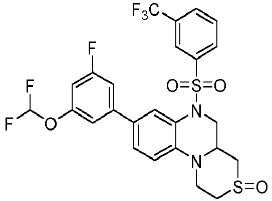

8-(3-(difluoromethoxy)-5-fluorophenyl)-6-((3-(trifluoromethyl)phenyl)sulfonyl)-1,2,4,4a,5,6-hexahydro-[1,4]thiazino[4,3-*a*]quinoxaline (60 mg, 0.1 mmol) was dissolved in dichlorometh ane (5 mL). 3-chloroperbenzoic acid (18 mg, 0.1 mmol) was added to thereto. The reactio n solution was stirred at room temperature for 16 hrs, and washed with saturated aqueous solution of sodium sulfite. The solvent was removed by concentration. The residue was se parated by a rapid silica gel column to obtain 8-(3-(difluoromethoxy)-5-fluorophenyl)-6-((3-(trifluoromethyl)phenyl)sulfonyl)-1,2,4,4a,5,6-hexahydro-[1,4]thiazino[4,3-*a*]quinoxaline 3-oxide (40 mg, yield 67.7%). ESI-MS: 591.2 [M+H]⁺.
¹H NMR (400 MHz, CDCl₃) δ 7.94 (dd, *J* = 55.9, 7.3 Hz, 2H), 7.80-7.58 (m, 3H), 7.42-7.32 (m, 1H), 7.13-7.01 (m, 2H), 6.91-6.77 (m, 2H), 6.75-6.37 (t, *J* = 3.3 Hz, 1H), 4.14 (d, *J* = 13.8 Hz, 1H), 3.97 (s, 1H), 3.75 (d, *J* = 13.8 Hz, 1H), 3.52 (d, *J* = 13.8 Hz, 1H),3.34(s, 1H), 3.08(s, 1H) 2.80 (t, *J* = 15.7 Hz, 1H), 2.73- 2.53 (m, 1H), 2.44 (s, 1H).

### Example 102: preparation of 8-(3-(difluoromethoxy)-5-fluorophenyl)-6-((3-(trifluoromethy l)phenyl)sulfonyl)-1,2,4,4a,5,6-hexahydro-[1,4]thiazino[4,3-a]quinoxaline 3,3-dioxide

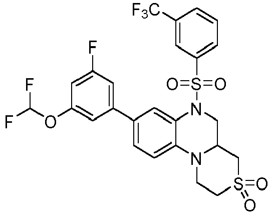

It was prepared according to the synthesis method of Example 101. 3 equivalents of 3-chloroperbenzoic acid were used. ESI-MS: 591.2 [M+H]⁺.
¹H NMR (400 MHz, CDCl₃) δ 8.14-8.00 (m, 3H), 8.00-7.91 (m, 2H), 7.78 (t, *J* = 7.7 Hz, 1H), 7.50 (d, *J* = 8.3 Hz, 1H), 7.12-7.02 (m, 2H), 6.96 (d, *J* = 9.0 Hz, 1H), 6.76-6.41(t, *J* = 3.1 Hz, 1H), 4.99 (s, 1H), 4.68 (s, 1H), 4.47 (t, *J* = 13.5 Hz, 1H), 4.20 (d, *J* = 21.0 Hz, 3H), 3.81 (t, *J* = 12.4 Hz, 1H), 3.19 (d, *J* = 14.5 Hz, 1H), 3.02 (d, *J* = 13.6 Hz, 1H).

### Example 103: preparation of (6aS)-3-(3-(difluoromethoxy)-5-fluorophenyl)-8-fluoro-5-((3-(trifluoromethyl)phenyl)sulfonyl)-6,6a,7,8,9,10-hexahydro-5H-pyrido[1,2-a]quinoxaline

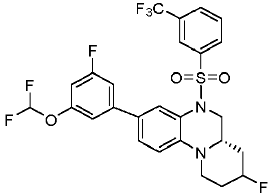

### Step 1: synthesis of (6aS)-3-bromo-8-fluoro-7,8,9,10-tetrahydro-5H-pyrido[1,2-a]quinoxali n-6(6aH)-one

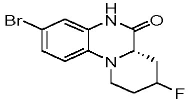

(6a*S*)-3-bromo-8-hydroxy-7,8,9,10-tetrahydro-5*H*-pyrido[1,2-*a*]quinoxalin-6(6a*H*)-one(60 mg, 0.2 mmol) was dissolved in dichloromethane (5 mL), and the mixture solution was co oled to -70 °C. DAST (33 mg, 0.2 mmol) was added thereto. The mixture solution was st irred at -30 °C for 2 hrs, then naturally warmed to room temperature and stirred for 16 h rs. The mixture solution was diluted with 15 mL of ethyl acetate, washed with brine (15 mL * 3), dried over anhydrous sodium sulfate, filtered and concentrated to remove the sol vent. The residue was separated by a rapid silica gel column to obtain (6a*S*)-3-bromo-8-flu oro-7,8,9,10-tetrahydro-5*H*-pyrido[1,2-*a*]quinoxalin-6(6a*H*)-one (40 mg, yield 66.8%). ESI-M S: 299, 300 [M+H]⁺.

### Step 2: synthesis of (6aS)-3-bromo-8-fluoro-6,6a,7,8,9,10-hexahydro-5H-pyrido[1,2-a]quinoxalin e

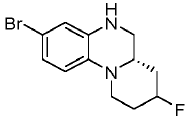

(6a,*S*)-3-bromo-8-fluoro-7,8,9,10-tetrahydro-5*H*-pyrido[1,2-*a*]quinoxalin-6(6a*H*)-one (40 m g, 0.12 mmol) was dissolved in 4M borane-tetrahydrofuran (5 mL). The reaction mixture was stirred at 30 °C for 2 hrs. Methanol was added dropwise to quench the reaction. The reaction mixture was concentrated to remove the solvent. The residue was separated by a r apid silica gel column to obtain (6a*S*)-3-bromo-8-fluorine-6,6a,7,8,9,10-hexahydro-5*H*-pyrido [1,2-*a*]quinoxaline (17 mg, yield 49.7%). ESI-MS: 285, 287 [M+H]⁺.

### Step 3: synthesis of (6aS)-3-bromo-8-fluoro-5-((3-(trifluoromethyl)phenyl)sulfonyl)-6,6a,7, 8,9,10-hexahydro-5H-pyrido[1,2-a]quinoxaline

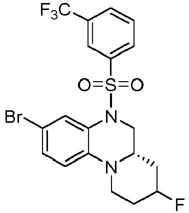

(6a,*S*)-3-bromo-8-fluoro-6,6a,7,8,9,10-hexahydro-5*H*-pyrido[1,2-*a*]quinoxaline (17 mg, 0.05 9 mmol) was dissolved in dichloromethane (5 mL). Pyridine (0.2 mL), dimethylaminopyrid ine (1 mg, 0.005 mmol) and 3-(trifluoromethyl)benzenesulfonyl chloride (18 mg, 0.072 mm ol) were added thereto. The mixture solution was reacted at 25 °C for 4 hrs. The solvent was removed by concentration. The residue was dissolved in ethyl acetate (15 mL), succes sively washed with water (10 mL), saturated aqueous solution of sodium hydrogencarbonat e (10 mL) and brine (10 mL), dried over anhydrous sodium sulfate, filtered and then conc entrated to remove the solvent. The residue was separated by a rapid silica gel column to obtain (6a*S*)-3-bromo-8-fluoro-5-((3-(trifluoromethyl)phenyl)sulfonyl)-6,6a,7,8,9,10-hexahydro-5 *H*-pyrido[1,2-*a*]quinoxaline (30 mg, yield 100%). ESI-MS: 493, 495 [M+H]⁺.

### Step 4: synthesis of (6aS)-3-(3-(difluoromethoxy)-5-fluorophenyl)-8-fluoro-5-((3-(trifluoro methyl)phenyl)sulfonyl)-6,6a,7,8,9,10-hexahydro-5H-pyrido[1,2-a]quinoxaline

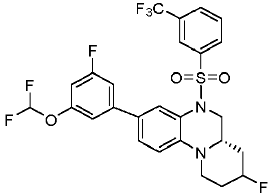

(6a*S*)-3-bromo-8-fluoro-5-((3-(trifluoromethyl)phenyl)sulfonyl)-6,6*a*,7,8,9,10-hexahydro-5*H-*pyrido[1,2-*a*]quinoxaline (30 mg, 0.059 mmol), [1,1'-bis(diphenylphosphino)ferrocene]palladiu m chloride (29 mg, 0.04 mmol), potassium carbonate (21 mg, 0.15 mmol) and 2-(3-(difluo romethoxy)-5-fluorophenyl)-4,4,5,5-tetramethyl-1,3-dioxalane (17 mg, 0.06 mmol) were place d in a three-necked flask; 1,4-dioxane (4 mL) and water (2 mL) were added thereto. Nitro gen was charged to replace three times by evacuation. The mixture soltuion was heated to 60 °C for 2 hrs. After the reaction was completed, the reaction mixture was concentrated to remove the solvent. The residue was separated by a rapid silica gel column to obtain (6a*S*)-3-(3-(difluoromethoxy)-5-fluorophenyl)-8-fluoro-5-((3-(trifluoromethyl)phenyl)sulfonyl)-6, 6a,7,8,9,10-hexahydro-5*H*-pyrido[1,2-*a*]quinoxaline (15 mg, yield 44.3%). ESI-MS: 575.6 [M +1]⁺.
¹H NMR (400 MHz, CDCl₃) δ 7.86-7.73 (m, 4H), 7.61 (t, *J* = 7.8 Hz, 1H), 7.35 (d d, *J* = 8.7, 2.3 Hz, 1H), 7.12 (dt, *J* = 9.5, 1.9 Hz, 1H), 7.09 (d, *J* = 2.1 Hz, 1H), 6.90 (d, *J* = 8.7 Hz, 1H), 6.81 (dt, *J* = 9.3, 2.3 Hz, 1H), 6.75-6.38 (t, *J* = 73.3 Hz, 1H), 4.45 (ddt, *J* = 48.6, 10.5, 5.6 Hz, 1H), 4.29 (dd, *J* = 14.4, 4.3 Hz, 1H), 3.82-3.70 (m, 1H), 3.41 (dd, *J* = 14.4, 10.4 Hz, 1H), 2.62 (s, 1H), 2.30 (t, *J* = 12.7 Hz, 1H), 2.14 (s, 2H), 1.65 (m, 1H), 1.37 (m, 1H).

### Example 104: preparation of (6aS)-3-(3-(difluoromethoxy)-5-fluorophenyl)-5-((3-(trifluoro methyl)phenyl)sulfonyl)-6,6a,7,8,9,10-hexahydro-5H-pyrido[1,2-a]quinoxalin-8-yl carbamat e

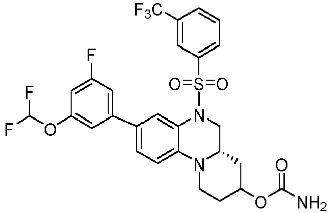

### Step 1: synthesis of (6aS)-3-bromo-5-((3-(trifluoromethyl)phenyl)sulfonyl)-6,6a,7,8,9,10-he xahydro-5H-pyrido[1,2-a]quinoxalin-8-yl carbamate

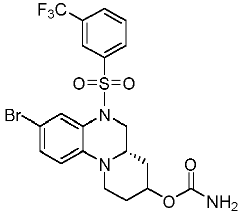

(6a*S*)-3-bromo-5-((3-(trifluoromethyl)phenyl)sulfonyl)-6,6a,7,8,9,10-hexahydro-5*H*-pyrido[1, 2-*a*]quinoxalin-8-ol (40 mg, 0.08 mmol) was dissolved in tetrahydrofuran (5 mL). Carbonyl diimidazole (20 mg, 0.12 mmol) was added thereto. The mixture solution was stirred at 2 5 °C for 16 hrs. Ammonia liquor (2 mL) was added thereto. The reaction mixture was sti rred for an additional 2 hrs, diluted with 15 mL of ethyl acetate, washed with water (15 mL * 3), dried over anhydrous sodium sulfate, filtered and concentrated to remove the sol vent. The residue was separated by a rapid silica gel column to obtain (6a*S*)-3-bromo-5-((3 -(trifluoromethyl)phenyl)sulfonyl)-6,6a,7,8,9,10-hexahydro-5*H*-pyrido[1,2-*a*]quinoxalin-8-yl carb amate (45 mg, yield 100%). ESI-MS: 534, 536 [M+H]⁺.

### Step 2: synthesis of (6aS)-3-(3-(difluoromethoxy)-5-fluorophenyl)-5-((3-(trifluoromethyl)p henyl)sulfonyl)-6,6a,7,8,9,10-hexahydro-5H-pyrido[1,2-a]quinoxalin-8-yl carbamate

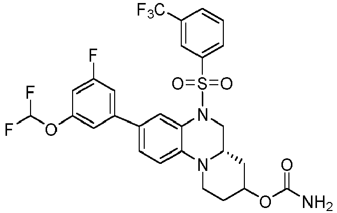

(6a*S*)-3-bromo-5-((3-(trifluoromethyl)phenyl)sulfonyl)-6,6a,7,8,9,10-hexahydro-5*H*-pyrido[1, 2-*a*]quinoxalin-8-yl carbamate (45 mg, 0.08 mmol), [1,1'-bis(diphenylphosphino)ferrocene]palladi um chloride (29 mg, 0.04 mmol), potassium carbonate (28 mg, 0.2 mmol) and 2-(3-(difluor omethoxy)-5-fluorophenyl)-4,4,5,5-tetramethyl-1,3-dioxalane (23 mg, 0.08 mmol) were placed in a three-necked flask; 1,4-dioxane (4 mL) and water (2 mL) were added thereto. The n itrogen was charged to replace three times by evacuation. The mixture solution was heated to 60 °C for 2 hrs. After the reaction was completed, the reaction mixture was concentra ted to remove the solvent. The residue was separated by a rapid silica gel column to obta in (6a*S*)-3-(3-(difluoromethoxy)-5-fluorophenyl)-5-((3-(trifluoromethyl)phenyl)sulfonyl)-6,6a,7,8, 9,10-hexahydro-5*H*-pyrido[1,2-*a*]quinoxalin-8-yl carbamate (20 mg, yield 40.6%). ESI-MS: 6 16.6 [M+1]⁺.
¹H NMR (400 MHz, CDCl₃) δ 7.82 (t, *J* = 7.0 Hz, 2H), 7.79 (d, *J* = 2.2 Hz, 1H), 7.67 (s, 1H), 7.63 (t, *J* = 7.7 Hz, 1H), 7.38-7.31 (m, 1H), 7.13 (dd, *J* = 9.6, 2.2 Hz, 1 H), 7.10 (s, 1H), 6.80 (d, *J* = 9.1 Hz, 1H), 6.75-6.38 (t, 1H), 6.66 (d, *J* = 73.3 Hz, 1H), 4.54 (d, *J* = 15.3 Hz, 2H), 4.25 (dd, *J* = 14.4, 4.3 Hz, 1H), 3.77 (d, *J* = 13.7 Hz, 1H), 3.37 (dd, *J* = 14.4, 10.1 Hz, 2H), 2.59 (s, 1H), 2.28 (t, *J* = 12.8 Hz, 1H), 2.04 (s, 2H).

### Example 105: preparation of (6aS)-3-(3-(difluoromethoxy)-5-fluorophenyl)-5-((3-(trifluoro methyl)phenyl)sulfonyl)-6,6a,7,8,9,10-hexahydro-5H-pyrido[1,2-a]quinoxalin-8-ol

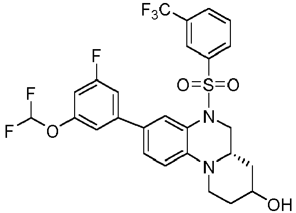

(6a*S*)-3-bromo-5-((3-(trifluoromethyl)phenyl)sulfonyl)-6,6a,7,8,9,10-hexahydro-5*H*-pyrido[1, 2-*a*]quinoxalin-8-ol (50 mg, 0.1 mmol), [1,1'-bis(diphenylphosphino)ferrocene]palladium chlor ide (29 mg, 0.04 mmol), potassium carbonate (35 mg, 0.25 mmol) and 2-(3-(difluorometho xy)-5-fluorophenyl)-4,4,5,5-tetramethyl-1,3-dioxalane (29 mg, 0.1 mmol) were placed in a th ree-necked flask; 1,4-dioxane (4 mL) and water (2 mL) were added thereto. Nitrogen was charged to replace three times by evacuation. The mixture soltuion was heated to 60 °C f or 2 hrs. After the reaction was completed, the reaction mixture was concentrated to remo ve the solvent. The residue was separated by a rapid silica gel column to obtain (6a*S*)-3-(3-(difluoromethoxy)-5-fluorophenyl)-5-((3-(trifluoromethyl)phenyl)sulfonyl)-6,6a,7,8,9,10-hexah ydro-5*H*-pyrido[1,2-*a*]quinoxalin-8-ol (50 mg, yield 87.3%). ESI-MS: 573.5 [M+1]⁺.
¹H NMR (400 MHz, CDCl₃) δ 7.82 (d, *J* = 8.4 Hz, 3H), 7.76 (d, *J* = 7.9 Hz, 1H), 7.60 (t, *J* = 7.7 Hz, 1H), 7.35 (dd, *J* = 8.7, 2.3 Hz, 1H), 7.15-7.06 (m, 2H), 6.96 (s, 1 H), 6.82 (d, *J* = 9.1 Hz, 1H), 6.75-6.38 (t, 1H),4.28 (dd, *J* = 14.4, 4.2 Hz, 1H), 3.79-3.5 8 (m, 2H), 3.50 (s, 1H), 2.66 (s, 1H), 2.05 (s, 2H), 1.47 (d, *J* = 12.6 Hz, 2H), 1.23 (d, *J* = 12.7 Hz, 1H).

### Example 106: preparation of 2-(((6aS)-3-(3-(difluoromethoxy)-5-fluorophenyl)-5-((3-(triflu oromethyl)phenyl)sulfonyl)-6,6a,7,8,9,10-hexahydro-5H-pyrido[1,2-a]quinoxalin-8-yl)oxy)ac etic acid

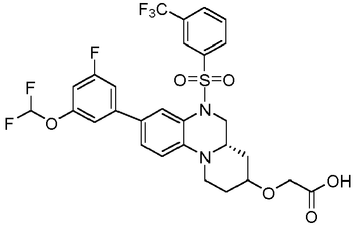

### Step 1: synthesis of tert-butyl 2-(((6aS)-3-bromo-5-((3-(trifluoromethyl)phenyl)sulfonyl)-6, 6a,7,8,9,10-hexahydro-5H-pyrido[1,2-a]quinoxalin-8-yl)oxy)acetate

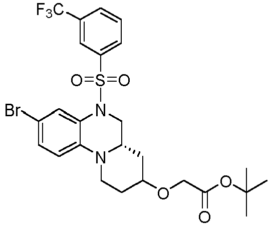

(6a*S*)-3-bromo-5-((3-(trifluoromethyl)phenyl)sulfonyl)-6,6a,7,8,9,10-hexahydro-5*H*-pyrido[1, 2-*a*]quinoxalin-8-ol (40 mg, 0.08 mmol) was dissolved in toluene (5 mL), 2M sodium hyd roxide aqueous solution (5 mL), tetrabutylammonium bromide (129 mg, 0.4 mmol) and *ter t*-butyl bromoacetate (78 mg, 0.4 mmol) were added thereto. The mixture solution was stir red at 25 °C for 24 hrs. The reaction mixture was diluted with 15 mL of ethyl acetate, w ashed with water (15 mL * 3), dried over anhydrous sodium sulfate, filtered and concentra ted to remove the solvent. The residue was separated by a rapid silica gel column to obta in tert-butyl 2-(((6a*S*)-3-bromo-5-((3-(trifluoromethyl)phenyl)sulfonyl)-6,6a,7,8,9,10-hexahydro-5*H*-pyrido[1,2-*a*]quinoxalin-8-yl)oxy)acetate (35 mg, yield 72.3%). ESI-MS: 605, 607 [M+ H]⁺.

### Step 2: synthesis of 2-(((6aS)-3-bromo-5-((3-(trifluoromethyl)phenyl)sulfonyl)-6,6a,7,8,9,10 -hexahydro-5H-pyrido[1,2-a]quinoxalin-8-yl)oxy)acetic acid

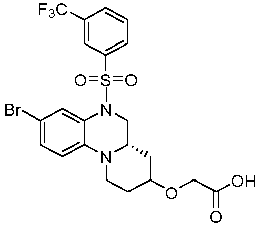

Tert-butyl 2-(((6a*S*)-3-bromo-5-((3-(trifluoromethyl)phenyl)sulfonyl)-6,6a,7,8,9,10-hexahydro-5 *H*-pyrido[1,2-*a*]quinoxalin-8-)oxy)acetate (35 mg, 0.057 mmol) was dissolved in dichloromet hane (3 mL). A 4M solution of HCl in dioxane (3 mL) was added thereto. The mixture s olution was stirred at 25 °C for 1 hr and concentrated to remove the solvent. The residue was separated by a rapid silica gel column to obtain 2-(((6a*S*)-3-bromo-5-((3-(trifluoromethy l)phenyl)sulfonyl)-6,6a,7,8,9,10-hexahydro-5*H*-pyrido[1,2-*a*]quinoxalin-8-yl)oxy)acetic acid (30 mg, yield 95.8%). ESI-MS: 547, 549 [M-H]⁻.

### Step 3: synthesis of 2-(((6aS)-3-(3-(difluoromethoxy)-5-fluorophenyl)-5-((3-(trifluoromethy l)phenyl)sulfonyl)-6,6a,7,8,9,10-hexahydro-5H-pyrido[1,2-a]quinoxalin-8-yl)oxy)acetic acid

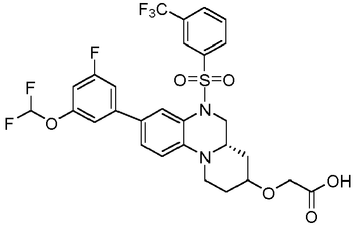

2-(((6a*S*)-3-bromo-5-((3-(trifluoromethyl)phenyl)sulfonyl)-6,6a,7,8,9,10-hexahydro-5*H*-pyrid o[1,2-*a*]quinoxalin-8-yl)oxy)acetic acid (30 mg, 0.054 mmol), [1,1'-bis(diphenylphosphino)fer rocene]palladium chloride (15 mg, 0.02 mmol), potassium carbonate (18 mg, 0.14 mmol) a nd 2-(3-(difluoromethoxy)-5-fluorophenyl)-4,4,5,5-tetramethyl-1,3-dioxalane (16 mg, 0.054 m mol) were placed in a three-necked flask; 1,4-dioxane (4 mL) and water (2 mL) were add ed thereto. Nitrogen was charged to replace three times by evacuation. The mixture soltuio n was heated to 60 °C for 2 hrs. After the reaction was completed, the solvent was remo ved by concentration. The residue was firstly separated by a rapid silica gel column, and t hen separated by a reversed-phase column to obtain 2-(((6a*S*)-3-(3-(difluoromethoxy)-5-fluor ophenyl)-5-((3-(trifluoromethyl)phenyl)sulfonyl)-6,6a,7,8,9,10-hexahydro-5*H*-pyrido[1,2-*a*]quino xalin-8-yl)oxy)acetic acid (9.8 mg, yield 28.7%). ESI-MS: 631.6 [M+1]⁺.
¹H NMR (400 MHz, CDCl₃) δ 7.82 (d, *J* = 6.7 Hz, 2H), 7.79-7.73 (m, 2H), 7.60 (t, *J* = 8.0 Hz, 1H), 7.35 (dd, *J* = 8.6, 2.3 Hz, 1H), 7.11 (d, *J* = 9.5 Hz, 1H), 7.08 (s, 1H), 6.87 (d, *J* = 8.8 Hz, 1H), 6.80 (d, *J* = 9.2 Hz, 1H), 6.74 (s, 1H), 6.74-6.38(t, 1H)4.28 (dd, *J* = 14.4, 4.1 Hz, 1H), 4.15 (s, 2H), 3.79 (d, *J* = 13.1 Hz, 1H), 3.38 (d, *J* = 10.4 Hz, 2H), 2.58 (s, 1H), 2.24 (d, *J* = 13.2 Hz, 1H), 2.08 (s, 2H),1.25-1.15(d, *J* = 11.24,1H) 1.21 (d, *J* = 11.2 Hz, 1H).

### Example 107: preparation of (6aS,8R)-3-(3-(difluoromethoxy)-5-fluorophenyl)-8-methyl-5-((3-(trifluoromethyl)phenyl)sulfonyl)-6,6a,7,8,9,10-hexahydro-5H-pyrido[1,2-a]quinoxalin-8-ol

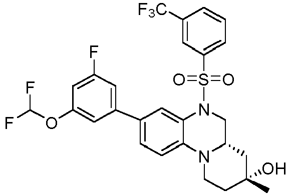

### Step 1: synthesis of (6aS, 8R)-3-bromo-8-methyl-5-((3-(trifluoromethyl)phenyl)sulfonyl)-6, 6a,7,8,9,10-hexahydro-5H-pyrido[1,2-a]quinoxalin-8-ol

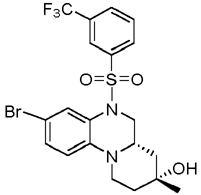

(*S*)-3-bromo-5-((3-(trifluoromethyl)phenyl)sulfonyl)-5,6,6a,7,9,10-hexahydro-8*H*-pyrido[1,2-*a*]quinoxalin-8-one (50 mg, 0.1 mmol) was dissolved in tetrahydrofuran (5 mL), and the m ixture solution was cooled to -15 °C. A 3M solution of methylmagnesium bromide in dim ethyltetrahydrofuran (0.04 mL, 0.12 mmol) was added under nitrogen protection. The mixtu re solution was naturally raised to 25 °C and stirred for 2 hrs, the reaction was quenched with ammonium chloride, diluted with 15 mL of ethyl acetate, washed with brine (15 mL * 3), dried over anhydrous sodium sulfate, filtered and concentrated to remove the solvent. The residue was separated by a rapid silica gel column to obtain (6aS, 8*R*)-3-bromo-8-me thyl-5-((3-(trifluoromethyl)phenyl)sulfonyl)-6,6a,7,8,9,10-hexahydro-5*H*-pyrido[1,2-*a*]quinoxalin-8-ol (25 mg, yield 50.0%). ESI-MS: 505, 507 [M+H]⁺.

### Step 2: synthesis of (6aS,8R)-3-(3-(difluoromethoxy)-5-fluorophenyl)-8-methyl-5-((3-(triflu oromethyl)phenyl)sulfonyl)-6,6a,7,S,9,10-hexahydro-5H-pyrido[1,2-a]quinoxalin-8-ol

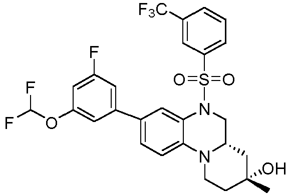

(6a*S*,8*R*)-3-bromo-8-methyl-5-((3-(trifluoromethyl)phenyl)sulfonyl)-6,6a,7,8,9,10-hexahydro -5*H*-pyrido[1,2-*a*]quinoxalin-8-ol (25 mg, 0.049 mmol), [1,1'-bis(diphenylphosphino)ferrocen e]palladium chloride (15 mg, 0.02 mmol), potassium carbonate (17 mg, 0.12 mmol) and 2-(3-(difluoromethoxy)-5-fluorophenyl)-4,4,5,5-tetramethyl-1,3-dioxalane (14 mg, 0.049 mmol) were placed in a three-necked flask; 1,4-dioxane (4 mL) and water (2 mL) were added th ereto. Nitrogen was charged to replace three times by evacuation. The mixture solution wa s heated to 60 °C for 2 hrs. After the reaction was completed, the solvent was removed b y concentration. The residue was separated by a rapid silica gel column and then separate d by a reversed-phase column to obtain (6a*S*,8*R*)-3-(3-(difluoromethoxy)-5-fluorophenyl)-8-m ethyl-5-((3-(trifluoromethyl)phenyl)sulfonyl)-6,6a,7,8,9,10-hexahydro-5*H*-pyrido[1,2-*a*]quinoxali n-8-ol (3.8 mg, yield 13.2%). ESI-MS: 587 [M+H]⁺.
¹H NMR (400 MHz, CDCl₃) δ 7.82 (dd, *J* = 7.7, 5.3 Hz, 3H), 7.67 (d, *J* = 7.8 Hz, 1H), 7.58 (t, *J* = 7.7 Hz, 1H), 7.35 (dd, *J* = 8.7, 2.3 Hz, 1H), 7.14 (dt, *J* = 9.6, 1.9 Hz, 1H), 7.11 (d, *J* = 2.1 Hz, 1H), 6.81 (t, *J* = 8.9 Hz, 2H), 6.75-6.38 (t, 1H), 4.23 (dd, *J* = 14.4, 4.0 Hz, 1H), 3.70 (dt, *J* = 13.0, 4.1 Hz, 1H), 3.25 (dd, *J* = 14.4, 10.4 Hz, 1H), 2.39 (t, *J* = 11.2 Hz, 1H), 2.22 (td, *J* = 12.6, 3.7 Hz, 1H), 1.27 (q, *J* = 11.7, 11.1 Hz, 2 H), 1.07 (s, 3H).

### Example 108 was prepared according to the synthesis method of Example 107.

| **Example No.** | **Structural formula** | **Chemical name** | **ESI-MS:** [M+1]⁺ |
|---|---|---|---|
| **108** | 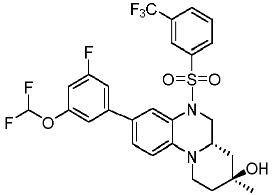 | (6a*S*,8*S*)-3-(3 -(difluoromethox y)-5-fluorophenyl)-8-methyl-5 -((3-(trifluoromethyl)phenyl)s ulfonyl)-6,6a,7,8,9,10-hexahy dro-5*H*-pyrido[1,2-*a*]quinoxali n-8-ol | 587 |

### ¹H NMR data of the compound prepared in Example 108 was as follows:

**Example 108:** ¹H NMR (400 MHz, CDCl₃) δ 7.83-7.75 (m, 3H), 7.70 (d, *J* = 7.9 Hz, 1H), 7.57 (t, *J* = 8.1 Hz, 1H), 7.33 (dd, *J* = 8.7, 2.3 Hz, 1H), 7.14 (dt, *J* = 9.5, 1.9 Hz, 1H), 7.11 (d, *J* = 2.1 Hz, 1H), 6.79 (t, *J* = 8.9 Hz, 2H), 6.75-6.38 (t, 1H), 4.20 (dd, *J* = 14.3, 4.3 Hz, 1H), 3.72 (q, *J* = 7.0 Hz, 1H), 3.49 (s, 1H), 3.27 (dd, *J* = 14.3, 10.4 Hz, 1H), 2.84 (td, *J* = 11.5, 9.1, 5.4 Hz, 1H), 2.49 (td, *J* = 12.0, 4.1 Hz, 1H), 1.3 (m, 1H), 1.23 (s, 3H), 1.15 (t, *J* = 12.4 Hz, 1H).

### Example 109: preparation of 3-(((6aS)-3-(3-(difluoromethoxy)-5-fluorophenyl)-5-((3-(triflu oromethyl)phenyl)sulfonyl)-6,6a,7,8,9,10-hexahydro-5H-pyrido[1,2-a]quinoxalin-8-yl)oxy)pr opanoic acid

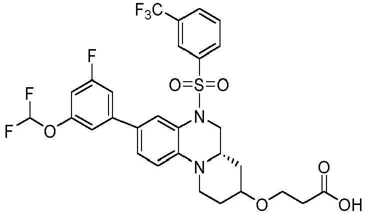

### Step 1: synthesis of tert-butyl 3-(((6aS)-3-bromo-5-((3-(trifluoromethyl)phenyl)sulfonyl)-6, 6a,7,8,9,10-hexahydro-5H-pyrido[1,2-a]quinoxalin-8-yl)oxy)propanoate

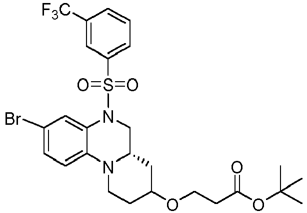

(6a*S*)-3-bromo-5-((3-(trifluoromethyl)phenyl)sulfonyl)-6,6a,7,8,9,10-hexahydro-5*H*-pyrido[1, 2-*a*]quinoxalin-8-ol (150 mg, 0.3 mmol) was dissolved in toluene (5 mL). Potassium hydro xide (17 mg, 0.3 mmol) and tert-butyl acrylate (192 mg, 1.5 mmol) were added thereto. T he mixture solution was stirred at 110 °C for 24 hrs. The reaction mixture was cooled, an d then diluted with 15 mL of ethyl acetate, washed with water (15 mL*3), dried over anh ydrous sodium sulfate, filtered and concentrated to remove the solvent. The residue was se parated by a rapid silica gel column to obtain tert-butyl 3-(((6a*S*)-3-bromo-5-((3-(trifluorom ethyl)phenyl)sulfonyl)-6,6a,7,8,9,10-hexahydro-5*H*-pyrido[1,2-*a*]quinoxalin-8-yl)oxy)propanoate (110 mg, yield 59.2%). ESI-MS: 619, 621 [M+H]⁺.

### Step 2: synthesis of tert-butyl 3-(((6aS)-3-(3-(difluoromethoxy)-5-fluorophenyl)-5-((3-(trifl uoromethyl)phenyl)sulfonyl)-6,6a,7,8,9,10-hexahydro-5H-pyrido[1,2-a]quinoxalin-8-yl)oxy)p ropanoate

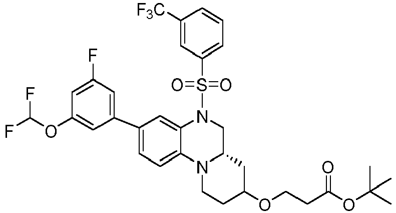

*Tert*-butyl 3-(((6a*S*)-3-bromo-5-((3-(trifluoromethyl)phenyl)sulfonyl)-6,6a,7,8,9,10-hexahydro-5 *H*-pyrido[1,2-*a*]quinoxalin-8-yl)oxy)propanoate (110 mg, 0.17 mmol), [1,1'-bis(diphenylphosphin o)ferrocene]palladium chloride (22 mg, 0.03 mmol), potassium carbonate (59 mg, 0.43 mmo 1) and 2-(3-(difluoromethoxy)-5-fluorophenyl)-4,4,5,5-tetramethyl-1,3-dioxalane (51 mg, 0.17 mmol) were placed in a three-necked flask; 1,4-dioxane (4 mL) and water (2 mL) were a dded thereto. Nitrogen was charged to replace three times by evacuation. The mixture solu tion was heated to 60 °C for 2 hrs. After the reaction was completed, the solvent was re moved by concentration. The residue was separated by a rapid silica gel column and then separated by a reversed-phase column to obtain *tert*-butyl 3-(((6a*S*)-3-(3-(difluoromethoxy)-5-fluorophenyl)-5-((3-(trifluoromethyl)phenyl)sulfonyl)-6,6a,7,8,9,10-hexahydro-5*H*-pyrido[1,2-*a*]qu inoxalin-8-yl)oxy)propanoate (100 mg, yield 84.0%), which was directly used in the next st ep.

### Step 3: synthesis of 3-(((6aS)-3-(3-(difluoromethoxy)-5-fluorophenyl)-5-((3-(trifluoromethy l)phenyl)sulfonyl)-6,6a,7,8,9,10-hexahydro-5H-pyrido[1,2-a]quinoxalin-8-yl)oxy)propanoic a cid

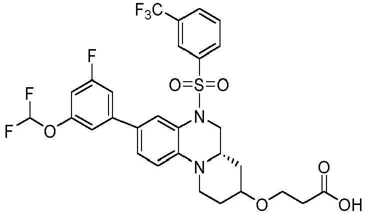

*Tert*-butyl 3-(((6aS)-3-(3-(difluoromethoxy)-5-fluorophenyl)-5-((3-(trifluoromethyl)phenyl)s ulfonyl)-6,6a,7,8,9,10-hexahydro-5*H*-pyrido[1,2-*a*]quinoxalin-8-yl)oxy)propanoate (100 mg, 0.1 4 mmol) was dissolved in dichloromethane (3 mL). Trifluoroacetic acid (3 mL) was added thereto. The mixture solution was stirred at 25 °C for 1 hr and concentrated to remove t he solvent. The residue was separated by a rapid silica gel column to obtain 3-(((6a*S*)-3-(3 -(difluoromethoxy)-5-fluorophenyl)-5-((3-(trifluoromethyl)phenyl)sulfonyl)-6,6a,7,8,9,10-hexahy dro-5*H*-pyrido[1,2-*a*]quinoxalin-8-yl)oxy)propanoic acid (30 mg, yield 33.3%). ESI-MS: 645. 2 [M+1]⁺.
¹H NMR (500 MHz, CDCl₃) δ 7.84-7.71 (m, 4H), 7.59 (t, *J* = 7.8 Hz, 1H), 7.33 (d d, *J* = 8.7, 2.3 Hz, 1H), 7.12 (dt, *J* = 9.5, 2.0 Hz, 1H), 7.09 (d, *J* = 2.0 Hz, 1H), 6.85-6.76 (m, 2H), 6.74-6.37 (t, 1H), 4.25 (dd, *J* = 14.4, 4.3 Hz, 1H), 3.73 (t, *J* = 6.1 Hz, 3 H), 3.31 (dd, *J* = 14.4, 10.3 Hz, 1H), 3.21 (td, *J* = 10.7, 5.2 Hz, 1H), 2.61 (t, *J* = 6.1 Hz, 2H), 2.50 (t, *J* = 10.7 Hz, 1H), 2.18 (t, *J* = 12.5 Hz, 1H), 1.99 (d, *J* = 12.2 Hz, 2 H), 1.36 (qd, *J* = 12.7, 4.5 Hz, 1H), 1.05 (q, *J* = 11.5 Hz, 1H).

### Example 110: preparation of (6aS)-3-(3-(difluoromethoxy)-5-fluorophenyl)-8-((methylsulfony l)methoxy)-5-((3-(trifluoromethyl)phenyl)sulfonyl)-6,6a,7,8,9,10-hexahydro-5H-pyrido[1,2-a]quinoxaline

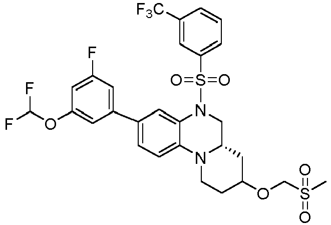

### Step 1: synthesis of (6aS)-3-bromo-8-((methylthio)methoxy)-5-((3-(trifluoromethyl)phenyl) sulfonyl)-6,6a,7,8,9,10-hexahydro-5H-pyrido[1,2-a]quinoxaline

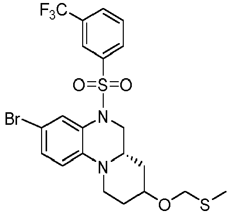

(6a*S*)-3-bromo-5-((3-(trifluoromethyl)phenyl)sulfonyl)-6,6a,7,8,9,10-hexahydro-5*H*-pyrido[1, 2-*a*]quinoxalin-8-ol (60 mg, 0.12 mmol) was dissolved in dimethyl sulfoxide (3 mL). Aceti c anhydride (3 mL) and acetic acid (1 mL) were added thereto. The mixture solution was stirred at 25 °C for 16 hrs. The reaction mixture was diluted with 15 mL of ethyl acetat e, washed with water (15 mL*3), dried over anhydrous sodium sulfate, filtered and concen trated to remove the solvent. The residue was separated by a rapid silica gel column to o btain (6a*S*)-3-bromo-8-((methylthio)methoxy)-5-((3-(trifluoromethyl)phenyl)sulfonyl)-6,6a,7,8,9,10-hexahydro-5*H*-pyrido[1,2-*a*]quinoxaline (60 mg, yield 90.7%). ESI-MS: 551, 553 [M+H]⁺.

### Step 2: synthesis of (6aS)-3-(3-(difluoromethoxy)-5-fluorophenyl)-8-((methylthio)methoxy) -5-((3-(trifluoromethyl)phenyl)sulfonyl)-6,6a,7,8,9,10-hexahydro-5H-pyrido[1,2-a]quinoxaline

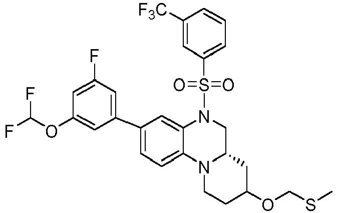

(6a*S*)-3-bromo-8-((methylthio)methoxy)-5-((3-(trifluoromethyl)phenyl)sulfonyl)-6,6a,7,8,9,1 0-hexahydro-5*H*-pyrido[1,2-*a*]quinoxaline (60 mg, 0.10 mmol), [1,1'-bis(diphenylphosphino)fe rrocene]palladium chloride (15 mg, 0.02 mmol), potassium carbonate (35 mg, 0.25 mmol) and 2-(3-(difluoromethoxy)-5-fluorophenyl)-4,4,5,5-tetramethyl-1,3-dioxalane (29 mg, 0.10mm ol) were placed in a three-necked flask. 1,4-dioxane (4 mL) and water (2 mL) were added thereto. Nitrogen was charged to replace three times by evacuation. The mixture solution was heated to 60 °C for 2 hrs. After the reaction was completed, the solvent was remove d by concentration. The residue was firstly separated by a rapid silica gel column, and the n separated by a reversed-phase column to obtain (6a*S*)-3-(3-(difluoromethoxy)-5-fluorophen yl)-8-((methylthio)methoxy)-5-((3-(trifluoromethyl)phenyl)sulfonyl)-6,6a,7,8,9,10-hexahydro-5*H-*pyrido[1,2-*a*]quinoxaline (40 mg, yield 63.3%). ESI-MS: 633 [M+H]⁺.

### Step 3: synthesis of (6aS)-3-(3-(difluoromethoxy)-5-fluorophenyl)-8-((methylsulfonyl)meth oxy)-5-((3-(trifluoromethyl)phenyl)sulfonyl)-6,6a,7,8,9,10-hexahydro-5H-pyrido[1,2-a]quinox aline

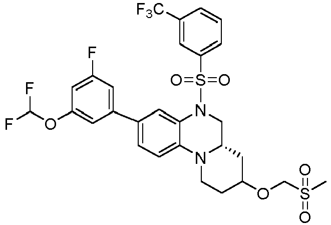

(6a*S*)-3-(3-(difluoromethoxy)-5-fluorophenyl)-8-((methylthio)methoxy)-5-((3-(trifluoromethy l)phenyl)sulfonyl)-6,6a,7,8,9,10-hexahydro-5*H*-pyrido[1,2-*a*]quinoxaline (40 mg, 0.06 mmol) was dissolved in dichloromethane (3 mL); 3-chloroperoxybenzoic acid (21 mg, 0.1 mmol) was added thereto. The mixture solution was stirred at 25 °C for 16 hrs, added with a sat urated aqueous solution of sodium sulfite (5 mL) and stirred for 30 mins. The organic lay er was dried, concentrated, and separated by a rapid silica gel column to obtain (6a*S*)-3-(3 -(difluoromethoxy)-5-fluorophenyl)-8-((methylsulfonyl)methoxy)-5-((3-(trifluoromethyl)phenyl)s ulfonyl)-6,6a,7,8,9,10-hexahydro-5*H*-pyrido[1,2-*a*]quinoxaline (5.4 mg, yield 13.5%). ESI-MS: 665.4 [M+1]⁺.
¹H NMR (500 MHz, CDCl₃) δ 7.82 (d, *J* = 7.9 Hz, 2H), 7.76 (d, *J* = 2.3 Hz, 1H), 7.71 (s, 1H), 7.62 (t, *J* = 7.9 Hz, 1H), 7.34 (d, *J* = 8.7 Hz, 1H), 7.12 (d, *J* = 10.2 Hz, 1H), 7.09 (s, 1H), 6.84-6.76 (m, 2H), 6.74-638 (t, *J* = 73.4 Hz, 1H), 4.41 (d, *J* = 24.4 H z, 2H), 4.26 (dd, *J* = 14.5, 4.5 Hz, 1H), 3.87-3.74 (m, 2H), 3.36 (dd, *J* = 14.4, 10.0 Hz, 1H), 2.89 (s, 3H), 2.54 (s, 1H), 2.30-2.14 (m, 1H), 2.09 (d, *J* = 12.9 Hz, 2H), 1.46-1.37 (m, 1H), 1.15 (q, *J* = 11.6 Hz, 1H).

### Example 111: preparation of (6aS)-3-(3-(difluoromethoxy)-5-fluorophenyl)-8-((methylsulfony l)ethoxy)-5-((3-(trifluoromethyl)phenyl)sulfonyl)-6,6a,7,8,9,10-hexahydro-5H-pyrido[1,2-a]q uinoxaline

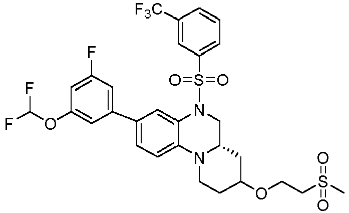

(6a,*S*)-3-(3-(difluoromethoxy)-5-fluorophenyl)-5-((3-(trifluoromethyl)phenyl)sulfonyl)-6,6a,7, 8,9,10-hexahydro-5*H*-pyrido[1,2-*a*]quinoxalin-8-ol (50 mg, 0.07 mmol) was dissolved in tolu ene. Methylsulfonyl ethylene (37 mg,0.35 mmol) and sodium hydride (5 mg, 0.11 mmol) were added thereto. The mixture solution was reacted at 25 °C for 16 hrs, then concentrat ed to remove the solvent. The residue was separated by a rapid silica gel column to obtai n (6a*S*)-3-(3-(difluoromethoxy)-5-fluorophenyl)-8-((methylsulfonyl)ethoxy)-5-((3-(trifluoromethy l)phenyl)sulfonyl)-6,6a,7,8,9,10-hexahydro-5*H*-pyrido[1,2-*a*]quinoxaline (10 mg, yield 21.1%). ESI-MS: 679.2 [M+1]⁺.
¹H NMR (500 MHz, CDCl₃) δ 7.86-7.70 (m, 4H), 7.61(s, 1H)7.34 (s, 1H), 7.11 (d, *J* = 9.5 Hz, 1H), 7.08 (s, 1H), 6.85 (s, 1H), 6.80 (d, *J* = 9.2 Hz, 1H), 6.74-6.38 (t, 1H), 4.27 (d, *J* = 14.4 Hz, 1H), 3.90 (s, 2H), 3.78 (s, 1H), 3.38 (s, 1H), 3.28 (s, 1H), 3.21 (s, 2H), 2.97 (s, 3H), 2.56 (s, 1H), 2.24 (s, 1H), 2.04 (s, 2H), 1.40 (s, 1H), 1.11 (s, 1H).

### Example 112: preparation of (2R,3R,4S,5S,6R)-2-(((6aS)-3-(3-(difluoromethoxy)-5-fluorop henyl)-5-((3-(trifluoromethyl)phenyl)sulfonyl)-6,6a,7,8,9,10-hexahydro-5H-pyrido[1,2-a]quin oxalin-8-yl)oxy)-6-(hydroxymethyl)tetrahydro-2H-pyran-3,4,5-triol

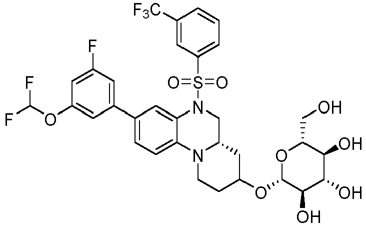

### Step 1: synthesis of (2R,3R,4S,5R)-2-(acetoxymethyl)-6-(2,2,2-trichloro-1-iminoethoxy)tetr ahydro-2H-pyran-3,4,5-triyl triacetate

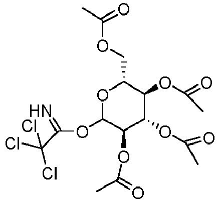

(2*R*,3*R*,4*S*,5*R*)-2-(acetoxymethyl)-6-hydroxytetrahydro-2*H*-pyran-3,4,5-triyl triacetate (350 mg, 1.0 mmol) was dissolved in dichloromethane. One drop of DBU, and trichloroacetonitr ile (450 mg, 3.0 mmol) were added thereto. The mixture solution was reacted at 25 °C fo r 6 hrs, then concentrated to remove the solvent. The residue was separated by a rapid sil ica gel column to obtain (2*R*,3*R*,4*S*,5*R*)-2-(acetoxymethyl)-6-(2,2,2-trichloro-1-iminoethoxy)tet rahydro-2*H*-pyran-3,4,5-triyl triacetate (390 mg, yield 79.1%), which was directly used in t he next step.

### Step 2: synthesis of (2R,3R,4S,5R,6R)-2-(acetoxymethyl)-6-(((6aS)-3-bromo-5-((3-(trifluoro methyl)phenyl)sulfonyl)-6,6a,7,8,9,10-hexahydro-5H-pyrido[1,2-a]quinoxalin-8-yl)oxy)tetrah ydro-2H-pyran-3,4,5-triyl triacetate

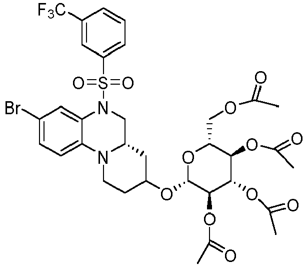

(2*R*,3*R*,4*S*,5*R*)-2-(acetoxymethyl)-6-hydroxytetrahydro-2*H*-pyran-3,4,5-triyltriacetate(2*R*,3*R*, 4*S*,5*R*)-2-(acetoxymethyl)-6-(2,2,2-trichloro-1-iminoethoxy)tetrahydro-2*H*-pyran-3,4,5-triyl triacetate (390 mg, 0.79 mmol) was dissolved in dichloromethane, TMSOTF (176 mg, 0.79 mmol) a nd (6a*S*)-3-bromo-5-((3-(trifluoromethyl)phenyl)sulfonyl)-6,6a,7,8,9,10-hexahydro-5*H*-pyrido[1,2 -*a*]quinoxalin-8-ol (387 mg, 0.79 mmol) were added thereto. The mixture solution was reac ted at 25 °C for 6 hrs, then concentrated to remove the solvent. The residue was separate d by a rapid silica gel column to obtain (2*R*,3*R*,4*S*,5*R*,6*R*)-2-(acetoxymethyl)-6-(((6a*S*)-3-bro mo-5-((3-(trifluoromethyl)phenyl)sulfonyl)-6,6a,7,8,9,10-hexahydro-5*H*-pyrido[1,2-*a*]quinoxalin-8-yl)oxy)tetrahydro-2*H*-pyran-3,4,5-triyl triacetate (250 mg, yield 38.5%). ESI-MS: 821, 823 [M+H]⁺.

### Step 3: synthesis of (2R,3R,4S,5R,6R)-2-(acetoxymethyl)-6-(((6aS)-3-(3-(difluoromethoxy)-5-fluorophenyl)-5-((3-(trifluoromethyl)phenyl)sulfonyl)-6,6a,7,8,9,10-hexahydro-5H-pyrido [1,2-a]quinoxalin-8-yl)oxy)tetrahydro-2H-pyran-3,4,5-triyl triacetate

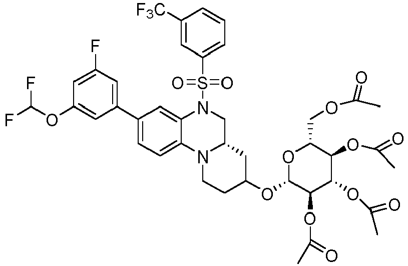

(2*R*,3*R*,4*S*,5*R*,6*R*)-2-(acetoxymethyl)-6-(((6a*S*)-3-bromo-5-((3-(trifluoromethyl)phenyl)sulfon yl)-6,6a,7,8,9,10-hexahydro-5*H*-pyrido[1,2-*a*]quinoxalin-8-yl)oxy)tetrahydro-2*H*-pyran-3,4,5-triyl triacetate (250 mg, 0.3 mmol) was dissolved in a mixture solvent of 1,4-dioxane (4 mL) a nd water (2 mL). 2-(3-(difluoromethoxy)-5-fluorophenyl)-4,4,5,5-tetramethyl-1,3,2-dioxaborola ne (87 mg, 0.3 mmol), [1,1'-bis(diphenylphosphino)ferrocene]palladium chloride (29 mg,0.04 mmol) and potassium carbonate (105 mg, 0.75 mmol) were added thereto. The mixture s oution was placed in a three-necked flask. Nitrogen was charged to replace three times by evacuation. The mixture solution was heated to 60 °C for 2 hrs. After the reaction was co mpleted, the solvent was removed by concentration. The residue was separated by a rapid silica gel column to obtain (2*R*,3*R*,4*S*,5*R*,6*R*)-2-(acetoxymethyl)-6-(((6a*S*)-3-(3-(difluorometho xy)-5-fluorophenyl)-5-((3-(trifluoromethyl)phenyl)sulfonyl)-6,6a,7,8,9,10-hexahydro-5*H*-pyrido [1,2-*a*]quinoxalin-8-yl)oxy)tetrahydro-2*H*-pyran-3,4,5-triyl triacetate (60 mg, 22.2% yield). ES I-MS: 903 [M+H]⁺.

### Step 4: synthesis of (2R,3R,4S,5S,6R)-2-(((6aS)-3-(3-(difluoromethoxy)-5-fluorophenyl)-5-((3-(trifluoromethyl)phenyl)sulfonyl)-6,6a,7,8,9,10-hexahydro-5H-pyrido[1,2-a]quinoxalin-8-yl)oxy)-6-(hydroxymethyl)tetrahydro-2H-pyran-3,4,5-triol

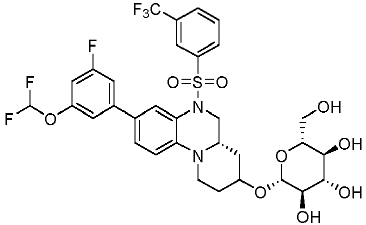

(2*R*,3*R*,4*S*,5*R*,6*R*)-2-(acetoxymethyl)-6-(((6a*S*)-3-(3-(difluoromethoxy)-5-fluorophenyl)-5-((3 -(trifluoromethyl)phenyl)sulfonyl)-6,6a,7,8,9,10-hexahydro-5*H*-pyrido[1,2-*a*]quinoxalin-8-yl)oxy) tetrahydro-2*H*-pyran-3,4,5-triyl triacetate (60 mg, 0.066 mmol) was dissolved in methanol (5 mL). Sodium methoxide (11 mg, 0.2 mmol) was added thereto. The mixture solution w as reacted at 25 °C for 1 hr; after the reaction was completed, hydrochloric acid was add ed dropwise to adjust the pH value ∼7.0, and the solvent was removed by concentration. The residue was separated by a rapid silica gel column to obtain (2R,3R,4S,5S,6R)-2-(((6a *S*)-3-(3-(difluoromethoxy)-5-fluorophenyl)-5-((3-(trifluoromethyl)phenyl)sulfonyl)-6,6a,7,8,9,10-h exahydro-5*H*-pyrido[1,2-*a*]quinoxalin-8-yl)oxy)-6-(hydroxymethyl)tetrahydro-2*H*-pyran-3,4,5-trio 1 (13.2 mg, 27.2% yield). ESI-MS: 735.2 [M+1]⁺.
¹H NMR (500 MHz, DMSO-*d*₆) δ 8.09 (d, *J* = 7.9 Hz, 1H), 7.97 (d, *J* = 8.0 Hz, 1 H), 7.86 (t, *J* = 7.9 Hz, 1H), 7.72-7.64 (m, 1H), 7.60 (d, *J* = 6.3 Hz, 1H), 7.53 (dd, *J* = 8.7, 2.3 Hz, 1H), 7.41 (d, *J* = 6.7 Hz, 1H), 7.30 (dd, *J* = 11.6, 9.5 Hz, 1H), 7.21 (s, 1 H), 7.04 (dd, *J* = 12.3, 9.1 Hz, 2H), 4.90 (dd, *J* = 8.3, 4.9 Hz, 3H), 4.44 (dt, *J* = 11.7, 5.9 Hz, 1H), 4.31-4.17 (m, 2H), 3.89 (d, *J* = 13.0 Hz, 1H), 3.68 (dd, *J* = 11.5, 5.8 Hz, 1H), 3.57-3.41 (m, 2H), 2.89 (s, 1H), 3.1(m, 4H), 2.52-2.43(d, 1H), 2.02 (dt, *J* = 29.3, 1 4.8 Hz, 3H), 1.31-1.10 (m, 1H), 1.03 (q, *J* = 11.6 Hz, 1H).

### Example 113: preparation of 3-(((6aS,8R)-3-(3-(difluoromethoxy)-5-fluorophenyl)-8-methy l-5-((3-(trifluoromethyl)phenyl)sulfonyl)-6,6a,7,8,9,10-hexahydro-5H-pyrido[1,2-a]quinoxali n-8-yl)oxy)propanoic acid

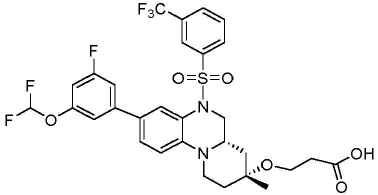

### Step 1: synthesis of tert-butyl 3-(((6aS,8R)-3-bromo-8-methyl-5-((3-(trifluoromethyl)pheny l)sulfonyl)-6,6a,7,8,9,10-hexahydro-5H-pyrido[1,2-a]quinoxalin-8-yl)oxy)propanoate

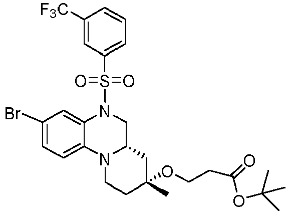

(6a*S*,8*R*)-3-bromo-8-methyl-5-((3-(trifluoromethyl)phenyl)sulfonyl)-6,6a,7,8,9,10-hexahydro -5*H*-pyrido[1,2-*a*]quinoxalin-8-ol (40 mg, 0.13 mmol) was dissolved in toluene (5 mL). Pot assium hydroxide (4 mg, 0.06 mmol) and tert-butyl acrylate (166 mg, 1.3 mmol) were add ed thereto. The mixture solution was stirred at 110 °C for 24 hrs. The reaction mixture w as cooled, and then diluted with 15 mL of ethyl acetate, washed with water (15 mL * 3), dried over anhydrous sodium sulfate, filtered and concentrated to remove the solvent. The residue was separated by a rapid silica gel column to obtain *tert*-butyl 3-(((6a*S*,8*R*)-3-bro mo-8-methyl-5-((3-(trifluoromethyl)phenyl)sulfonyl)-6,6a,7,8,9,10-hexahydro-5*H*-pyrido[1,2-*a*]qu inoxalin-8-yl)oxy)propanoate (25 mg, yield 30.3%). ESI-MS: 633, 635 [M+H]⁺.

### Step 2: synthesis of tert-butyl 3-(((6aS,8R)-3-(3-(difluoromethoxy)-5-fluorophenyl)-8-meth yl-5-((3-(trifluoromethyl)phenyl)sulfonyl)-6,6a,7,8,9,10-hexahydro-5H-pyrido[1,2-a]quinoxali n-8-yl)oxy)propanoate

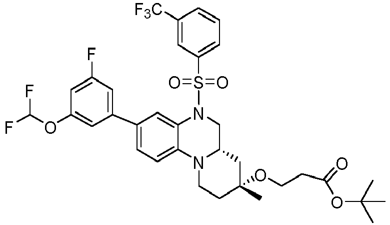

*Tert*-butyl 3-(((6a*S*,8*R*)-3-bromo-8-methyl-5-((3-(trifluoromethyl)phenyl)sulfonyl)-6,6a,7,8, 9, 10-hexahydro-5*H*-pyrido[1,2-*a*]quinoxalin-8-yl)oxy)propanoate (25 mg, 0.039 mmol), [1,1'-bis(diphenylphosphino)ferrocene]palladium chloride (22 mg, 0.03 mmol), potassium carbonat e (14 mg, 0.1 mmol) and 2-(3-(difluoromethoxy)-5-fluorophenyl)-4,4,5,5-tetramethyl-1,3-diox alane (12 mg, 0.039 mmol) were placed in a three-necked flask. 1,4-dioxane (4 mL) and w ater (2 mL) were added thereto. Nitrogen was charged to replace three times by evacuatio n. The mixture solution was heated to 60 °C for 2 hrs. After the reaction was completed, the solvent was removed by concentration. The residue was separated by a rapid silica gel column and then separated by a reversed-phase column to obtain *tert*-butyl 3-(((6a*S*,8*R*)-3-(3-(difluoromethoxy)-5-fluorophenyl)-8-methyl-5-((3-(trifluoromethyl)phenyl)sulfonyl)-6,6a,7,8, 9,10-hexahydro-5*H*-pyrido[1,2-*a*]quinoxalin-8-yl)oxy)propanoate (27.8 mg, yield 100%). ESI-MS: 715 [M+H]⁺.

### Step 3: synthesis of 3-(((6aS,8R)-3-(3-(difluoromethoxy)-5-fluorophenyl)-8-methyl-5-((3-(tr ifluoromethyl)phenyl)sulfonyl)-6,6a,7,8,9,10-hexahydro-5H-pyrido[1,2-a]quinoxalin-8-yl)ox y)propanoic acid

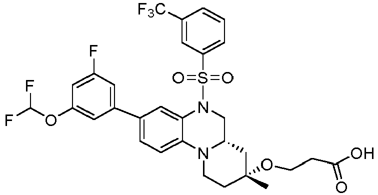

*Tert*-butyl 3-(((6a*R*)-3-(3-(difluoromethoxy)-5-fluorophenyl)-5-((3-(trifluoromethyl)phenyl)s ulfonyl)-6,6a,7,8,9,10-hexahydro-5*H*-pyrido[1,2-*a*]quinoxalin-8)oxy)propanoate (27.8 mg, 0.039 mmol) was dissolved in dichloromethane (3 mL). Trifluoroacetic acid (3 mL) was added thereto. The mixture solution was stirred at 25 °C for 1 hr and concentrated to remove th e solvent. The residue was separated by a rapid silica gel column to obtain 3-(((6aS,8R)-3-(3-(difluoromethoxy)-5-fluorophenyl)-8-methyl-5-((3-(trifluoromethyl)phenyl)sulfonyl)-6,6a,7,8, 9,10-hexahydro-5*H*-pyrido[1,2-*a*]quinoxalin-8-yl)oxy)propanoic acid (5.5 mg, yield 21.4%). E SI-MS: 659.5 [M+1]⁺.
¹H NMR (500 MHz, CDCl₃) δ 7.86-7.77 (m, 3H), 7.65 (d, *J* = 7.9 Hz, 1H), 7.58 (t, *J* = 7.8 Hz, 1H), 7.34 (dd, *J* = 8.6, 2.3 Hz, 1H), 7.14 (dd, *J* = 9.6, 1.9 Hz, 1H), 7.11 (s, 1H), 6.80 (d, *J* = 8.7 Hz, 2H), 6.75-6.38 (t, *J* = 73.4 Hz, 1H), 4.22 (dd, *J* = 14.4, 4. 0 Hz, 1H), 3.69 (s, 1H), 3.64 (t, *J* = 6.3 Hz, 2H), 3.23 (dd, *J* = 14.4, 10.4 Hz, 1H), 2.5 6 (t, *J* = 6.1 Hz, 2H), 2.44-2.36 (m, 1H), 2.26-2.18 (m, 1H), 1.61 (s, 3H), 1.25 (t, *J* = 1 2.1 Hz, 1H), 1.03 (s, 3H).

### Example 114 was prepared according to the synthesis method of Example 113.

| **Example No.** | **Structural formula** | **Chemical name** | **ESI-MS:** [M+1]⁺ |
|---|---|---|---|
| **114** | 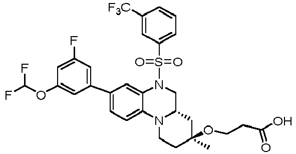 | 3-(((6a*S*,8*S*)-3-(3-(difluoro methoxy)-5-fluorophenyl)-8-methyl-5-((3-(trifluorom ethyl)phenyl)sulfonyl)-6,6 a,7,8,9,10-hexahydro-5*H*-p yrido[1,2-*a*]quinoxalin-8-y l)oxy)propanoic acid | 659 |

¹H NMR (500 MHz, CDCl₃) δ 7.85-7.75 (m, 3H), 7.70 (s, 1H), 7.61 (t, *J* = 7.8 Hz, 1H), 7.32 (dd, *J* = 8.6, 2.3 Hz, 1H), 7.12 (dt, *J* = 9.6, 1.9 Hz, 1H), 7.09 (s, 1H), 6.78 (d, *J* = 8.9 Hz, 2H), 6.74-6.37 (t, 1H), 4.18 (dd, *J* = 14.2, 4.3 Hz, 1H), 3.51 (t*, J* = 6.2 Hz, 2H), 3.49-3.39 (m, 1H), 3.26 (dd, *J* = 14.2, 10.2 Hz, 1H), 2.82 (s, 1H), 2.50 (t, *J* = 6.0 Hz, 3H), 1.81 (dd, *J* = 13.9, 2.9 Hz, 1H), 1.73 (dt, *J* = 13.3, 2.7 Hz, 1H), 1.36 (td, *J* = 13.4, 4.7 Hz, 1H), 1.15 (s, 3H), 1.04 (dd, *J* = 13.4, 11.6 Hz, 1H).

### Example 115 was prepared according to the synthesis method of Example 109.

| **Example No.** | **Structural formula** | **Chemical name** | **ESI-MS:** [M+1]⁺ |
|---|---|---|---|
| **115** | 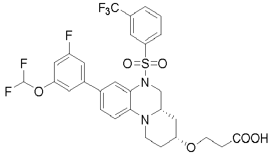 | 3-(((6a*S*,8*R*)-3-(3-(difluoromet hoxy)-5-fluorophenyl)-5-((3-(t rifluoromethyl)phenyl)sulfony l)-6,6a,7,8,9,10-hexahydro-5*H* -pyrido[1,2-*a*]quinoxalin-8-yl) oxy)propanoic acid | 645 |

¹H NMR (400 MHz, CDCl₃) δ 7.82-7.74 (m, 3H), 7.71 (s, 1H), 7.60 (t, *J* = 7.9 Hz, 1H), 7.32 (dd, *J* = 8.7, 2.3 Hz, 1H), 7.13 (dt, *J* = 9.8, 1.9 Hz, 1H), 7.09 (d, *J* = 2.0 Hz, 1H), 6.78 (dd, *J* = 9.1, 2.1 Hz, 2H), 6.74-6.38 (t*, J* = 73.5 Hz, 1H), 4.19 (dd, *J* = 14.3, 4.3 Hz, 1H), 3.74-3.57 (m, 3H), 3.43 (d, *J* = 10.5 Hz, 1H), 3.25 (dd, *J* = 14.3, 10.5 Hz, 1H), 2.83 (t, *J* = 10.9 Hz, 1H), 2.58 (t, *J* = 6.0 Hz, 2H), 2.51-2.44 (m, 1H), 1.89-1.77 (m, 2H), 1.55 (t*, J* = 13.8 Hz, 1H), 1.27-1.17 (m, 1H).

### Example 116: preparation of (6aS,8S)-3-(3-(difluoromethoxy)-5-fluorophenyl)-5-((3-(triflu oromethyl)phenyl)sulfonyl)-6,6a,7,8,9,10-hexahydro-5H-pyrido[1,2-a]quinoxaline-8-carbo xylic acid

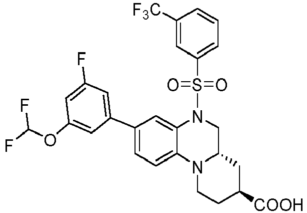

### Step 1: synthesis of (6aS,8S)-3-bromo-5-((3-(trifluoromethyl)phenyl)sulfonyl)-6,6a,7,8,9,10 -hexahydro-5H-pyrido[1,2-a]quinoxaline-8-carbonitrile

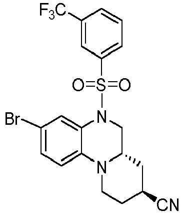

(*S*)-3-bromo-5-((3-(trifluoromethyl)phenyl)sulfonyl)-6a,7,9,10-tetrahydro-5*H*-pyrido[1,2-*a*]q uinoxalin-8(6*H*)-one (100 mg, 0.2 mmol) was dissolved in tetrahydrofuran (10 mL). Potassi um *tert*-butoxide (25 mg, 0.22 mmol) was added, and the mixture solution was stirred at 25 °C for 30 mins, 1-((isocyanomethyl)sulfonyl)-4-methylbenzene (43 mg, 0.22 mmol) was added, and the mixture solution was stirred at 25 °C for 16 hrs. The reaction mixture was diluted with 15 mL of ethyl acetate, washed with brine (15 mL * 3), dried over anhydro us sodium sulfate, filtered and concentrated to remove the solvent. The residue was separat ed by a rapid silica gel column to obtain (6a*S*,8*S*)-3-bromo-5-((3-(trifluoromethyl)phenyl)sulf onyl)-6,6a,7,8,9,10-hexahydro-5*H*-pyrido[1,2-*a*]quinoxaline-8-carbonitrile (35 mg, yield 35%), which was directly used in the next step.

### Step 2: synthesis of (6aS,8S)-3-bromo-5-((3-(trifluoromethyl)phenyl)sulfonyl)-6,6a,7,8,9,10 -hexahydro-5H-pyrido [1,2-a]quinoxalin-8-carboxylic acid

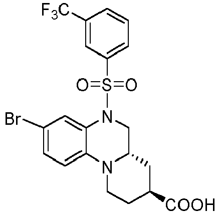

(6a*S*,8*S*)-3-bromo-5-((3-(trifluoromethyl)phenyl)sulfonyl)-6,6a,7,8,9,10-hexahydro-5*H*-pyrid o[1,2-*a*]quinoxaline-8-carbonitrile (35 mg, 0.07 mmol) was dissolved in methanol (10 mL). Thionyl chloride (25 mg, 0.22 mmol) was added under ice bath. The mixture solution was heated to 65 °C and stirred for 16 hrs, then concentrated to remove the solvent. The resi due was added with 2M aqueous potassium hydroxide solution (15 mL), stirred at 80 °C f or 4 hrs, cooled to room temperature, added with aqueous HCl to adjust pH to 6, extracte d with ethyl acetate, washing with brine (15 mL * 3), dried over anhydrous sodium sulfat e, filtered and concentrated to remove the solvent. The residue was separated by a rapid s ilica gel column to obtain (6a*S*,8*S*)-3-bromo-5-((3-(trifluoromethyl)phenyl)sulfonyl)-6,6a,7,8,9, 10-hexahydro-5*H*-pyrido[1,2-*a*]quinoxalin-8-carboxylic acid (30 mg, yield 82.5%). ESI-MS: 519, 521 [M+H]⁺.

### Step 3: synthesis of (6aS,8S)-3-(3-(difluoromethoxy)-5-fluorophenyl)-5-((3-(trifluoromethy l)phenyl)sulfonyl)-6,6a,7,8,9,10-hexahydro-5H-pyrido[1,2-a]quinoxaline-8-carboxylic acid

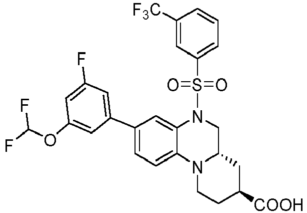

(6a*S*,8*S*)-3-bromo-5-((3-(trifluoromethyl)phenyl)sulfonyl)-6,6a,7,8,9,10-hexahydro-5*H*-pyrid o[1,2-*a*]quinoxaline-8-carboxylic acid (30 mg, 0.057 mmol), [1,1'-bis(diphenylphosphino)ferro cene]palladium chloride (22 mg, 0.03 mmol), potassium carbonate (20 mg, 0.14 mmol) and 2-(3-(difluoromethoxy)-5-fluorophenyl)-4,4,5,5-tetramethyl-1,3-dioxalane (17 mg, 0.057 mmo 1) were placed in a three-necked flask. 1,4-dioxane (4 mL) and water (2 mL) were added thereto. The nitrogen was charged to replace three times by evacuation. The mixture soluti on was heated to 60 °C for 2 hrs. After the reaction was completed, the solvent was rem oved by concentration. The residue was firstly separated by a rapid silica gel column, and then separated by a reversed-phase column to obtain (6a*S*,8*S*)-3-(3-(difluoromethoxy)-5-fluor ophenyl)-5-((3-(trifluoromethyl)phenyl)sulfonyl)-6,6a,7,8,9,10-hexahydro-5*H*-pyrido[1,2-*a*]quino xaline-8-carboxylic acid (15 mg, yield 43.8%). ESI-MS: 599.4 [M-1]⁺.
¹H NMR (500 MHz, DMSO-*d*₆) δ 8.08 (d, *J* = 7.7 Hz, 1H), 7.92 (d, *J* = 8.0 Hz, 1 H), 7.84 (t, *J* = 7.8 Hz, 1H), 7.71 (d, *J* = 2.3 Hz, 1H), 7.64 (s, 1H), 7.59-7.22 (t,1H), 7. 53 (dd, *J* = 8.8, 2.4 Hz, 1H), 7.32 (dt, *J* = 10.2, 1.8 Hz, 1H), 7.22 (d, *J* = 2.8 Hz, 1H), 7.05 (dt, *J* = 9.6, 2.3 Hz, 1H), 6.99 (d, *J* = 8.9 Hz, 1H), 4.26 (dd, *J* = 14.5, 4.5 Hz, 1 H), 3.85 (d, *J* = 12.6 Hz, 1H), 2.36 (s, 2H), 2.11-1.95 (m, 2H), 1.83 (dd, *J* = 26.3, 13.3 Hz, 2H), 1.39-1.22 (m, 1H), 1.03 (q, *J* = 12.1 Hz, 1H).

### Example 117 was prepared according to the synthesis method of Example 116.

| **Example No.** | **Structural formula** | **Chemical name** | **ESI-MS:** [M+1]⁺ |
|---|---|---|---|
| **117** | 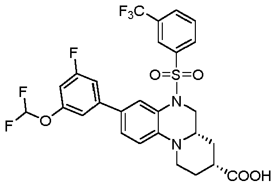 | (6a*S*,8*R*)-3-(3-(difluoromethoxy)-5-fluorophenyl)-5-((3-(trifluoromethyl)phenyl)sulfonyl)-6,6a,7,8,9,10-hexahydro-5*H*-pyrido[1,2-*a*]quinoxaline-8-carboxylic acid | 599 |

¹H NMR (400 MHz, DMSO-*d*₆) δ 8.08 (d, *J* = 7.7 Hz, 1H), 7.92 (d, *J* = 8.0 Hz, 1H), 7.84 (t, *J* = 7.8 Hz, 1H), 7.71 (d, *J* = 2.3 Hz, 1H), 7.64 (s, 1H), 7.59-7.22(t, 1H), 7.53 (dd, *J* = 8.8, 2.4 Hz, 1H), 7.32 (dt, *J* = 10.2, 1.8 Hz, 1H), 7.22 (d, *J* = 2.8 Hz, 1H), 7.05 (dt, *J* = 9.6, 2.3 Hz, 1H), 6.99 (d, *J* = 8.9 Hz, 1H), 4.26 (dd, *J* = 14.5, 4.5 Hz, 1H), 3.85 (d, *J* = 12.6 Hz, 1H), 2.36 (s, 2H), 2.11-1.95 (m, 2H), 1.83 (dd, *J* = 26.3, 13.3 Hz, 2H), 1.39-1.22 (m, 1H), 1.03 (q, *J* = 12.1 Hz, 1H).

### Example 118: preparation of (S)-3-(1-methyl-6-(2,2,6,6-tetramethyl-3,6-dihydro-2H-pyran -4-yl)-4-((3-(trifluoromethyl)phenyl)sulfonyl)-1,2,3,4-tetrahydroquinoxalin-2-yl)propanoic acid

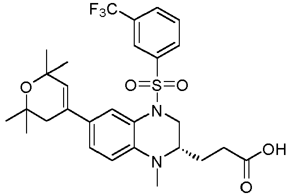

### Step 1: synthesis of methyl (S)-3-(1-methyl-6-(2,2,6,6-tetramethyl-3,6-dihydro-2H-pyran-4 -yl)-4-((3-(trifluoromethyl)phenyl)sulfonyl)-1,2,3,4-tetrahydroquinoxalin-2-yl)propanoate

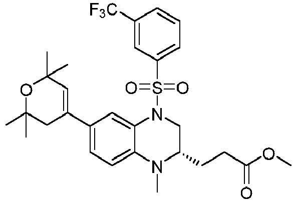

Methyl (*S*)-3-(6-bromo-1-methyl-4-((3-(trifluoromethyl)phenyl)sulfonyl)-1,2,3,4-tetrahydroq uinoxalin-2-yl)propanoate (160 mg, 0.307 mmol) was dissolved in the mixture solvent of t oluene : ethanol : water = 2:1:1 (12 mL). Sodium carbonate (50 mg, 0.460 mmol), 4,4,5,5 -tetramethyl-2-(2,2,6,6-tetramethyl-3,6-dihydro-2*H*-pyran-4-yl)-1,3,2-dioxaborolane(123 mg, 0.46 0 mmol) and tetratriphenylphosphine palladium (50 mg) were added under nitrogen protecti on, the mixture solution was heated to 80 °C and stirred for 5 hrs, then cooled, , the mix ture solution was extracted twice with water and ethyl acetate. The organic phases were dr ied over anhydrous sodium sulfate, filtered and concentrated to remove the solvent. The cr ude product was separated by a rapid silica gel column to obtain methyl (*S*)-3-(1-methyl-6-(2,2,6,6-tetramethyl-3,6-dihydro-2*H*-pyran-4-yl)-4-((3-(trifluoromethyl)phenyl)sulfonyl)-1,2,3,4-te trahydroquinoxalin-2-yl)propanoate (85 mg, 48%).

### Step 2: synthesis of (S)-3-(1-methyl-6-(2,2,6,6-tetramethyl-3,6-dihydro-2H-pyran-4-yl)-4-((3-(trifluoromethyl)phenyl)sulfonyl)-1,2,3,4-tetrahydroquinoxalin-2-yl)propanoic acid

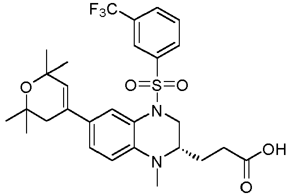

Methyl (*S*)-3-(1-methyl-6-(2,2,6,6-tetramethyl-3,6-dihydro-2*H*-pyran-4-yl)-4-((3-(trifluoromethy 1)phenyl)sulfonyl)-1,2,3,4-tetrahydroquinoxalin-2-yl)propanoate (85 mg, 0.146 mmol) was diss olved in the mixture solvent of tetrahydrofuran : methanol : water = 1:1:1 (10 mL). Lithiu m hydroxide monohydrate (31 mg) was added thereto. The mixture solution was stirred at room temperature overnight. The reaction mixture was concentrated to remove the solvent, and acidified with dilute hydrochloric acid. The residue was separated by a rapid silica gel column to obtain (*S*)-3-(1-methyl-6-(2,2,6,6-tetramethyl-3,6-dihydro-2*H*-pyran-4-yl)-4-((3-(trifl uoromethyl)phenyl)sulfonyl)-1,2,3,4-tetrahydroquinoxalin-2-yl)propanoic acid (45 mg, 54%). ESI-MS: 567.6 [M+H]⁺.
¹H NMR (500 MHz, DMSO-*d₆*) δ 8.41 (s, 1H), 8.13 (dd, *J* = 16.5, 7.9 Hz, 2H), 7.9 0 (dd, *J* = 17.1, 9.2 Hz,2H), 7.11 (dd, *J* = 8.6, 2.2 Hz, 1H), 7.05 (d, *J* = 2.1 Hz, 1H), 6.60 (d, *J* = 8.8 Hz, 1H), 5.80 (d, *J* = 1.5 Hz,1H), 4.00 (dd, *J* = 13.6, 5.3 Hz, 1H), 3.70 (dd, *J* = 13.6, 4.0 Hz, 1H), 3.26 (d, *J* = 9.0 Hz, 1H), 2.71 (s, 3H), 2.23 (dt, *J* = 14.3, 8.3 Hz, 2H), 2.15-2.03 (m, 2H), 1.82 (d, *J* = 7.7 Hz, 1H), 1.60-1.51 (m, 1H), 1.21 (d, *J* = 4.3 Hz, 6H), 1.17 (s, 6H).

### Example 119: preparation of (S)-3-(1-methyl-6-(2,2,6,6-tetramethyltetrahydro-2H-pyran-4 -yl)-4-((3-(trifluoromethyl)phenyl)sulfonyl)-1,2,3,4-tetrahydroquinoxalin-2-yl)propanoic acid

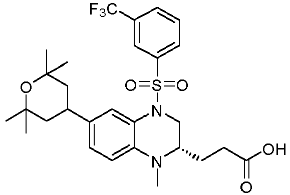

(*S*)-3-(1-methyl-6-(2,2,6,6-tetramethyl-3,6-dihydro-2*H*-pyran-4-yl)-4-((3-(trifluoromethyl)ph enyl)sulfonyl)-1,2,3,4-tetrahydroquinoxalin-2-yl)propanoic acid (30 mg, 0.053 mmol) was dis solved in methanol (10 mL), 10% wet palladium on carbon (10 mg) was added thereto. T he reaction mixture was stirred under hydrogen at room temperature overnight. The reactio n mixture was filtered, concentrated to remove the solvent, and lyophilized to obtain (S)-3-(1-methyl-6-(2,2,6,6-tetramethyltetrahydro-2*H*-pyran-4-yl)-4-((3-(trifluoromethyl)phenyl)sulfony 1)-1,2,3,4-tetrahydroquinoxalin-2-yl)propanoic acid (17.3 mg, 57%). ESI-MS: 569.5 [M+H]⁺.
¹H NMR (500 MHz, DMSO-*d₆*) δ 8.09 (t, *J* = 8.5 Hz, 2H), 7.87 (t, *J* = 7.8 Hz, 1 H), 7.77 (s, 1H), 6.99 (d, *J* = 2.1 Hz, 1H), 6.92 (dd, *J* = 8.5, 2.1 Hz, 1H), 6.53 (d, *J =* 8.5 Hz, 1H), 3.88-3.74(m, 2H), 3.15 (s, 1H), 2.96 -2.87 (m, 1H), 2.59 (s, 3H), 2.24-2.09 (m, 2H), 1.78 (d, *J* = 11.5 Hz, 1H), 1.63-1.44 (m, 3H), 1.26 (s, 6H), 1.18 (dd, *J* = 18.5, 12.8 Hz, 2H), 1.12 (d, *J* = 7.3 Hz, 6H).

### Example 120: preparation of methyl (S,E)-3-(6-(2-(2-chloro-6-fluorophenyl)prop-1-en-1-y l)-1-methyl-4-((3-(trifluoromethyl)phenyl)sulfonyl)-1,2,3,4-tetrahydroquinoxalin-2-yl)propanoat e

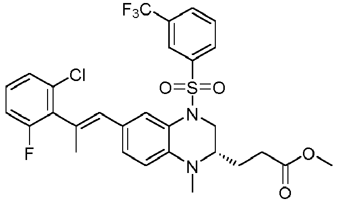

Methyl (*S*)-3-(6-bromo-1-methyl-4-((3-(trifluoromethyl)phenyl)sulfonyl)-1,2,3,4-tetrahydroq uinoxalin-2-yl)propanoate (1.4 g, 2.69 mmol) was dissolved in the mixture solvent of tolue ne : ethanol : water = 2:1:1 (30 mL). Sodium carbonate (286 mg, 2.69 mmol), (*E*)-2-(2-(2 -chloro-6-fluorophenyl)prop-1-en-1-yl)-4,4,5,5-tetramethyl-1,3,2-dioxaborolane (1.6 g, 5.38 m mol) and tetrakis(triphenylphosphine) palladium (200 mg) were added. The mixture solution was heated to 80 °C under nitrogen and stirred overnight, then cooled, the mixture soluti on was extracted twice with water and ethyl acetate. The organic phases were dried over anhydrous sodium sulfate, filtered and concentrated to remove the solvent. The crude prod uct was separated by a rapid silica gel column to obtain methyl (*S,E*)-3-(6-(2-(2-chloro-6-fl uorophenyl)prop-1-en-1-yl)-1-methyl-4-((3-(trifluoromethyl)phenyl)sulfonyl)-1,2,3,4-tetrahydroqu inoxalin-2-yl)propanoate (1.0 g, 61%). ESI-MS: 610.7 [M+1]⁺.
¹H NMR (500 MHz, CDCl₃) δ 7.91 (s, 1H), 7.83 (d, *J* = 8.0 Hz, 1H), 7.74 (d, *J =* 8.0 Hz, 1H), 7.55 (t, *J* = 7.7 Hz, 1H), 7.35 (d, *J* = 2.0 Hz, 1H), 7.16-7.08 (m, 2H), 7.04 (dd, *J* = 8.6, 2.1 Hz, 1H), 6.97-6.91 (m, 1H), 6.52 (d, *J* = 8.5 Hz, 1H), 6.22 (d, *J* = 1. 6 Hz, 1H), 3.81 (dd, *J* = 5.3, 2.5 Hz, 2H), 3.63 (s, 3H), 3.19-3.10 (m, 1H), 2.65 (d, *J =* 3.8 Hz, 3H), 2.39-2.21 (m, 2H), 2.05 (d, *J* = 1.5 Hz, 3H), 1.92-1.89 (m, 1H), 1.73-1.66 (m, 1H).

### Example 121 was prepared according to the synthesis method of Example 113.

| **Example No.** | **Structural formula** | **Chemical name** | **ESI-MS:** [M+1]⁺ |
|---|---|---|---|
| **121** | 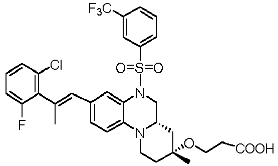 | 3 -((6a*S*,8*R*)-3-((E)-2-(2-chloro-6-fluorophenyl)prop-1-en-1 -yl)-8-methyl-5 -((3 -(trifluoromethy l)phenyl)sulfonyl)-6,6a,7,8,9,10 -hexahydro-5*H*-pyrazino[1,2-*a*] quinoxalin-8-yl)oxy)propanoic acid | 667 |

¹H NMR (400 MHz, CDCl₃) δ 7.95-7.88 (s, 1H), 7.85-7.78 (d, *J* = 7.7 Hz, 1H), 7.74-7.70 (d, *J* = 1.9 Hz, 1H), 7.70-7.64 (d, *J* = 7.9 Hz, 1H), 7.62-7.55 (t, *J* = 7.8 Hz, 1H), 7.23-7.20 (m, 1H), 7.20 -7.13 (ddd, *J* = 9.1, 4.9, 2.4 Hz, 2H), 7.06-6.97 (m, 1H), 6.90-6.76 (s, 1H), 6.38-6.32 (d, *J* = 1.7 Hz, 1H), 4.28-4.16 *(dd, J=* 14.4, 3.8 Hz, 1H), 3.69-3.60 (t*, J* = 6.1 Hz, 3H), 3.47-3.29 (s, 1H), 2.65-2.55 (t, *J* = 6.1 Hz, 2H), 2.53-2.26 (d, *J* = 77.6 Hz, 2H), 2.25-2.17 (d, *J* = 1.4 Hz, 3H), 1.75-1.66(m, 3H), 1.38-1.26 (s, 1H), 1.10-1.01 (s, 3H).

### Example 122 was prepared according to the synthesis method of Example 59.

| **Example No.** | **Structural formula** | **Chemical name** | **ESI-MS:** [M+1]⁺ |
|---|---|---|---|
| **122** | 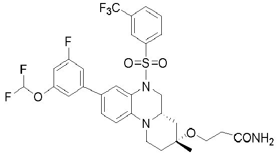 | 3-(((6a*S*, 8*R*)-3-(3-(difluorometho xy)-5-fluorophenyl)-8-methyl-5-((3 -(trifluoromethyl)phenyl)sulfo nyl)-6,6a,7,8,9,10-hexahydro-5*H* -pyrido[1,2-*a*]quinoxalin-8-yl)ox y)propanamide | 658 |

¹H NMR (400 MHz, DMSO-*d₆*) δ 8.14-8.07 (d, *J* = 7.9 Hz, 1H), 7.89-7.81 (t, *J* = 7.8 Hz, 1H), 7.81-7.72 (dd, J= 8.5, 6.3 Hz, 3H), 7.60-7.23 (t, 1H), 7.58-7.51 (dd, J= 8.8, 2.3 Hz, 1H), 7.42-7.38 (s, 0H), 7.38-7.30 (dt, *J* = 10.0, 1.9 Hz, 1H), 7.28-7.17 (m, 2H), 7.09-6.98 (m, 2H), 6.77-6.69 (s, 1H), 4.29-4.18 (m, 1H), 3.88-3.75 (d, *J* = 13.3 Hz, 1H), 3.54-3.45 (t, *J* = 6.6 Hz, 2H),3.3-3.2(m, 1H), 2.24-2.13 (t, J= 6.4 Hz, 3H), 2.12-2.00 (dd, *J* = 13.9, 11.0 Hz, 1H), 1.74-1.64 (d, J= 12.7 Hz, 1H), 1.64-1.52 (d, *J* = 12.8 Hz, 1H), 1.49-1.36 (dt, *J* = 15.2, 7.5 Hz, 1H), 1.14-1.04 (t, *J* = 12.1 Hz, 1H), 0.94-0.86 (s, 3H).

### Biological Test Evaluation

### I. Time-Resolved Fluorescence Resonance Energy-Transfer Assay (TR-FRET)

This experiment was a screening experiment for RORyt nuclear receptor agonists based on TR-FRET technology. When His-labeled RORyt-LBD receptor binds to receptor agonists, it may increase the recruitment of biotin-labeled co-activator peptides. Europium-His-RORyt-LBD is indirectly labeled by donor (Eu) by binding to Eu-anti-His antibody. Once Eu is activated by an energy source (such as flashlight or laser), the energy will be transferred to the co-activator indirectly labeled by allophycocyanin by binding of allophycocyanin-streptavidin and biotin-labeled co-activator.
1. 10X buffer (500 mM Tris-HCl, 500 mM KCl, and 10 mM Na-EDTA) was prepared, pH value was adjusted to 7.0. The buffer was stored at 4 °C for later use and was restored to normal temperature before experiment;
2. The 10X buffer was diluted to 1X with pure water. Final concentration of 0.01% Triton X-100 and 1 mM DTT (dithiothreitol) were added to prepare the assay buffer;
3. DMSO was used to prepare 1000X compound stock solution. The compound solution was serial-diluted in 5-fold for 7 concentrations from the top concentration of 1000 nM/10000 nM. Then the 10X compound solution was prepared with assay buffer. An amount of 2 µL was aliquoted into the 20 µL system;
4. 5X RORyt-LBD protein solution was unfreezed, and a solution of 5X concentration was prepared with assay buffer to obtain a final concentration of 30 nM. The whole operation was carried out on ice;
5. 5X SRC peptide was unfreezed, and a solution of 5X concentration was prepared with assay buffer to obtain a final concentration of 500 nM. The whole operation was carried out on ice;
6. 4 µL/well of RORyt-LBD receptor was added into the testing wells of 384-well plate, and the same amount of assay buffer was added to the control wells of non-RORyt-LBD group;
7. 2 µL of compound was added into each well of 384-well plate;
8. 4 µL of SRC peptide was added into each well of 384-well plate;
9. 2X Eu-anti-6X His/APC-Streptavidin was diluted with Lance detection buffer to a final concentration of 0.25 nM and 5 nM, respectively, and then 10 µL of 2X Eu-anti-6X His/APC-Streptavidin was added into each well of 384-well plate;
10. The mixed solution was incubated at 4 °C overnight;
11. On the morning of the next day, the 384-well plate was incubated at room temperature for 1 hr. Then the corresponding signal values were read with PerkinElmer EnVision at a wavelength of 665/615 nM. The agonistic activity of the corresponding compound was calculated with Graphpad Prism 7.0 software. The specific test results were shown in Table 1.

### II. RORγt Cellular Reporter Gene Detection Assay

In this experiment, RORyt Reporter Gene Detection method was used to evaluate the activation and specificity of compounds to RORyt in cells. The plasmid of pFN26A-RORyt-LBD and pG14.35 (Promega, Cat. No. E1370) were co-transfected into HEK 293 cells (Cat. No. GNHu18). The efficacy of compounds was evaluated with the presence of an antagonist Ursolic acid (Selleck, Cat. No. S2370-100 mg). The specific experimental process was as follow:
1. The plasmids-co-transfected cells were inoculated into a 96-well plate (Corning, Cat. No 3610) in a fresh DMEM medium (Gibco, cat. No. 1773536) containing 10% fetal bovine serum (Gibco, Cat. No. 10099-141) at 30000 cells/40 µL/well;
2. 5 µL of medium containing 20 µM Ursolic acid was added thereto;
3. The dose effect was evaluated by a 4-fold dilution of the test compound, starting from 50 µM;
4. 5 µL of medium containing a compound concentration of 10-fold of its final test concentration was added;
5. After the cells were incubated at 37 °C and 5% CO₂ for 24 hrs, 50 µL of detection reagent was added to detect firefly fluorescence using PerkinElmerEnVision, and 50 µL of the second detection reagent was added to detect sea kidney fluorescence;
6. A four-parameter curve simulated in GraphPad Prism was used to determine the concentration for 50% activation (EC₅₀) and the upper activation limit (Amax) for each compound. The specific test results were shown in Table 1.

**Table 1: Test Results**

| Example No. | TR-FRET Biochemical | | Cellular Reporter Assay | |
|---|---|---|---|---|
| | EC₅₀/(nM) | Amax | EC₅₀/(nM) | Amax |
| 1 | 18.40 | 1.61 | 517.6 | 3.3 |
| 2 | 18.12 | 1.85 | 163.3 | 3.1 |
| 3 | 29.11 | 1.20 | 1739.3 | 4.4 |
| 4 | 28.39 | 1.25 | 3550.5 | 4.9 |
| 5 | 9.68 | 1.39 | 1028.0 | 5.0 |
| 6 | 16.09 | 1.54 | 528.4 | 4.2 |
| 7 | 17.51 | 1.45 | 144.6 | 3.7 |
| 8 | 13.32 | 1.22 | 90.4 | 5.5 |
| 9 | 11.61 | 0.63 | 289.6 | 3.5 |
| 10 | 13.62 | 0.81 | 578.2 | 2.8 |
| 11 | 21.65 | 0.82 | 428.7 | 3.2 |
| 12 | 136.25 | 0.67 | 2387.2 | 4.3 |
| 13 | 35.76 | 0.74 | 1423.0 | 1.7 |
| 14 | 30.51 | 0.89 | 69.7 | 3.1 |
| 15 | 24.99 | 0.83 | 134.4 | 3.2 |
| 16 | 9.52 | 0.78 | 59.0 | 2.0 |
| 17 | 29.57 | 0.82 | 603.5 | 4.2 |
| 18 | 15.13 | 0.62 | 177.7 | 4.3 |
| 19 | 17.81 | 0.97 | 25.7 | 2.0 |
| 20 | 32.00 | 1.01 | 418.2 | 6.3 |
| 21 | 17.63 | 0.94 | 273.3 | 5.5 |
| 22 | 1.51 | 0.62 | 244.8 | 3.3 |
| 23 | 17.17 | 0.79 | 1075.0 | 3.8 |
| 24 | 31.48 | 0.92 | 48.5 | 2.4 |
| 25 | 6.35 | 0.84 | 441.1 | 4.4 |
| 26 | 29.56 | 0.64 | 403.7 | 2.3 |
| 27 | 5.25 | 0.61 | 228.4 | 3.9 |
| 28 | 14.69 | 0.86 | 354.0 | 2.6 |
| 29 | 17.33 | 0.84 | 1980.0 | 6.7 |
| 30 | 8.34 | 0.80 | 404.0 | 4.0 |
| 31 | 11.53 | 0.97 | 419.1 | 3.4 |
| 32 | 10.77 | 0.93 | 599.0 | 4.3 |
| 33 | 45.71 | 0.93 | 2041.0 | 5.2 |
| 34 | 3.91 | 1.11 | 156.1 | 1.7 |
| 35 | 23.97 | 1.15 | 467.5 | 4.2 |
| 36 | 24.92 | 1.23 | 500.8 | 4.1 |
| 37 | 37.49 | 1.21 | 366.0 | 3.7 |
| 38 | 40.69 | 1.23 | 289.6 | 3.7 |
| 39 | 33.59 | 0.58 | 1245.0 | 2.2 |
| 40 | 12.97 | 0.76 | 732.8 | 2.5 |
| 41 | 6.73 | 1.01 | NT | NT |
| 42 | 0.84 | 1.28 | 123.1 | 1.4 |
| 43 | 17.21 | 0.87 | 161.6 | 3.0 |
| 44 | 23.54 | 1.04 | 256.4 | 4.2 |
| 45 | 10.49 | 1.06 | 375.2 | 3.9 |
| 46 | 5.29 | 0.72 | 924.2 | 4.0 |
| 47 | 3.51 | 0.77 | 750.2 | 2.1 |
| 48 | 10.67 | 0.67 | 282.6 | 1.9 |
| 49 | 5.47 | 0.61 | 238.3 | 5.2 |
| 50 | 8.13 | 0.64 | 676.5 | 6.3 |
| 51 | 48.71 | 0.77 | 3941.0 | 1.5 |
| 52 | 183.90 | 0.50 | 50000.0 | NT |
| 53 | 256.50 | 0.55 | 50000.0 | NT |
| 54 | 20.07 | 0.72 | 297.4 | 3.9 |
| 55 | 46.56 | 0.78 | 507.6 | 2.7 |
| 56 | 44.52 | 0.71 | 621.7 | 3.2 |
| 57 | 6.54 | 0.89 | 304.3 | 3.2 |
| 58 | 19.45 | 0.79 | 1396.0 | 4.0 |
| 59 | 100.30 | 1.10 | 601.7 | 3.5 |
| 60 | 61.43 | 0.98 | 248.7 | 2.8 |
| 61 | 85.99 | 0.69 | 468.6 | 3.0 |
| 62 | 25.19 | 0.69 | 296.7 | 3.8 |
| 63 | 23.51 | 0.89 | 308.1 | 5.0 |
| 64 | 47.81 | 0.80 | 562.9 | 2.7 |
| 65 | 8.40 | 0.89 | 391.3 | 2.8 |
| 66 | 33.57 | 0.99 | 522.5 | 5.6 |
| 67 | 19.69 | 0.87 | 157.6 | 3.3 |
| 68 | 30.47 | 1.03 | 151.4 | 2.5 |
| 69 | 44.36 | 1.05 | 599.5 | 5.8 |
| 70 | 64.19 | 0.99 | 530.8 | 4.2 |
| 71 | 40.45 | 0.97 | 497.5 | 3.5 |
| 72 | 42.29 | 0.65 | 238.1 | 2.6 |
| 73 | 39.98 | 0.96 | 725.4 | 4.6 |
| 74 | 45.12 | 1.20 | 1037.0 | 6.2 |
| 75 | 55.69 | 0.87 | 1194.7 | 3.7 |
| 76 | 6.54 | 0.99 | 1176.5 | 3.2 |
| 77 | 18.29 | 0.95 | 1466.0 | 3.7 |
| 78 | 5.20 | 0.67 | 821.5 | 2.4 |
| 79 | 28.23 | 1.15 | 266.9 | 2.8 |
| 80 | 39.33 | 1.04 | 209.2 | 3.4 |
| 81 | 21.17 | 1.19 | 354.8 | 4.0 |
| 82 | 28.06 | 1.09 | 393.6 | 4.5 |
| 83 | 28.90 | 0.98 | 978.9 | 7.0 |
| 84 | 206.60 | 0.76 | 1141.0 | 4.7 |
| 85 | 75.43 | 1.01 | 279.5 | 1.8 |
| 86 | 78.40 | 0.69 | 2344.0 | 12.6 |
| 87 | 384.00 | 0.96 | 50000.0 | NT |
| 88 | 191.00 | 0.72 | 50000.0 | NT |
| 89 | 0.53 | 0.40 | 1094.0 | 2.7 |
| 90 | 20.63 | 0.86 | 368.1 | 1.8 |
| 91 | 34.13 | 0.47 | 3755.0 | 4.1 |
| 92 | 848.00 | NT | 50000.0 | NT |
| 93 | 46.44 | 0.51 | 264.3 | 2.1 |
| 94 | 159.00 | 0.27 | 3553.0 | 1.8 |
| 95 | 41.86 | 0.40 | 648.7 | 1.4 |
| 96 | 31.39 | 0.43 | 2092.0 | 1.2 |
| 97 | 37.03 | 0.61 | 604.9 | 2.5 |
| 98 | 20.58 | 0.75 | 1543.0 | 5.4 |
| 99 | 40.26 | 0.74 | 4840.0 | 6.3 |
| 100 | 91.70 | 0.84 | 3453.5 | 4.3 |
| 101 | 40.93 | 0.45 | 913.3 | 3.9 |
| 102 | 808.72 | 0.81 | 2149.4 | 3.7 |
| 103 | 41.08 | 0.49 | 1305.0 | 2.7 |
| 104 | 46.31 | 0.81 | 344.3 | 2.3 |
| 105 | 170.10 | 0.69 | 955.3 | 4.8 |
| 106 | 72.53 | 0.48 | 704.1 | 4.1 |
| 107 | 98.28 | 1.31 | 643.0 | 3.0 |
| 108 | 55.42 | 0.99 | 1981.0 | 4.0 |
| 109 | 17.54 | 0.65 | 467.7 | 5.9 |
| 110 | 12.09 | 0.76 | 214.2 | 2.8 |
| 111 | 18.93 | 0.64 | 564.3 | 3.2 |
| 112 | 273.40 | 0.33 | 1939.0 | 5.6 |
| 113 | 21.85 | 0.71 | 149.5 | 2.8 |
| 114 | 113.40 | 0.51 | 969.3 | 5.3 |
| 115 | 67.88 | 0.71 | 177.9 | 5.4 |
| 116 | 43.44 | 0.65 | 301.3 | 5.4 |
| 117 | 148.50 | 0.61 | 627.3 | 5.5 |
| 118 | 204.13 | 0.36 | 223.0 | 1.0 |
| 119 | 31.54 | 0.41 | 294.9 | 1.1 |
| 120 | 64.90 | 1.07 | 157.1 | 5.7 |
| 121 | 12.14 | 0.98 | 377.7 | 2.4 |
| 122 | 98.44 | 0.68 | 446.8 | 1.1 |
| Notes | 1. "NT" is an abbreviation of "Not Tested", and means that an object has not been detected yet. | | | |
| | 2. The above values are averages of one or more measurements. | | | |

It can be concluded from the data of biochemical and cellular activity of compounds in the specific examples that the series of compounds of the present invention have obvious agonistic effects and specificity on RORyt nuclear receptor, and are expected to be developed into a new generation of RORyt agonists, thus meeting the needs of clinical application.

All documents mentioned in the present invention are incorporated as references, just as each document is individually cited as a reference. In addition, it should be understood that various modifications or changes may be made by those skilled in the art after reading the above teachings of the present invention, and these equivalent forms also fall within the scope defined by the claims appended hereto.

## Claims

1. A compound of formula (I), a stereoisomer, prodrug or pharmaceutically acceptable salt thereof: 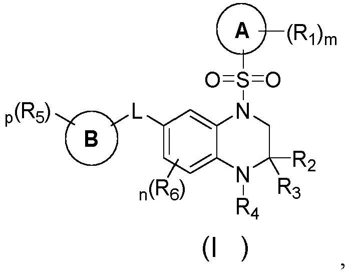 wherein,
L is selected from the group consisting of a bond, -C(R₇)=C(R₈)-, -(CR₉R₁₀)ₘ₁-, -(CR₁₁R₁₂)ₘ₂-O-, -O-(CR₁₃R₁₄)ₘ₃-, -N(R₁₅)-C(O)-, -C(O)-N(R₁₆)-, -(CR₁₇R₁₈)ₘ₄-N(R₁₉)-, -N(R₂₀)-(CR₂₁R₂₂)ₘ₅-, - (CR₂₃R₂₄)ₘ₆-S(O)ᵣ,- and -S(O)ᵣ-(CR₂₅R₂₆)ₘ₇-;
ring A is 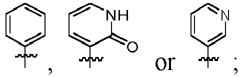
ring B is 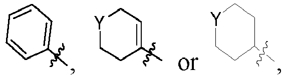 wherein Y is -O- or -N(R₂₇)-;
R₁ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, - C₀₋₈-C(O)NR₃₁R₃₂ and -C₀₋₈-N(R₃₁)-C(O)R₃₀;
R₂ and R₃ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, or R₂ and R₃, together with the carbon atom directly attached thereto, form C(O), 3-10 membered cycloalkyl or 3-10 membered heterocyclyl, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
R₄ is selected from the group consisting of hydrogen, deuterium, hydroxy, C₁₋₄ alkyl, vinyl, propenyl, allyl, ethynyl, C₃₋₆ cycloalkyl, 3-6 membered heterocyclyl, phenyl, benzyl, diazole, triazole, methylsulfonyl, isopropylsulfonyl, aminosulfonyl, carboxyl, methoxycarbonyl, ethoxycarbonyl and acetyl, said C₁₋₄ alkyl, C₃₋₆ cycloalkyl, 3-6 membered heterocyclyl, phenyl, benzyl, diazole and triazole are unsubstituted or substituted by one or more substituents selected from the group consisting of deuterium, halogen, cyano, methyl, ethyl, isopropyl, trifluoromethyl, difluoromethyl, trideuteriomethyl, cyclopropyl, oxacyclobutyl, =O, methoxy, carboxyl, methoxycarbonyl, acetyl, amino, dimethylamino and acetylamino,
or R₄ and R₃, together with the carbon atom directly attached thereto, form 5-10 membered heterocyclyl, the 5-10 membered heterocyclyl is unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈₋C(O)OR₂₉, -C₀₋₈-C(O)R₃₀, -C₀₋₈-C(S)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-C(S)R₃₀, -C₀₋₈-0-C(O)R₃₀, -C₀₋₈-NR₃₁R₃₂, -C₀₋₈-C(O)NR₃₁R₃₂ and - C₀₋₈-N(R₃₁)-C(O)R₃₀;
each R₅ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -SF₅, -C₀₋₈-S(O)ᵣR₂₈, -C₀₋₈-O-R₂₉, -C₀₋₈-C(O)OR₂₉, -C₀₋₈-C(O)R₃₀, -C₀₋₈-0-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
each R₆ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, - C₀₋₈-C(O)R₃₀, -C₀₋₈-0-C(O)R₃₀, -C₀₋₈-NR₃₁R₃₂, -C₀₋₈-C(O)NR₃₁R₃₂ and -C₀₋₈-N(R₃₁)-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
R₇ and R₅ are each independently selected from the group consisting of hydrogen, deuterium, fluorine, C₁₋₄ alkyl, C₁₋₄ deuterioalkyl and C₁₋₄ fluoroalkyl;
R₉, R₁₀, R₁₁, R₁₂, R₁₃, R₁₄, R₁₇, R₁₈, R₂₁, R₂₂, R₂₃, R₂₄, R₂₅ and R₂₆ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-O-R₂₉, -C₀₋₈-C(O)OR₂₉, -C₀₋₈-C(O)R₃₀, -C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀, or R₉ and R₁₀, R₁₁ and R₁₂, R₁₃ and R₁₄, R₁₇ and R₁₈, R₂₁ and R₂₂, R₂₃ and R₂₄, R₂₅ and R₂₆, together with the carbon atom directly attached thereto, each independently form C(O), 3-6 membered cycloalkyl, 3-6 membered heterocyclyl, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
R₁₅, R₁₆, R₁₉, R₂₀ and R₂₇ are each independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, -C₀₋₈-S(O)ᵣR₂₈, -C₀₋₈-C(O)OR₂₉ and -C₀₋₈-C(O)R₃₀, above groups are unsubstituted or substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -C₀₋₈-S(O)ᵣR₂₈, -C₀₋₈-O-R₂₉, -C₀₋₈-C(O)OR₂₉, -C₀₋₈-C(O)R₃₀, - C₀₋₈-O-C(O)R₃₀, -C₀₋₈-NR₃₁R₃₂, -C₀₋₈-C(O)NR₃₁R₃₂ and -C₀₋₈-N(R₃₁)-C(O)R₃₀;
each R₂₈ is independently selected from the group consisting of hydrogen, deuterium, hydroxy, C₁₋₁₀ alkyl, C₁₋₁₀ alkoxy, C₂₋₁₀ alkenyl, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂, above groups are unsubstituted or substituted by one or more substituents selected from the group consisting of deuterium, halogen, hydroxy, =O, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂;
each R₂₉ is independently selected from the group consisting of hydrogen, deuterium, C₁₋₁₀ alkyl, C₂₋₁₀ alkenyl, C₃₋₁₀ cycloalkyl, 3-10 membered heterocyclyl, C₅₋₁₀ aryl and 5-10 membered heteroaryl, above groups are unsubstituted or substituted by one or more substituents selected from the group consisting of deuterium, halogen, hydroxy, =O, cyano, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂;
each R₃₀ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂, above groups are unsubstituted or substituted by one or more substituents selected from the group consisting of deuterium, halogen, hydroxy, cyano, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy and -NR₃₁R₃₂;
each R₃₁ and each R₃₂ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₅₋₁₀ aryl, 5-10 membered heteroaryl, sulfonyl, methylsulfonyl, isopropylsulfonyl, cyclopropylsulfonyl, p-toluenesulfonyl, amino, monoalkylamino, dialkylamino and C₁₋₁₀ alkanoyl, above groups are unsubstituted or substituted by one or more substituents selected from the group consisting of deuterium, halogen, hydroxy, carboxyl, C₁₋₈ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, amino, monoalkylamino, dialkylamino and C₁₋₁₀ alkanoyl;
or R₃₁ and R₃₂, together with nitrogen atom directly attached thereto, form 4-10 membered heterocyclyl, above groups are unsubstituted or substituted by one or more substituents selected from the group consisting of deuterium, halogen, hydroxy, C₁₋₁₀ alkyl, C₁₋₁₀ alkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, amino, monoalkylamino, dialkylamino and C₁₋₁₀ alkanoyl;
m is an integer of 0 to 5; n is an integer of 0 to 3; p is an integer of 0 to 5;
m1, m3, m5 and m7 are each independently 1 or 2;
m2, m4 and m6 are each independently 0, 1 or 2;
each r is independently 0, 1 or 2.

2. The compound of formula (I), the stereoisomer, prodrug or pharmaceutically acceptable salt thereof of claim 1, wherein,
each R₆ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀; R₂₈, R₂₉, R₃₀, R₃₁ and R₃₂ are defined as in claim 1;
**preferably,** each R₆ is selected from the group consisting of hydrogen, deuterium, fluorine, chlorine, cyano, methyl, ethyl, isopropyl, vinyl, allyl, ethynyl, cyclopropyl, 3-oxacyclobutyl, 3-azacyclobutyl, phenyl, pyridyl, diazole, triazole, methylsulfonyl, aminosulfonyl, methoxy, methoxyacyl, carboxyl, acetyl, acetoxy, amino, dimethylamino, aminoacyl and acetylamino, above groups are unsubstituted or substituted by one or more substituents selected from the group consisting of deuterium, fluorine, chlorine, cyano, methyl, trifluoromethyl, cyclopropyl, phenyl, pyridyl, methylsulfonyl, hydroxy, methoxy, carboxyl and amino.

3. The compound of formula (I), the stereoisomer, prodrug or pharmaceutically acceptable salt thereof of claim 1, wherein, the compound of formula (I) is a compound having formula (IIa): 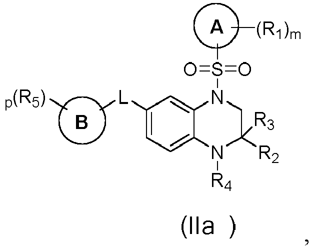
wherein, R₂ and R₃ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀, or R₂ and R₃, together with the carbon atom directly attached thereto, form C(O), 3-10 membered cycloalkyl or 3-10 membered heterocyclyl;
R₄ is selected from the group consisting of hydrogen, deuterium, hydroxy, C₁₋₄ alkyl, C₃₋₆ cycloalkyl, 3-6 membered heterocyclyl, phenyl, methylsulfonyl, isopropylsulfonyl, aminosulfonyl, carboxy, methoxycarbonyl, ethoxycarbonyl and acetyl, and said C₁₋₄ alkyl, C₃₋₆ cycloalkyl, 3-6 membered heterocyclyl and phenyl are unsubstituted or substituted by one or more substituents selected from the group consisting of deuterium, fluoro, chloro, cyano, methyl, ethyl, isopropyl, trifluoromethyl, difluoromethyl, trideuteromethyl, cyclopropyl, oxacyclobutyl, methoxy, carboxy, methoxycarbonyl, acetyl, amino, dimethylamino and acetylamino;
ring A, ring B, L, R₁, R₅, R₂₈, R₂₉, R₃₀, R₃₁, R₃₂, r, m and p are defined as in claim 1.

4. The compound of formula (I), the stereoisomer, prodrug or pharmaceutically acceptable salt thereof of claim 3, wherein, the compound of formula (I) is a compound having formula (IIIa1), formula (IIIa2), formula (IIIa3) or formula (IIIa4): 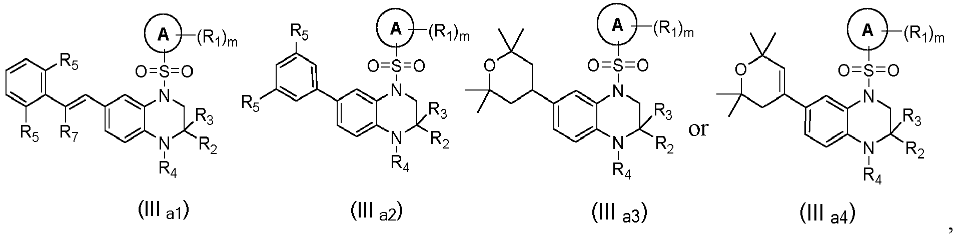
wherein R₂ and R₃ are each independently selected from the group consisting of hydrogen, deuterium, C₁₋₄ alkyl, C₃₋₆ cycloalkyl, 3-6 membered heterocyclyl, -C₀₋₄-O-R₂₉, -C₀₋₄-C(O)OR₂₉, - C₀₋₄-C(O)R₃₀ and -C₀₋₄-O-C(O)R₃₀, or R₂ and R₃, together with the carbon atom directly attached thereto, form C(O), 3-6 membered cycloalkyl or 3-6 membered heterocyclyl;
R₄ is selected from the group consisting of hydrogen, deuterium, C₁₋₄ alkyl, C₃₋₆ cycloalkyl and 3-6 membered heterocyclyl, said C₁₋₄ alkyl, C₃₋₆ cycloalkyl, 3-6 membered heterocyclyl are unsubstituted or substituted by one or more substituents selected from the group consisting of deuterium, fluoro, chloro, cyano, methyl, ethyl, isopropyl, trifluoromethyl, difluoromethyl, trideuteromethyl, cyclopropyl, oxacyclobutyl, methoxy, carboxy, methoxycarbonyl, acetyl, amino, dimethylamino and acetylamino;
each R₅ is independently selected from the group consisting of hydrogen, deuterium, halogen, cyano, C₁₋₄ alkyl, C₃₋₆ cycloalkyl, 3-6 membered heterocyclyl and -O-R₂₉, above groups are unsubstituted or substituted by one or more substituents selected from the group consisting of deuterium, fluoro, chloro, cyano, methyl, ethyl, isopropyl, trifluoromethyl, difluoromethyl, trideuteromethyl, dideuteromethyl, cyclopropyl, oxacyclobutyl, =O, methoxy and carboxy;
R₇ is selected from the group consisting of hydrogen, deuterium, fluoro, methyl, ethyl, trifluoromethyl, difluoromethyl, trideuteromethyl and dideuteromethyl;
ring A, R₁, R₂₉, R₃₀ and m are defined as in claim 3.

5. The compound of formula (I), the stereoisomer, prodrug or pharmaceutically acceptable salt thereof of claim 1, wherein, the compound of formula (I) is a compound having formula (IIb): 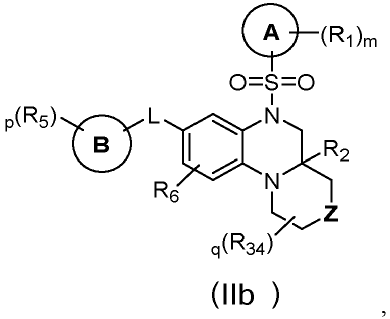
wherein Z is selected from the group consisting of a bond, -O-, -S-, -S(O)-, -S(O)₂-, -N(R₃₃)- and -(CR₃₅R₃₆)-;
R₃₃ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-C(S)R₃₀, -C₀₋₄-O-C(O)R₃₀ and -C₀₋₄-C(O)NR₃₁R₃₂, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-C(S)R₃₀, -C₀₋₄-O-C(O)R₃₀ and -C₀₋₄-C(O)NR₃₁R₃₂;
each R₃₄ is independently selected from the group consisting of hydrogen, deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and - C₀₋₄-N(R₃₁)-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀;
R₃₅ is selected from the group consisting of hydrogen, deuterium, halogen, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, - C₀₋₄-C(S)R₃₀ and -C₀₋₄-O-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀;
R₃₆ is selected from the group consisting of hydrogen, deuterium, halogen, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, -C₀₋₄-S(O)ᵣR₂₈, -C₀₋₄-O-R₂₉, -C₀₋₄-C(O)OR₂₉, -C₀₋₄-C(O)R₃₀, - C₀₋₄-C(S)R₃₀ and -C₀₋₄-O-C(O)R₃₀, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₅₋₈ aryl, 5-8 membered heteroaryl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀, -C₀₋₄-NR₃₁R₃₂, -C₀₋₄-C(O)NR₃₁R₃₂ and -C₀₋₄-N(R₃₁)-C(O)R₃₀;
q is an integer of 0 to 4; ring A, ring B, L, R₁, R₂, R₅, R₆, R₂₈, R₂₉, R₃₀, R₃₁, R₃₂, m, r and p are defined as in claim 1.

6. The compound of formula (I), the stereoisomer, prodrug or pharmaceutically acceptable salt thereof of claim 5, wherein, the compound of formula (I) is a compound having formula (IIIb1), formula (IIIb2), formula (IIIb3), formula (IIIb4) or formula (IIIb5): 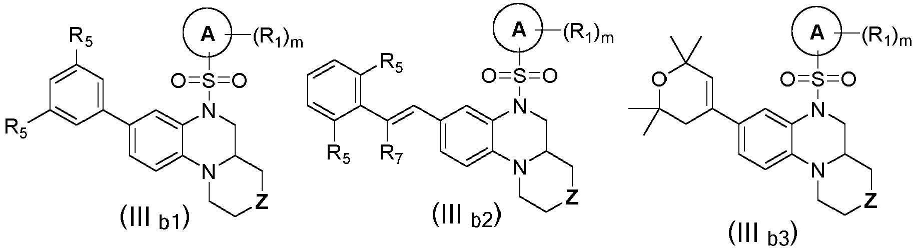 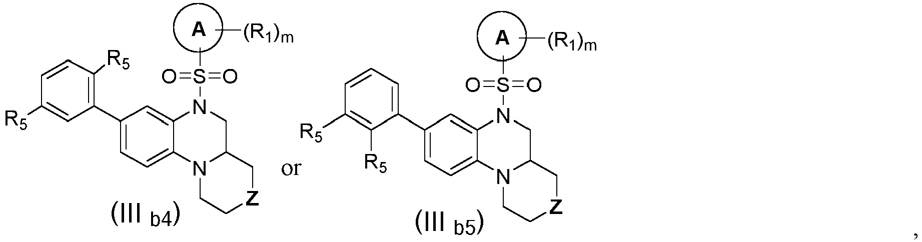 wherein Z is selected from the group consisting of a bond, -O-, -S-, -S(O)-, -S(O)₂-, -N(R₃₃)- and - (CR₃₅R₃₆)-;
each R₅ is independently selected from the group consisting of hydrogen, deuterium, halogen, cyano, C₁₋₄ alkyl, C₃₋₆ cycloalkyl, 3-6 membered heterocyclyl and -O-R₂₉, above groups are unsubstituted or substituted by one or more substituents selected from the group consisting of deuterium, fluoro, chloro, cyano, methyl, ethyl, isopropyl, trifluoromethyl, difluoromethyl, trideuteromethyl, dideuteromethyl, cyclopropyl, oxacyclobutyl, =O, methoxy and carboxy;
R₇ is selected from the group consisting of hydrogen, deuterium, fluoro, methyl, ethyl, trifluoromethyl, difluoromethyl, trideuteromethyl and dideuteromethyl;
R₃₃ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, -C₀₋₄-S(O)ᵣR₂₈, -C₀₋₄-O-R₂₉, -C₀₋₄-C(O)OR₂₉, -C₀₋₄-C(O)R₃₀, -C₀₋₄-C(S)R₃₀, -C₀₋₄-O-C(O)R₃₀ and -C₀₋₄-C(O)NR₃₁R₃₂, above groups are unsubstituted or substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, =O, -C₀₋₄-S(O)ᵣR₂₈, -C₀₋₄-O-R₂₉, -C₀₋₄-C(O)OR₂₉, -C₀₋₄-C(O)R₃₀, -C₀₋₄-O-C(O)R₃₀ and -C₀₋₄-C(O)NR₃₁R₃₂;
R₃₅ is selected from the group consisting of hydrogen, deuterium, halogen, C₁₋₄ alkyl, C₁₋₄ haloalkyl, C₁₋₄ deuterioalkyl, C₃₋₆ cycloalkyl, 3-6 membered heterocyclyl, -O-R₂₉, -C(O)OR₂₉, -O-C(O)R₃₀ and -C(O)NR₃₁R₃₂, above groups are unsubstituted or substituted by one or more substituents selected from the group consisting of deuterium, halogen, cyano, nitro, azido, C₁₋₄ alkyl, C₁₋₄ haloalkyl, C₁₋₄ deuterioalkyl, C₃₋₆ cycloalkyl, 3-6 membered heterocyclyl, =O, -S(O)ᵣR₂₈, -C₀₋₄-O-R₂₉, -C(O)OR₂₉, -C(O)R₃₀ and -C(O)NR₃₁R₃₂;
R₃₆ is selected from the group consisting of hydrogen, deuterium, halogen, C₁₋₄ alkyl, C₂₋₄ alkenyl, C₂₋₄ alkynyl, C₁₋₄ haloalkyl, C₁₋₄ deuterioalkyl and C₃₋₆ cycloalkyl, above groups are unsubstituted or substituted by one or more substituents selected from the group consisting of deuterium, fluoro, chloro, cyano, nitro, azido, methyl, ethyl, hydroxy, methoxy and carboxy;
ring A, R₁, R₂₈, R₂₉, R₃₀, R₃₁, R₃₂ and m are defined as in claim 5.

7. The compound of formula (I), the stereoisomer, prodrug or pharmaceutically acceptable salt thereof of any one of claims 1 to 6, wherein ring A, together with -(R₁)ₘ, forms the structure as follows: 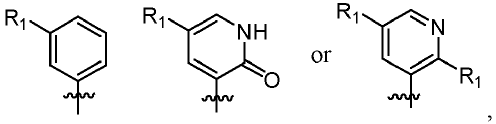
wherein each R₁ is independently selected from the group consisting of hydrogen, deuterium, halogen, cyano, C₁₋₄ alkyl, C₃₋₆ cycloalkyl, 3-6 membered heterocyclyl and -O-R₂₉, above groups are unsubstituted or substituted by one or more substituents selected from the group consisting of deuterium, fluoro, chloro, cyano, methyl, ethyl, isopropyl, trifluoromethyl, difluoromethyl, trideuteromethyl, dideuteromethyl, cyclopropyl, oxacyclobutyl, =O, methoxy, carboxy, methoxycarbonyl, acetyl, amino, dimethylamino and acetylamino;
each R₂₈ is independently selected from the group consisting of hydrogen, deuterium, C₁₋₄ alkyl, C₂₋₄ alkenyl, C₃₋₈ cycloalkyl, 3-8 membered heterocyclyl, C₅₋₈ aryl, 5-8 membered heteroaryl and - NR₃₁R₃₂, above groups are unsubstituted or substituted by one or more substituents selected from the group consisting of deuterium, halogen, hydroxy, =O, cyano, C₁₋₄ alkyl, C₁₋₄ alkoxy, C₃₋₈ cycloalkyl, C₃₋₈ cycloalkoxy and 3-8 membered heterocyclyl;
each R₂₉ is independently selected from the group consisting of hydrogen, deuterium, C₁₋₄ alkyl, C₂₋₄ alkenyl, C₃₋₈ cycloalkyl, 3-8 membered heterocyclyl, C₅₋₈ aryl and 5-8 membered heteroaryl, above groups are unsubstituted or substituted by one or more substituents selected from the group consisting of deuterium, halogen, hydroxy, =O, cyano, C₁₋₄ alkyl, C₁₋₄ alkoxy, C₃₋₈ cycloalkyl, C₃₋₈ cycloalkoxy and 3-8 membered heterocyclyl;
each R₃₀ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₈ cycloalkyl, C₃₋₈ cycloalkyloxy, 3-8 membered heterocyclyl, 3-8 membered heterocyclyloxy, C₅₋₈ aryl, C₅₋₈ aryloxy, 5-8 membered heteroaryl, 5-8 membered heteroaryloxy and -NR₃₁R₃₂, above groups are unsubstituted or substituted by one or more substituents selected from the group consisting of deuterium, halogen, hydroxy, cyano, C₁₋₄ alkyl, C₁₋₄ alkoxy, C₃₋₈ cycloalkyl, C₃₋₈ cycloalkyloxy, 3-8 membered heterocyclyl, 3-8 membered heterocyclyloxy, C₅₋₈ aryl, C₅₋₈ aryloxy, 5-8 membered heteroaryl, 5-8 membered heteroaryloxy and -NR₃₁R₃₂;
each R₃₁ and each R₃₂ are independently selected from the group consisting of hydrogen, deuterium, hydroxy, C₁₋₄ alkyl, C₂₋₄ alkenyl, C₂₋₄ alkynyl, C₃₋₈ cycloalkyl, 3-8 membered heterocyclyl, amino, monoalkylamino and dialkylamino.

8. The compound of formula (I), the stereoisomer, prodrug or pharmaceutically acceptable salt thereof of any one of claims 1 to 7, wherein, the compound is selected from the following compounds: 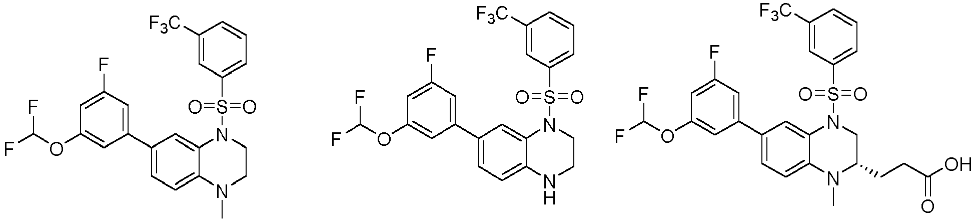 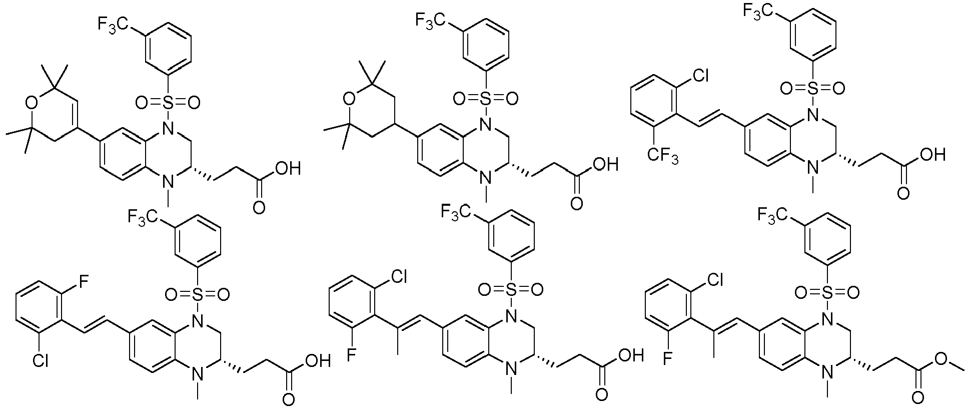 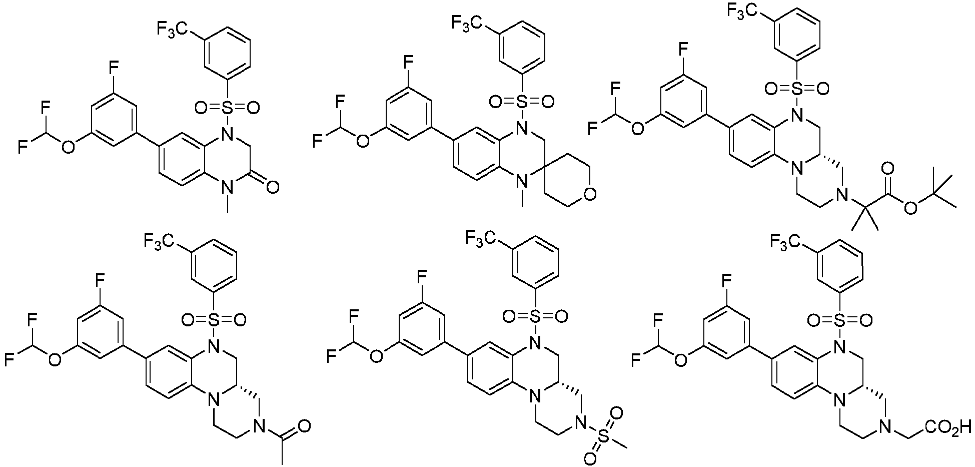 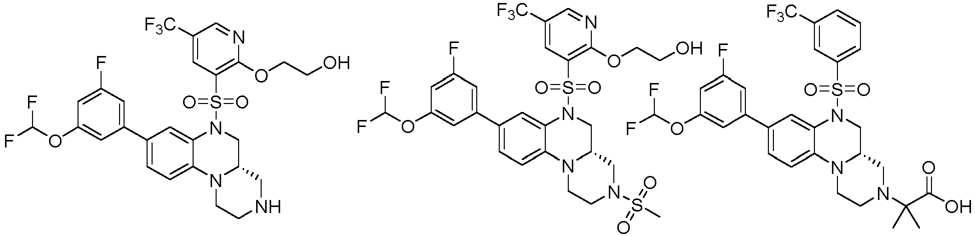 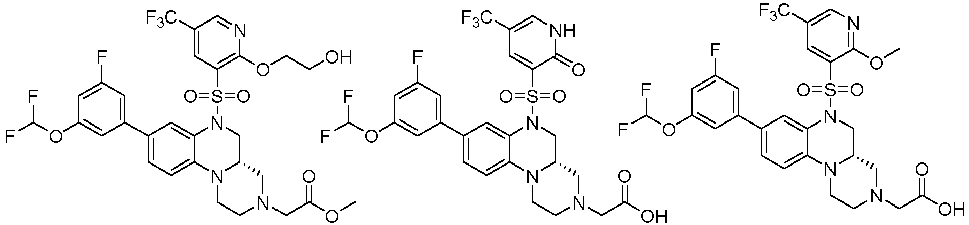 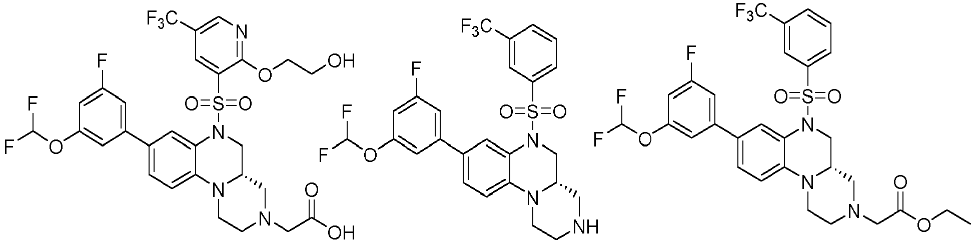 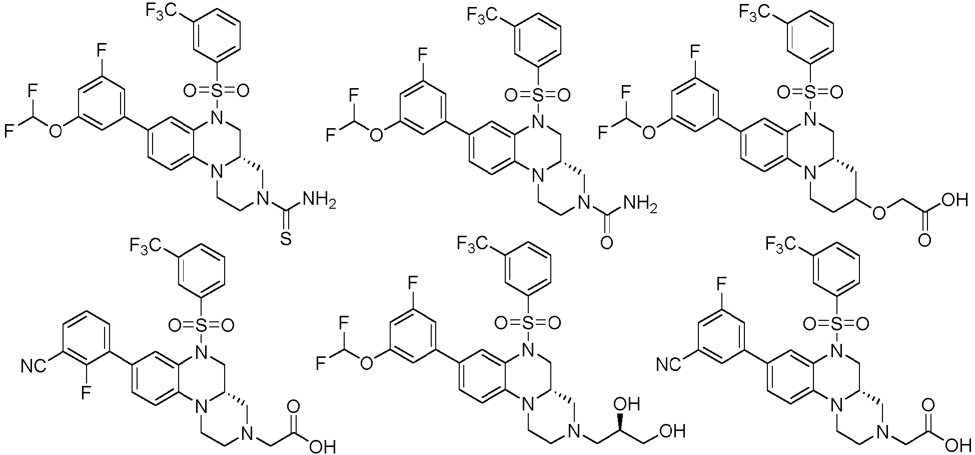 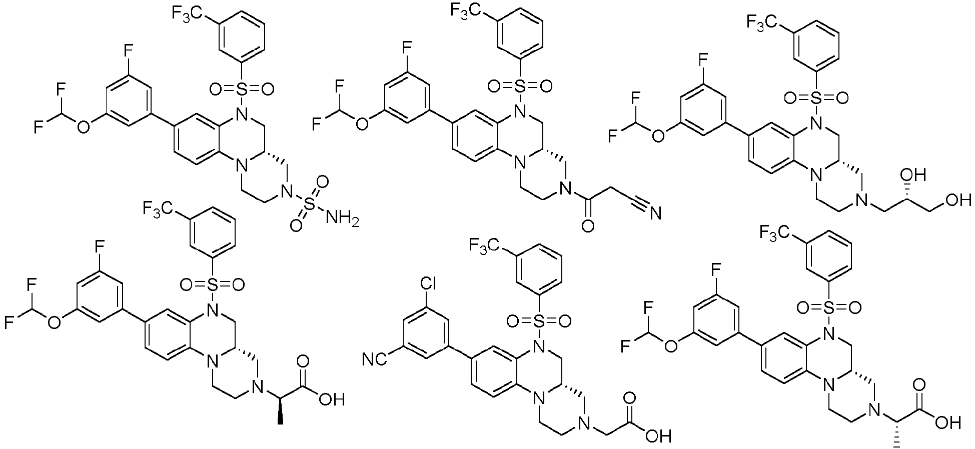 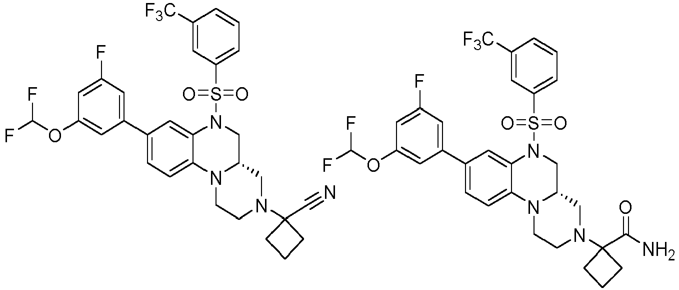 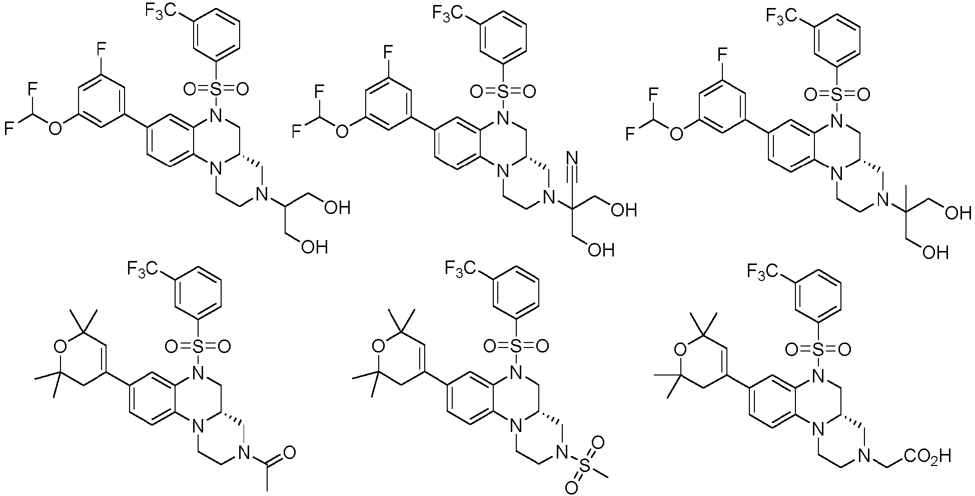 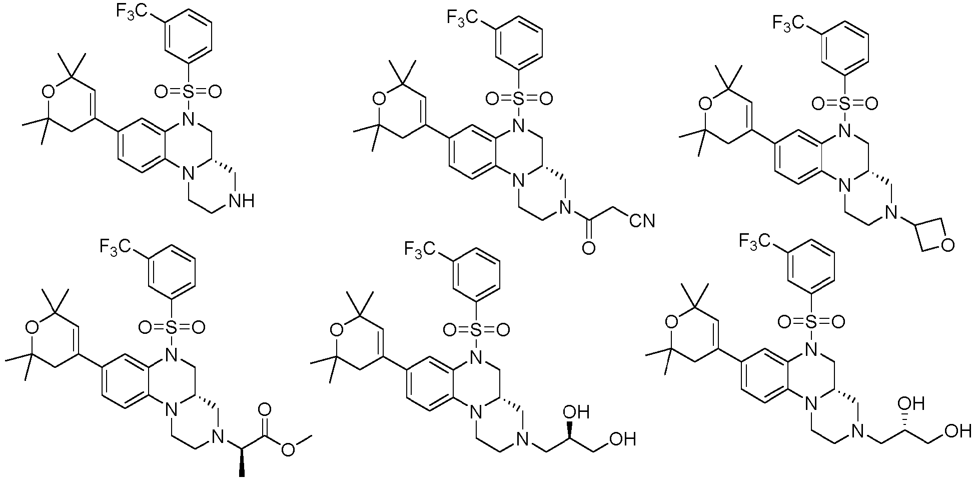 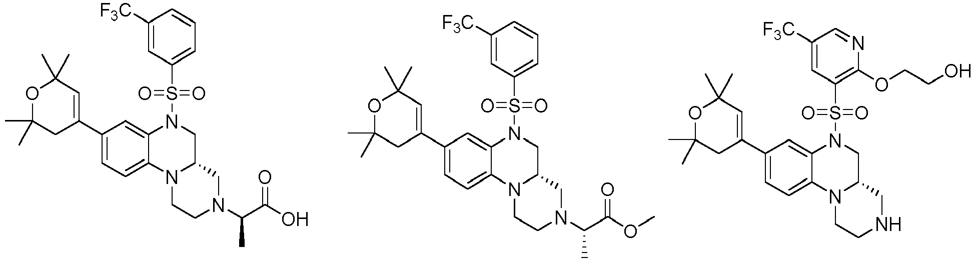 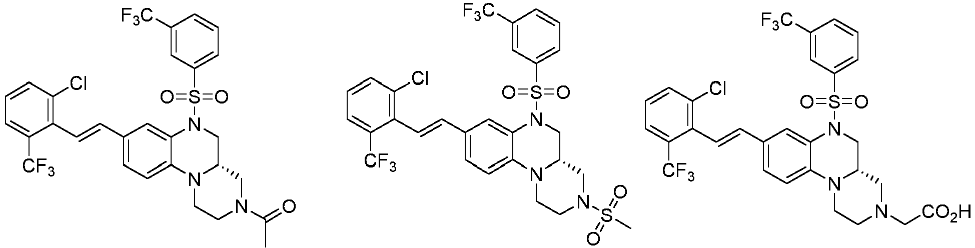 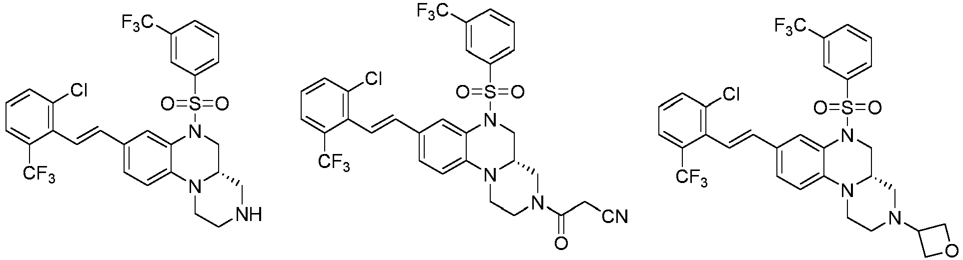 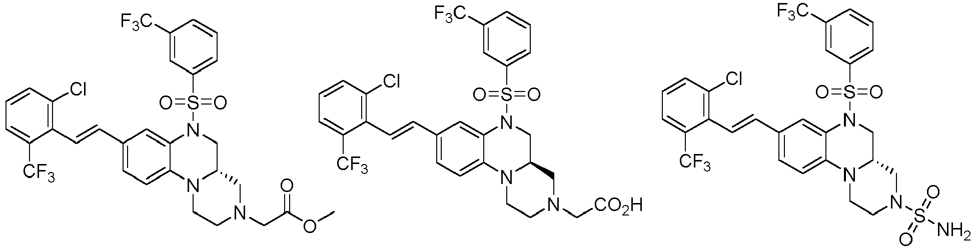 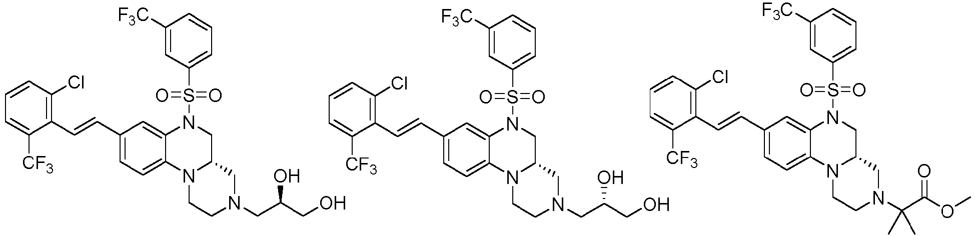 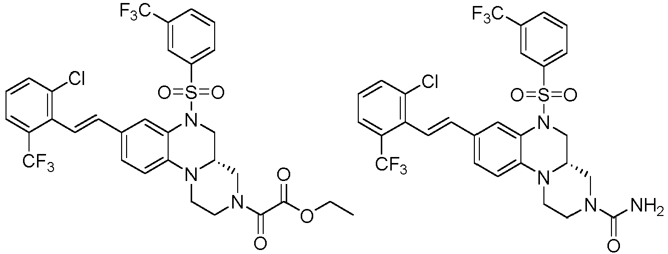 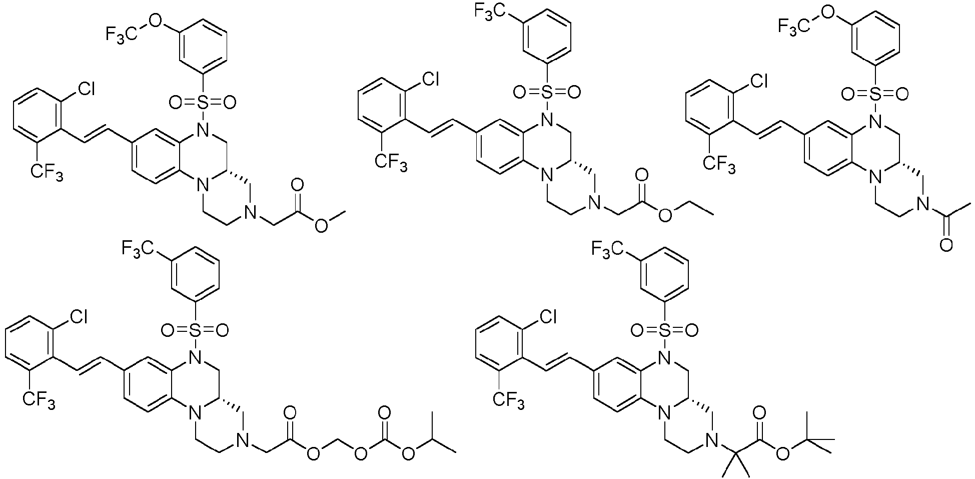 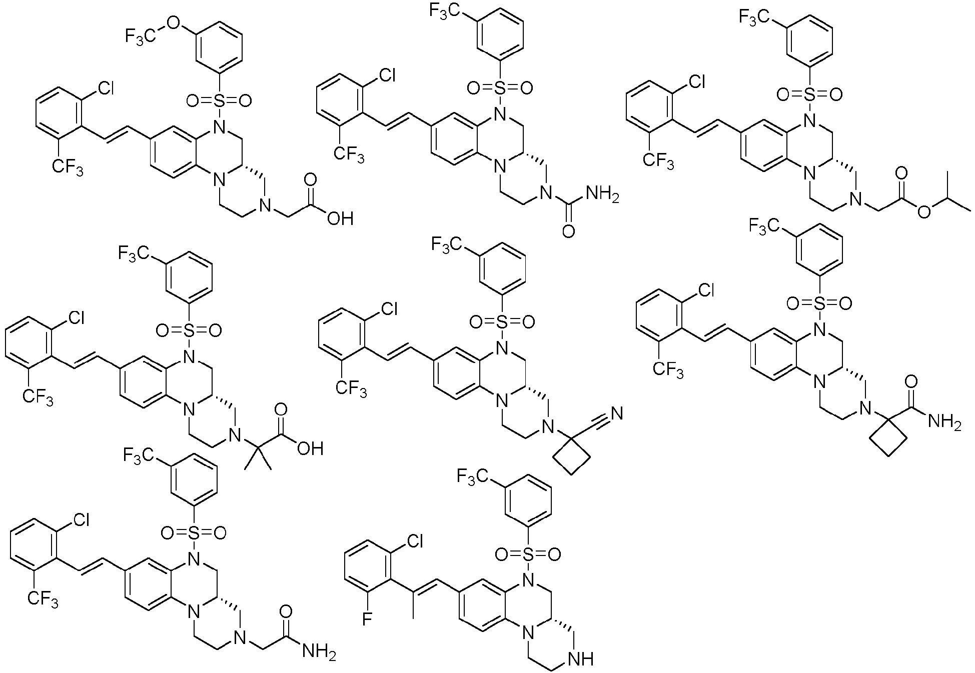 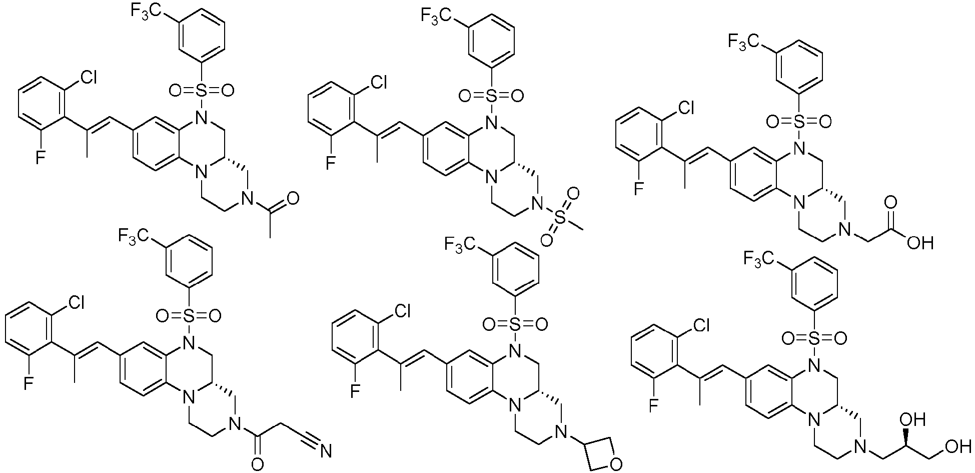 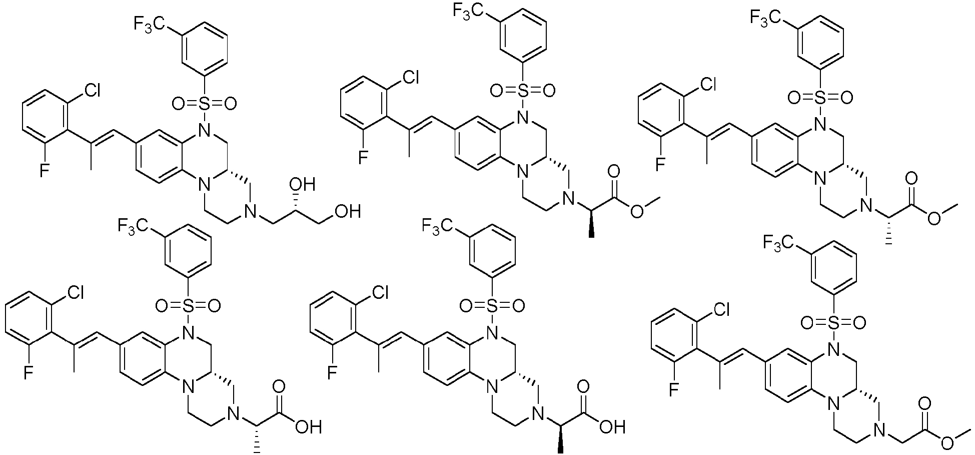 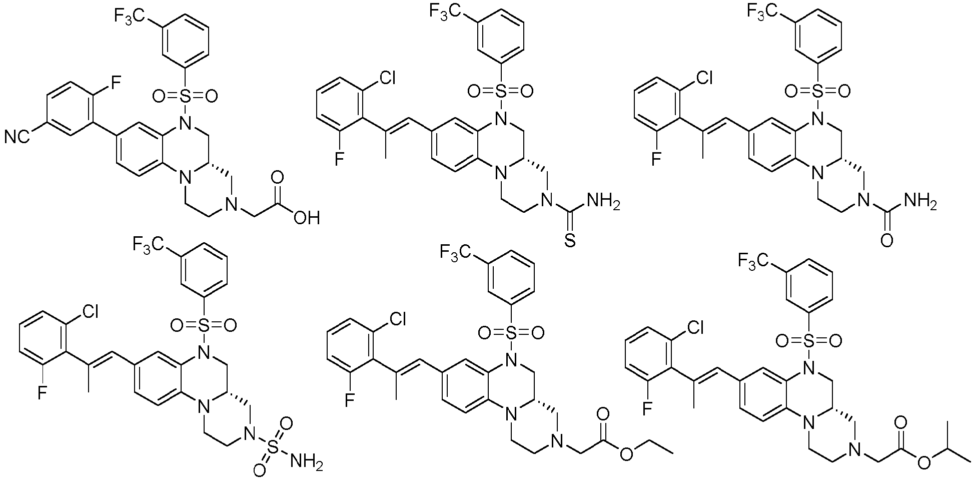 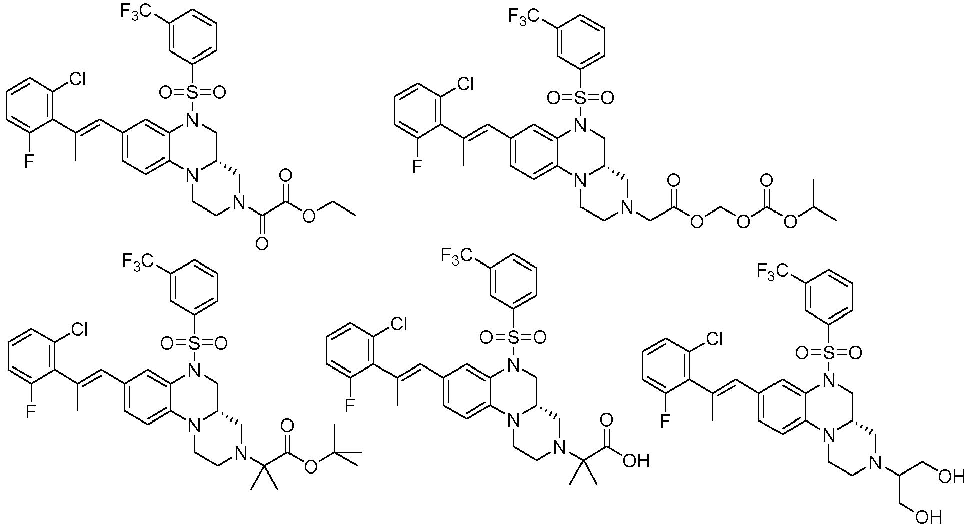 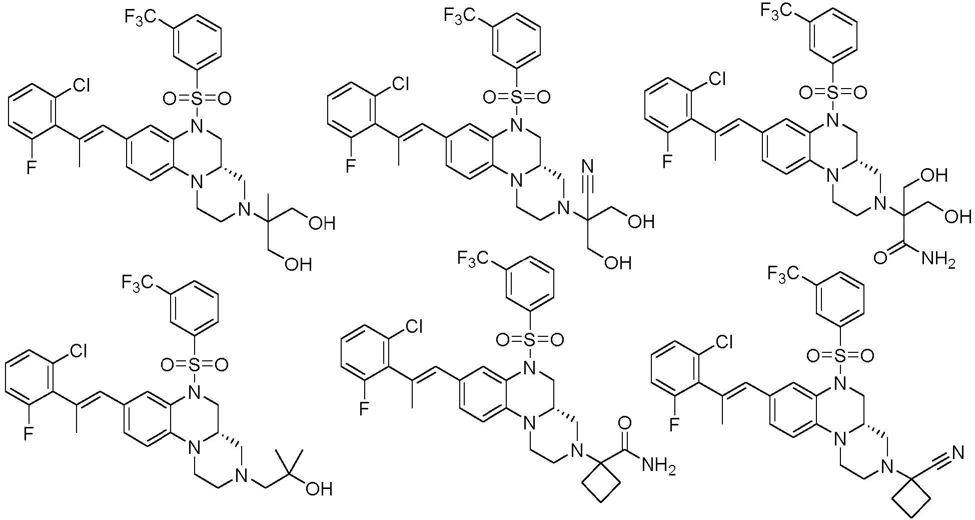 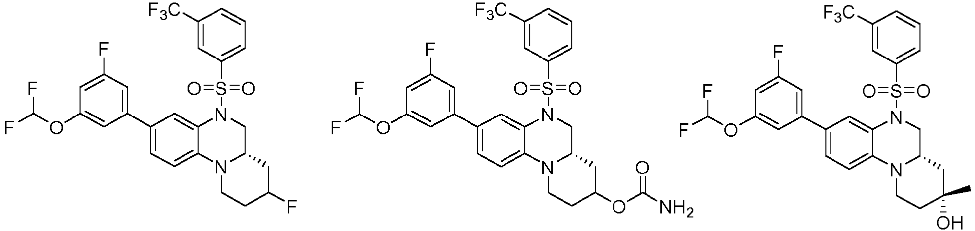 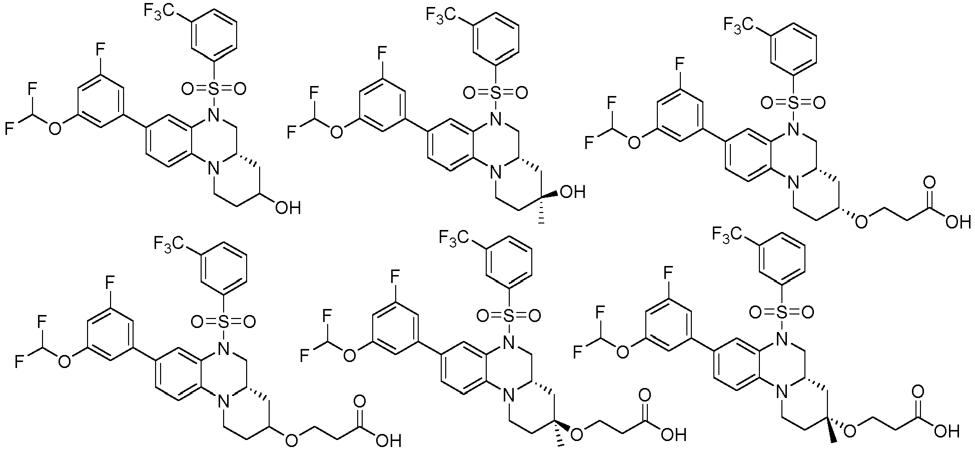 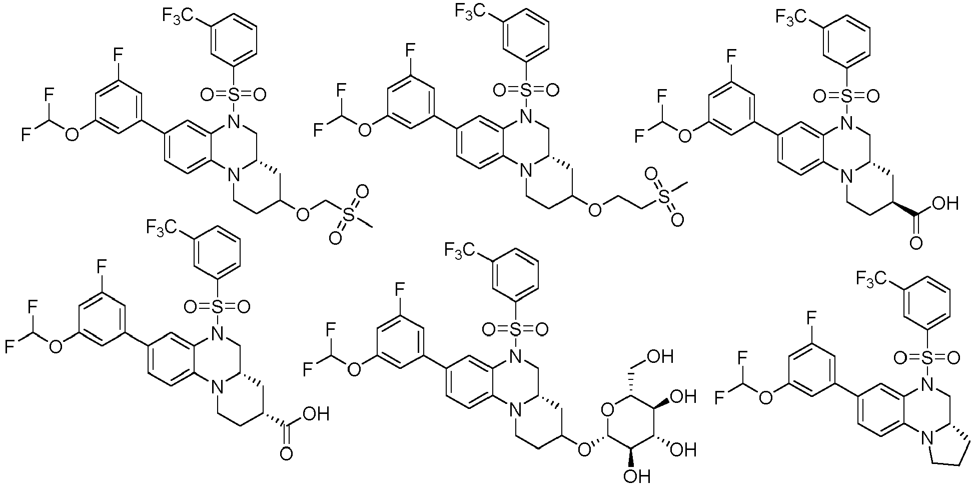 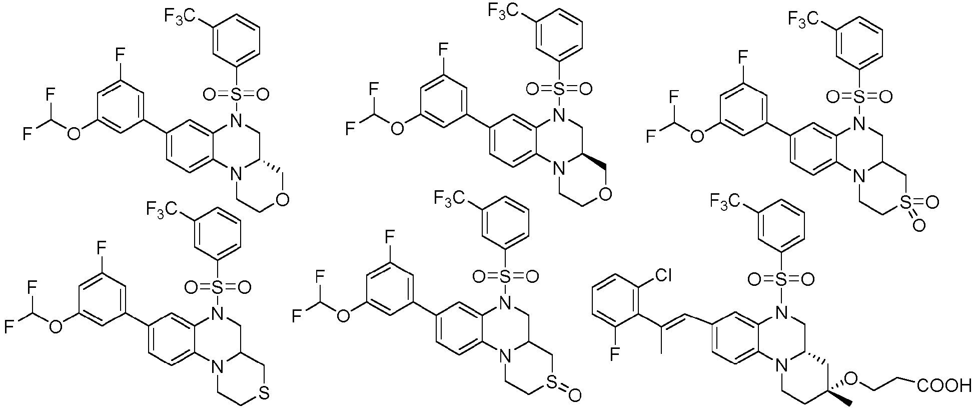 or

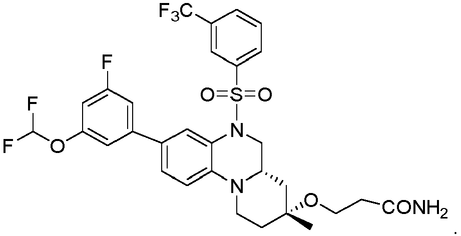

9. A preparation method for the compound of formula (I), the stereoisomer, prodrug or pharmaceutically acceptable salt thereof of any one of claims 1 to 8, comprising the following step: 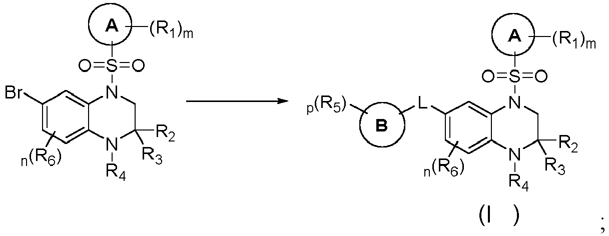 or, 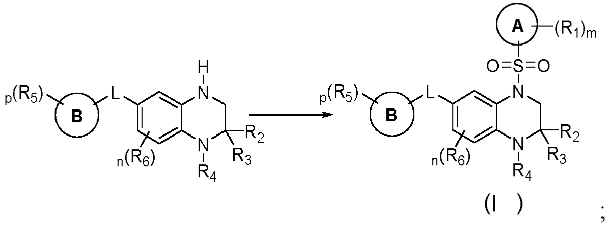 optionally, the compound of formula (I) can be obtained by further substitution reaction according to the definitions of substituents R₂, R₃ and R₄;
wherein ring A, ring B, L, R₁, R₂, R₃, R₄, R₅, R₆, m, n and p are defined as in claim 1.

10. A pharmaceutical composition, comprising the compound of formula (I), the stereoisomer, prodrug or pharmaceutically acceptable salt thereof of any one of claims 1 to 8 and pharmaceutically acceptable carrier.

11. Use of the compound of formula (I), the stereoisomer, prodrug or pharmaceutically acceptable salt thereof of any one of claims 1 to 8 in the preparation of medicaments for the treatment of one or more tumors, cancers, metabolic diseases, and autoimmune diseases or disorders.

12. The use of claim 11, wherein the metabolic disease, and autoimmune disease or disorder are selected from the group consisting of atopic dermatitis, contact dermatitis, allergic dermatitis, comedo, acne, cystic fibrosis, allograft rejection, multiple sclerosis, scleroderma, Systemic Lupus Erythematosus (SLE), psoriasis, Hashimoto's disease, arthritis, rheumatoid arthritis, psoriatic arthritis, juvenile idiopathic arthritis, juvenile rheumatoid arthritis, osteoarthritis, ankylosing spondylitis, Psoriatic Arthritis (PsA), autoimmune diabetes, diabetes mellitus type I, diabetes mellitus type II, obesity, fatty liver, adipose tissue-related inflammation, pancreatitis, thyroiditis, autoimmune thyroid disease, biliary cirrhosis, liver fibrosis, Non-alcoholic Fatty Liver Disease (NAFLD), ulcerative colitis, Crohn's disease, regional enteritis, Inflammatory Bowel Disease (IBD), Inflammatory Bowel Syndrome (IBS), Jogging Syndrome (S Jogging Syndrome), original sclerosing cholangitis, autoimmune polyendocrine syndrome type I, autoimmune polyendocrine syndrome type II, celiac disease, neuritis, systemic sclerosis, endometriosis, Behcet's syndrome, myocarditis, dermatomyositis, polymyositis, graft-versus-host disease, sarcoidosis, myocardial infarction, pulmonary hypertension, cutaneous leishmaniasis, Crohn's disease, autoimmune ocular disease, optic neuritis, neuromyelitis optica, xerophthalmia, uveitis, insulin resistance, myasthenia gravis, age-related macular degeneration, Guillain-Barre syndrome, glomerulonephritis, scleritis, major depressive disorder, seasonal affective disorder, Post-Traumatic Stress (Mental) Disorder (PTSD), bipolar disorder, autism, epilepsy, Alzheimer's disease, asthma, Chronic Obstructive Pulmonary Disease (COPD), bronchitis, allergic rhinitis, anaphylactic rhinitis, steroid-resistant asthma, toxic diffuse goiter, Obstructive Sleep Apnea Syndrome (OSAS), sinus polyps and a central nervous system disorder associated with changes in sleep and/or circadian rhythm.

13. The use of claim 11, wherein the tumor or cancer is selected from the group consisting of fallopian tube tumor, ovarian tumor, peritoneal tumor, stage IV melanoma, solid tumor, glioma, glioblastoma, papillary renal carcinoma, head and neck tumor, lymphoma, myeloma, non-Hodgkin's lymphoma, diffuse large B-cell lymphoma, follicular lymphoma, synovial sarcoma, hepatocellular carcinoma, breast cancer, uterine cancer, colon cancer, lung cancer, gastric cancer, rectal cancer, pancreatic cancer, brain cancer, skin cancer, oral cancer, prostate cancer, bone cancer, renal cancer, ovarian cancer, bladder cancer, liver cancer, leukemia and non-small cell lung cancer.

14. The compound of formula (I), the stereoisomer, prodrug or pharmaceutically acceptable salt thereof of any one of claims 1 to 8 for use as medicaments for treating one or more tumors, cancers, metabolic diseases, autoimmune diseases or disorders.
